(19) Europäisches Patentamt
European Patent Office
Office européen des brevets

(11) **EP 0 483 667 B1**

(12) **EUROPÄISCHE PATENTSCHRIFT**

(45) Veröffentlichungstag und Bekanntmachung des Hinweises auf die Patenterteilung:
**04.02.1998 Patentblatt 1998/06**

(51) Int. Cl.$^6$: **C07D 207/267**, C07D 207/27, A61K 31/40

(21) Anmeldenummer: **91118148.5**

(22) Anmeldetag: **24.10.1991**

(54) **Cyclische Iminoderivate, diese Verbindungen enthaltende Arzneimittel und Verfahren zu ihrer Herstellung**

Cyclic imino derivatives, process for their preparation and drugs containing them

Composés imino cycliques, procédé pour leur préparation et médicament les contenant

(84) Benannte Vertragsstaaten:
**AT BE CH DE DK ES FR GB GR IT LI LU NL SE**

(30) Priorität: **02.11.1990 DE 4035961**

(43) Veröffentlichungstag der Anmeldung:
**06.05.1992 Patentblatt 1992/19**

(73) Patentinhaber:
**Dr. Karl Thomae GmbH**
**D-88397 Biberach (DE)**

(72) Erfinder:
• **Himmelsbach, Frank, Dr. Dipl.-Chem.**
  **W-7951 Mittelbiberach (DE)**
• **Austel, Volkhard, Dr. Dipl.-Chem.**
  **W-7950 Biberach 1 (DE)**
• **Pieper, Helmut, Dr. Dipl.-Chem.**
  **W-7950 Biberach 1 (DE)**
• **Eisert, Wolfgang, Prof. Dr. Dr. Dr.**
  **W-7950 Biberach 1 (DE)**
• **Müller, Thomas, Dr. Dipl.-Chem.**
  **W-7950 Biberach 1 (DE)**
• **Weisenberger, Johannes, Dr. Dipl.-Chem.**
  **W-7950 Biberach 1 (DE)**
• **Linz, Günter, Dr. Dipl.-Chem.**
  **W-7951 Mittelbiberach (DE)**
• **Krüger, Gerd, Dr. Dipl.-Chem.**
  **W-7950 Rissegg (DE)**

(74) Vertreter: **Laudien, Dieter, Dr.**
**c/o Boehringer Ingelheim GmbH**
**55216 Ingelheim (DE)**

(56) Entgegenhaltungen:
**EP-A- 0 194 548     EP-A- 0 196 184**
**EP-A- 0 350 437     US-A- 4 247 466**

Bemerkungen:
Die Akte enthält technische Angaben, die nach dem Eingang der Anmeldung eingereicht wurden und die nicht in dieser Patentschrift enthalten sind.

**Beschreibung**

In der EP-A-0,350,437 werden bereits 2-Pyrrolidinone beschrieben, die in 1-Stellung durch ein Wasserstoffatom, eine $C_{1-4}$-Alkyl-, Acyl- oder Arylgruppe, in 4-Stellung durch ein Wasserstoffatom oder eine $C_{1-4}$-Alkylgruppe und durch eine substituierte Phenylgruppe substituiert sind, in der US-A-4,247,466 5-gliedrige Lactone, die in 3- und 4-Stellung durch ein Wasserstoffatom oder durch eine Hydroxygruppe und in 5-Stellung durch eine Biphenylylgruppe substituiert sind, in der EP-A-0,196,184 Hydroxamsäure-Derivate und in der EP-A-0,194,548 u.a. 4,5-Dihydro-pyridazin-(2H)-one.

Es wurde nun gefunden, daß die neuen cyclische Iminoderivate der allgemeinen Formel

$$B - X - A - Y - E, \tag{I}$$

welche sich immer durch die Strukturmermale der vorstehenden Y-E-Gruppe und/oder der Reste A und B von denen des Standes der Technik unterscheiden, wertvolle pharmakologische Eigenschaften besitzen.

Gegenstand der vorligenden Erfindung sind somit die cyclische Iminoderivate der obigen allgemeinen Formel I, deren Stereoisomere, deren Gemische und deren Additionssalze, insbesondere deren physiologisch verträgliche Additionssalze mit anorganischen oder organischen Säuren oder Basen, welche vorzugsweise aggregationshemmende Wirkungen aufweisen, diese Verbindungen enthaltende Arzneimittel und Verfahren zu ihrer Herstellung.

In der obigen allgemeinen Formel I bedeutet

A einen gegebenenfalls durch die Reste $R_1$ und $R_2$ substituierten Pyrrolidin- oder 2-Pyrrolidinonring, in denen

$R_1$ eine Phenylgruppe, die durch eine Carboxy-, Methoxycarbonyl-, Aminocarbonyl-, Methylaminocarbonyl-, Ethylaminocarbonyl-, Dimethylaminocarbonyl-, Methansulfonylamino- oder Acetylaminogruppe substituiert sein kann,

eine Alkylgruppe mit 1 bis 4 Kohlenstoffatomen, die durch eine Hydroxy-, Methoxy-, Phenoxy-, Methylsulfenyl-, Methylsulfinyl-, Methylsulfonyl-, Phenylsulfenyl-, Phenylsulfinyl-, Phenylsulfonyl-, Amino-, Acetylamino-, Benzoylamino-, N-Methyl-acetylamino-, Methansulfonylamino- oder Benzolsulfonylaminogruppe substituiert sein kann, wobei diese Substituenten nicht in 1-Stellung stehen können, wenn $R_1$ an das Ringstickstoffatom des Restes A gebunden ist,

eine Alkylgruppe mit 1 bis 4 Kohlenstoffatomen, die durch zwei Phenylgruppen, durch eine Cyclohexylgruppe oder durch eine Phenylgruppe substituiert ist, wobei die letztere durch ein Fluor-, Chlor- oder Bromatom, durch ein Alkylgruppe mit 1 bis 4 Kohlenstoffatomen, durch eine Alkoxygruppe mit 1 bis 6 Kohlenstoffatomen, durch eine Phenyl-, Phenylmethyl-, Hydroxy-, Benzyloxy-, Methylsulfenyl-, Methylsulfonyl- oder Trifluormethylgruppe, durch zwei Methoxygruppen oder durch zwei Chloratome substituiert sein kann,

eine Methylgruppe, die durch eine Carboxy-, Methoxycarbonyl-, Aminocarbonyl-, Methylaminocarbonyl-, Ethylaminocarbonyl-, Dimethylaminocarbonyl-, Benzylaminocarbonyl-, Pyrrolidinocarbonyl-, Piperidinocarbonyl-, Hexamethyleniminocarbonyl-, Morpholinocarbonyl-, Thiomorpholinocarbonyl-, 1-Oxido-thiomorpholinocarbonyl-, 1,1-Dioxido-thiomorpholinocarbonyl-, Piperazinocarbonyl-, N-Methyl-piperazinocarbonyl-, N-Acetylpiperazinocarbonyl- oder N-Methansulfonyl-piperazinocarbonylgruppe substituiert ist,

oder auch, sofern $R_1$ nicht in 1-Stellung eines 2-Pyrrolidinonringes steht, eine Carbonylgruppe, die durch eine Methyl-, Phenyl-, Methoxymethyl-, Amino-, Methylamino-, Ethylamino-, Aminomethyl-, Dimethylamino-, Carboxy-, Methoxycarbonyl- oder Dimethylaminocarbonylgruppe substituiert ist,

oder auch, sofern $R_1$ auch nicht an einem zum Ringstickstoffatom benachbarten Kohlenstoffatom des 5-giedrigen Alkyleniminoringes A steht, eine durch eine Methyl-, Dimethylamino-, Phenyl- oder Methoxyphenylgruppe substituierte Sulfonylgruppe und

$R_2$ eine gegebenenfalls durch eine Phenylgruppe substituierte Alkylgruppe mit 1 bis 4 Kohlenstoffatomen darstellen,

B eine Aminoalkylgruppe mit 1 bis 5 Kohlenstoffatomen, eine Amino-, Amidino-, Guanidino- oder Guanidinoalkylgruppe mit 1 bis 5 Kohlenstoffatomen, wobei die vorstehend erwähnten Amino-, Aminoalkyl- oder Amidinogruppen an einem der Stickstoffatome durch eine Hydroxygruppe, durch eine Alkylgruppe mit 1 bis 4 Kohlenstoffatomen, durch eine Alkyloxycarbonylgruppe mit insgesamt 2 bis 6 Kohlenstoffatomen, durch eine Benzyloxycarbonyl-, Phenyloxycarbonyl- oder Benzoylgruppe substituiert sein können,

2

die Y-E-Gruppe eine geradkettige Alkylgruppe mit 1 bis 3 Kohlenstoffatomen, die durch eine Carboxy-, Phosphono-, O-Methylphosphono- oder Hydroxymethylgruppe, durch eine Alkoxycarbonylgruppe mit insgesamt 2 bis 7 Kohlenstoffatomen, durch eine Dialkylaminocarbonylmethoxycarbonylgruppe, in welcher jeder Alkylteil 1 bis 3 Kohlenstoffatome enthalten kann, durch eine Morpholinoethoxycarbonyl- oder (2-Oxo-1-pyrrolidinyl)ethoxycarbonylgruppe, durch eine Phenylalkoxycarbonylgruppe mit 1 bis 3 Kohlenstoffatomen im Alkoxyteil, wobei der Phenylkern durch eine oder zwei Methoxygruppen substituiert sein kann, oder durch eine Pyridylmethyloxycarbonylgruppe substituiert ist,
wobei der kürzeste Abstand zwischen dem Substituenten E und dem ersten Stickstoffatom der Gruppe B mindestens 10 Bindungen beträgt, und

X eine Gruppe der Formel

$$- X_1 - X_2 - ,$$

wobei $X_1$ an die Gruppe A und $X_2$ an die Gruppe B gebunden ist,

$X_1$ eine Bindung, eine Methylen- oder Ethylengruppe, wobei zwischen der Methylengruppe, sofern diese nicht an das Ringstickstoffatom der Gruppe A gebunden ist, und der benachbarten Gruppe $X_2$ zusätzlich noch ein Sauerstoff- oder Schwefelatom, eine Sulfonyl-, Imino-, -N(COCH$_3$)-, -N(SO$_2$CH$_3$)-, -CONH-, -NH-CO-, -NH-SO$_2$- oder -NH-CO-NH-Gruppe
oder zwischen der Ethylengruppe und der benachbarten Gruppe $X_2$ zusätzlich noch eine Imino-, -NHCO- oder -N(C$_2$H$_5$)CO-Gruppe stehen kann,

$X_2$ eine Phenylen- oder Biphenylylengruppe, die durch ein Fluor-, Chlor- oder Bromatom, durch eine Methyl-, Methoxy-, Ethoxy-, Trifluormethyl-, Methylsulfenyl-, Methylsulfinyl-, Methylsulfonyl-, Nitro-, Acetylamino- oder Methansulfonylaminogruppe oder durch eine weitere Methylgruppe substituiert sein können,

eine gegebenenfalls einfach oder mehrfach ungesättigte geradkettige Phenylenalkylengruppe mit 1 bis 3 Kohlenstoffatomen im Alkylteil, wobei jedoch eine Doppel- oder Dreifachbindung nicht benachbart zu einem Heteroatom stehen kann,

eine Phenylencycloalkylen- oder Cycloalkylenphenylengruppe mit jeweils 4 bis 6 Kohlenstoffatomen im Cycloalkylteil, eine Phenylennaphtylen-, Phenanthrenylen- oder Dihydrophenanthrenylengruppe oder eine Naphthylengruppe, die ganz oder teilweise hydriert sein kann, eine Fluorenylengruppe, in der die Methylengruppe durch eine Hydroxymethylen- oder Carbonylgruppe ersetzt sein kann, eine Indanylen-, Spiroundecylen- oder Phenylenbicycloheptylengruppe oder auch eine Phenylen-W-Phenylengruppe, in welcher W ein Sauerstoff- oder Schwefelatom, eine Imino-, Carbonyl-, Hydroxymethylen-, Sulfinyl- oder Sulfonylgruppe darstellt,
oder, falls auf $X_2$ nicht unmittelbar ein Heteroatom oder ein ungesättigtes Kohlenstoffatom der Gruppe B folgt, auch eine Oxyphenylen- oder Carbonylaminophenylengruppe.

Bevorzugte Verbindungen der obigen allgemeinen Formel I sind diejenigen, in denen

A einen gegebenenfalls durch die Reste $R_1$ und $R_2$ substituierten Pyrrolidin- oder 2-Pyrrolidinonring, in denen

$R_1$ eine Phenylgruppe, die durch eine Carboxy-, Methoxycarbonyl- oder Dimethylaminocarbonylgruppe substituiert sein kann,

eine geradkettige Alkylgruppe mit 1 bis 4 Kohlenstoffatomen, die endständig durch eine Phenylgruppe, welche durch eine Alkylgruppe mit 1 bis 4 Kohlenstoffatomen, durch eine Alkoxygruppe mit 1 bis 6 Kohlenstoffatomen, durch eine Phenyl-, Benzyl-, Methylsulfenyl-, Methylsulfonyl- oder Trifluormethylgruppe, durch zwei Methoxygruppen oder durch zwei Chloratome substituiert sein kann, oder durch eine Cyclohexylgruppe oder durch zwei Phenylgruppen substituiert sein kann,

eine Alkylgruppe mit 2 bis 4 Kohlenstoffatomen, die in 2-, 3- oder 4-Stellung durch eine Hydroxy-, Methoxy- oder Phenoxygruppe substituiert ist,

eine Methylgruppe, die durch eine Carboxy-, Methoxycarbonyl-, Aminocarbonyl-, Ethylaminocarbonyl-, Dimethylaminocarbonyl-, Benzylaminocarbonyl-, Pyrrolidinocarbonyl- oder Morpholinocarbonylgruppe substituiert ist,

oder auch, sofern $R_1$ nicht am Ringstickstoffatom des 2-Pyrrolidinonringes steht, eine Carbonylgruppe, die durch eine Methyl-, Phenyl-, Ethylamino-, Dimethylamino-, Methoxymethyl- oder Aminomethylgruppe substituiert ist,

oder auch, sofern $R_1$ auch nicht an einem zum Ringstickstoff benachbarten Kohlenstoffatom des 5-gliedrigen Alkyleniminoringes steht, eine durch eine Methyl-, Methoxyphenyl- oder Dimethylaminogruppe substituierte Sulfonylgruppe und

$R_2$ eine Alkylgruppe mit 1 bis 4 Kohlenstoffatomen darstellen,

B eine Guanidinomethylgruppe oder eine Amidinogruppe, die an einem der Stickstoffatome durch eine Alkylgruppe mit 1 bis 4 Kohlenstoffatomen oder durch eine Methoxycarbonyl-, Ethoxycarbonyl-, Isopropyloxycarbonyl-, Isobutyloxycarbonyl-, Phenyloxycarbonyl-, Benzyloxycarbonyl- oder Benzoylgruppe substituiert sein kann,

die Y-E-Gruppe eine Methylgruppe, die durch eine Carboxy-, Phosphono-, O-Methyl-phosphono- oder Dimethylaminocarbonylmethoxycarbonylgruppe oder durch eine Alkoxycarbonylgruppe mit insgesamt 2 bis 7 Kohlenstoffatomen, in der ein Methoxyteil durch eine Phenyl- oder Pyridylgruppe, der Ethoxyteil endständig durch eine Phenyl-, Dimethoxyphenyl-, Morpholino- oder 2-Oxo-1-pyrrolidinylgruppe und der n-Propoxyteil endständig durch einen Phenylrest substituiert sein kann, substituiert ist,
wobei der kürzeste Abstand zwischen dem Substituenten E und dem ersten Stickstoffatom der Gruppe B mindestens 10 Bindungen beträgt, und

X eine Gruppe der Formel

$$- X_1 - X_2 -,$$

in welcher

$X_1$ eine Bindung, eine Methylengruppe, die, sofern diese nicht an das Ringstickstoffatom der Gruppe A gebunden ist, über ein Sauerstoffatom, eine Sulfonyl-, Imino-, $-N(COCH_3)-$, $-NH-CO-$ oder $-NH-SO_2$-Gruppe an die benachbarte $X_2$-Gruppe gebunden ist, oder eine Ethylengruppe, die über eine $-NH-CO$-Gruppe an die benachbarte $X_2$-Gruppe gebunden ist, und

$X_2$ eine Biphenylylengruppe, die durch ein Fluor-, Chlor- oder Bromatom, durch eine Methyl-, Methoxy-, Ethoxy-, Trifluormethyl-, Methylsulfenyl-, Methylsulfinyl-, Methylsulfonyl-, Nitro-, Acetylamino- oder Methansulfonylaminogruppe oder durch eine weitere Methylgruppe substituiert sein kann, eine Phenylencycloalkylengruppe mit insgesamt 10 bis 12 Kohlenstoffatomen, eine Phenylensulfenylphenylen-, Phenylensulfinylphenylen-, Dihydrophenanthrenylen-, Indanylen- oder Naphthylengruppe oder eine Fluorenylengruppe, in der die Methylengruppe durch eine Hydroxymethylen- oder Carbonylgruppe ersetzt sein kann, bedeuten,
deren geometrische Isomere und deren Salze.

Besonders bevorzugte Verbindungen der obigen allgemeinen Formel I sind diejenigen, in denen

$R_1$ mit dem Ringstickstoffatom oder der Position 3 der Gruppe A verknüpft ist, deren geometrische Isomere und deren Salze.

Als besonders bevorzugte Verbindungen der obigen allgemeinen Formel I seien folgende erwähnt:

(3S,5S)-5-[(4'-Amidino-4-biphenylyl)oxymethyl]-3-[(methoxycarbonyl)methyl]-2-pyrrolidinon,

1-(4'-Amidino-4-biphenylyl)-4-phosphonomethyl-2-pyrrolidinon,

(3S,5S)-5-[(4'-Amidino-4-biphenylyl)oxymethyl)-3-carboxymethyl-1-(3-phenylpropyl)-2-pyrrolidinon,

(3S,5S)-5-[(4'-Amidino-4-biphenylyl)oxymethyl]-3-carboxymethyl-2-pyrrolidinon,

(3S,5S)-5-[(4'-Amidino-4-biphenylyl)oxymethyl)-3-carboxymethyl-1-phenyl-2-pyrrolidinon,

(3S,5S)-5-[(4'-Amidino-2,3-dimethyl-4-biphenylyl)oxymethyl]-3-carboxymethyl-1-(3-phenylpropyl)-2-pyrrolidinon,

(3S,5S)-5-[(7-Amidino-9-keto-2-fluorenyl)oxymethyl]-3-carboxymethyl-1-(3-phenylpropyl)-2-pyrrolidinon,

(3S,5S)-5-[[N-(4'-Amidino-4-biphenylyl)-N-acetyl]aminomethyl]-3-carboxymethyl-1-(3-phenylpropyl)-2-pyrrolidinon,

(3S,5S)-5-[[2-[(4-Amidinophenyl)amino]phenyl]carbonylaminomethyl]-3-carboxylmethyl-1-(3-phenylpropyl)-2-pyrrolidinon,

(3S,5S)-5-[[4-[(3-Amidinophenyl)carbonyl]phenyl]oxymethyl]-3-carboxymethyl-1-(3-phenylpropyl)-2-pyrrolidinon,

(3S,5S)-5-[(4'-Amidino-4-biphenylyl)oxymethyl]-3-carboxymethyl-1-[(dimethylaminocarbonyl)methyl]-2-pyrrolidinon,

(3S,5S)-1-Acetyl-5-[(4'-amidino-4-biphenylyl)oxymethyl]-3-carboxymethyl-pyrrolidin,

(3S,5S)-5-[(4'-Amidino-4-biphenylyl)oxymethyl]-3-carboxymethyl-1-(dimethylaminosulfonyl)-pyrrolidin,

(3S,5S)-5-[(4'-Amidino-4-biphenylyl)sulfonyl]aminomethyl]-3-carboxymethyl-1-(3-phenylpropyl)-2-pyrrolidinon,

(3R,S;4R,S)-4-[(4'-Amidino-4-biphenylyl)oxymethyl]-3-carboxymethyl-2-pyrrolidinon,

(3S,5S)-5-[(4'-Amidino-3'-fluor-4-biphenylyl)oxymethyl]-3-carboxymethyl-2-pyrrolidinon,

(3S,5S)-5-[[4-(4-Aminobutyl)phenyl]oxymethyl]-3-carboxymethyl-1-(3-phenylpropyl)-2-pyrrolidinon,

(3S,R;5S,R)-3-Carboxymethyl-5-[4-[(5-guanidinopentyl)oxy]-phenyl]-1-(3-phenylpropyl)-2-pyrrolidinon,

(3S,5S)-5-[2-[(6-Amino-5,6,7,8-tetrahydro-2-naphtylcarbonyl)amino]ethyl]-3-carboxymethyl-1-(3-phenylpropyl)-2-pyrrolidinon,

(3S,5S)-5-[[4-[(3-Aminopropyl)carbonylamino]phenyl]oxymethyl]-3-carboxymethyl-1-(3-phenylpropyl)-2-pyrrolidinon,

(3S,5S)-3-Carboxymethyl-5-[[4'-(N-hydroxyamidino)-4-biphenylyl)oxymethyl]-2-pyrrolidinon,

(3S,5S)-5-[[4'-(N-Methoxycarbonylamidino)-4-biphenylyl]-oxymethyl]-3-[(methoxycarbonyl)methyl]-2-pyrrolidinon,

(3S,5S)-5-[[4'-(N-Ethoxycarbonylamidino)-4-biphenylyl]-oxymethyl]-3-[(methoxycarbonyl)methyl]-2-pyrrolidinon,

(3S,5S)-5-[[4'-(N-Ethoxycarbonylamidino)-4-biphenylyl]-oxymethyl]-3-[(ethoxycarbonyl)methyl]-2-pyrrolidinon und

(3S,5S)-3-[(Ethoxycarbonyl)methyl]-5-[[4'-(N-methoxycarbonylamidino)-4-biphenylyl]-oxymethyl]-2-pyrrolidinon insbesondere

(3S,5S)-5-[(4'-Amidino-4-biphenylyl)oxymethyl]-3-carboxymethyl-2-pyrrolidinon und

(3S,5S)-5-[[4'-(N-Methoxycarbonylamidino)-4-biphenylyl]-oxymethyl]-3-[(methoxycarbonyl)methyl]-2-pyrrolidinon und deren Salze.

Erfindungsgemäß erhält man die neuen Verbindungen der allgemeinen Formel I beispielsweise nach folgenden an und für sich bekannten Verfahren:

a) Zur Herstellung von Verbindungen der allgemeinen Formel I, in der B eine Amidinogruppe darstellt, welche an einem Stickstoffatom durch eine Hydroxygruppe oder durch eine Alkylgruppe mit 1 bis 4 Kohlenstoffatomen substituiert sein kann:

Umsetzung einer gegebenenfalls im Reaktionsgemisch gebildeten Verbindung der allgemeinen Formel

$$Z_1 - C(=NR_{10}) - X - A - Y - E \ , \qquad\qquad (II)$$

in der

A, E, X und Y wie eingangs definiert sind,
$R_{10}$ ein Wasserstoffatom oder eine Alkylgruppe mit 1 bis 4 Kohlenstoffatomen und
$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellt, mit einem Amin der allgemeinen Formel

$$R_{11} - NH - R_{12} \ , \qquad\qquad (III)$$

in der
$R_{11}$ ein Wasserstoffatom, eine Hydroxygruppe oder eine Alkylgruppe mit 1 bis 4 Kohlenstoffatomen und
$R_{12}$ ein Wasserstoffatom bedeuten, oder mit deren Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einem entsprechenden freien Amin oder mit einem entsprechenden Säureadditionssalz wie beispielsweise Ammoniumcarbonat durchgeführt.

Eine Verbindung der allgemeinen Formel II erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50°C, vorzugsweise jedoch bei 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

b) Zur Herstellung von Verbindungen der allgemeinen Formel I, in der B eine Aminogruppe enthält:
Reduktion einer Verbindung der allgemeinen Formel

$$NC - X - A - Y - E \ , \qquad\qquad (IV)$$

in der

A, E, X und Y wie eingangs definiert sind.

Die Reduktion wird vorzugsweise in einem geeigneten Lösungsmittel wie Methanol, Methanol/Wasser, Methanol/Wasser/Ammoniak, Methanol/Salzsäure, Ethanol, Ether, Tetrahydrofuran, Dioxan oder Eisessig in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100°C, vorzugsweise bei Temperaturen zwischen 20 und 80°C, durchgeführt.

c) Zur Herstellung von Verbindungen der allgemeinen Formel I, in der B einen Guanidinorest darstellt:
Umsetzung einer Verbindung der allgemeinen Formel

$$H_2N - X - A - Y - E \ , \qquad\qquad (V)$$

in der

A, E, X und Y wie eingangs definiert sind, oder dessen Säureadditionssalz mit Cyanamid.

Die Umsetzung wird zweckmäßigerweise in einem Lösungsmittel wie Dioxan, Dioxan/Wasser oder Tetrahydrofuran vorzugsweise bei Temperaturen zwischen 80 und 120°C, z.B. bei der Siedetemperatur des Reaktionsgemisches, durchgeführt.

d) Zur Herstellung von Verbindungen der allgemeinen Formel I, in der B einen Guanidinorest darstellt, welcher an einem der Stickstoffatome durch eine Hydroxygruppe oder durch eine Alkylgruppe mit 1 bis 4 Kohlenstoffatomen oder durch eine Methoxycarbonyl-, Ethoxycarbonyl-, Isopropyloxycarbonyl-, Isobutyloxycarbonyl-, Benzyloxycarbonyl- oder Phenyloxycarbonylgruppe substituiert sein kann:

Umsetzung einer Verbindung der allgemeinen Formel

$$R_{13} - NH - X - A - Y - E \,, \hspace{3cm} (VI)$$

in der

A, E, X und Y wie eingangs definiert sind und
$R_{13}$ ein Wasserstoffatom oder eine Alkylgruppe mit 1 bis 4 Kohlenstoffatomen darstellt, mit einem Amidin der allgemeinen Formel

$$R_{14} - Z_2 \,, \hspace{3cm} (VII)$$

in der
$R_{14}$ eine Amidinogruppe, die an einem der Stickstoffatome durch eine Hydroxygruppe oder durch eine Alkylgruppe mit 1 bis 4 Kohlenstoffatomen oder oder durch eine Methoxycarbonyl-, Ethoxycarbonyl-, Isopropyloxycarbonyl-, Isobutyloxycarbonyl-, Benzyloxycarbonyl- oder Phenyloxycarbonylgruppe substituiert sein kann, und
$Z_2$ einen abspaltbaren Rest wie eine 3,5-Dimethylpyrazol-1-yl-, Sulfo-, Methoxy- oder Methylthiogruppe darstellt.

Die Umsetzung wird zweckmäßigerweise in einem Lösungsmittel wie Dimethylformamid, Wasser, Dimethylformamid/Wasser, Dioxan, Dioxan/Wasser oder Tetrahydrofuran gegebenenfalls in Gegenwart einer tertiären organischen Base wie Triethylamin bei Temperaturen zwischen 0 und 100°C, vorzugsweise bei Temperaturen zwischen 20 und 50°C, durchgeführt.
e) Zur Herstellung von Verbindungen der allgemeinen Formel I, in der B eine Aminogruppe darstellt:
Reduktion einer gegebenenfalls im Reaktionsgemisch gebildeten Verbindung der allgemeinen Formel

$$Z_3 - X - A - Y - E \,, \hspace{3cm} (VIII)$$

in der

A, E, X und Y wie eingangs definiert sind und
$Z_3$ eine Nitro- oder Azidogruppe darstellt.

Die Reduktion wird vorzugsweise in einem Lösungsmittel wie Wasser, Methanol, Methanol/Wasser, Ethanol, Ethanol/Wasser, Eisessig, Essigsäurethylester oder Dimethylformamid zweckmäßigerweise mit Wasserstoff in Gegenwart eines Hydrierungskatalysators wie Raney-Nickel, Platin oder Palladium/Kohle, oder mit Hydrazin in Gegenwart von Raney-Nickel bei Temperaturen zwischen 0 und 100°C, vorzugsweise jedoch bei Temperaturen zwischen 20 und 80°C, durchgeführt.
Die Reduktion einer Nitrogruppe kann auch mit Metallen wie Eisen, Zinn oder Zink in Gegenwart einer Säure wie Zink/Essigsäure oder Zink/Calciumchlorid oder mit Salzen wie Eisen(II)sulfat, Zinn(II)chlorid, Natriumsulfid, Natriumhydrogensulfit oder Natriumdithionit in einem Lösungsmittel wie Eisessig, Ethanol, Ethanol/Wasser, Wasser, Wasser/Salzsäure oder Wasser/Schwefelsäure durchgeführt werden.
f) Zur Herstellung von Verbindungen der allgemeinen Formel I, in der E eine Carboxygruppe darstellt:
Überführung einer Verbindung der allgemeinen Formel

$$B - X - A - Y - E' \,, \hspace{3cm} (IX)$$

in der

A, B, X und Y wie eingangs definiert sind und
E', das an ein Kohlenstoffatom gebunden ist, eine mittels Hydrolyse, Behandlung mit einer Säure, Thermolyse oder Hydrogenolyse in eine Carboxygruppe überführbare Gruppe darstellt, mittels Hydrolyse, mittels Behandlung mit einer Säure, Thermolyse oder Hydrogenolyse in eine entsprechende Carboxylverbindung und gegebenenfalls anschließende Decarboxylierung.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe, Ester mit tertiären Alkoholen, z.B. der tert.-Butylester, mittels Behandlung mit einer Säure oder Thermolyse in eine Carboxylgruppe, Ester mit Aralkanolen, z.B. der Benzylester, mittels Hydrogenolyse in eine Carboxylgruppe, und Bis(alkoxycarbonyl)methylgruppen mittels Hydrolyse oder Behandlung mit einer Säure in eine Bis(hydroxycarbonyl)methylgruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure, in Gegenwart einer Base wie Natriumhydroxid oder Kaliumhydroxid in einem geeigneten Lösungsmittel wie Wasser, Wasser/Methanol, Ethanol, Wasser/Ethanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet E' in einer Verbindung der Formel IX eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxylgruppe übergeführt werden.

Bedeutet E' in einer Verbindung der Formel IX beispielsweise die tert.-Butyloxycarbonylgruppe, so kann die tert.-Butylgruppe auch durch Behandlung mit einer Säure wie Trifluoressigsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10°C und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet E' in einer Verbindung der Formel IX beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50°C, z.B. bei Raumtemperatur, und einem Wasserstoffdruck von 1 bis 10 bar abgespalten werden. Bei der Hydrogenolyse können gleichzeitig andere Reste, z.B. eine Nitrogruppe zur Aminogruppe, oder eine Benzyloxygruppe zur Hydroxygruppe mitreduziert werden.

Die gegebenenfalls anschließende Decarboxylierung wird vorzugsweise in einem Lösungsmittel wie Eisessig bei erhöhten Temperaturen, z.B. bei der Siedetemperatur des Reaktionsgemisches, durchgeführt.

g) Zur Herstellung von Verbindungen der allgemeinen Formel I, in der E einen der eingangs erwähnten Esterreste darstellt:

Umsetzung einer Verbindung der allgemeinen Formel

$$B - X - A - Y - COOH , \qquad (X)$$

in der

A, B, X und Y wie eingangs definiert sind, oder deren reaktionsfähige Derivate mit einer Verbindung der allgemeinen Formel

$$H - R_{15} , \qquad (XI)$$

in der

$R_{15}$ eine Alkoxygruppe mit 1 bis 6 Kohlenstoffatomen, eine Dialkylaminocarbonylmethoxygruppe, in welcher jeder Alkylteil 1 bis 3 Kohlenstoffatome enthalten kann, eine Morpholinoethoxy- oder (2-Oxo-1-pyrrolidinyl)ethoxygruppe, eine Phenylalkoxygruppe mit 1 bis 3 Kohlenstoffatomen im Alkoxyteil, wobei der Phenylkern durch eine oder zwei Methoxygruppen substituiert sein kann, oder eine Pyridylmethyloxygruppe darstellt.

Die Umsetzung wird zweckmäßigerweise in einem Lösungsmittel wie Tetrahydrofuran, Chloroform, Dimethylformamid oder in einem entsprechenden Alkohol in Gegenwart eines die Säure aktivierenden Mittels oder eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer Base wie Natriumcarbonat, Kaliumcarbonat, Kalium-tert.butylat oder 1-Hydroxy-benztriazol/Triethylamin oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 80°C, durchgeführt. Die Umsetzung kann jedoch auch mit einem entsprechenden Säurehalogenid oder Säureanhydrid gegebenenfalls in Gegenwart eines säurebindenden Mittels wie vorstehend beschrieben durchgeführt werden.

h) Zur Herstellung von Verbindungen der allgemeinen Formel I, in der E eine Carboxygruppe darstellt:
Oxidation einer Verbindung der allgemeinen Formel

$$B - X - A - Y - CHO , \qquad (XII)$$

in der

A, B, X und Y wie eingangs definiert sind.

Die Oxidation wird vorzugsweise in einem Lösungsmittel wie Methylenchlorid, Acetonitril, Tetrahydrofuran/Wasser, Dioxan/Wasser oder Aceton/Wasser in Gegenwart eines Oxidationsmittels wie Kaliumpermanganat oder Chromtrioxid und in Gegenwart einer Säure wie Schwefelsäure, Salzsäure oder Trifluoressigsäure bei Temperaturen zwischen 0 und 50°C, vorzugsweise bei Raumtemperatur durchgeführt.

i) Zur Herstellung von Verbindungen der allgemeinen Formel I, in denen der B-X-A-Rest entweder eine $B-G_1-T-G_2$-A-Gruppe oder eine $B-G_1$-A-Gruppe darstellt, wobei $G_2$ einem Teil von X und $G_1$-T dem anderen Teil von X entspricht und zusätzlich $G_1$ und $G_2$ oder $G_1$-T auch eine Bindung darstellen können und $G_1$ auch für X stehen kann, und T ein Sauerstoff- oder Schwefelatom, eine Imino-, $-N(COCH_3)-$ oder $-N(SO_2CH_3)$-Gruppe bedeutet:
Umsetzung einer Verbindung der allgemeinen Formel

$$B - G_1 - T - H , \qquad (XIII)$$

mit einer Verbindung der allgemeinen Formel

$$Z_5 - G_2 - A - Y - E , \qquad (XIV)$$

oder einer Verbindung der allgemeinen Formel

$$H - T - G_2 - A - Y - E , \qquad (XV)$$

mit einer Verbindung der allgemeinen Formel

$$B - G_1 - Z_5 , \qquad (XVI)$$

oder einer Verbindung der allgemeinen Formel

$$H - A - Y - E , \qquad (XVII)$$

mit einer Verbindung der allgemeinen Formel

$$B - G_1 - Z_5 , \qquad (XVI)$$

in denen

A, B, E und Y wie eingangs definiert sind,
$G_2$ einem Teil von X und $G_1$-T dem anderen Teil von X entspricht,
wobei zusätzlich $G_1$ und $G_2$ oder $G_1$-T eine Bindung darstellen können und $G_1$ auch für X stehen kann sowie X jeweils wie eingangs definiert ist,
T ein Sauerstoff- oder Schwefelatom, eine Imino-, $-N(COCH_3)-$ oder $-N(SO_2CH_3)$-Gruppe und
$Z_5$ eine Austrittsgruppe bedeuten, sowie mit den Alkali-, Erdalkalimetall- oder MgHal-Salzen einer Verbindung

EP 0 483 667 B1

der allgemeinen Formeln XIII, XV oder XVII.

Ist $Z_5$ an eine gegebenenfalls durch eine Alkyl- oder Arylgruppe substituierte Methylgruppe gebunden, so kommt als Austrittsgruppe vorzugsweise ein Halogenatom, z.B. ein Chlor-, Brom- oder Jodatom, oder eine substituierte Hydroxygruppe, z.B. eine Methansulfonyloxy-, Ethansulfonyloxy-, Benzolsulfonyloxy-, p-Toluolsulfonyloxy- oder Triphenylphosphoniooxygruppe, oder ist $Z_5$ an eine Carbonyl- oder Sulfonylgruppe gebunden, so kommt als Austrittsgruppe beispielsweise ein Halogenatom, z.B. ein Chlor- oder Bromatom, eine Alkoxy-, Aryloxy-, Alkylthio- , Arylthio-, Azido-, Imidazolyl-, Alkylcarbonyloxy-, Arylcarbonyloxy- oder Alkoxycarbonyloxygruppe in Betracht, wobei die vorstehend erwähnten Alkyl- und Arylteile wie eingangs definiert sind.

Die Umsetzung wird zweckmäßigerweise in einem Lösungsmittel wie Methanol, Methylenchlorid, Chloroform, Tetrachlorkohlenstoff, Ether, Tetrahydrofuran, Dioxan, Benzol, Toluol, Acetonitril, Dimethylsulfoxid, Sulfolan oder Dimethylformamid gegebenenfalls in Gegenwart einer anorganischen oder organischen Base, gegebenenfalls in Gegenwart eines Reaktionsbeschleunigers wie Kupfer oder Kupfer(I)chlorid oder auch, falls $Z_5$ eine an eine Carbonylgruppe gebundene Hydroxygruppe darstellt, gegebenenfalls in Gegenwart eines die Säure aktivierenden Mittels, gegebenenfalls in Gegenwart eines wasserentziehenden Mittels oder gegebenenfalls in Gegenwart eines die Aminogruppe aktivierenden Mittels bei Temperaturen zwischen -20 und 200°C, vorzugsweise jedoch bei Temperaturen zwischen -10 und 160°C, durchgeführt.

Die Alkylierung wird vorzugsweise in einem Lösungsmittel wie Tetrahydrofuran, Aceton, Dioxan, Dimethylsulfoxid, Sulfolan, Dimethylformamid oder Dimethylacetamid in Gegenwart einer anorganischen Base wie Kaliumcarbonat, Cäsiumcarbonat, Natriumhydroxid, Kaliumhydroxid, Natriumhydrid oder Kalium-tert.butylat oder in Gegenwart tertiärer organischer Basen wie N-Ethyl-diisopropylamin, welche gegebenenfalls auch als Lösungsmittel dienen können, und gegebenenfalls in Gegenwart eines Phasentransfer-Katalysators wie Polyethylenglykol-750-monomethylether an Polystyrol oder Hexadecyl-trimethylammoniumchlorid bei Temperaturen zwischen 0 und 180°C, vorzugsweise jedoch bei Temperaturen zwischen 10 und 160°C, durchgeführt.

Umsetzungen, bei denen $Z_5$ eine an eine Carbonyl- oder Sulfonylgruppe gebundene Austrittsgruppe darstellt, werden zweckmäßigerweise in einem Lösungsmittel wie Tetrahydrofuran, Methylenchlorid, Chloroform, Acetonitril, Sulfolan oder Dimethylformamid und, falls $Z_5$ eine an eine Carbonylgruppe gebundene Hydroxygruppe darstellt, in Gegenwart eines die Säure aktivierenden Mittels oder eines wasserentziehenden Mittels, z.B. in Gegenwart von Chlorameisensäureisobutylester, Thionylchlorid, Phosphortrichlorid, Phosphorpentoxid, N,N'-Dicyclohexyl-carbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer Base wie Natriumcarbonat, Kaliumcarbonat oder Kalium-tert.butylat oder in Gegenwart einer tertiären organischen Base wie 4-Dimethylaminopyridin, Triethylamin, N-Ethyl-diisopropylamin, N-Methyl-morpholin oder Pyridin, welche gleichzeitig als Lösungsmittel dienen können, bei Temperaturen zwischen -50 und 100°C, vorzugsweise jedoch bei Temperaturen zwischen -30 und 50°C, durchgeführt. Die Acylierungen und Sulfonierungen können jedoch auch mit einem entsprechenden Säurehalogenid oder Säureanhydrid gegebenenfalls in Gegenwart eines säurebindenden Mittels wie vorstehend beschrieben durchgeführt werden.

j) Zur Herstellung von Verbindungen der allgemeinen Formel I, in der $R_1$ einen der für $R_1$ eingangs erwähnten Alkylreste darstellt:

Alkylierung einer Verbindung der allgemeinen Formel

$$B - X - A' - Y - E \,, \qquad \text{(XVIII)}$$

in der

B, E, X und Y wie eingangs definiert sind und
A' einen der im Anpruch 1 erwähnten gegebenenfalls im Kohlenstoffgerüst durch $R_2$ substituierten 5-gliedrigen cyclische Alkyleniminogruppe darstellt, wobei $R_2$ wie eingangs definiert ist, mit einer Verbindung der allgemeinen Formel

$$R_1' - Z_6 \,, \qquad \text{(XIX)}$$

in der
$R_1'$ einen der für $R_1$ eingangs erwähnten Alkylreste darstellt und
$Z_6$ eine Austrittsgruppe wie ein Halogenatom, z. B. ein Chlor-, Brom- oder Jodatom, oder eine substituierte Sulfonyloxygruppe, z.B. eine Methansulfonyloxy-, Ethansulfonyloxy-, Phenylsulfonyloxy- oder p-Toluolsulfonyloxygruppe, darstellt.

10

Die Alkylierung wird vorzugsweise in einem Lösungsmittel wie Methanol, Methylenchlorid, Chloroform, Tetrachlorkohlenstoff, Ether, Tetrahydrofuran, Dioxan, Benzol, Toluol, Acetonitril, Sulfolan oder Dimethylformamid in Gegenwart einer anorganischen Base wie Kaliumcarbonat, Cäsiumcarbonat, Natriumhydroxid, Kaliumhydroxid, Natriumhydrid oder Kalium-tert.butylat oder in Gegenwart einer tertiären organischen Base wie N-Ethyl-diisopropylamin, welche gegebenenfalls auch als Lösungsmittel dienen kann, und gegebenenfalls in Gegenwart eines Phasentransfer-Katalysators wie Polyethylenglykol-750-monomethylether an Polystyrol oder Hexadecyl-trimethylammoniumchlorid bei Temperaturen zwischen 0 und 180°C, vorzugsweise jedoch bei Temperaturen zwischen 10 und 160°C, durchgeführt.

k) Zur Herstellung von Verbindungen der allgemeinen Formel I, in der $R_1$ einen der eingangs erwähnten Acyl- oder Sulfonylreste und A einen Pyrrolidinring darstellen:
Alkylierung einer Verbindung der allgemeinen Formel

$$B - X - A'' - Y - E , \qquad (XX)$$

in der

B, E, X und Y wie eingangs definiert sind und
A'' einen gegebenenfalls im Kohlenstoffgerüst durch $R_2$ substituierten Pyrrolidinring darstellt, wobei $R_2$ wie eingangs definiert ist, mit einer Verbindung der allgemeinen Formel

$$R_1'' - Z_7 , \qquad (XXI)$$

in der
$R_1''$ einen der für $R_1$ in den Ansprüchen 1 bis 6 erwähnten Acyl- oder Sulfonylreste darstellt und
$Z_7$ eine Hydroxygruppe, eine Austrittsgruppe wie ein Halogenatom, z.B. ein Chlor- oder Bromatom, eine Azidogruppe oder eine Acyloxygruppe, z.B. die Acetoxy-, Methoxycarbonyloxy-, Ethoxycarbonyloxy- oder Isobutoxycarbonyloxygruppe, oder auch $Z_7$ zusammen mit dem Wasserstoffatom einer zur Carbonylgruppe benachbarten Iminogruppe eine weitere Kohlenstoff-Stickstoff-Bindung bedeuten.

Die Umsetzung wird zweckmäßigerweise in einem Lösungsmittel wie Methanol, Methylenchlorid, Chloroform, Tetrachlorkohlenstoff, Ether, Tetrahydrofuran, Dioxan, Benzol, Toluol, Acetonitril, Sulfolan oder Dimethylformamid gegebenenfalls in Gegenwart einer anorganischen oder organischen Base, gegebenenfalls in Gegenwart eines die Säure aktivierenden Mittels, gegebenenfalls in Gegenwart eines wasserentziehenden Mittels oder gegebenenfalls eines die Aminogruppe aktivierenden Mittels bei Temperaturen zwischen -20 und 200°C, vorzugsweise jedoch bei Temperaturen zwischen -10 und 160°C, durchgeführt.

Bedeutet $Z_7$ eine Hydroxygruppe, so wird die Acylierung zweckmäßigerweise in einem Lösungsmittel wie Tetrahydrofuran, Methylenchlorid, Chloroform, Sulfolan oder Dimethylformamid in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer Base wie Natriumcarbonat, Kaliumcarbonat, Kalium-tert.butylat oder 1-Hydroxy-benztriazol/Triethylamin oder in Gegenwart einer tertiären organischen Base wie 4-Dimethylamino-pyridin, Triethylamin, N-Ethyl-diisopropylamin, N-Methyl-morpholin oder Pyridin, welche gleichzeitig auch als Lösungsmittel dienen können, bei Temperaturen zwischen -10 und 100°C, vorzugsweise jedoch bei Temperaturen zwischen 0 und 50°C, durchgeführt.

Die Acylierung oder Sulfonylierung wird jedoch vorzugsweise mit einem entsprechenden Säurehalogenid oder Säureanhydrid gegebenenfalls in Gegenwart eines säurebindenden Mittels wie vorstehend beschrieben durchgeführt.

l) Zur Herstellung von Verbindungen der allgemeinen Formel I, in der E eine Hydroxycarbonylgruppe, eine Alkoxycarbonylgruppe mit insgesamt 2 bis 7 Kohlenstoffatomen, eine Pyridylmethyloxycarbonyl- oder Phenylalkoxycarbonylgruppe mit 1 bis 3 Kohlenstoffatomen im Alkoxyteil, in der der Phenylkern durch eine oder zwei Methoxygruppen substituiert sein kann, darstellt:
Oxidation einer Verbindung der allgemeinen Formel

$$B - X - A - Y - E'' , \qquad (XXII)$$

in der

A, B, X und Y wie eingangs definiert sind und

E" eine Vinyl- oder 1,2-Dihydroxyalkylgruppe darstellt, und erforderlichenfalls anschließende Veresterung einer so erhaltenen Verbindung mit einem entsprechenden Alkohol.

Die Oxidation wird in einem Lösungsmittel wie Methylenchlorid, Acetonitril, Acetonitril/Wasser, Methylenchlorid/Acetonitril/Wasser oder Tetrachlorkohlenstoff/Acetonitril/Wasser in Gegenwart eines Oxidationsmittels wie Kaliumpermanganat oder Rutheniumtetroxid, wobei das Rutheniumtetroxid vorzugsweise im Reaktionsgemisch durch Umsetzung eines Rutheniumsalzes wie Rutheniumtrichlorid mit einem Oxidationsmittel wie Natriumperjodat gebildet wird, bei Temperaturen zwischen -10 und 60°C, vorzugsweise bei Temperaturen zwischen 0 und 40°C, durchgeführt.

Die gegebenenfalls anschließende Veresterung wird zweckmäßigerweise in einem geeigneten Lösungsmittel, z.B. in einem entsprechenden Alkohol, Pyridin, Toluol, Methylenchlorid, Tetrahydrofuran oder Dioxan, in Gegenwart eines säureaktivierenden und/oder wasserentziehenden Mittels wie Chlorwasserstoff, konzentrierte Schwefelsäure, Thionylchlorid, Chlorameisensäureethylester, Carbonyldiimidazol oder N,N'-Dicyclohexyl-carbodiimid oder dessen Isoharnstoffestern, gegebenenfalls in Gegenwart eines Reaktionsbeschleunigers wie Kupferchlorid, oder durch Umesterung, z.B. mit einem entsprechenden Kohlensäurediester, bei Temperaturen zwischen 0 und 100°C, vorzugsweise jedoch bei Temperaturen zwischen 20°C und der Siedetemperatur des betreffenden Lösungsmittels, durchgeführt.

m) Zur Herstellung von Verbindungen der allgemeinen Formel I, in der E eine der eingangs erwähnten durch eine Alkoxy-, Phenylalkoxy- oder Pyridylmethyloxygruppe substituierte Carbonylgruppe darstellt:

Umsetzung einer Verbindung der allgemeinen Formel

$$B - X - A - Y - COOH , \qquad (XI)$$

in der

A, B, X und Y wie eingangs definiert sind, mit einem Formamidacetal der allgemeinen Formel

$$(R_{16})_2N - CH(OR_{17})_2 , \qquad (XXIII)$$

in der

$R_{16}$ eine niedere Alkylgruppe und

$R_{17}$ eine Alkylgruppe mit 1 bis 6 Kohlenstoffatomen, eine im Phenylteil gegebenenfalls durch eine oder zwei Methoxygruppen substituierte Phenylalkylgruppe oder eine Pyridylmethylgruppe bedeuten.

Die Umsetzung wird zweckmäßigerweise in einem Lösungsmittel wie Tetrahydrofuran, Dioxan oder Toluol bei Temperaturen zwischen 40 und 160°C, vorzugsweise jedoch bei Temperaturen zwischen 60 und 120°C, durchgeführt.

n) Zur Herstellung von Verbindungen der allgemeinen Formel I, in der A einen Pyrrolidinring und $R_1$ in 1-Stellung des Pyrrolidinringes eine Carbonylgruppe darstellt, die durch eine Amino-, Methylamino-, Ethylamino- oder Dimethylaminogruppe substituiert ist:

Umsetzung einer Verbindung der allgemeinen Formel

$$B - X - A''' - Y - E , \qquad (XXIV)$$

in der

B, E, X und Y wie eingangs definiert sind und

A''' einen gegebenenfalls durch $R_2$ substituierten Pyrrolidinring darstellt, welcher in 1-Stellung durch eine Halogencarbonyl- oder N-Azolylcarbonylgruppe substituiert ist, mit einem Amin der allgemeinen Formel

$$H - N(R_5)_2 , \qquad (XXV)$$

in der

$R_5$ ein Wasserstoffatom, eine Methyl- oder Ethylgruppe darstellt, umgesetzt wird.

Die Umsetzung wird zweckmäßigerweise in einem Lösungsmittel wie Methylenchlorid, Dioxan, Tetrahydrofuran oder Toluol gegebenenfalls in Gegenwart einer Base wie Triethylamin, N-Ethyl-diisopropylamin, N-Methyl-morpholin

EP 0 483 667 B1

oder Pyridin, welche gleichzeitig als Lösungsmittel dienen können, bei Temperaturen zwischen -10 und 60°C, vorzugsweise jedoch bei Raumtemperatur, durchgeführt.

Erhält man erfindungsgemäß eine Verbindung der allgemeinen Formel I, die eine Amino-, Methylamino- oder Iminogruppe enthält, so kann diese mittels Acylierung, Sulfonylierung oder Alkylierung in eine entsprechende Verbindung der allgemeinen Formel I übergeführt werden und/oder

eine Verbindung der allgemeinen Formel I, die eine Hydroxygruppe enthält, so kann diese mittels Alkylierung oder Acylierung in eine entsprechende Verbindung der allgemeinen Formel I übergeführt werden und/oder

eine Verbindung der allgemeinen Formel I, die eine Carbonylbrücke enthält, so kann diese mittels Reduktion in eine entsprechende Hydroxymethylenverbindung der allgemeinen Formel I übergeführt werden und/oder

eine Verbindung der allgemeinen Formel I, die eine Carbonylbrücke enthält, so kann diese mittels Reduktion in eine entsprechende Methylenverbindung der allgemeinen Formel I übergeführt werden und/oder

eine Verbindung der allgemeinen Formel I, die eine Sulfenyl- oder Sulfenylgruppe oder eine Thioetherbrücke enthält, so kann diese mittels Oxidation in eine entsprechende S-Oxidverbindung der allgemeinen Formel I übergeführt werden und/oder

eine Verbindung der allgemeinen Formel I, die eine Sulfenyl- oder Sulfinylgruppe oder eine Thioetherbrücke enthält, oder eine S-Oxidverbindung der allgemeinen Formel I, so kann diese mittels Oxidation in eine entsprechende S,S-Dioxidverbindung der allgemeinen Formel I übergeführt werden und/oder

eine Verbindung der allgemeinen Formel I, die einen durch eine Aminogruppe substituierten aromatischen Rest enthält, so kann diese mittels Sandmeyer-Reaktion in eine entsprechende Cyanoverbindung der allgemeinen Formel I übergeführt werden und/oder

eine Verbindung der allgemeinen Formel I, die eine Estergruppe enthält, so kann diese durch Umsetzung mit einem Amin in ein entsprechendes Amid übergeführt werden und/oder

eine Verbindung der allgemeinen Formel I, die eine Estergruppe enthält, so kann diese mittels Reduktion eine eine entsprechende Hydroxymethylverbindung der allgemeinen Formel I übergeführt werden und/oder

eine Verbindung der allgemeinen Formel I, die eine Estergruppe enthält, so kann diese mittels Umesterung in einen entsprechenden Ester übergeführt werden und/oder

eine Verbindung der allgemeinen Formel I, die eine Amidino- oder Guanodinogruppe enthält, so kann diese mittels Acylierung in eine entsprechende Verbindung der allgemeinen Formel I übergeführt werden.

Die nachträgliche Acylierung oder Sulfonylierung einer Amino-, Alkylamino-, Imino- oder Hydroxygruppe wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, gegebenenfalls in Gegenwart einer anorganischen Base wie Natriumcarbonat oder einer tertiären organischen Base wie Triethylamin, Pyridin oder 4-Dimethylaminopyridin, welche gleichzeitig als Lösungsmittel dienen können, bei Temperaturen zwischen -25 und 150°C, vorzugsweise jedoch bei Temperaturen zwischen -10°C und der Siedetemperatur des verwendeten Lösungsmittels, durchgeführt. Die nachträgliche Acylierung oder Sulfonylierung wird jedoch wie vorstehend beschrieben vorzugsweise mit einem entsprechenden Säurehalogenid oder Säureanhydrid durchgeführt, wobei diese auch ohne Lösungsmittel durchgeführt werden kann.

Die nachträgliche Alkylierung einer Amino-, Alkylamino- oder Iminoverbindung wird beispielsweise in einem Lösungsmittel oder Lösungsmittelgemisch wie Methylenchlorid, Dimethylformamid, Dimethylsulfoxid, Benzol, Chlorbenzol, Tetrahydrofuran, Benzol/Tetrahydrofuran oder Dioxan in Gegenwart eines Alkylierungsmittels wie Methyljodid, Methylbromid, Ethylbromid, Dimethylsulfat oder Benzylchlorid beispielsweise in Gegenwart eines säurebindenden Mittels, z.B. eines Alkoholats wie Kalium-tert.-butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid zweckmäßigerweise bei Temperaturen zwischen 0 und 150°C, vorzugsweise bei Temperaturen zwischen 0 und 50°C, durchgeführt.

Die nachträgliche Alkylierung einer Amino- oder Alkylaminoverbindung kann auch mittels reduktiver Aminierung in einem geeigneten Lösungsmittel wie Methanol, Ethanol, Tetrahydrofuran, Dioxan, Acetonitril oder deren Gemische mit Wasser in Gegenwart eines geeigneten Reduktionsmittels wie eines geeigneten komplexen Metallhydrids, vorzugsweise jedoch in Gegenwart von Natriumcyanborhydrid, oder mit Wasserstoff in Gegenwart eines Hydrierungskatalysators wie Palladium/Kohle bei Temperaturen zwischen 0 und 50°C, vorzugsweise jedoch bei Raumtemperatur, durchgeführt werden.

Die nachträgliche O-Alkylierung wird zweckmäßigerweise in einem Lösungsmittel oder Lösungsmittelgemisch wie Methylenchlorid, Dimethylformamid, Dimethylsulfoxid, Benzol, Chlorbenzol, Tetrahydrofuran, Benzol/Tetrahydrofuran oder Dioxan in Gegenwart eines Alkylierungsmittels wie Methyljodid, Methylbromid, Ethylbromid, Dimethylsulfat oder Benzylchlorid vorzugsweise in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid zweckmäßigerweise bei Temperaturen zwischen 0 und 150°C, vorzugsweise bei Temperaturen zwischen 0 und 50°C, durchgeführt.

Die nachträgliche O-Acylierung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, gegebenenfalls in Gegenwart einer anorganischen Base wie Natriumcarbonat oder einer tertiären organischen Base wie Pyridin, 4-Dimethylaminopyridin oder Triethylamin, welche gleichzeitig als Lösungsmittel dienen können, bei Temperaturen zwischen -25 und 150°C, vorzugsweise jedoch bei Temperaturen zwischen -10°C und der Siedetemperatur des verwendeten Lösungsmittels, durchgeführt. Die nachträgliche O-Acylierung wird jedoch wie vorstehend beschrieben vorzugsweise mit einem entsprechenden Säurehalogenid oder Säureanhydrid durchgeführt, wobei diese auch ohne Lösungsmittel durchgeführt werden kann.

Die nachträgliche Reduktion der Carbonylbrücke zu einer Hydroxymethylbrücke wird in einem geeigneten Lösungsmittel wie Methanol, Methanol/Wasser, Ethanol, Ether, Tetrahydrofuran, Dioxan oder Eisessig in Gegenwart von katalytisch angeregtem Wasserstoff, z.B. von Wasserstoff in Gegenwart von Platin oder Palladium/Kohle, oder in Gegenwart eines Metallhydrids wie Natriumborhydrid, Lithiumborhydrid oder Lithiumaluminiumhydrid bei Temperaturen zwischen 0 und 50°C, vorzugsweise jedoch bei Raumtemperatur, durchgeführt.

Die nachträgliche Reduktion der Carbonylbrücke zu einer Methylenbrücke wird in einem geeigneten Lösungsmittel wie Methanol, Ethanol, Ether, Tetrahydrofuran, Dioxan oder Eisessig in Gegenwart von katalytisch angeregtem Wasserstoff, z.B. von Wasserstoff in Gegenwart von Platin oder Palladium/Kohle, der in Gegenwart eines Metallhydrids wie Natriumborhydrid, Lithiumborhydrid oder Lithiumaluminiumhydrid bei erhöhten Temperaturen, z.B. bei Temperaturen zwischen 30 und 150°C, vorzugsweise bei Temperaturen zwischen 50 und 100°C, durchgeführt.

Die nachträgliche Oxidation eines Thioethers wird vorzugsweise in einem Lösungsmittel oder Lösungsmittelgemisch, z.B. in Wasser, Wasser/Pyridin, Aceton, Eisessig, Methylenchlorid, Eisessig/Acetanhydrid, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer entsprechenden S-Oxidverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wäßrigem Methanol oder Ethanol bei -15 bis 25°C, mit Brom in Eisessig oder wäßriger Essigsäure, mit N-Brom-succinimid in Ethanol, mit tert.Butylhypochlorit in Methanol bei -80 bis -30°C, mit Jodbenzodichlorid in wäßrigem Pyridin bei 0 bis 50°C, mit Salpetersäure in Eisessig bei 0 bis 20°C, mit Chromsäure in Eisessig der in Aceton bei 0 bis 20°C und mit Sulfurylchlorid in Methylenchlorid bei -70°C, der hierbei erhaltene Thioether-Chlor-Komplex wird zweckmäßigerweise mit wäßrigem Ethanol hydrolysiert.

Zur Herstellung einer S,S-Dioxidverbindung der allgemeinen Formel I wird die Oxidation zweckmäßigerweise mit einem bzw. mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

Die nachträgliche Umsetzung eines Diazoniumsalzes, z.B. des Hydrogensulfates in Schwefelsäure oder des Chlorids in wäßriger Salzsäure wird zweckmäßigerweise in Gegenwart von Trinatrium-Kupfer-(I)-tetracyanid in einem Lösungsmittel wie Wasser, Methanol/Wasser oder Wasser/Salzsäure durchgeführt. Das hierzu erforderliche Diazoniumsalz wird zweckmäßigerweise in einem Lösungsmittel, z.B. in Wasser/Salzsäure, Wasser/Schwefelsäure, Methanol/Salzsäure, Ethanol/Salzsäure oder Dioxan/Salzsäure, durch Diazotierung einer entsprechenden Aminoverbindung mit einem Nitrit, z.B. Natriumnitrit oder einem Ester der salpetrigen Säuren, bei niederen Temperaturen, z.B. bei Temperaturen zwischen -10 und 5°C, hergestellt.

Die nachträgliche Umsetzung eines Esters mit einem Amin erfolgt vorzugsweise in einem Lösungsmittel wie Methanol, Ethanol, Tetrahydrofuran, Dioxan oder in einem Überschuß des eingesetzten Amins bei Temperaturen zwischen 0 und 70°C, vorzugsweise bei Temperaturen zwischen 20 und 50°C.

Die nachträgliche Reduktion einer Estergruppe wird vorzugsweise in einem Lösungsmittel wie Diethylether, Tetrahydrofuran oder Dioxan mit einem komplexen Metallhydrid wie Lithiumborhydrid oder Lithiumaluminiumhydrid bei Temperaturen zwischen -20 und 80°C, vorzugsweise jedoch mit Lithiumborhydrid oder Lithiumaluminiumhydrid in Tetrahydrofuran/Methanol bei Temperaturen zwischen 0°C und 25°C, durchgeführt.

Die nachträgliche Umsetzung einer Estergruppe mit einem Alkohol wird vorzugsweise in einem entsprechenden Alkohol als Lösungsmittel gegebenenfalls in Gegenwart eines weiteren Lösungsmittel wie Methylenchlorid oder Ether vorzugsweise in Gegenwart einer Säure wie Salzsäure bei Temperaturen zwischen 0 und 100°C, vorzugsweise bei Temperaturen zwischen 20 und 80°C, durchgeführt.

Die nachträgliche Acylierung einer Amidino- oder Guanidinogruppe wird zweckmäßigerweise in einem Lösungsmittel wie Methylenchlorid, Chloroform, Dioxan, Tetrahydrofuran oder Dimethylformamid vorzugsweise mit einem Säurehalogenid oder -anhydrid, insbesondere mit einem Kohlensäureesterchlorid, oder mit einer Halogencyanverbindung in Gegenwart einer anorganischen Base wie Natriumcarbonat oder Natronlauge oder einer tertiären organischen Base wie Triethylamin, N-Ethyl-diisopropylamin, Pyridin oder 4-Dimethylaminopyridin, welche gleichzeitig auch als Lösungsmittel dienen können, bei Temperaturen zwischen -25 und 100°C, vorzugsweise jedoch bei Temperaturen zwischen 0 und 50°C, durchgeführt.

Bei den vorstehend beschriebenen Umsetzungen a) bis n) und bei den nachträglichen Umsetzungen können gegebenenfalls vorhandene reaktive Gruppen wie Hydroxy-, Carboxy-, Phosphono-, Amidino-, Guanidino-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl- , Benzyl- oder Tetrahydropyranylgruppe,

als Schutzreste für eine Carboxylgruppe die Trimethylsilyl-, Methyl-, Ethyl-, tert.Butyl-, Benzyl- oder Tetrahydropyranylgruppe,

als Schutzreste für eine Amidino- oder Guanidinogruppe die Benzyloxycarbonylgruppe und für die Guanidinogruppe zusätzlich die 4-Methoxy-2,3,6-trimethyl-phenylsulfonylgruppe,

als Schutzreste für eine Phosphonogruppe die Trimethylsilyl-, Methyl-, Ethyl- oder Benzylgruppe und

als Schutzrest für eine Amino-, Alkylamino- oder Iminogruppe die Acetyl-, Benzoyl-, Ethoxycarbonyl-, tert.Butoxycarbonyl-, Benzyloxycarbonyl-, Benzyl- oder Methoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Methanol/Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysatorswie Palladium/Kohle in einem Lösungsmittel wie Methanol, Ethanol, Dioxan, Essigsäureethylester oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Essigsäureethylester oder Ether.

Die Abspaltung nur eines Alkylrestes von einer O,O'-Dialkylphosphonogruppe erfolgt beispielsweise mit Natriumjodid in einem Lösungsmittel wie Aceton, Ethylmethylketon, Acetonitril oder Dimethylformamid bei Temperaturen zwischen 40 und 150°C, vorzugsweise jedoch bei Temperaturen zwischen 60 und 100°C.

Die Abspaltung beider Alkylreste von einer O,O'-Dialkylphosphonogruppe erfolgt beispielsweise mit Jodtrimethylsilan, Bromtrimethylsilan oder Chlortrimethylsilan/Natriumjodid in einem Lösungsmittel wie Methylenchlorid, Chloroform oder Acetonitril bei Temperaturen zwischen 0 C und der Siedetemperatur des Reaktionsgemisches, vorzugsweise jedoch bei Temperaturen zwischen 20 und 60°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalischen-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze bildenden optisch aktiven Substanz, insbesondere Säuren, und Trennen des auf diese Weise erhaltenen diastereomeren Salzgemisches, z.B. auf Grund von verschiedenen Löslichkeiten, wobei aus den reinen diastereomeren Salzen die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure.

Desweiteren können die erhaltenen Verbindungen der Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsstoffe verwendeten Verbindungen der Formeln II bis XXV sind teilweise literaturbekannt oder man erhält diese nach literaturbekannten Verfahren (siehe Beispiele).

Die als Ausgangsstoffe verwendeten Verbindungen der allgemeinen Formel XXIV erhält man durch Umsetzung einer Verbindung der allgemeinen Formel XX mit einem Carbonyldihalogenid oder mit einem N,N'-Carbonyl-bis-azol.

Wie bereits eingangs erwähnt, weisen die neuen cyclischen Iminoderivate der allgemeinen Formel I und deren Additionssalze, insbesondere deren physiologisch verträgliche Additionssalze mit anorganischen oder organischen Säuren oder Basen, wertvolle pharmakologische Eigenschaften auf. So weisen die Verbindungen der allgemeinen Formel I, in denen B eine gegebenenfalls substituierte Amino- oder Iminogruppe oder eine gegebenenfalls in vivo in eine gegebenenfalls substituierte Amino- oder Iminogruppe überführbare Gruppe, z.B. eine durch eine Alkoxycarbonylgruppe substituierte Amino- oder Iminogruppe, enthält und Y-E eine Carboxyl-, Sulfo-, Phosphono- oder O-Methyl-phosphonogruppe oder eine in vivo in eine Carboxyl-, Sulfo-, Phosphono- oder O-Methyl-phosphonogruppe überführbare Gruppe, z.B. eine durch eine Alkoxygruppe substituierte Carbonylgruppe, enthält, wertvolle pharmakologische Eigenschaften auf, nämlich neben einer entzündungshemmenden und den Knochenabbau hemmenden Wirkung insbesondere antithrombotische, antiaggregatorische und tumor- bzw. metastasenhemmende Wirkungen.

Beispielsweise wurden die Verbindungen der allgemeinen Formel I auf ihre biologischen Wirkungen wie folgt untersucht:

1. Fibrinogen-Bindung an Humanthrombozyten

Das durch Punktion einer Antekubitalvene gewonnene Blut wird mit Trinatriumcitrat (Endkonzentration: 13 mM) antikoaguliert und 10 Minuten bei 170 *g zentrifugiert. Das überstehende plättchenreiche Plasma wird auf eine Sepharose 2B-Säule (Pharmacia) gegeben und mit einer Lösung aus 90 mM Kochsalz, 14 mM Trinatriumcitrat, 5 mM Glucose und 50 mM Tris(hydroxymethyl)aminomethan, eingestellt auf pH 7,4, eluiert. Die vor den Plasmaproteinen erscheinenden gelfiltrierten Plättchen (GFP) werden für die Bindungsversuche verwendet.

50 $\mu$l einer 60 mM Calziumchlorid-Lösung, 50 $\mu$l einer 0,6 mM Adenosindiphosphat-Lösung, 100 $\mu$l Substanzlösung bzw. Lösungsmittel und 50 $\mu$l Fibrinogenlösung (enthaltend 3 $\mu$g 125-J-Fibrinogen) werden zu 750 $\mu$l GFP gegeben und bei Raumtemperatur 20 Minuten inkubiert. Die unspezifische Bindung wird in Gegenwart von 3 mg/ml kaltem Fibrinogen bestimmt.

900 $\mu$l des Inkubates werden vorsichtig auf 250 $\mu$l Silikonöl (AP 38: AR 20, 1:2 v/v, Wacker Chemie) in Eppendorf-Gefäße pipettiert und 2 Minuten bei 10 000 *g zentrifugiert. Der wäßrige Überstand und ein Teil des Öls werden abgezogen, die Gefäßspitze mit dem Plättchenpellet abgeschnitten und im Gamma-Zähler die Menge des gebundenen Fibrinogens bestimmt. Aus einer Konzentrationsreihe wird die Substanzkonzentration ermittelt, welche die Fibrinogenbindung zu 50 % hemmt und als $IC_{50}$ angegeben.

2. <u>Antithrombotische Wirkung</u>

<u>Methodik</u>

Die Thrombozytenaggregation wird nach der Methode von Born und Cross (J. Physiol. <u>170</u>, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

<u>Collagen-induzierte Aggregation</u>

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München. Vor der Collagen-Zugabe wird das Plasma jeweils 10 Minuten mit der Substanz bei 37°C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungs-test IC$_{50}$[μM] | Hemmung der Plättchen-aggregation EC$_{50}$[μM] |
|---|---|---|
| 3(2) | 2,3 | 1,9 |
| 3(166) | 0,42 | 1,0 |
| 4 | 0,032 | 0,9 |
| 4(1) | 0,024 | 0,17 |
| 4(3) | 0,031 | 0,06 |
| 4(10) | 0,05 | 2,6 |
| 4(36) | 0,041 | 0,41 |
| 4(40) | 0,17 | 2,0 |
| 4(41) | 2,8 | 19,0 |
| 4(46) | 0,69 | 12,0 |
| 4(51) | 0,027 | 0,08 |
| 4(56) | 0,02 | 0,06 |
| 4(58) | 0,02 | 0,21 |
| 4(70) | 0,4 | 19,0 |
| 4(88) | 0,23 | 2,8 |
| 4(92) | 0,026 | 0,1 |
| 14(8) | 4,6 | 20,0 |
| 19(1) | 4,7 | 66,0 |
| 20 | 1,2 | 5,8 |
| 20(1) | 2,2 | 33,0 |
| 35 | 4,6 | 11,0 |

Außerdem hemmt beispielsweise die Verbindung des Beispiels 34(1) die Collagen-induzierte Thrombozytenaggregation ex vivo am Rhesusaffen nach oraler Gabe von 1 mg/kg länger als 8 Stunden.

Die neuen Verbindungen sind gut verträglich, da beispielsweise die approximative LD$_{50}$ der Verbindung des Beispiels 4(3) an der Ratte oberhalb 100 mg/kg bei intravenöser Gabe und die der Verbindung des Beispiels 34(1) an der Ratte oberhalb 2000 mg/kg nach oraler Gabe liegt.

Auf Grund ihrer Hemmwirkung auf Zell-Zell- bzw. Zell-Matrix-Wechselwirkungen eignen sich die neuen cyclischen Iminoderivate der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z. B. bei der Bekämpfung bzw. Verhütung von venösen und arteriellen Thrombosen, von zerebrovasculären Erkrankungen, von Lungenembolien, des Herzinfarkts, der Arteriosklerose, der Osteoporose und der Metastasierung von Tumoren und der Therapie genetisch bedingter oder auch erworbener Störungen der Interaktion von Zellen untereinander oder mit soliden Strukturen. Weiterhin eignen sich diese bei der Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 μg und 30 mg/kg Körpergewicht, vorzugsweise bei 1 μg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, α-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose, Cyclodextrine wie Hydroxypropyl-β-cyclodextrin oder fetthaltigen Substanzen wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Herstellung der Ausgangsverbindungen:

Beispiel I

(S)-1-(4-Phenylbutyl)-5-[(trityloxy)methyl]-2-pyrrolidinon

Zu 6 g Natriumhydrid (55-60%ige Dispersion in Paraffinöl) in 50 ml trockenem Dimethylformamid werden unter Rühren und Eiskühlung 40 g (S)-5-[(Trityloxy)methyl]-2-pyrrolidinon in 150 ml trockenem Dimethylformamid zugetropft. Anschließend wird 2 Stunden bei Raumtemperatur gerührt und dann 32 g 4-Phenylbutylbromid in 50 ml trockenem Dimethylformamid zugetropft. Nach 18 Stunden Rühren bei Raumtemperatur wird der Ansatz auf Eis gegossen, die Mischung dreimal mit Essigester ausgeschüttelt, die vereinigten organischen Phasen mit Wasser gewaschen, über Natriumsulfat getrocknet und eingeengt. Das Rohprodukt wird über eine Kieselgelsäule mit Toluol/Aceton (4:1) gereinigt.

Ausbeute: 51,5 g (94 % der Theorie),
$R_f$-Wert: 0,41 (Kieselgel; Toluol/Aceton = 4:1)

Analog werden erhalten:

(1) (5S)-1-(4-Methoxybenzyl)-5-[(trityloxy)methyl]-2-pyrrolidinon

$R_f$-Wert: 0,50 (Kieselgel; Cyclohexan/Essigester = 1:1)

| Ber.: | C 80,48 | H 6,54 | N 2,93 |
|-------|---------|--------|--------|
| Gef.: | 80,21 | 6,74 | 2,75 |

(2) (S)-1-[3-(4-Benzyloxyphenyl)propyl]-5-[(trityloxy)methyl]-2-pyrrolidinon

$R_f$-Wert: 0,47 (Kieselgel; Toluol/Aceton = 4:1)

Beispiel II

(S)-1-(3-Phenylpropyl)-5-[(trityloxy)methyl]-2-pyrrolidinon

203,7 g (S)-5-[(Trityloxy)methyl]-2-pyrrolidinon, 3 l Toluol, 1,4 l 50%ige wäßrige Natronlauge, 139,4 g 3-Phenylpropylbromid und 10 g Methyl-trioctylammoniumchlorid werden unter Argon 25 Stunden bei Raumtemperatur kräftig gerührt. Dann werden 40 ml 3-Phenylpropylbromid zugefügt und weitere 24 Stunden gerührt. Der Ansatz wird zwischen Toluol und Wasser verteilt, die organische Phase abgetrennt, über Magnesiumsulfat getrocknet, dann eine halbe Stunde mit 5 Löffeln Aktivkohle gerührt, filtriert und einrotiert. Der Eindampfrückstand wird über eine Kieselgelsäule mit Methylenchlorid und dann mit Essigester chromatographiert. Der kristalline Eindampfrückstand wird dann aus Isopropanol/Wasser (4:1) umkristallisiert.

Ausbeute: 234,3 g (86 % der Theorie),
Schmelzpunkt: 103-104°C
$R_f$-Wert: 0,71 (Kieselgel; Essigester)

Analog werden erhalten:

(1) (S)-1-(3-Cyclohexylpropyl)-5-[(trityloxy)methyl]-2-pyrrolidinon

R_f-Wert: 0,14 (Kieselgel; Methylenchlorid/Methanol = 100:1)

(2) (R)-1-(3-Phenylpropyl)-5-[(trityloxy)methyl]-2-pyrrolidinon

Schmelzpunkt: 109°C

R_f-Wert: 0,37 (Kieselgel; Cyclohexan/Essigester = 2:1)

| Ber.: | C 83,33 | H 6,99 | N 2,94 |
|-------|---------|--------|--------|
| Gef.: | 83,31   | 7,15   | 2,95   |

(3) (R,S)-5-(4-Methoxyphenyl)-1-(3-phenylpropyl)-2-pyrrolidinon

R_f-Wert: 0,09 (Kieselgel; Cyclohexan/Essigester = 2:1)

| Ber.: | C 77,64 | H 7,49 | N 4,53 |
|-------|---------|--------|--------|
| Gef.: | 77,33   | 7,58   | 4,53   |

(4) (S)-1-Benzyl-5-[(trityloxy)methyl]-2-pyrrolidinon

Schmelzpunkt: 103-104°C

(5) (S)-1-Isobutyl-5-[(trityloxy)methyl]-2-pyrrolidinon

Schmelzpunkt: 91-93°C
R_f-Wert: 0,68 (Kieselgel; Essigester)

(6) (S)-1-(2-Phenylethyl)-5-[(trityloxy)methyl]-2-pyrrolidinon

Schmelzpunkt: 95-98°C

(7) (S)-1-(4-Phenyloxybutyl)-5-[(trityloxy)methyl]-2-pyrrolidinon

Schmelzpunkt: 69-72°C

(8) (S)-1-[2-(Benzyloxy)ethyl-5-[(trityloxy)methyl]-2-pyrrolidinon

Schmelzpunkt: 78-80°C,
R_f-Wert: 0,60 (Kieselgel; Essigester)

(9) (S)-1-Methyl-5-[(trityloxy)methyl]-2-pyrrolidinon

R_f-Wert: 0,41 (Kieselgel; Essigester)

Beispiel III

(S)-1-(3-Phenylpropyl)-5-[(trityloxy)methyl]-2-pyrrolidinon

Zu 2255 g (S)-5-[(Trityloxy)methyl]-2-pyrrolidinon und 1005 ml 3-Phenylpropylbromid in 4,5 l Toluol werden unter kräftigem Rühren bei Raumtemperatur portionsweise 741 g Kalium-tert.butylat eingetragen und dann 17 Stunden bei Raumtemperatur gerührt. Das Reaktionsgemisch wird mit 4 l Wasser versetzt und mit Zitronensäure angesäuert. Die wäßrige Phase wird abgetrennt, mit 1 l Toluol extrahiert und die vereinigten Toluolphasen werden dreimal mit je 1,5 l Wasser gewaschen. Die organische Phase wird mit Natriumsulfat getrocknet, filtriert und in 2 gleiche Portionen aufgeteilt. Die Lösungen werden getrennt einrotiert und die Eindampfrückstände mit je 4 l heißem Cyclohexan versetzt. Unter Rühren wird abgekühlt und das Kristallisat jeweils abgesaugt. Die beiden Portionen werden mit Cyclohexan nachgewaschen und getrocknet.

Ausbeute: insgesamt 2532 g (88,7 % der Theorie),
Schmelzpunkt: 103°C
$R_f$-Wert: 0,26 (Kieselgel; Methylenchlorid/Methanol = 50:1)

Beispiel IV

(3R,5S)-3-Allyl-1-(3-phenylpropyl)-5-[(trityloxy)methyl]-2-pyrrolidinon

225 ml Diisopropylamin werden in 250 ml Tetrahydrofuran gelöst und unter Kühlung in einem Trockeneis/Methanol-Bad portionsweise mit 1000 ml einer 1,6-molaren Lösung von n-Butyllithium in n-Hexan versetzt. Man kühlt auf -75°C ab und tropft eine Lösung von (S)-1-(3-Phenylpropyl)-5-[(trityloxy)methyl]-2-pyrrolidinon in 2000 ml Tetrahydrofuran so zu, daß die Temperatur zwischen -75 und -65°C bleibt. Man rührt noch 3 Stunden bei -75°C nach, fügt anschließend innerhalb von einer Stunde eine Lösung von 134 ml Allylbromid in 150 ml Tetrahydrofuran zu, wobei die Temperatur zwischen -75 und -65°C gehalten wird, und rührt noch eine Stunde bei -75°C nach. Man läßt auf Raumtemperatur kommen und gießt die Reaktionslösung auf 2000 ml halbgesättigte Kochsalzlösung. Man extrahiert mit Essigester, wäscht die organischen Phasen mit Wasser, trocknet mit Natriumsulfat und destilliert das Lösungsmittel im Vakuum ab. Das zurückbleibende Rohprodukt wird ohne weitere Reinigung weiter umgesetzt, dieses enthält noch wenige Prozent des (3S,5S)-Isomeren [$R_f$-Wert: 0,55 (Kieselgel; Cyclohexan, Essigester = 2:1)]

Ausbeute: 788 g (100 % der Theorie),
$R_f$-Wert: 0,63 (Kieselgel; Cyclohexan/Essigester = 2:1)

Analog werden hergestellt:

(1) (3R,5S)-3-Allyl-1-phenyl-5-[(trityloxy)methyl]-2-pyrrolidinon

$R_f$-Wert: 0,53 (Kieselgel; Cyclohexan/Essigester = 1:1)

| Ber.: | 83,69 | H 6,60 | N 2,96 |
|---|---|---|---|
| Gef.: | 83,40 | 7,08 | 2,83 |

(2) (3R,5S)-3-Allyl-1-(3-cyclohexylpropyl)-5-[(trityloxy)methyl]-2-pyrrolidinon

$R_f$-Wert: 0,50 (Kieselgel; Cyclohexan/Essigester = 8:2)

| Ber.: | 82,88 | H 8,31 | N 2,68 |
|---|---|---|---|
| Gef.: | 82,68 | 8,56 | 2,46 |

(3) (3R,5S)-3-Allyl-1-(4-methoxybenzyl)-5-[(trityloxy)methyl]-2-pyrrolidinon

Schmelzpunkt: 130-132°C
$R_f$-Wert: 0,38 (Kieselgel; Cyclohexan/Essigester = 8:2)

| Ber.: | 81,21 | H 6,81 | N 2,71 |
|---|---|---|---|
| Gef.: | 81,16 | 6,85 | 2,53 |

(4) (3S,5R)-3-Allyl-1-(3-phenylpropyl)-5-[(trityloxy)methyl]-2-pyrrolidinon

$R_f$-Wert: 0,60 (Kieselgel; Cyclohexan/Essigester = 2:1)

(5) (3R,S;5S,R)-3-Allyl-5-(4-methoxyphenyl)-1-(3-phenylpropyl)-2-pyrrolidinon

$R_f$-Wert: 0,23 (Kieselgel; Cyclohexan/Essigester = 2:1)

| Ber.: | 79,05 | H 7,79 | N 4,01 |
|-------|-------|--------|--------|
| Gef.: | 78,82 | 7,60 | 4,18 |

(6) (3R,5S)-3-Allyl-1-(4-phenylbutyl)-5-[(trityloxy)methyl]-2-pyrrolidinon

$R_f$-Wert: 0,53 (Kieselgel; Toluol/Aceton = 4:1)

(7) (3R,5S)-3-Allyl-1-isobutyl-5-[(trityloxy)methyl]-2-pyrrolidinon

Schmelzpunkt: 75-77°C
$R_f$-Wert: 0,25 (Kieselgel; Petrolether/tert.Butyl-methylether = 2:1)

(8) (6R,8S)-6-Allyl-3-cyclohexyl-perhydropyrrolo[1,2-c]oxazol-5-on

$R_f$-Wert: 0,43 (Kieselgel; Cyclohexan/Essigester = 2:1)
Nebenprodukt: (8S)-3-Cyclohexyl-6,6-diallyl-perhydropyrrolo-[1,2-c]oxazol-5-on
$R_f$-Wert: 0,52 (Kieselgel; Cyclohexan/Essigester = 4:1)

(9) (6R,8S)-6-Allyl-3-tert.butyl-perhydropyrrolo[1,2-c]oxazol-5-on

$R_f$-Wert: 0,29 (Kieselgel; Cyclohexan/Essigester = 4:1)

(10) (3R,5S)-3-Allyl-3-methyl-1-(3-phenylpropyl)-5-[(trityloxy)methyl]-2-pyrrolidinon

$R_f$-Wert: 0,63 (Kieselgel; Cyclohexan/Essigester = 2:1)

(11) (3R,5S)-3-Methyl-1-(3-phenylpropyl)-5-[(trityloxy)methyl]-2-pyrrolidinon

Alkylierungsmittel: Methyljodid.
$R_f$-Wert: 0,59 (Kieselgel; Cyclohexan/Essigester = 2:1)

(12) (3R,5S)-3-Allyl-1-benzyl-5-[(trityloxy)methyl]-2-pyrrolidinon

Schmelzpunkt: 113-116°C

(13) (3R,5S)-3-Allyl-1-(2-phenylethyl)-5-[(trityloxy)methyl]-2-yrrolidinon

$R_f$-Wert: 0,47 (Kieselgel; Toluol/Aceton = 8:1)

(14) (3R,5S)-3-Allyl-1-(4-phenylbutyl)-5-[(trityloxy)methyl]-2-pyrrolidinon

$R_f$-Wert: 0,53 (Kieselgel; Toluol/Aceton = 4:1)

(15) (3R,5S)-3-Allyl-1-(4-phenyloxybutyl)-5-[(trityloxy)methyl]-2-pyrrolidinon

$R_f$-Wert: 0,38 (Kieselgel; Toluol/Aceton = 8:1)

(16) (3R,5S)-3-Allyl-1,3-bis-(3-phenylpropyl)-5-[(trityloxy)methyl]-2-pyrrolidinon

$R_f$-Wert: 0,66 (Kieselgel; Cyclohexan/Essigester = 2:1)

(17) (3R,5S)-1,3-Bis-(3-phenylpropyl)-5-[(trityloxy)methyl]-2-pyrrolidinon

Alkylierungsmittel: 3-Phenylpropylbromid
$R_f$-Wert: 0,66 (Kieselgel; Cyclohexan/Essigester = 2:1)

(18) (3R,5S)-3-Allyl-3-(n-butyl)-1-(3-phenylpropyl)-5-[(trityloxy)methyl]-2-pyrrolidinon

$R_f$-Wert: 0,70 (Kieselgel; Cyclohexan/Essigester = 2:1)

(19) (3R,5S)-3-(n-Butyl)-1-(3-phenylpropyl)-5-[(trityloxy)methyl]-2-pyrrolidinon

Alkylierungsmittel: n-Butyljodid
$R_f$-Wert: 0,69 (Kieselgel; Cyclohexan/Essigester = 2:1)

(20) (3R,5S)-3-Allyl-1-[3-(4-benzyloxyphenyl)propyl]-5-[(trityloxy)methyl]-2-pyrrolidinon

$R_f$-Wert: 0,63 (Kieselgel; Toluol/Aceton = 4:1)

(21) (3R,S;5R,S)-3-Allyl-3-methyl-1-(3-phenylpropyl)-5-[(trityloxy)methyl]-2-pyrrolidinon

Alkylierungsmittel: Methyljodid
$R_f$-Wert: 0,72 (Kieselgel; Methylenchlorid/Ethanol = 15:1)

(22) (3R,S;5S,R)-3-Allyl-1-(3-phenylpropyl)-5-[(trityloxy)methyl]-2-pyrrolidinon

$R_f$-Wert: 0,36 (Kieselgel; Cyclohexan/Essigester = 5:1)

(23) (3R,5S)-3-(1-Buten-4-yl)-1-(3-phenylpropyl)-5-[(trityloxy)methyl]-2-pyrrolidinon

$R_f$-Wert: 0,60 (Kieselgel; Methylenchlorid/Methanol = 19:1)

(24) (3R,S;4R,S)-3-Allyl-1-(3-phenylpropyl)-4-[(trityloxy)methyl]-2-pyrrolidinon

$R_f$-Wert: 0,87 (Kieselgel; Methylenchlorid/Methanol = 30:1)

(25) (6S,8S)-6-Allyl-3-tert.butyl-perhydropyrrolo[1.2-c]oxazol-5-on (Chromatographisch abgetrenntes Nebenprodukt von Beispiel IV (11))

$R_f$-Wert: 0,48 (Kieselgel; Cyclohexan/Essigester = 4:1)

(26) (6R,8S)-6-Allyl-3-cyclohexyl-6-methyl-perhydropyrrolo-[1.2-c]oxazol-5-on

$R_f$-Wert: 0,53 (Kieselgel; Cyclohexan/Essigester = 7:3)

| Ber.: | C 72,96 | H 9,57 | N 5,32 |
|-------|---------|--------|--------|
| Gef.: | 73,10 | 9,74 | 5,50 |

(27) (6R,S;8S)-3-Cyclohexyl-6-methyl-perhydropyrrolo[1.2-c]oxazol-5-on

$R_f$-Wert: 0,33 und 0,46 (Kieselgel; Cyclohexan/Essigester = 7:3)

(28) (3R,5S)-3-Allyl-1-[2-(benzyloxy)ethyl]-5-[(trityloxy)methyl]-2-pyrrolidinon

$R_f$-Wert: 0,50 (Kieselgel; Cyclohexan/Essigester = 7:3)

| Ber.: | C 81,30 | H 7,00 | N 2,60 |
|-------|---------|--------|--------|
| Gef.: | 81,15 | 7,20 | 2,49 |

(29) (6S,8R)-6-Allyl-3-cyclohexyl-perhydropyrrolo[1,2-c]oxazol-5-on

$R_f$-Wert: 0,29 (Kieselgel; Cyclohexan/Essigester = 4:1)

| Ber.: | C 72,25 | H 9,30 | N 5,62 |
|-------|---------|--------|--------|
| Gef.: | 71,96 | 9,46 | 5,44 |

(30) (3S,5S)-1-(Benzyloxycarbonyl)-3-[(tert.butyloxycarbonyl)methyl]-5-[(trityloxy)methyl]-2-pyrrolidinon
Als Base wurde Lithiumhexamethyldisilazid und als Alkylierungsmittel Bromessigsäure-tert.butylester verwendet.

$R_f$-Wert: 0,66 (Kieselgel; Cyclohexan/Essigester = 2:1)

| Ber.: | C 75,35 | H 6,49 | N 2,31 |
|-------|---------|--------|--------|
| Gef.: | 75,17 | 6,65 | 2,50 |

(31) (3R,5S)-3-(3-Cyanopropyl)-1-methyl-3-[(trityloxy)methyl]-2-pyrrolidinon

Alkylierungsmittel: 4-Jodbuttersäure-nitril
$R_f$-Wert: 0,66 (Kieselgel; Essigester)

(32) (3R,5S)-3-(3-Cyanopropyl)-1-isobutyl-5-[(trityloxy)methyl]-2-pyrrolidinon

Alkylierungsmittel: 4-Jodbuttersäure-nitril
$R_f$-Wert: 0,50 (Kieselgel; Toluol/Aceton = 4:1)

(33) (3R,5S)-3-(1-Penten-5-yl)-1-(3-phenylpropyl)-5-[(trityloxy)methyl]-2-pyrrolidinon

Alkylierungsmittel: 5-Brom-1-penten
$R_f$-Wert: 0,29 (Kieselgel; Ether/Hexan = 1:1)

(34) (3R,S;4R,S)-3-Allyl-1-(4-methoxybenzyl)-4-[(trityloxy)methyl]-2-pyrrolidinon

$R_f$-Wert: 0,22 (Kieselgel; Cyclohexan/Essigester = 4:1)

(35) (3S,5S)-1-(Benzyloxycarbonyl)-3-[(methoxycarbonyl)methyl]-5-[(trityloxy)methyl]-2-pyrrolidinon
Verwendung von Lithiumhexamethyldisilazid als Base und Bromessigsäuremethylester als Alkylierungsmittel

$R_f$-Wert: 0,54 (Kieselgel; Cyclohexan/Essigester = 2:1)

| Ber.: | C 74,58 | H 5,90 | N 2,49 |
|-------|---------|--------|--------|
| Gef.: | 74,61 | 6,09 | 2,43 |

(36) (3R,S;4R,S)-3-Allyl-1-(4-methoxybenzyl)-3-methyl-4-[(trityloxy)methyl]-2-pyrrolidinon

$R_f$-Wert: 0,41 (Kieselgel; Cyclohexan/Essigester = 3:1)

(37) (3R,S;4R,S)-1-(4-Methoxybenzyl)-3-methyl-4-[(trityloxy)methyl]-2-pyrrolidinon
Verwendung von 1,3-Dimethyl-3,4,5,6-tetrahydro-2(1H)-pyrimidon als Kosolvens.

$R_f$-Wert: 0,36 (Kieselgel; Cyclohexan/Essigester = 2:1)

Beispiel V

(3R,5S)-3-Allyl-5-hydroxymethyl-1-(3-phenylpropyl)-2-pyrrolidinon

Eine Lösung von 690 g (3R,5S)-3-Allyl-1-(3-phenylpropyl)5-[(trityloxy)methyl]-2-pyrrolidinon in 2000 ml Methanol wird mit 650 ml 5N Salzsäure versetzt, 3 Stunden bei 40°C und 2 Tage bei Raumtemperatur gerührt. Man löst den entstandenen Niederschlag durch Erwärmen auf 50°C wieder auf und rührt nochmals 16 Stunden bei Raumtemperatur. Man filtriert vom gebildeten Niederschlag ab und engt das Filtrat bis zur beginnenden Kristallisation ein (auf etwa 1500 ml). Nach Abfiltrieren des Niederschlags wird das Filtrat eingeengt, der Rückstand mit Methylenchlorid aufgenommen, die organisches Phase mit Wasser gewaschen und eingeengt.

Ausbeute: 365 g (100 % der Theorie),
$R_f$-Wert: 0,12 (Kieselgel; Methylenchlorid/Methanol = 30:1)

Analog werden erhalten:

(1) (3R,5S)-3-Allyl-1-(3-cyclohexylpropyl)-5-hydroxymethyl-2-pyrrolidinon

$R_f$-Wert: 0,20 (Kieselgel; Cyclohexan/Essigester = 1:1)

(2) (3R,5S)-3-Allyl-1-(4-methoxybenzyl)-5-hydroxymethyl-2-pyrrolidinon

$R_f$-Wert: 0,33 (Kieselgel; Cyclohexan/Essigester = 1:2)

(3) (3S,5R)-3-Allyl-5-hydroxymethyl-1-(3-phenylpropyl)-2-pyrrolidinon

$R_f$-Wert: 0,43 (Kieselgel; Methylenchlorid/Methanol = 15:1)

| Ber.: | C 74,69 | H 8,48 | N 5,12 |
|-------|---------|--------|--------|
| Gef.: | 74,85   | 8,56   | 5,00   |

(4) (3R,5S)-3-Allyl-5-hydroxymethyl-1-(4-phenylbutyl)-2-pyrrolidinon

$R_f$-Wert: 0,18 (Kieselgel; Toluol/Aceton = 4:1)

(5) (3R,5S)-3-Allyl-5-hydroxymethyl-1-isobutyl-2-pyrrolidinon

$R_f$-Wert: 0,26 (Kieselgel; Essigester)

(6) (3R,5S)-3-Allyl-5-hydroxymethyl-3-methyl-1-(3-phenylpropyl)-2-pyrrolidinon

$R_f$-Wert: 0,16 (Kieselgel; Cyclohexan/Essigester = 2:1)

(7) (3R,5S)-3-Allyl-1-benzyl-5-hydroxymethyl-2-pyrrolidinon

$R_f$-Wert: 0,63 (Kieselgel; Methylenchlorid/Methanol = 10:1)

(8) (3R,5S)-3-Allyl-5-hydroxymethyl-1-(2-phenylethyl)-2-pyrrolidinon

$R_f$-Wert: 0,49 (Kieselgel; Methylenchlorid/Methanol = 10:1)

(9) (3R,5S)-3-Allyl-5-hydroxymethyl-1-(4-phenylbutyl)-2-pyrrolidinon

$R_f$-Wert: 0,28 (Kieselgel; Toluol/Aceton = 4:1)

(10) (3R,5S)-3-Allyl-5-hydroxymethyl-1-(4-phenyloxybutyl)-2-pyrrolidinon

$R_f$-Wert: 0,36 (Kieselgel; Methylenchlorid/Methanol = 10:1)

(11) (3R,5S)-3-Allyl-5-hydroxymethyl-1,3-bis(3-phenylpropyl)-2-pyrrolidinon

$R_f$-Wert: 0,28 (Kieselgel; Cyclohexan/Essigester = 2:1)

(12) (3R,5S)-3-Allyl-3-(n-butyl)-5-hydroxymethyl-1-(3-phenylpropyl)-2-pyrrolidinon

$R_f$-Wert: 0,25 (Kieselgel; Cyclohexan/Essigester = 2:1)

(13) (3R,5S)-3-Allyl-1-[3-(4-benzyloxyphenyl)propyl]-5-hydroxymethyl-2-pyrrolidinon

$R_f$-Wert: 0,13 (Kieselgel; Toluol/Aceton = 4:1)

(14) (3R,S;5R,S)-3-Allyl-5-hydroxymethyl-3-methyl-1-(3-phenylpropyl)-2-pyrrolidinon

$R_f$-Wert: 0,39 (Kieselgel; Methylenchlorid/Ethanol = 15:1)

(15) (3R,5S)-3-(1-Buten-4-yl)-5-hydroxymethyl-1-(3-phenylpropyl)-2-pyrrolidinon

$R_f$-Wert: 0,70 (Kieselgel; Methylenchlorid/Methanol = 9:1)

(16) (3R,S;4R,S)-3-Allyl-4-hydroxymethyl-1-(3-phenylpropyl)-2-yrrolidinon

$R_f$-Wert: 0,47 (Kieselgel; Methylenchlorid/Methanol = 9:1)

(17) (3R,5S)-3-Allyl-1-[2-(benzyloxy)ethyl]-5-hydroxymethyl-2-pyrrolidinon

$R_f$-Wert: 0,47 (Kieselgel; Essigester)

| Ber. x 0,5 $H_2O$: | C 68,43 | H 8,11 | N 4,69 |
|---|---|---|---|
| Gef.: | 68,59 | 8,14 | 4,41 |

(18) (3R,S;4R,S)-3-Allyl-4-hydroxymethyl-1-(4-methoxybenzyl)-2-pyrrolidinon

$R_f$-Wert: 0,31 (Kieselgel; Cyclohexan/Essigester = 1:2)

| Ber.: | C 69,80 | H 7,69 | N 5,09 |
|---|---|---|---|
| Gef.: | 69,84 | 7,84 | 4,96 |

(19) (3R,S;4R,S)-3-Allyl-4-hydroxymethyl-1-(4-methoxybenzyl)-3-methyl-2-pyrrolidinon

$R_f$-Wert: 0,44 (Kieselgel; Cyclohexan/Essigester = 1:2)

| Ber.: | C 70,56 | H 8,01 | N 4,84 |
|---|---|---|---|
| Gef.: | 70,40 | 8,07 | 4,82 |

(20) (3R,5S)-3-(3-Cyanopropyl)-5-hydroxymethyl-1-methyl-2-pyrrolidinon

$R_f$-Wert: 0,33 (Kieselgel; Essigester/Methanol = 9:1)

(21) (3R,5S)-3-(3-Cyanopropyl)-5-hydroxymethyl-1-isobutyl-2-pyrrolidinon

Schmelzpunkt: 46-48°C,
$R_f$-Wert: 0,45 (Kieselgel; Essigester/Methanol = 9:1)

Beispiel VI

(3R,5S)-3-Allyl-5-[(methansulfonyloxy)methyl]-1-(3-phenylpropyl)-2-pyrrolidinon

Eine Lösung von 54,7 g (3R,5S)-3-Allyl-5-hydroxymethyl-1-(3-phenylpropyl)-2-pyrrolidinon in 500 ml Methylenchlorid wird auf -10°C gekühlt und unter Rühren zunächst 16 ml Methansulfonylchlorid und anschließend eine Lösung von 29,4 ml Triethylamin in 50 ml Methylenchlorid zugesetzt, wobei die Temperatur auf -10°C gehalten wird. Man läßt auf Raumtemperatur kommen und eine Stunde nachreagieren. Die erhaltene Lösung wird nacheinander mit Wasser, 0,5 N wäßrigem Ammoniak und Wasser gewaschen und eingedampft.

Ausbeute: 70 g (100 % der Theorie),
$R_f$-Wert: 0,61 (Kieselgel; Methylenchlorid/Methanol = 30:1) (nach zweifacher Entwicklung)

Analog werden erhalten:

(1) (3S,5S)-5-[(Methansulfonyloxy)methyl]-3-[(methoxycarbonyl)methyl]-1-phenyl-2-pyrrolidinon

$R_f$-Wert: 0,63 (Kieselgel; Chloroform/Methanol = 95:5)

(2) (3R,5S)-3-Allyl-1-(3-cyclohexylpropyl)-5-[(methansulfonyloxy)methyl]-2-pyrrolidinon

$R_f$-Wert: 0,26 (Kieselgel; Cyclohexan/Essigester = 6:4)

| Ber.: | C 60,47 | H 8,74 | N 3,92 | S 8,97 |
|-------|---------|--------|--------|--------|
| Gef.: | 60,30 | 8,79 | 3,79 | 8,92 |

(3) (3R,5S)-3-Allyl-1-(4-methoxybenzyl)-5-[(methansulfonyloxy)methyl]-2-pyrrolidinon

$R_f$-Wert: 0,38 (Kieselgel; Cyclohexan/Essigester = 1:1)

| Ber.: | C 57,77 | H 6,56 | N 3,96 | S 9,07 |
|-------|---------|--------|--------|--------|
| Gef.: | 57,89 | 6,76 | 3,75 | 8,93 |

(4) (3S,5R)-3-Allyl-5-[(4-methansulfonyloxy)methyl]-1-(3-phenylpropyl)-2-pyrrolidinon

$R_f$-Wert: 0,63 (Kieselgel; Cyclohexan/Essigester = 1:2)

| Ber.: | C 61,51 | H 7,17 | N 3,99 | S 9,12 |
|-------|---------|--------|--------|--------|
| Gef.: | 61,30 | 7,37 | 3,70 | 8,88 |

(5) (3R,5S)-3-Allyl-5-[(methansulfonyloxy)methyl]-1-(4-phenylbutyl)-2-pyrrolidinon

$R_f$-Wert: 0,38 (Kieselgel; Toluol/Aceton = 4:1)

(6) (3R,5S)-3-Allyl-1-isobutyl-5-[(methansulfonyloxy)methyl]-2-pyrrolidinon

$R_f$-Wert: 0,69 (Kieselgel; Essigester)

(7) (3R,5S)-3-Allyl-5-[(methansulfonyloxy)methyl]-2-pyrrolidinon

Schmelzpunkt: 62-63°C
$R_f$-Wert: 0,40 (Kieselgel; Essigester)

(8) (3R,5S)-3-Allyl-5-[(methansulfonyloxy)methyl]-3-methyl-1-(3-phenylpropyl)-2-pyrrolidinon

$R_f$-Wert: 0,50 (Kieselgel; Methylenchlorid/Methanol = 15:1)

(9) (3R,5S)-3-Allyl-1-benzyl-5-[(methansulfonyloxy)methyl]-2-pyrrolidinon

$R_f$-Wert: 0,75 (Kieselgel; Essigester)

(10) (3R,5S)-3-Allyl-5-[(methansulfonyloxy)methyl]-1-(2-phenylethyl)-2-pyrrolidinon

$R_f$-Wert: 0,73 (Kieselgel; Essigester)

(11) (3R,5S)-3-Allyl-5-[(methansulfonyloxy)methyl]-1-(4-phenylbutyl)-2-pyrrolidinon

$R_f$-Wert: 0,38 (Kieselgel; Toluol/Aceton = 4:1)

(12) (3R,5S)-3-Allyl-5-[(methansulfonyloxy)methyl]-1-(4-phenyloxybutyl)-2-pyrrolidinon

$R_f$-Wert: 0,64 (Kieselgel; Essigester)

(13) (3R,5S)-3-Allyl-5-[(methansulfonyloxy)methyl]-1,3-bis(3-phenylpropyl)-2-pyrrolidinon

$R_f$-Wert: 0,53 (Kieselgel; Methylenchlorid/Methanol = 40:1)

(14) (3R,5S)-3-Allyl-3-(n-butyl)-5-[(methansulfonyloxy)methyl]-1-(3-phenylpropyl)-2-pyrrolidinon

$R_f$-Wert: 0,53 (Kieselgel; Methylenchlorid/Methanol = 40:1)

(15) (3R,5S)-3-Allyl-1-[3-(4-benzyloxyphenyl)propyl]-5-[(methansulfonyloxy)methyl]-2-pyrrolidinon

$R_f$-Wert: 0,47 (Kieselgel; Toluol/Aceton = 2:1)

(16) (3S,5S)-5-[(Methansulfonyloxy)methyl]-3-[(methoxycarbonyl)methyl]-1-(3-phenylpropyl)-2-pyrrolidinon

$R_f$-Wert: 0,36 (Kieselgel; Methylenchlorid/Ethanol = 15:1)

(17) (3R,S;5R,S)-3-Allyl-5-[(methansulfonyloxy)methyl]-3-methyl-1-(3-phenylpropyl)-2-pyrrolidinon
Als Base wird Pyridin verwendet

$R_f$-Wert: 0,58 (Kieselgel; Methylenchlorid/Ethanol = 15:1)

(18) 1-Benzyloxy-3-[3-(methansulfonyloxy)propyl]benzol Durchführung in Pyridin.

$R_f$-Wert: 0,92 (Kieselgel; Methylenchlorid)

(19) 1-Benzyloxy-4-[3-(methansulfonyloxy)propyl]benzol Durchführung in Pyridin.

$R_f$-Wert: 0,75 (Kieselgel; Methylenchlorid)

(20) 4-[4-(Methansulfonyloxy)butyl]anisol
Als Lösungsmittel wird Tetrahydrofuran und als Base Pyridin verwendet

$R_f$-Wert: 0,81 (Kieselgel; Methylenchlorid)

(21) (3R,5S)-3-Allyl-1-(tert.butyloxycarbonyl)-5-[(methansulfonyloxy)methyl]-pyrrolidin

$R_f$-Wert: 0,65 (Kieselgel; Cyclohexan/Essigester = 1:1)

(22) (3S,5S)-3-Allyl-5-[(methansulfonyloxy)methyl]-2-pyrrolidinon

Schmelzpunkt: 74-76°C,
$R_f$-Wert: 0,32 (Kieselgel; Essigester)

(23) (3R,5S)-3-Allyl-5-[(methansulfonyloxy)methyl]-3-methyl-2-pyrrolidinon

$R_f$-Wert: 0,46 (Kieselgel; Essigester)

(24) (3R,5S)-3-Allyl-1-[2-(benzyloxy)ethyl]-5-[(methansulfonyloxy)methyl]-2-pyrrolidinon

$R_f$-Wert: 0,59 (Kieselgel; Chloroform/Methanol = 95:5)

(25) (3S,5R)-3-Allyl-5-[(methansulfonyloxy)methyl]-2-pyrrolidinon

$R_f$-Wert: 0,38 (Kieselgel; Essigester)

(26) (3S,5S)-3-[(tert.Butyloxycarbonyl)methyl]-5-[(methansulfonyloxy)methyl]-2-pyrrolidinon

Schmelzpunkt: 114-116°C,
$R_f$-Wert: 0,47 (Kieselgel; Toluol/Aceton = 1:3)

| Ber.: | C 46,89 | H 6,89 | N 4,56 | S 10,43 |
|-------|---------|--------|--------|---------|
| Gef.: | 46,90 | 6,79 | 4,84 | 10,17 |

(27) (3R,5S)-3-(3-Cyanopropyl)-5-[(methansulfonyloxy)methyl]-1-methyl-2-pyrrolidinon

$R_f$-Wert: 0,29 (Kieselgel; Essigester)

(28) (3R,5S)-3-(3-Cyanopropyl)-1-isobutyl-5-[(methansulfonyloxy)methyl]-2-pyrrolidinon

$R_f$-Wert: 0,63 (Kieselgel; Essigester/Methanol = 9:1)

(29) (3S,5S)-5-[2-(Methansulfonyloxy)ethyl]-3-[(methoxycarbonyl)methyl]-1-(3-phenylpropyl)-2-pyrrolidinon

$R_f$-Wert: 0,68 (Kieselgel; Methylenchlorid/Methanol = 19:1) (nach zweifacher Entwicklung)

(30) (3R,5S)-5-[(Methansulfonyloxy)methyl]-3-[3-(methoxycarbonyl)propyl]-1-(3-phenylpropyl)-2-pyrrolidinon

$R_f$-Wert: 0,36 (Kieselgel; Methylenchlorid/Methanol = 19:1)

(31) (S)-3,3-Diallyl-5-[(methansulfonyloxy)methyl]-2-pyrrolidinon

$R_f$-Wert: 0,56 (Kieselgel; Essigester)

(32) (3R,S;4R,S)-3-Allyl-4-[(methansulfonyloxy)methyl]-1-(4-methoxybenzyl)-2-pyrrolidinon

R$_f$-Wert: 0,38 (Kieselgel; Cyclohexan/Essigester = 1:2)

| Ber.: | C 57,77 | H 5,56 | N 3,96 | S 9,07 |
|---|---|---|---|---|
| Gef.: | 57,60 | 6,40 | 4,02 | 9,31 |

(33) (3S,5S)-5-[(Methansulfonyloxy)methyl]-3-[(methoxycarbonyl)methyl]-2-pyrrolidinon

Schmelzpunkt: 85-87°C
R$_f$-Wert: 0,42 (Kieselgel; Aceton/Petrolether = 4:1)

| Ber.: | C 40,75 | H 5,70 | N 5,28 | S 12,09 |
|---|---|---|---|---|
| Gef.: | 40,63 | 5,50 | 5,45 | 12,01 |

(34) (3R,S;4R,S)-3-Allyl-4-[(methansulfonyloxy)methyl]-1-(4-methoxybenzyl)-3-methyl-2-pyrrolidinon

R$_f$-Wert: 0,53 (Kieselgel; Cyclohexan/Essigester = 1:2)

| Ber.: | C 58,83 | H 6,86 | N 3,81 | S 8,73 |
|---|---|---|---|---|
| Gef.: | 58,52 | 6,70 | 3,71 | 8,70 |

(35) 1-(4'-Cyano-4-biphenylyl)-4-[(methansulfonyloxy)methyl]-2-pyrrolidinon

Schmelzpunkt: 183-186°C
R$_f$-Wert: 0,47 (Kieselgel; Essigester)

| Ber.: | C 61,61 | H 4,90 | N 7,56 | S 8,65 |
|---|---|---|---|---|
| Gef.: | 61,43 | 4,90 | 7,47 | 8,62 |

Beispiel VII

(3R,5S)-3-Allyl-5-[(4-nitrophenyl)oxymethyl]-1-(3-phenylpropyl)-2-pyrrolidinon

Hergestellt analog Beispiel CVIII aus (3R,5S)-3-Allyl-5-[(methansulfonyloxy)methyl]-1-(3-phenylpropyl)-2-pyrrolidinon und 4-Nitrophenol.

R$_f$-Wert: 0,50 (Kieselgel; Cyclohexan/Essigester = 2:1)

| Ber.: | C 70,03 | H 6,64 | N 7,10 |
|---|---|---|---|
| Gef.: | 69,88 | 6,72 | 7,04 |

Beispiel VIII

(3S,5S)-5-[(4'-Aminocarbonyl-3'-chlor-4-biphenylyl)oxymethyl]-3-[(methoxycarbonyl)methyl]-1-(3-phenylpropyl)-2-pyrrolidinon

Hergestellt analog Beispiel CIX aus (3S,5S)-5-[(Methansulfonyloxy)methyl]-3-[(methoxycarbonyl)methyl]-1-(3-phenylpropyl)-2-pyrrolidinon und 4'-Aminocarbonyl-3'-chlor-4-hydroxybiphenyl.

$R_f$-Wert: 0,40 (Kieselgel; Essigester)

| Ber.: | C 67,34 | H 5,84 | N 5,24 | Cl 6,63 |
|---|---|---|---|---|
| Gef.: | 67,25 | 5,92 | 5,23 | 6,52 |

Beispiel IX

(3S,5S)-3-[(Methoxycarbonyl)methyl]-5-[(4-nitrophenyl)oxymethyl]-1-(3-phenylpropyl)-2-pyrrolidinon

Hergestellt analog Beispiel CXI durch Oxidation von (3R,5S)-3-Allyl-5-[(4-nitrophenyl)oxymethyl]-1-(3-phenylpropyl)-2-pyrrolidinon und nachfolgende Veresterung mit Methanol.

$R_f$-Wert: 0,40 (Kieselgel; Cyclohexan/Essigester = 1:1)

| Ber.: | C 64,78 | H 6,15 | N 6,57 |
|---|---|---|---|
| Gef.: | 64,95 | 6,31 | 6,51 |

Zwischenprodukt:

(3S,5S)-3-Carboxymethyl-5-[(4-nitrophenyl)oxymethyl]-1-(3-phenylpropyl)-2-pyrrolidinon

$R_f$-Wert: 0,90 (Kieselgel; Methylenchlorid/Methanol = 10:1)

| Ber.: | C 64,07 | H 5,87 | N 6,79 |
|---|---|---|---|
| Gef.: | 63,88 | 5,91 | 6,63 |

Beispiel X

(S)-1-Phenyl-5-[(trityloxy)methyl]-2-pyrrolidinon

Ein 500 ml Zweihalskolben wird mit einem Magnetrührer, einem Soxhlet-Aufsatz, der eine Extraktionshülse mit 10 g Kaliumcarbonat und 20 g Kieselgel enthält, und einem Rückflußkühler versehen. In dieser Apparatur werden 17 g (S)-5-[(Trityloxy)methyl]-2-pyrrolidinon, 24,3 g Jodbenzol, 1,3 g Kupferpulver und 11,8 g Kaliumacetat mit 120 ml Dimethylformamid 7 Stunden unter Rückfluß erhitzt. Es wird abgekühlt, die Extraktionshülse ausgewechselt und dann weitere 1,5 Stunden unter Rückfluß gekocht. Nach dieser Zeit wird das Dimethylformamid im Wasserstrahlvakuum abrotiert und der Rückstand mit 400 ml Essigester und 400 ml Wasser versetzt und 30 Minuten gerührt. Danach wird abgesaugt und der Filterrückstand mit Essigester und Methanol gewaschen. Die organische Phase des Filtrats wird abgetrennt und mit Wasser und gesättigter Kochsalzlösung gewaschen, getrocknet und eingeengt. Der dunkle, ölige Rückstand wird über Kieselgel mit Cyclohexan/Essigester (1:1) chromatographiert. Die Produktfraktionen werden einrotiert, der Rückstand mit Diisopropylether verrührt und abgesaugt. Nach dem Trocknen verbleiben 10,5 g (51 % der Theorie) kristallines Produkt.

Schmelzpunkt: 144-146°C,
$R_f$-Wert: 0,24 (Kieselgel; Cyclohexan/Essigester = 1:1)

| Ber.: | C 83,11 | H 6,28 | N 3,23 |
|---|---|---|---|
| Gef.: | 83,06 | 6,46 | 3,28 |

Beispiel XI

(3S,5S)-3-Carboxymethyl-1-phenyl-5-[(trityloxy)methyl]-2-pyrrolidinon

8 g (3R,5S)-3-Allyl-1-phenyl-5-[(trityloxy)methyl]-2-pyrrolidinon werden in 40 ml Methylenchlorid und 40 ml Acetonitril gelöst und die Lösung auf 5°C abgekühlt. Nach Zugabe von 0,45 g Ruthenium(III)chlorid-trihydrat werden 19,8 g Natriummetaperjodat in 160 ml Wasser so zugegeben, daß die Temperatur unter 10°C bleibt. Nach 2 Stunden wird mit 20 g Kieselgur verrührt und dann abgesaugt. Der Filterrückstand wird mit Wasser und Methylenchlorid ausgewaschen und die Phasen des Filtrats getrennt. Die wäßrige Phase wird zweimal mit Methylenchlorid extrahiert, die vereinigten organischen Phasen mit Wasser gewaschen, getrocknet und eingeengt. Es verbleiben 11,7 g Rohprodukt als dunkles zähes Harz.

$R_f$-Wert: 0,60 (Kieselgel; Essigester)
$R_f$-Wert: 0,62 (Kieselgel; Toluol/Dioxan/Ethanol/Eisessig = 90:10:10:6)

Analog werden erhalten:

(1) (3S,5S)-3-Carboxymethyl-1-(3-phenylpropyl)-5-(N-phthalimidomethyl)-2-pyrrolidinon

$R_f$-Wert: 0,51 (Kieselgel; Methylenchlorid/Methanol = 8:1)

(2) (3S,5S)-3-Carboxymethyl-1-(3-phenylpropyl)-5-[(trityloxy)methyl]-2-pyrrolidinon

$R_f$-Wert: 0,32 (Kieselgel; Methylenchlorid/Cyclohexan/Methanol/konz. wäßriges Ammoniak = 68:15:15:2)

(3) (3S,5S)-3-Carboxymethyl-1-(3-phenylpropyl)-5-[[[2(N-phthalimidomethyl)-5-indanyl]sulfonylamino]methyl]-2-pyrrolidinon

$R_f$-Wert: 0,15 (Kieselgel; Methylenchlorid/Cyclohexan/Methanol/konz. wäßriges Ammoniak = 68:15:15:2)

(4) (3S,5S)-3-Carboxymethyl-1-(3-phenylpropyl)-5-[[[2(N-phthalimido)-5-indanyl]sulfonylamino]methyl]-2-pyrrolidinon

$R_f$-Wert: 0,25 (Kieselgel; Methylenchlorid/Cyclohexan/Methanol/konz. wäßriges Ammoniak = 68:15:15:2)

(5) (3R,S;4R,S)-3-Carboxymethyl-1-(3-phenylpropyl)-4-(N-phthalimidomethyl)-2-pyrrolidinon

$R_f$-Wert: 0,48 (Kieselgel; Methylenchlorid/Methanol = 9:1)

(6) (3R,5S)-3-(3-Carboxypropyl)-1-(3-phenylpropyl)-5-[(trityloxy)methyl]-2-pyrrolidinon

$R_f$-Wert: 0,33 (Kieselgel; Methylenchlorid/Methanol = 19:1)

(7) (3S,5S)-5-Aminocarbonylmethyl-3-carboxymethyl-1-(3-phenylpropyl)-2-pyrrolidinon

$R_f$-Wert: 0,18 (Kieselgel; Methylenchlorid/Methanol/konz. wäßriges Ammoniak = 4:1:0,25)

(8) (3S,5S)-3-Carboxymethyl-5-cyanomethyl-1-(3-phenylpropyl)-2-pyrrolidinon

$R_f$-Wert: 0,30 (Kieselgel; Methylenchlorid/Methanol = 19:1)

Beispiel XII

(3S,5S)-5-Hydroxymethyl-3-[(methoxycarbonyl)methyl]-1-phenyl-2-pyrrolidinon

11,7 g (3S,5S)-3-Carboxymethyl-1-phenyl-5-[(trityloxy)methyl]-2-pyrrolidinon werden mit 50 ml Methanol versetzt. Unter Rühren wird 5 Minuten lang Chlorwasserstoff eingeleitet, wobei sich eine dunkle Lösung bildet. Nach weiteren 30 Minuten Rühren wird die Lösung eingeengt und das zurückbleibende Harz in der Hitze dreimal mit Essigester ausgezogen. Die Essigesterfiltrate werden eingeengt und der Rückstand mit Cyclohexan/Essigester (1:1), Cyclohexan/Essigester (4:6) und Essigester über Kieselgel chromatographiert. Es werden 1,2 g (25 % der Theorie) eines Öls erhalten, das langsam durchkristallisiert.

$R_f$-Wert: 0,16 (Kieselgel; Cyclohexan/Essigester = 1:1)

| Ber.: | C 63,86 | H 6,51 | N 5,32 |
|---|---|---|---|
| Gef.: | 63,60 | 6,56 | 5,06 |

Analog werden erhalten:

(1) (3S,5S)-5-Hydroxymethyl-3-[(methoxycarbonyl)methyl]-1-(3-phenylpropyl)-2-pyrrolidinon

$R_f$-Wert: 0,27 (Kieselgel; Methylenchlorid/Ethanol = 15:1) Reaktion in methanolischer Salzsäure/konz. wäßriger Salzsäure

(2) (3R,5S)-5-Hydroxymethyl-3-[3-(methoxycarbonyl)propyl]-1-(3-phenylpropyl)-2-pyrrolidinon

$R_f$-Wert: 0,45 (Kieselgel; Methylenchlorid/Methanol = 9:1)

Beispiel XIII

(3R,5S)-3-Allyl-1-(3-phenylpropyl)-5-(N-phthalimidomethyl)-2-pyrrolidinon

Zu 28,7 g Phthalimid, 50 g (3R,5S)-3-Allyl-5-hydroxymethyl-1-(3-phenylpropyl)-2-pyrrolidinon und 51,15 g Triphenylphosphin in 900 ml trockenem Tetrahydrofuran werden unter Eiskühlung langsam 34 g Azodicarbonsäurediethylester zugetropft. Danach wird 18 Stunden bei Raumtemperatur gerührt. Die Lösung wird eingeengt und der Rückstand in wenig Toluol aufgenommen. Das ungelöste Material wird abgesaugt, mit Toluol gewaschen und das Filtrat eingeengt. Der erhaltene Rückstand wird durch Chromatographie über Kieselgel mit Toluol/Aceton (4:1) gereinigt. Es werden 76 g (100 % der Theorie) des Produkts erhalten, dem noch etwas Toluol anhaftet.

$R_f$-Wert: 0,57 (Kieselgel; Toluol/Aceton = 4:1)

Analog werden erhalten:

(1) (3R,5S)-3-Allyl-1-(4-phenoxybutyl)-5-(N-phthalimidomethyl)-2-pyrrolidinon

$R_f$-Wert: 0,56 (Kieselgel; Toluol/Aceton = 4:1)

(2) (3R,5S)-3-Allyl-1-(2-phenylethyl)-5-(N-phthalimidomethyl)-2-pyrrolidinon

$R_f$-Wert: 0,87 (Kieselgel; Methylenchlorid/Methanol = 10:1)

(3) (3R,5S)-3-Allyl-1-benzyl-5-(N-phthalimidomethyl)-2-pyrrolidinon

$R_f$-Wert: 0,69 (Kieselgel; Toluol/Aceton = 2:1)

(4) (3R,5S)-3-Allyl-1-isobutyl-5-(N-phthalimidomethyl)-2-pyrrolidinon

Schmelzpunkt: 75-87°C
$R_f$-Wert: 0,78 (Kieselgel; tert.Butyl-methylether)

(5) (3R,5S)-3-(1-Buten-4-yl)-1-(3-phenylpropyl)-5-(N-phthalimidomethyl)-2-pyrrolidinon

$R_f$-Wert: 0,70 (Kieselgel; Methylenchlorid/Methanol = 9:1)

(6) (3R,S;4R,S)-3-Allyl-1-(3-phenylpropyl)-4-(N-phthalimidomethyl)-2-pyrrolidinon

$R_f$-Wert: 0,45 (Kieselgel; Methylenchlorid/Methanol = 30:1)

(7) (3R,S;4R,S)-3-Allyl-1-(4-methoxybenzyl)-4-(N-phthalimidomethyl)-2-pyrrolidinon

$R_f$-Wert: 0,63 (Kieselgel; Cyclohexan/Essigester = 1:2)

Beispiel XIV

(3S,5S)-3-[(Methoxycarbonyl)methyl]-5-[[(3'-nitro-3-biphenylyl)carbonyl]aminomethyl]-1-(3-phenylpropyl)-2-pyrrolidinon

Hergestellt analog Beispiel CXVII aus 3'-Nitrobiphenyl-3-carbonsäure, (3S,5S)-5-Aminomethyl-3-[(methoxycarbonyl)methyl]-1-(3-phenylpropyl)-2-pyrrolidinon-hydrochlorid und Triethylamin.

$R_f$-Wert: 0,47 (Kieselgel; Methylenchlorid/Essigester = 1:1)

| Ber.: | C 68,04 | H 5,90 | N 7,93 |
|---|---|---|---|
| Gef.: | 67,84 | 6,00 | 7,66 |

Beispiel XV

(3R,S;5S,R)-3-Allyl-5-(4-hydroxyphenyl)-1-(3-phenylpropyl)-2-pyrrolidinon

Zu einer auf -50°C abgekühlten Lösung von 39 g (3R,S;5S,R)-3-Allyl-5-(4-methoxyphenyl)-1-(3-phenylpropyl)-2-pyrrolidinon in 1 l trockenem 1,2-Dichlorethan wird unter kräftigem Rühren 62,6 g Bortribromid getropft. Nach 2 Stunden bei -50°C läßt man den Ansatz auf Raumtemperatur kommen und rührt weitere 3 Stunden. Die klare Lösung wird portionsweise in 1 l 50 % wäßriges Ethanol eingetragen und das Gemisch im Vakuum auf etwa 600 ml einrotiert. Das Konzentrat wird auf 1,5 kg Eis und die Mischung über Nacht stehengelassen. Der gebildete Niederschlag wird abgesaugt, in Ethylenchlorid aufgenommen und die Lösung mit Magnesiumsulfat getrocknet, filtriert und einrotiert. Das zurückbleibende Öl erstarrt beim Stehen über Nacht. Der Kristallkuchen wird mit Diethylether digeriert, abgesaugt und getrocknet.

Ausbeute: 35,6 g (95 % der Theorie),
Schmelzpunkt: 108-111°C
$R_f$-Wert: 0,18 (Kieselgel; Cyclohexan/Essigester = 2:1)

| Ber.: | C 78,77 | H 7,52 | N 4,18 |
|---|---|---|---|
| Gef.: | 78,55 | 7,31 | 4,32 |

Analog werden erhalten:

(1) 4'-Cyano-3-fluor-4-hydroxybiphenyl x 0,2 Wasser

Schmelzpunkt: 203-204°C
$R_f$-Wert: 0,50 (Kieselgel; Methylenchlorid)

| Ber.: | C 72,02 | H 3,90 | N 6,57 |
|---|---|---|---|
| Gef.: | 72,19 | 3,91 | 6,39 |

(2) 4'-Cyano-4-hydroxy-3-trifluormethylbiphenyl

Schmelzpunkt: 201-203°C
$R_f$-Wert: 0,67 (Kieselgel; Methylenchlorid/Essigester = 9:1)

(3) 4'-Aminocarbonyl-3'-chlor-4-hydroxybiphenyl

Schmelzpunkt: 215-216°C
$R_f$-Wert: 0,67 (Kieselgel; Methylenchlorid/Methanol = 9:1)

(4) 4'-Cyano-4-hydroxy-2'-methylbiphenyl

$R_f$-Wert: 0,12 (Kieselgel; Methylenchlorid/Petrolether = 1:1)

(5) 4'-Cyano-2,3-dimethyl-4-hydroxybiphenyl

Schmelzpunkt: 174-176°C
$R_f$-Wert: 0,72 (Kieselgel; Methylenchlorid)

(6) 6-Cyano-2-hydroxynaphthalin

Schmelzpunkt: 160-164°C
$R_f$-Wert: 0,40 (Kieselgel; Cyclohexan/Essigester = 2:1)

(7) 4-(4-Hydroxyphenyl)butyronitril

Schmelzpunkt: 52-54°C

(8) 2-Cyano-7-hydroxy-fluorenon

Schmelzpunkt: 275-277°C

(9) 4-(4-Cyanobenzoylamino)phenol

Schmelzpunkt: 190-192°C

| Ber.: | C 70,58 | H 4,53 | N 11,76 |
|---|---|---|---|
| Gef.: | 70,73 | 4,38 | 11,50 |

(10) 4-(4-Hydroxybenzoylamino)benzonitril

Schmelzpunkt: 228-231°C

(11) 4-[(4-Hydroxyphenyl)sulfonylamino]benzonitril

Schmelzpunkt: 230-233°C

| Ber.: | C 56,88 | H 3,67 | N 10,21 | S 11,69 |
|---|---|---|---|---|
| Gef.: | 56,88 | 3,75 | 10,03 | 11,87 |

(12) 3-Cyano-4'-hydroxy-diphenylsulfid

Schmelzpunkt: 120-124°C

(13) 3-Cyano-4'-hydroxy-benzophenon

Schmelzpunkt: 165-168°C

| Ber.: | C 75,33 | H 4,06 | N 6,28 |
|-------|---------|--------|--------|
| Gef.: | 75,25   | 4,04   | 6,42   |

(14) 4'-Cyano-4-hydroxy-3-methylsulfenyl-biphenyl

$R_f$-Wert: 0,39 (Kieselgel; Cyclohexan/Essigester = 2:1)

(15) 2-Cyano-7-hydroxy-9,10-dihydrophenanthren

$R_f$-Wert: 0,42 (Aluminiumoxid; Methylenchlorid/Methanol = 30:1)

(16) 4'-Cyano-3'-fluor-4-hydroxybiphenyl

Schmelzpunkt: 200-202°C
$R_f$-Wert: 0,45 (Kieselgel; Cyclohexan/Essigester = 7:3)

| Ber.: | C 73,23 | H 3,78 | N 6,57 |
|-------|---------|--------|--------|
| Gef.: | 72,97   | 3,83   | 6,54   |

Beispiel XVI

(8S)-3-Cyclohexyl-perhydro-pyrrolo[1,2-c]oxazol-5-on

150 g (S)-5-Hydroxymethyl-2-pyrrolidinon und 209 ml Cyclohexanaldehyd werden mit 5 g p-Toluolsulfonsäure und 1,5 l Toluol 3 Stunden am Wasserabscheider unter Rühren gekocht. Nach dem Abkühlen wird von einem zähen Rückstand abdekantiert, die Toluollösung mit gesättigter Natriumhydrogensulfit-Lösung Wasser und gesättigter Bicarbonatlösung gewaschen, mit Natriumsulfat getrocknet und eingeengt. Nach Vakuumdestillation werden 197 g (71 % der Theorie) erhalten.

Siedepunkt: 105-111°C (0,05 mbar)
Schmelzpunkt: 42-43°C
$R_f$-Wert: 0,30 (Kieselgel; Cyclohexan/Essigester = 15:1)

Analog werden erhalten:

(1) (8S)-3-tert.Butyl-perhydro-pyrrolo[1,2-c]oxazol-5-on

Siedepunkt: 90-94°C (0,2 mbar)
$R_f$-Wert: 0,40 (Kieselgel; Cyclohexan/Essigester = 1:1)

| Ber.: | C 65,54 | H 9,35 | N 7,64 |
|-------|---------|--------|--------|
| Gef.: | 65,36   | 9,64   | 7,64   |

(2) (8R)-3-Cyclohexyl-perhydro-pyrrolo[1,2-c]oxazol-5-on

$R_f$-Wert: 0,42 (Kieselgel; Cyclohexan/Essigester = 1:1)

| Ber.: | C 68,87 | H 9,15 | N 6,69 |
|-------|---------|--------|--------|
| Gef.: | 68,73 | 9,30 | 6,55 |

Beispiel XVII

(3R,5S)-3-Allyl-5-hydroxymethyl-2-pyrrolidinon

Zu 94,7 g (6R,8S)-6-Allyl-3-cyclohexyl-perhydro-pyrrolo[1,2-c]oxazol-5-on in 900 ml Aceton werden 800 ml halbkonzentrierte Salzsäure gegeben und 18 Stunden bei Raumtemperatur gerührt. Das Aceton wird dann abgezogen, die wäßrige Phase mit Essigester gewaschen, die Essigesterphase mit Wasser extrahiert und die vereinigten wäßrigen Phasen zur Trockne eingeengt. Der Rückstand wird in 750 ml Methylenchlorid aufgenommen, mit Kaliumhydrogencarbonat und wenig Wasser neutralisiert. Anschließend wird von den Salzen abgesaugt, die organische Phase mit Natriumsulfat getrocknet, über Aktivkohle filtriert und einrotiert. Es verbleiben 58,9 g (100 % der Theorie) eines Öls, das langsam durchkristallisiert.

Schmelzpunkt: 53-55°C
$R_f$-Wert: 0,51 (Kieselgel; Essigester/Methanol = 4:1)

Die Spaltung von (6R,8S)-6-Allyl-3-tert.butyl-perhydropyrrolo[1,2-c]oxazol-5-on mit halbkonzentrierter Salzsäure und wenig Dioxan verläuft analog und liefert das gleiche Produkt.

Analog werden erhalten:

(1) (3S,5S)-3-Allyl-5-hydroxymethyl-2-pyrrolidinon

$R_f$-Wert: 0,15 (Kieselgel; Essigester)

(2) (3R,5S)-3-Allyl-5-hydroxymethyl-3-methyl-2-pyrrolidinon

$R_f$-Wert: 0,22 (Kieselgel; Essigester)

(3) (3S,5R)-3-Allyl-5-hydroxymethyl-2-pyrrolidinon

$R_f$-Wert: 0,12 (Kieselgel; Essigester)

(4) (S)-3,3-Diallyl-5-hydroxymethyl-2-pyrrolidinon

$R_f$-Wert: 0,35 (Kieselgel; Essigester)

Beispiel XVIII

(3R,5S)-3-Allyl-1-(3-phenylpropyl)-5-[(p-toluolsulfonyloxy)methyl]-2-pyrrolidinon

Zu einer Mischung aus 54,6 g (3R,5S)-3-Allyl-5-hydroxymethyl-1-(3-phenylpropyl)-2-pyrrolidinon, 500 ml Methylenchlorid, 32 ml Pyridin und 0,2 g 4-Dimethylaminopyridin werden bei -20°C portionsweise 76,4 g 4-Toluolsulfochlorid gegeben. Man hält die Mischung noch 2 Stunden bei -20°C und läßt 64 Stunden bei Raumtemperatur stehen. Das Methylenchlorid wird im Vakuum abgedampft, der Rückstand in Ether aufgenommen und mit 1N Salzsäure gewaschen. Das nach dem Eindampfen erhaltene Rohprodukt wird durch Chromatographie über Kieselgel gereinigt (Elutionsmittel: Ether/Petrolether = 10:1)

Ausbeute: 56,6 g (66 % der Theorie),
$R_f$-Wert: 0,68 (Kieselgel; Ether)

Beispiel XIX

(3R,5S)-3-Allyl-1-(3-phenylpropyl)-5-[[2-(N-phthalimidomethyl)-5-indanyl]sulfonylaminomethyl]-2-pyrrolidinon

6,8 g (3R,5S)-3-Allyl-5-aminomethyl-1-(3-phenylpropyl)-2-pyrrolidinon werden in einer Mischung von 150 ml Tetrahydrofuran und 7,5 ml Triethylamin gelöst, auf 0°C abgekühlt und mit 9,4 g 2-(N-Phthalimidomethyl)indan-5-sulfochlorid versetzt. Man rührt 4 Stunden bei Raumtemperatur, gießt in verdünnte Salzsäure und extrahiert mit Essigester. Die Essigesterphase wird eingedampft und der Rückstand über Kieselgel säulenchromatographisch gereinigt (Elutionsmittel: Methylenchlorid/Ethanol = 15:1).

Ausbeute: 11,7 g (81 % der Theorie),
$R_f$-Wert: 0,86 (Kieselgel; Methylenchlorid/Ethanol = 15:1)

Analog werden erhalten:

(1) (3R,5S)-3-Allyl-1-(3-phenylpropyl)-5-[[2-(N-phthalimido)-5-indanyl)sulfonylaminomethyl]-2-pyrrolidinon

$R_f$-Wert: 0,69 (Kieselgel; Methylenchlorid/Ethanol = 15:1)

(2) 4-[(4-Methoxyphenyl)sulfonylamino]benzonitril

Lösungsmittel: Pyridin
Schmelzpunkt: 187-189°C

| Ber.: | C 58,32 | H 4,20 | N 9,72 | S 11,12 |
|---|---|---|---|---|
| Gef.: | 58,11 | 4,27 | 9,44 | 11,07 |

Beispiel XX

1-(3-Phenylpropyl)-4-[(trityloxy)methyl]-2-pyrrolidinon

Eine Mischung aus 74,1 g 4-Hydroxymethyl-1-(3-phenylpropyl)-2-pyrrolidinon, 81 ml Triethylamin, 1,8 g 4-Dimethylaminopyridin, 97,1 g Triphenylchlormethan und 1100 ml Dimethylformamid wird 16 Stunden bei Raumtemperatur gerührt. Das Reaktionsgemisch wird eingedampft, der Rückstand auf 2500 ml Eiswasser gegossen und mit Methylenchlorid extrahiert. Die organische Phase wird mit gesättigter Ammoniumchloridlösung gewaschen, mit Aktivkohle behandelt, eingedampft, und der Rückstand über Kieselgel (Elutionsmittel; Methylenchlorid/Methanol = 50:1) gereinigt.

Ausbeute: 109,9 g (73 % der Theorie),
$R_f$-Wert: 0,69 (Kieselgel; Methylenchlorid/Methanol = 9:1)

Analog wird erhalten:

(1) -(4-Methoxybenzyl)-4-[(trityloxy)methyl]-2-pyrrolidinon

Schmelzpunkt: 171-173°C
$R_f$-Wert: 0,80 (Kieselgel; Essigester)

Beispiel XXI

4-Hydroxymethyl-1-(3-phenylpropyl)-2-pyrrolidinon

88,5 g 1-(3-Phenylpropyl)-2-pyrrolidinon-4-carbonsäure und 36,2 g Triethylamin werden in 1400 ml Tetrahydrofuran gelöst. Man kühlt auf -20°C ab, tropft 38,8 g Chlorameisensäureethylester zu, rührt 30 Minuten nach und filtriert den ausgefallenen Niederschlag ab. Das Filtrat wird bei -10°C zu einer Lösung von 33,8 g Natriumborhydrid in 700 ml Wasser getropft. Man rührt noch 2 Stunden, wobei man die Mischung auf Raumtemperatur kommen läßt, stellt mit konzentrierter Salzsäure auf pH 4 und läßt 16 Stunden bei Raumtemperatur stehen. Die Lösung wird mit Natriumhydrogencarbonat alkalisch gestellt und mit Methylenchlorid extrahiert. Der nach dem Eindampfen der Methylenchloridphase verbleibende Rückstand wird ohne weitere Reinigung umgesetzt.

Ausbeute: 57,7 g (69 % der Theorie),
$R_f$-Wert: 0,53 (Kieselgel; Methylenchlorid/Methanol = 9:1)

Beispiel XXII

1-(3-Phenylpropyl)-2-pyrrolidinon-4-carbonsäure

Eine Mischung aus 48 g Itaconsäure und 50 g 3-Phenylpropylamin wird eine Stunde auf 160°C erhitzt. Nach dem Abkühlen nimmt man mit 500 ml 2N Natronlauge auf, wäscht mit Essigester und säuert mit Salzsäure an. Man extrahiert mit Essigester und dampft die organische Phase ein.

Ausbeute: 88,5 g (97 % der Theorie),
$R_f$-Wert: 0,86 (Kieselgel; Methylenchlorid/Methanol = 5:1)

Analog wird erhalten:

(1) 1-(4-Methoxybenzyl)-2-pyrrolidinon-4-carbonsäuremethylester Die rohe 1-(4-Methoxybenzyl)-2-pyrrolidinon-4-carbonsäure wird direkt mit Methanol/Thionylchlorid in den Methylester übergeführt.

$R_f$-Wert: 0,54 (Kieselgel; Essigester)

| Ber.: | C 63,86 | H 6,51 | N 5,32 |
|-------|---------|--------|--------|
| Gef.: | 63,65 | 6,46 | 5,20 |

Beispiel XXIII

4-Amino-4'-cyano-biphenyl

Hergestellt analog Beispiel CXIV durch Reduktion von 4'-Cyano-4-nitrobiphenyl.

Schmelzpunkt: 171-173°C

Analog werden erhalten:

(1) 3-Amino-4'-cyano-biphenyl

Schmelzpunkt: 120-121°C

(2) 2-Amino-7-methoxy-9,10-dihydro-phenanthren

$R_f$-Wert: 0,63 (Kieselgel; Methylenchlorid/Methanol/konz.wäßriges Ammoniak = 19:1:0,1)

Beispiel XXIV

3-[4-(Aminomethyl)phenyl]propionsäure-methylester-hydrochlorid

3,7 g 4-(Aminomethyl)zimtsäure werden in 200 ml Methanol und 10 ml etherischer Salzsäure gelöst. Man fügt 0,3 g 10%ige Palladiumkohle zu und hydriert 4 Stunden bei Raumtemperatur und einem Wasserstoffdruck von 3 bar. Nach Abfiltrieren des Katalysators dampft man die Lösung ein und reinigt den Rückstand durch Chromatographie an Kieselgel (Elutionsmittel: Methylenchlorid/Methanol = 9:1).

Ausbeute: 2,1 g (50 % der Theorie),
$R_f$-Wert: 0,28 (Kieselgel; Methylenchlorid/Methanol = 9:1)

Beispiel XXV

4-(4-Cyanobenzoylamino)anisol

Hergestellt analog Beispiel CXIX aus 4-Cyanobenzoylchlorid und 4-Aminoanisol.

Schmelzpunkt: 178-181°C

| Ber.: | C 71,42 | H 4,79 | N 11,11 |
|-------|---------|--------|---------|
| Gef.: | 71,44 | 4,89 | 11,00 |

Analog werden erhalten:

(1) 4-(4-Methoxybenzoylamino)benzonitril

Lösungsmittel: Pyridin
Schmelzpunkt: 152-155°C

| Ber.: | C 71,42 | H 4,79 | N 11,11 |
|-------|---------|--------|---------|
| Gef.: | 71,23 | 4,67 | 11,19 |

(2)　(3S,5S)-3-[(Methoxycarbonyl)methyl]-5-[2-[[(3'-nitro-4-biphenylyl)carbonyl]amino]ethyl]-1-(3-phenylpropyl)-2-pyrrolidinon

$R_f$-Wert: 0,46 (Kieselgel; Methylenchlorid/Methanol/konz. wäßriges Ammoniak = 19:1:0,1)

(3)　(3S,5S)-3-[(Methoxycarbonyl)methyl]-5-[[(3'-nitro-4-biphenylyl)carbonyl]aminomethyl]-1-(3-phenylpropyl)-2-pyrrolidinon

$R_f$-Wert: 0,53 (Kieselgel; Methylenchlorid/Methanol = 9:1)

Beispiel XXVI

4-(5-Aminopentyl)phenol-hydrochlorid

20,8 g 4-(5-Aminopentyl)anisol werden mit 350 ml halbkonzentrierter Salzsäure in einer Glasbombe 10 Stunden auf 180°C erhitzt. Die Lösung wird im Vakuum eingedampft, der Rückstand in Ethanol gelöst und mit Aktivkohle behandelt. Der nach dem Eindampfen verbleibende Rückstand wird als Rohprodukt weiter verwendet.

Ausbeute: 23 g (99 % der Theorie).

Beispiel XXVII

3-(4-Aminobutyl)-1-benzyloxy-benzol

Hergestellt analog Beispiel 14 durch Reduktion von 1-Benzyloxy-3-(3-cyanopropyl)benzol.

$R_f$-Wert: 0,50 (Kieselgel; Methylenchlorid/Cyclohexan/Methanol/konz. wäßriges Ammoniak = 68:15:15:2)

Analog werden erhalten:

(1) 4-(4-Aminobutyl)-1-benzyloxy-benzol
Durchführung in methanolischem Ammoniak

$R_f$-Wert: 0,18 (Kieselgel; Methylenchlorid/Methanol = 8:1)

(2) 2-Aminomethyl-indan

$R_f$-Wert: 0,44 (Kieselgel; Methylenchlorid/Cyclohexan/Methanol/konz. wäßriges Ammoniak = 68:15:15:2)

(3) 4-(5-Aminopentyl)anisol

$R_f$-Wert: 0,59 (Kieselgel; Methylenchlorid/Cyclohexan/Methanol/konz. wäßriges Ammoniak = 68:15:15:2)

(4) 4-Aminomethyl-zimtsäure

$R_f$-Wert: 0,22 (Kieselgel; Ethanol/Wasser = 9:1)

(5) (3S,5S)-5-(2-Aminoethyl)-3-carboxymethyl-1-(3-phenylpropyl)-2-pyrrolidinon

$R_f$-Wert: 0,23 (Kieselgel; Methylenchlorid/Methanol/Eisessig = 1:1:0,1)

Beispiel XXVIII

6-Carboxy-5,6,7,8-tetrahydronaphthalin-2-carbonsäuremethylester

Hergestellt analog Beispiel 16 durch Hydrolyse von 6-Methoxycarbonyl-5,6,7,8-tetrahydronaphthalin-2-carbonsäuremethylester.

Schmelzpunkt: 187-189°C
$R_f$-Wert: 0,68 (Kieselgel; Toluol/Dioxan/Ethanol/Eisessig = 90:10:10:6)

Beispiel XXIX

6-Cyano-2-naphthylcarbonylchlorid

2,8 g Naphtalin-2,6-dicarbonsäure-monoamid, 10 ml Thionylchlorid und 1 Tropfen Dimethylformamid werden 5 Stunden unter Rückfluß gekocht. Die klare gelbe Lösung wird im Vakuum eingeengt und der feste Eindampfrückstand mit 20 ml Diethylether und 20 ml Petrolether versetzt, verrührt und abgesaugt. Der Filterkuchen wird mit Petrolether und Diethylether gewaschen und im Vakuum getrocknet.

Ausbeute: 2,5 g (89 % der Theorie),
Schmelzpunkt: 150-155°C

Beispiel XXX

Naphtalin-2,6-dicarbonsäure-monoamid

3,5 g Naphtalin-2,6-dicarbonsäure-monoamid-methylester, 40 ml Ethanol und 2 ml 15N Natronlauge werden 1 Stunde unter Rückfluß erhitzt. Das Reaktionsgemisch wird eingeengt, der Rückstand in 100 ml Wasser gelöst und zweimal mit je 50 ml Methylenchlorid/Methanol (4:1) ausgeschüttelt. Die wäßrige Phase wird filtriert und das Filtrat in der Wärme mit Salzsäure angesäuert. Der Niederschlag wird abgesaugt, mit Wasser gewaschen und bei 100°C getrocknet.

Ausbeute: 2,9 (88,6 % der Theorie),
Schmelzpunkt: >260°C
$R_f$-Wert: 0,35 (Kieselgel; Toluol/Dioxan/Ethanol/Eisessig = 90:10:10:6)

| Ber.: | C 66,97 | H 4,21 | N 6,51 |
|-------|---------|--------|--------|
| Gef.: | 66,46 | 4,16 | 6,25 |

Analog werden erhalten:

(1) 7-Cyano-2-naphtalincarbonsäure

Schmelzpunkt: 294-297°C
$R_f$-Wert: 0,62 (Kieselgel; 1,2-Dichlorethan/Essigester/Eisessig = 100:30:5)

(2) 6-Cyano-5,6,7,8-tetrahydronaphtalin-2-carbonsäure

Schmelzpunkt: 231-236°C
$R_f$-Wert: 0,58 (Kieselgel; Cyclohexan/Essigester = 1:1)

(3) 6-Aminocarbonyl-5,6,7,8-tetrahydronaphtalin-2-carbonsäure

Schmelzpunkt: >260°C (Zers.)
$R_f$-Wert: 0,34 (Kieselgel; Toluol/Dioxan/Ethanol/Eisessig = 90:10:10:6)

(4) 3'-Nitro-3-biphenylcarbonsäure

Schmelzpunkt: 271-272°C
$R_f$-Wert: 0,51 (Kieselgel; Cyclohexan/Essigester = 1:2)

| Ber.: | C 64,20 | H 3,73 | N 5,76 |
|-------|---------|--------|--------|
| Gef.: | 63,96 | 3,78 | 5,98 |

Beispiel XXXI

Naphtalin-2,6-dicarbonsäure-monoamid-methylester

3,7 g Naphtalin-2,6-dicarbonsäure-monomethylester, 15 ml Thionylchlorid und 1 Tropfen Dimethylformamid werden 1,5 Stunden unter Rückfluß erhitzt. Anschließend wird das überschüssige Thionylchlorid im Vakuum abdestilliert und der feste Rückstand in 120 ml Dioxan suspendiert. In diese Suspension werden bei Raumtemperatur unter Rühren 15 Minuten Ammoniak durchgeleitet und dann 18 Stunden bei Raumtemperatur weitergerührt. Danach wird das Lösungsmittel abrotiert, der Rückstand mit Wasser versetzt und die Suspension abgesaugt. Nach waschen mit wenig Wasser/Methanol und Trocknen verbleiben 3,66 g (99,8 % der Theorie).

Schmelzpunkt: 226-228°C
$R_f$-Wert: 0,45 (Kieselgel; Toluol/Dioxan/Ethanol/Eisessig = 90:10:10:6)

| Ber.: | C 68,11 | H 4,84 | N 6,11 |
|-------|---------|--------|--------|
| Gef.: | 67,98 | 4,87 | 6,28 |

Analog wird erhalten:

(1) 6-Aminocarbonyl-5,6,7,8-tetrahydronaphtalin-2-carbonsäuremethylester

Schmelzpunkt: 162-164°C
$R_f$-Wert: 0,44 (Kieselgel; Toluol/Dioxan/Ethanol/Eisessig = 90:10:10:6)

Beispiel XXXII

Naphtalin-2,6-dicarbonsäure-monomethylester

Zu einer 70°-75°C warmen Lösung von 5 g Naphtalin-2,6-dicarbonsäure-dimethylester in 80 ml Dimethylformamid wird rasch eine Suspension von 1,96 g Natrium-n-propylmercaptid (hergestellt aus n-Propylmercaptan und Natriumhydrid) in 20 ml Dimethylformamid zugegeben. Nach 2 Stunden Rühren bei Raumtemperatur wird das Reaktionsgemisch etwas abgekühlt und mit Eiswasser und Wasserstoffperoxid-Lösung versetzt. Das Reaktionsgemisch wird mit Salzsäure angesäuert, der Niederschlag abgesaugt und mit Wasser/Methanol (3:1) gewaschen und dann getrocknet. Nach Umkristallisation aus Dioxan verbleiben 3,2 g (69 % der Theorie),

Schmelzpunkt: 277-280°C (Zers.)
$R_f$-Wert: 0,59 (Kieselgel; Toluol/Dioxan/Ethanol/Eisessig = 90:10:10:6)

| Ber.: | C 67,82 | H 4,38 |
|-------|---------|--------|
| Gef.: | 67,64 | 4,17 |

Beispiel XXXIII

7-Cyano-2-naphtalincarbonsäure-methylester

8,4 g 7-Brom-2-naphtalincarbonsäure-methylester und 3,0 g Kupfer(I)-cyanid werden in 30 ml Dimethylformamid 20 Stunden unter Rückfluß erhitzt. Nach dem Abkühlen wird mit Wasser versetzt, mit Salzsäure angesäuert und mit Essigester gerührt. Die ungelösten Anteile werden abfiltriert, die Phasen getrennt und die wäßrige Phase viermal mit Essigester extrahiert. Die vereinigten organischen Phasen werden mit Wasser gewaschen, über Magnesiumsulfat getrocknet und einrotiert. Der Rückstand wird über eine Kieselgelsäule mit Cyclohexan/Essigester chromatographiert.

Ausbeute: 1,4 g (21 % der Theorie),
Schmelzpunkt: 137-139°C
$R_f$-Wert: 0,52 (Kieselgel; Cyclohexan/Essigester = 8:2)

| Ber.: | C 73,92 | H 4,30 | N 6,63 |
|-------|---------|--------|--------|
| Gef.: | 73,94 | 4,39 | 6,65 |

Analog werden erhalten:

(1) 3-(4-Cyanophenyl)cyclobutancarbonsäure

$R_f$-Wert: 0,48 (Kieselgel; Cyclohexan/Essigester/Eisessig = 40:20:1)

| Ber.: | C 71,63 | H 5,51 | N 6,96 |
|-------|---------|--------|--------|
| Gef.: | 71,97 | 5,51 | 7,04 |

(2) 4-(4-Cyanophenyl)cyclohexanon-ethylenketal

Schmelzpunkt: 132-133°C
$R_f$-Wert: 0,40 (Kieselgel; Petrolether/Essigester = 3:1)

| Ber.: | C 74,04 | H 7,04 | N 5,76 |
|-------|---------|--------|--------|
| Gef.: | 73,84 | 7,10 | 5,58 |

(3) 4'-Cyano-3-biphenylylcarbonsäure-methylester

$R_f$-Wert: 0,39 (Kieselgel; Cyclohexan/Essigester = 4:1)

Beispiel XXXIV

7-Brom-2-naphtalincarbonsäure-methylester

15,7 g 2,7-Dibromnaphtalin in 160 ml trockenem Tetrahydrofuran werden bei -70°C langsam mit 22 ml 2,5 M n-Butyllithium-Lösung in Hexan versetzt. Nach 1 Stunde Rühren bei -70°C wird ein schwacher Strom getrocknetes Kohlendioxid eingeleitet, wobei die Temperatur unter -60°C gehalten wird. Nach Abklingen der exothermen Reaktion wird die Kohlendioxid-Einleitung beendet. Das Reaktionsgemisch wird mit Diethylether versetzt und der Niederschlag abgesaugt und mit Ether gewaschen. Der Niederschlag wird in Wasser suspendiert, mit konz. Salzsäure angesäuert, 20 Minuten gerührt, abgesaugt, mit Wasser gewaschen und getrocknet. Das Zwischenprodukt wird dann 3 Stunden mit 400 ml Methanol und 7 ml Thionylchlorid unter Rückfluß erhitzt. Nach dem Abkühlen wird vom Ungelösten abgenutscht und das Filtrat einrotiert. Nach Chromatographie über eine Kieselgelsäule mit Cyclohexan/Essigester (98:2) werden 7,6 g Produkt (52 % der Theorie) isoliert.

Schmelzpunkt: 120-122°C,
$R_f$-Wert: 0,47 (Kieselgel; Cyclohexan/Essigester = 95:5)

Beispiel XXXV

6-Cyano-5,6,7,8-tetrahydronaphtalin-2-carbonsäure-methylester

6,8 g 6-Aminocarbonyl-5,6,7,8-tetrahydronaphtalin-2-carbonsäure-methylester, 9,65 g Triphenylphosphin, 4,7 g Tetrachlorkohlenstoff, 3,1 g Triethylamin und 40 ml Chloroform werden zusammengegeben und 4 Stunden bei 60°C gerührt. Nach Zugabe von weiteren 2,7 g Triphenylphosphin wird noch 1 Stunde bei 60°C gerührt. Nach dem Abkühlen wurde die Reaktionslösung eingeengt, der Rückstand mit Essigester verrührt und dann vom Ungelösten abgesaugt. Das Filtrat wird eingeengt und über eine Kieselgelsäule mit Cyclohexan/Essigester (85:15) und dann (1:1) chromatographiert.

Ausbeute: 5,4 g (86 % der Theorie),
Schmelzpunkt: 79-81°C
$R_f$-Wert: 0,70 (Kieselgel; Cyclohexan/Essigester = 1:1)

| Ber.: | C 72,53 | H 6,09 | N 6,51 |
|---|---|---|---|
| Gef.: | 72,66 | 6,18 | 6,57 |

Beispiel XXXVI

N-[4-(4-Benzyloxyphenyl)butyl]-N-tert.butyloxycarbonyl-amin

16,8 g 4-(4-Benzyloxyphenyl)butylamin werden in 200 ml Methanol gelöst. Die Lösung wird bis zum Sättigungspunkt abgekühlt und dann eine Lösung von 17 g Pyrokohlensäure-di-tert.butylester zugetropft. Man rührt 2 Stunden bei Raumtemperatur nach, dampft ein, versetzt mit gesättigter Kochsalzlösung und extrahiert mit Methylenchlorid. Die Methylenchloridphase wird eingeengt und das zurückbleibende Produkt ohne weitere Reinigung weiterverwendet.

$R_f$-Wert: 0,57 (Kieselgel; Cyclohexan/Essigester = 5:2)

Analog werden erhalten:

(1) 4-[(tert.Butyloxycarbonylamino)methyl]phenol

Lösungsmittel: Methanol/Tetrahydrofuran
$R_f$-Wert: 0,54 (Kieselgel; Methylenchlorid/Methanol/konz. wäßriges Ammoniak = 90:10:1)

(2) 4-[2-(tert.Butyloxycarbonylamino)ethyl]phenol

Lösungsmittel: Methanol/Tetrahydrofuran

$R_f$-Wert: 0,61 (Kieselgel; Methylenchlorid/Methanol/konz. wäßriges Ammoniak = 90:10:1)

(3) 1-Benzyloxy-3-[4-(tert.butyloxycarbonylamino)butyl]benzol

Lösungsmittel: Tetrahydrofuran
$R_f$-Wert: 0,69 (Kieselgel; Methylenchlorid)

(4) 1-Benzyloxy-4-[4-(tert.butyloxycarbonylamino)butyl]benzol Reduktion in Tetrahydrofuran/Methanol

$R_f$-Wert: 0,57 (Kieselgel; Cyclohexan/Essigester)

(5) (3R,5S)-3-[4-(tert.Butyloxycarbonylamino)butyl]-5-[(methansulfonyloxy)methyl]-1-methyl-2-pyrrolidinon

$R_f$-Wert: 0,30 (Kieselgel; Essigester/Methanol = 20:1)

Beispiel XXXVII

6-(tert.Butyloxycarbonylamino)-5,6,7,8-tetrahydronaphtalin-2-carbonsäure

180 mg 6-Amino-5,6,7,8-tetrahydronaphtalin-2-carbonsäure wird mit 6 ml Dioxan, 2 ml Wasser und 0,9 ml 1N Natronlauge versetzt. Dazu werden unter Eiskühlung 220 mg Pyrokohlensäure-di-tert.butylester gegeben. Nach 2 Stunden Rühren bei Raumtemperatur wird das Dioxan im Vakuum abgezogen und der Rückstand mit etwas Eiswasser verdünnt. Das Produkt wird durch Zugabe von 200 mg Zitronensäure in 2 ml Wasser ausgefällt und dann getrocknet.

Ausbeute: 210 mg (81 % der Theorie),
Schmelzpunkt: 185-187°C
$R_f$-Wert: 0,71 (Kieselgel; Toluol/Dioxan/Ethanol/Eisessig = 90:10:10:6)

| Ber.: | C 65,96 | H 7,26 | N 4,81 |
|-------|---------|--------|--------|
| Gef.: | 65,95 | 7,48 | 4,83 |

Analog werden erhalten:

(1) 4-[3-(tert.Butyloxycarbonylamino)cyclobutyl]benzoesäure

$R_f$-Wert: 0,52 (Kieselgel; Cyclohexan/Essigester = 1:1)

(2) 4-[3-(tert.Butyloxycarbonylamino)phenyl]buttersäure

$R_f$-Wert: 0,20 (Kieselgel; Methylenchlorid/Methanol = 19:1)

(3) 2-(tert.Butyloxycarbonylamino)indan-5-carbonsäure

$R_f$-Wert: 0,44 (Kieselgel; Methylenchlorid/Ethanol = 15:1)

(4) 2-[(tert.Butyloxycarbonylamino)methyl]indan-5-essigsäure

Schmelzpunkt: 123-124°C
$R_f$-Wert: 0,56 (Kieselgel; Methylenchlorid/Ethanol = 10:1)

(5) 3-[3-(tert.Butoxycarbonylamino)propyl]phenol

$R_f$-Wert: 0,45 (Kieselgel; Methylenchlorid/Ethanol = 15:1)

(6) 4-[5-(tert.Butyloxycarbonylamino)pentyl]phenol

$R_f$-Wert: 0,70 (Kieselgel; Methylenchlorid/Cyclohexan/Methanol/konz. wäßriges Ammoniak = 68:15:15:2)

(7) 3-[3-(tert.Butyloxycarbonylamino)phenyl]propionsäure

$R_f$-Wert: 0,40 (Kieselgel; Methylenchlorid/Methanol = 9:1)

(8) 3-[4-(tert.Butyloxycarbonylamino)phenyl]propionsäure

$R_f$-Wert: 0,54 (Kieselgel; Methylenchlorid/Methanol = 9:1)

(9) 4-[4-(tert.Butyloxycarbonylamino)phenyl]buttersäure

$R_f$-Wert: 0,54 (Kieselgel; Methylenchlorid/Methanol = 9:1)

(10) cis-4-[4-(tert.Butyloxycarbonylamino)cyclohexyl]phenol Durch Umsetzung des cis/trans-Gemisches und Ausfällung der cis-Verbindung mit 2N Salzsäure.

Schmelzpunkt: 210-213°C
$R_f$-Wert: 0,53 (Kieselgel; Methylenchlorid/Methanol = 20:1)

(11) trans-4-[4-(tert.Butyloxycarbonylamino)cyclohexyl]phenol Durch Umsetzung des cis/trans-Gemisches, Ausfällung der cis-Verbindung mit 2N Salzsäure und Aufarbeitung des Filtrates.

Schmelzpunkt: 211-214°C (Ethanol)
$R_f$-Wert: 0,53 (Kieselgel; Methylenchlorid/Methanol = 20:1)

(12) 4-[cis/trans-4-(tert.Butyloxycarbonylamino)cyclohexyl]benzoesäure

$R_f$-Wert: 0,52 (Kieselgel; Methylenchlorid/Methanol/konz. wäßriges Ammoniak = 4:1:0,25)

(13) 4-[cis-4-[(tert.Butyloxycarbonyl)aminomethyl])cyclohexyl]phenol

$R_f$-Wert: 0,47 (Kieselgel; Cyclohexan/Essigester = 2:1)

(14) 4-[trans-4-[(tert.Butyloxycarbonyl)aminomethyl]cyclohexyl]phenol

$R_f$-Wert: 0,46 (Kieselgel; Cyclohexan/Essigester = 2:1)

(15) 4-(tert.Butyloxycarbonylamino)zimtsäure

$R_f$-Wert: 0,38 (Kieselgel; Methylenchlorid/Methanol = 15:1)

<u>Beispiel XXXVIII</u>

<u>2-[(tert.Butyloxycarbonylamino)methyl]indan-5-carbonsäure</u>

4,5 g 2-Aminomethylindan-5-carbonsäure-hydrochlorid werden in einer Mischung aus 75 ml Tetrahydrofuran, 75 ml Wasser und 5,6 ml Triethylamin gelöst und unter Rühren mit 5,2 g Pyrokohlensäure-di-tert.butylester versetzt. Man rührt 64 Stunden bei Raumtemperatur nach, dampft das Tetrahydrofuran im Vakuum ab, extrahiert die wäßrige Phase mit Ether und säuert mit Zitronensäure an. Man extrahiert mit Ether und dampft die erhaltene organische Phase ein.

Ausbeute: 5,0 g (86 % der Theorie),
$R_f$-Wert: 0,58 (Kieselgel; Methylenchlorid/Methanol/Eisessig = 9:1:0,1)

Analog werden erhalten:

(1) 3-[(tert.Butyloxycarbonylamino)methyl]phenol

Lösungsmittel: Methanol/Tetrahydrofuran
$R_f$-Wert: 0,43 (Kieselgel; Methylenchlorid/Methanol/konz. wäßriges Ammoniak = 30:10:1)

(2) 3-[2-(tert.Butyloxycarbonylamino)methyl]phenol

Lösungsmittel: Methanol/Tetrahydrofuran
$R_f$-Wert: 0,49 (Kieselgel; Methylenchlorid/Methanol = 12:1)

(3) 3-[2-(tert.Butyloxycarbonylamino)ethyl]benzoesäure

Lösungsmittel: Methanol/Tetrahydrofuran
Schmelzpunkt: 115-120°C
$R_f$-Wert: 0,51 (Kieselgel; Methylenchlorid/Methanol = 10:1)

(4) 4-[2-(tert.Butyloxycarbonylamino)ethyl]benzoesäure

Lösungsmittel: Methanol/Tetrahydrofuran
Schmelzpunkt: 152-155°C
$R_f$-Wert: 0,42 (Kieselgel; Methylenchlorid/Methanol = 10:1)

(5) 2-(tert.Butyloxycarbonylamino)indan-5-essigsäure

Lösungsmittel: Methanol
$R_f$-Wert: 0,38 (Kieselgel; Methylenchlorid/Methanol = 10:1)

(6) (3R,5S)-3-Allyl-1-(tert.butyloxycarbonyl)-5-hydroxymethylpyrrolidin

$R_f$-Wert: 0,60 (Kieselgel; Cyclohexan/Essigester = 1:1)

## Beispiel XXXIX

### 3-[4-[(tert.Butyloxycarbonylamino)methyl]phenyl]propionsäure

2 g 3-[4-(Aminomethyl)phenyl]propionsäure-methylester werden in 80 ml Methanol gelöst, mit 8,7 ml 2N Natronlauge versetzt und 30 Minuten bei Raumtemperatur gerührt. Man dampft im Vakuum ein, nimmt den Rückstand mit 30 ml einer 2:1 Mischung Dioxan/Wasser auf, fügt 2,1 g Pyrokohlensäure-di-tert.butylester zu und läßt 16 Stunden bei Raumtemperatur stehen. Man engt auf etwa 10 ml ein, stellt mit Zitronensäure auf pH 2 bis 3 und extrahiert mit Essigester. Die Essigesterphase wird mit Wasser gewaschen, mit Aktivkohle behandelt und eingedampft. Ausbeute: 1,7 g (71 % der Theorie), $R_f$-Wert: 0,63 (Kieselgel; Methylenchlorid/Methanol = 9:1)

## Beispiel XL

### 6-Amino-5,6,7,8-tetrahydronaphtalin-2-carbonsäure

500 mg 6-Aminocarbonyl-5,6,7,8-tetrahydronaphtalin-2-carbonsäure, 1,42 g [Bis-(trifluoracetoxy)jod]benzol, 3 ml Wasser und 10 ml Acetonitril werden 38 Stunden bei Raumtemperatur gerührt. Das Acetonitril wird abrotiert, der Rückstand mit Wasser verdünnt und das Gemisch zweimal mit Essigester extrahiert. Die wäßrige Phase wird etwas konzentriert und dann mit 2N Natronlauge auf pH 4-5 eingestellt. Der Niederschlag wird abgesaugt, mit Wasser gewaschen und getrocknet.

Ausbeute: 250 mg (54 % der Theorie),
Schmelzpunkt: >260°C
$R_f$-Wert: 0,04 (Kieselgel; Toluol/Dioxan/Ethanol/Eisessig = 90:10:10:6)

Analog wird erhalten:

(1) 4-(3-Aminocyclobutyl)benzonitril

$R_f$-Wert: 0,27 (Kieselgel; Essigester/Methanol = 3:1)

| Ber.: | C 76,71 | H 7,02 | N 16,26 |
|-------|---------|--------|---------|
| Gef.: | 76,40   | 7,18   | 15,95   |

Beispiel XLI

3-(4-Bromphenyl)cyclobutancarbonsäure

2 g 3-(4-Bromphenyl)cyclobutandicarbonsäure werden 15 Minuten unter Stickstoff auf 210°C erhitzt. Nach Abkühlen auf Raumtemperatur wird der Rückstand in 20 ml 2N Natronlauge aufgenommen und dreimal mit Methylenchlorid extrahiert. Die wäßrige Phase wird unter Eisbildung mit 20 ml 2N Salzsäure versetzt und mit Methylenchlorid extrahiert. Die organische Phase wird mit Natriumsulfat getrocknet und einrotiert.

Ausbeute: 1,6 g (90 % der Theorie),
Schmelzpunkt: 85-90°C
$R_f$-Wert: 0,51 (Kieselgel; Cyclohexan/Essigester = 2:1)

| Ber.: | C 51,79 | H 4,35 | Br 31,32 |
|-------|---------|--------|----------|
| Gef.: | 51,60   | 4,30   | 31,37    |

Beispiel XLII

3-(4-Bromphenyl)cyclobutandicarbonsäure

17,5 g 1,3-[Bis(p-tosyloxy)]-2-(4-bromphenyl)-propan, 5,2 ml Malonsäurediethylester und 100 ml trockenes Dioxan werden unter Stickstoff auf 90°C erwärmt. Dazu gibt man vorsichtig (starkes Aufschäumen!) portionsweise 2,83 g Natriumhydrid (55%ig in Öl) und kocht dann 18 Stunden unter Rückfluß. Nach dem Abkühlen wird das Lösungsmittel abrotiert, der Rückstand mit 25 ml Ethanol/Wasser (1:1) und 4 g Kaliumhydroxid versetzt und 3 Stunden unter Rückfluß gekocht. Danach wird zur Trockene einrotiert, der Rückstand in Wasser aufgenommen und zweimal mit tert.Butylmethylether extrahiert. Die wäßrige Phase wird unter Eiskühlung mit konz. Salzsäure sauer gestellt und der Niederschlag abgesaugt und getrocknet.

Ausbeute: 7,58 g (78 % der Theorie),
$R_f$-Wert: 0,27 (Kieselgel; Cyclohexan/Essigester = 1:1)

| Ber.: | C 48,19 | H 3,71 | Br 26,71 |
|-------|---------|--------|----------|
| Gef.: | 48,10   | 3,84   | 26,96    |

Beispiel XLIII

4-(3-Aminocyclobutyl)benzoesäure-hydrochlorid

1,8 g 4-(3-Aminocyclobutyl)benzonitril werden mit 20 ml konz. Salzsäure unter Rückfluß erhitzt. Nach dem Abkühlen wird der Niederschlag abgesaugt, mit 10 ml Eiswasser nachgewaschen und bei 60°C getrocknet.

Ausbeute: 2,4 g (100 % der Theorie),
Schmelzpunkt: >200°C

| Ber.: | C 58,03 | H 6,20 | N 6,15 | Cl 15,57 |
|-------|---------|--------|--------|----------|
| Gef.: | 57,85 | 6,36 | 6,25 | 15,59 |

Analog wird erhalten:

(1) 3'-Nitro-biphenyl-4-carbonsäure
Man verwendet 75%ige Schwefelsäure und erhitzt 16 Stunden auf 150°C.

Schmelzpunkt: 311-314°C (Zers.)

Beispiel XLIV

4-[3-(Aminocarbonyl)cyclobutyl]benzonitril

6,22 g 3-(4-Cyanophenyl)cyclobutancarbonsäure werden in 20 ml trockenem Tetrahydrofuran gelöst, mit 5,01 g Carbonyldiimidazol versetzt und 2 Stunden bei 50°C gerührt. Das Reaktionsgemisch wird dann auf ein Gemisch aus 15 ml konz. wäßeriges Ammoniak und 50 g Eis gegeben. Der Niederschlag wird abgesaugt, mit Eiswasser gewaschen und getrocknet.

Ausbeute: 5,39 g (87 % der Theorie),
Schmelzpunkt: >200°C
$R_f$-Wert: 0,34 (Kieselgel; Essigester)

| Ber.: | C 71,98 | H 6,04 | N 13,99 |
|-------|---------|--------|---------|
| Gef.: | 72,00 | 6,15 | 14,00 |

Beispiel XLV

3-Brom-4'-cyano-4-hydroxybiphenyl

3,9 g 4'-Cyano-4-hydroxybiphenyl werden in 250 ml Chloroform bei Siedehitze gelöst. Zu dieser Lösung wird unter weiterem Rückflußkochen 1 ml Brom in 20 ml Chloroform getropft. Die farblose Lösung wird abgekühlt und eingedampft.

Ausbeute: 5,48 g (100 % der Theorie),
Schmelzpunkt: 186-189°C
$R_f$-Wert: 0,66 (Kieselgel; 1,2-Dichlorethan/Essigester = 9:1)

| Ber.: | C 56,96 | H 2,94 | N 5,11 | Br 29,15 |
|-------|---------|--------|--------|----------|
| Gef.: | 57,07 | 3,15 | 5,03 | 29,14 |

Beispiel XLVI

4'-Cyano-4-hydroxy-3-nitrobiphenyl

Zu 15,3 g 4'-Cyano-4-hydroxybiphenyl in 900 ml Eisessig wird eine Mischung aus 5,3 ml 65%iger Salpetersäure und 5,3 ml Wasser langsam zugetropft. Dann wird 1,5 Stunden bei 100°C gerührt. Nach dem Abkühlen wird der Ansatz 18 Stunden bei Raumtemperatur stehengelassen. Die Reaktionsmischung wird auf 3 l Wasser gegeben, der Niederschlag abgesaugt, mit Wasser gewaschen und in Methylenchlorid gelöst. Nach Trocknen über Natriumsulfat und Abziehen des Lösungsmittels verbleiben 17,8 g (100 % der Theorie).

Schmelzpunkt: 179-180°C
$R_f$-Wert: 0,83 (Kieselgel; 1,2-Dichlorethan/Essigester = 9:1)

| Ber.: | C 64,99 | H 3,36 | N 11,66 |
|-------|---------|--------|---------|
| Gef.: | 64,89   | 3,47   | 11,69   |

Analog wird erhalten:

(1) 4-Cyano-4'-nitro-biphenyl
Durchführung in rauchender Salpetersäure bei maximal 30°C

Schmelzpunkt: 187-190°C

Beispiel XLVII

4'-Cyano-3-fluor-4-methoxybiphenyl

6,7 g 3-Fluor-4-methoxyphenylboronsäure/3-Fluor-4-methoxyphenylboronsäure-anhydrid, 4,9 g 4-Brombenzonitril, 11,3 ml Triethylamin, 0,5 g Tri-o-tolylphosphin und 0,2 g Palladium(II)-acetat werden in 110 ml Dimethylformamid 3 Tage bei 100°C gerührt. Danach wird der Ansatz im zwischen eiskalter verdünnter Salzsäure und Essigester verteilt, die organische Phase wird abgetrennt und die wäßrige Phase mit Essigester nachextrahiert. Die organischen Phasen werden vereinigt, mit Natriumsulfat getrocknet und eingeengt. Der Rückstand wird mit Methylenchlorid verrieben und filtriert. Das Filtrat wird eingeengt und über eine Kieselgelsäule mit Methylenchlorid/Petrolether (1:1) gereinigt.

Ausbeute: 2,75 g (45 % der Theorie),
Schmelzpunkt: 103-104°C
$R_f$-Wert: 0,55 (Kieselgel; Methylenchlorid/Petrolether = 1:1)

Analog werden erhalten:

(1) 4'-Cyano-4-methoxy-3-trifluormethylbiphenyl

Schmelzpunkt: 126-127°C
$R_f$-Wert: 0,51 (Kieselgel; Methylenchlorid/Petrolether = 1:1)

| Ber.: | C 64,98 | H 3,64 | N 5,05 |
|-------|---------|--------|--------|
| Gef.: | 64,75   | 3,79   | 4,97   |

(2) 4'-Aminocarbonyl-3'-chlor-4-methoxybiphenyl

Schmelzpunkt: 207-208°C
$R_f$-Wert: 0,78 (Kieselgel; Methylenchlorid/Methanol = 9:1)

(3) 4'-Cyano-4-methoxy-2'-methylbiphenyl

Schmelzpunkt: 77-78°C
$R_f$-Wert: 0,50 (Kieselgel; Methylenchlorid/Petrolether = 1:1)

| Ber.: | C 80,69 | H 5,87 | N 6,27 |
|-------|---------|--------|--------|
| Gef.: | 80,48   | 5,94   | 5,98   |

(4) 4'-Cyano-2,3-dimethyl-4-methoxybiphenyl

Schmelzpunkt: 114-115°C
$R_f$-Wert: 0,65 (Kieselgel; Methylenchlorid/Petrolether = 1:1)

| Ber.: | C 80,97 | H 6,37 | N 5,30 |
|---|---|---|---|
| Gef.: | 81,17 | 6,56 | 5,86 |

(5) 3'-Nitro-3-biphenylcarbonsäure-methylester

Schmelzpunkt: 88-89°C
$R_f$-Wert: 0,61 (Kieselgel; Methylenchlorid)

(6) 4'-Cyano-3-nitro-biphenyl

Schmelzpunkt: 164-165°C

(7) 3-Nitro-3'-(N-phthalimido)biphenyl

Schmelzpunkt: 222°C (Zers.)

(8) 4'-Brom-3-biphenylylcarbonsäure-methylester

$R_f$-Wert: 0,59 (Kieselgel; Cyclohexan/Essigester = 4:1)

(9) 4'-Cyano-3'-fluor-4-methoxybiphenyl
Durchführung bei Raumtemperatur

Schmelzpunkt: 154-156°C

| Ber.: | C 74,00 | H 4,44 | N 6,16 |
|---|---|---|---|
| Gef.: | 73,93 | 4,55 | 6,20 |

Beispiel XLVIII

3-Fluor-4-methoxyphenylboronsäure/3-Fluor-4-methoxy-phenylboronsäure-anhydrid

Zu 1,8 g Magnesiumspänen in 15 ml Toluol/Tetrahydrofuran (8:2) werden einige Tropfen 4-Brom-2-fluoranisol gegeben. Nachdem die Reaktion gestartet ist, werden 15 g 4-Brom-2-fluoranisol in 85 ml Toluol/Tetrahydrofuran (8:2) zugetropft. Dabei wird die Temperatur auf 35-40°C gehalten. Es wird noch eine halbe Stunde bei 40°C nachgerührt, dann wird die Lösung abgekühlt und unter kräftigem Rühren zu einer auf -70°C abgekühlten Lösung von 33,5 ml Triisopropylborat in 100 ml Toluol/Tetrahydrofuran (1:1) eingetropft. Nach 2 Stunden Rühren bei -70°C wird über Nacht auf Raumtemperatur erwärmt. Der Ansatz wird auf Eiswasser gegeben und die Mischung dreimal mit Essigester extrahiert. Die vereinigten organischen Phasen werden getrocknet und eingeengt. Der Rückstand wird mit tert.Butylmethylether verrieben und die Hauptmenge des Produkts (5,15 g) abgesaugt. Aus der Mutterlauge werden durch eine Nachfällung und dann durch Säulenchromatographie über Kieselgel mit Essigester/Methylenchlorid (1:1) weitere 1,5 g erhalten.

Gesamtausbeute: 6,75 g (69 % der Theorie),
Schmelzpunkt: 216-218°C
$R_f$-Wert: 0,68 (Kieselgel; Methylenchlorid/Essigester = 1:1)

Analog werden hergestellt:

(1) 4-Methoxy-3-trifluormethylphenylboronsäure/4-Methoxy-3-trifluormethylphenylboronsäure-anhydrid

Schmelzpunkt: 217-218°C

$R_f$-Wert: 0,70 (Kieselgel; Methylenchlorid/Essigester = 1:1)

(2) 2,3-Dimethyl-4-methoxyphenylboronsäure/2,3-Dimethyl-4-methoxyphenylboronsäure-anhydrid

Schmelzpunkt: 251-253°C, sintert ab 239°C
$R_f$-Wert: 0,65 (Kieselgel; Methylenchlorid/Essigester = 1:1)

Beispiel XLIX

trans-4-(4-Cyanophenyl)cyclohexanol

Zu 1 g 4-(4-Cyanophenyl)cyclohexanon in 40 ml Methanol werden unter Eiskühlung und Rühren 0,8 g Natriumborhydrid portionsweise zugegeben. Nachdem 4 Stunden ohne Kühlung weitergerührt wurde, wird eingeengt, der Rückstand mit Eiswasser versetzt, 5 ml 2N Natronlauge zugegeben und 15 Minuten gerührt. Das Gemisch wird mit tert.Butylmethylether extrahiert, die organische Phase getrocknet und eingeengt. Nach Chromatographie über eine Kieselgelsäule mit Essigester/Petrolether (3:1) werden 0,8 g (80 % der Theorie) erhalten.

$R_f$-Wert: 0,61 (Kieselgel; Essigester)

Beispiel L

4-(4-Cyanophenyl)cyclohexanon

4 g 4-(4-Cyanophenyl)cyclohexanon-ethylenketal, 120 ml Aceton, 12 ml Wasser und 0,5 g Toluolsulfonsäure-Pyridiniumsalz werden 24 Stunden unter Rückfluß gekocht. Der Ansatz wird eingeengt und in tert.Butylmethylether aufgenommen. Die Lösung wird mit gesättigter Kochsalzlösung und Wasser gewaschen, mit Magnesiumsulfat getrocknet und einrotiert. Durch Säulenchromatographie auf Kieselgel mit Petrolether/Essigester (3:1) werden 2,4 g Ausgangsmaterial und 1,1 g (33,5 % der Theorie) Endprodukt erhalten.

Schmelzpunkt: 127-128°C
$R_f$-Wert: 0,33 (Kieselgel; Petrolether/Essigester = 2:1)

Beispiel LI

4-(4-Bromphenyl)cyclohexanon-ethylenketal

8 g 4-(4-Bromphenyl)cyclohex-3-enon-ethylenketal werden in 80 ml Ethanol mit 0,8 g Platindioxid bei Raumtemperatur unter einem Wasserstoffdruck von 3 bar 8 Minuten lang hydriert. Die Lösung wird mit festem Kaliumhydrogencarbonat versetzt und eingeengt. Der Rückstand wird zwischen Essigester und Wasser verteilt, die organische Phase abgetrennt, getrocknet und einrotiert. Nach Säulenchromatographie auf Kieselgel mit Petrolether/Essigester (9:1) werden 1,6 g (20 % der Theorie) erhalten.

Schmelzpunkt: 70-71°C
$R_f$-Wert: 0,44 (Kieselgel; Petrolether/Essigester = 9:1)

| Ber.: | C 56,58 | H 5,76 | Br 26,89 |
|-------|---------|--------|----------|
| Gef.: | 56,84   | 5,94   | 26,72    |

Beispiel LII

4-(4-Bromphenyl)cyclohex-3-enon-ethylenketal

34,2 4-(4-Bromphenyl)-4-hydroxycyclohexanon-ethylenketal, 0,4 g p-Toluolsulfonsäure, 40 ml Ethylenglykol und 350 ml Toluol werden 3 Stunden am wasserabscheider gekocht. Nach dem Abkühlen wird mit Essigester verdünnt und mit gesättigter wäßriger Kaliumcarbonat-Lösung und gesättigter wäßriger Kochsalzlösung gewaschen. Die organische Phase wird getrocknet, eingeengt und über eine kurze Kieselgelsäule mit Methylenchlorid chromatographiert.

Ausbeute: 25 g (77 % der Theore),
Schmelzpunkt: 108-109°C
$R_f$-Wert: 0,52 (Kieselgel; Methylenchlorid)

Beispiel LIII

4-(4-Bromphenyl)-4-hydroxycyclohexanon-ethylenketal

Zu 38,1 g 1,4-Dibrombenzol in 220 ml trockenem Ether werden bei 0-5°C 64,8 ml einer 2,5 M n-Butyllithiumlösung in Hexan zugetropft und dann noch 20 Minuten bei 0°C nachgerührt. Dann werden bei -10 bis +5°C 25,2 g 1,4-Cyclohexandion-monoethylenketal in 100 ml trockenem Tetrahydrofuran zugetropft und über Nacht bei Raumtemperatur gerührt. Der Ansatz wird auf Eis gegeben und mit Essigester extrahiert. Die organische Phase wird mit gesättigter wäßriger Kochsalzlösung gewaschen, getrocknet, bis auf 80 ml eingeengt und mit 700 ml Petrolether versetzt. Das Kristallisat (34,4 g) wird abgesaugt. Aus der Mutterlauge werden durch Einengen weitere 4,3 g Produkt erhalten. Gesamtausbeute: 38,7 g (76 % der Theorie),

Schmelzpunkt: 156-158°C
$R_f$-Wert: 0,50 (Kieselgel; Essigester/Petrolether = 2:3)

Analog werden erhalten:

(1) 1-Benzyloxy-4-[cis/trans-4-(dibenzylamino)-1-hydroxycyclohexyl]benzol

$R_f$-Wert: 0,52 und 0,36 (Kieselgel; Cyclohexan/Essigester = 4:1)

(2) 2-[4-[4-(Dibenzylamino)-1-hydroxy-cyclohexyl]phenyl]-4,4-dimethyl-oxazolin

trans-Produkt:     $R_f$-Wert: 0,30 (Kieselgel; Cyclohexan/Essigester = 2:1)
cis-Produkt:       $R_f$-Wert: 0,16 (Kieselgel; Cyclohexan/Essigester = 2:1)

(3) 1-Benzyloxy-4-[cis/trans-4-(dibenzylaminomethyl)-1-hydroxycyclohexyl]benzol

$R_f$-Wert: 0,45 (Kieselgel; Cyclohexan/Essigester = 4:1)

Beispiel LIV

4'-(N-Benzyloxycarbonylamidino)-4-hydroxybiphenyl x 0,25 Wasser

Zu 2,5 g 4'-Amidino-4-hydroxybiphenyl-hemicarbonat suspendiert in 30 ml Dioxan werden 3,6 g Chlorameisensäure-benzylester gegeben. Anschließend werden 3,05 g Triethylamin in 5 ml Dioxan bei Raumtemperatur zugetropft. Nachdem eine Stunde bei Raumtemperatur gerührt wurde, wird die Temperatur für weitere 30 Minuten auf 80°C erhöht. Nach dem Abkühlen wird das Reaktionsgemisch am Rotationsverdampfer eingeengt und der Eindampfrückstand in 50 ml Essigester aufgenommen. Beim Ausschütteln mit Eiswasser dem etwas verdünnter Salzsäure zugefügt wurde, beginnt das diacylierte Zwischenprodukt auszukristallisieren. Das Kristallisat wird abgesaugt, in Methanol aufgenommen, mit 1,5 ml 15N Natronlauge versetzt und dann 10 Minuten auf dem Dampfbad erhitzt. Danach wird das Reaktionsgemisch mit Eisessig angesäuert und eingeengt. Der Eindampfrückstand wird in Essigester/Methanol gelöst, mit Wasser und wäßriger Kochsalzlösung gewaschen, wobei immer etwas Methanol zugegeben wird, und die organische Phase getrocknet und eingeengt. Dieser Rückstand wird mit wenig Essigester verrührt, die Suspension abgekühlt und der Feststoff abgesaugt und mit Essigester/Petrolether nachgewaschen. Das Rohprodukt wird dann mit Aceton ausgekocht, es wird filtriert und das Acetonfiltrat eingedampft.

Ausbeute: 1,45 g (46 % der Theorie),
Schmelzpunkt: 178-180°C (Zers.)
$R_f$-Wert: 0,38 (Kieselgel; Cyclohexan/Essigester = 1:1)

| Ber.: | C 71,89 | H 5,32 | N 7,98 |
|-------|---------|--------|--------|
| Gef.: | 71,82 | 5,25 | 7,88 |

## Beispiel LV

1,3-[Bis-(p-tosyloxy)]-2-(4-bromphenyl)propan

Zu 89,7 g 2-(4-Bromphenyl)-1,3-dihydroxypropan in 300 ml Pyridin wird bei 5°C portionsweise 153,9 g p-Toluolsulfonsäurechlorid zugegeben und dann 18 Stunden bei Raumtemperatur gerührt. Dann werden 200 ml Eiswasser zugetropft, das Reaktionsgemisch mit Salzsäure auf pH 3 eingestellt und eine Stunde im Eisbad gerührt. Der Niederschlag wird abgesaugt, mit Eiswasser/2N Salzsäure (1:1) ausgewaschen, getrocknet und aus Ethanol umkristallisiert.

Ausbeute: 61,1 g (30 % der Theorie),
Schmelzpunkt: 128-130°C
$R_f$-Wert: 0,45 (Kieselgel; Cyclohexan/Essigester = 3:1)

| Ber.: | C 51,21 | H 4,30 | Br 14,81 | S 11,88 |
|-------|---------|--------|----------|---------|
| Gef.: | 51,46 | 4,35 | 14,52 | 12,10 |

## Beispiel LVI

2-(4-Bromphenyl)-1,3-dihydroxypropan

Zu 17,7 g Lithiumaluminiumhydrid in Diethylether werden 111,5 g (4-Bromphenyl)malonsäure-dimethylester in 200 ml Tetrahydrofuran innerhalb von 1 3/4 Stunden bei einer Temperatur von 25°C zugetropft. Nach weiteren 3 Stunden Rühren bei Raumtemperatur werden vorsichtig 50 ml Wasser unter Eiskühlung zugetropft. Anschließend gibt man 1 l 15%ige Schwefelsäure zum Ansatz, extrahiert mit Essigester und trocknet die organische Phase mit Magnesiumsulfat. Nach dem Abrotieren des Lösungsmittels verbleiben 90,9 g (100 % der Theorie) an Rohprodukt. $R_f$-Wert: 0,39 (Kieselgel; Essigester)

Analog werden erhalten:

(1) 3-(4-Benzyloxyphenyl)propanol

Lösungsmittel: Tetrahydrofuran unter Rückfluß
Schmelzpunkt: 52-56°C
$R_f$-Wert: 0,15 (Kieselgel; Methylenchlorid)

(2) 3-(3-Benzyloxyphenyl)propanol

Lösungsmittel: Tetrahydrofuran unter Rückfluß
$R_f$-Wert: 0,23 (Kieselgel; Methylenchlorid)

## Beispiel LVII

(4-Bromphenyl)malonsäure-dimethylester

3,8 g Natriumhydrid (55%ig in Paraffinöl) wird mit trockenem Toluol gewaschen und dann in 100 ml trockenem Toluol suspendiert. Bei 60°C tropft man innerhalb von 30 Minuten eine Lösung von 10 g (4-Bromphenyl)essigsäuremethylester und 11 ml Kohlensäure-dimethylester in 10 ml Toluol zu. Das Reaktionsgemisch wird noch 3 1/2 Stunden bei 60°C gerührt und der ausgefallene Niederschlag abgesaugt. Nach Waschen des Niederschlags mit Toluol wird er portionsweise in ein Gemisch aus 100 ml gesättigter wäßriger Kochsalzlösung und 25 ml Eisessig eingetragen. Das Gemisch wird mit Diethylether extrahiert, die Etherphase getrocknet und einrotiert. Der Rückstand wird mit Petrolether verrieben, abgesaugt und getrocknet.

Ausbeute: 7 g (56 % der Theorie),
Schmelzpunkt: 77-79°C
$R_f$-Wert: 0,41 (Kieselgel; Cyclohexan/Essigester = 3:1)

| Ber.: | C 46,02 | H 3,86 | Br 27,83 |
|-------|---------|--------|----------|
| Gef.: | 45,99   | 3,86   | 27,88    |

Beispiel LVIII

2-Aminoindan-5-essigsäure-hydrochlorid

3,6 g 2-Aminoindan-5-essigsäure-methylester-hydrochlorid werden in 160 ml halbkonzentrierter Salzsäure 3 Stunden unter Rückfluß gekocht. Die Salzsäure wird abdestilliert und das zurückbleibende Produkt mit wenig Ethanol verrieben, abgesaugt und im Vakuum getrocknet.

Ausbeute: 2,5 g (73,5 % der Theorie),
Schmelzpunkt: 225-230°C

Beispiel LIX

3-[4-(tert.Butyloxycarbonylamino)butyl]phenol

13,5 g 1-Benzyloxy-3-[4-(tert.butyloxycarbonylamino)butyl]benzol werden mit 6 g Palladium (10 % auf Aktivkohle) in 200 ml Essigester bei 40°C unter einem Wasserstoffdruck von 3 bar geschüttelt. Nach 5 Minuten ist die theoretische Wasserstoffaufnahme beendet. Der Ansatz wird abgekühlt, filtriert und das Filtrat einrotiert.

Ausbeute: 9,6 g (95 % der Theorie),
$R_f$-Wert: 0,51 (Kieselgel; Methylenchlorid/Ethanol = 15:1)

Analog wird erhalten:

(1) 4-[4-(tert.Butyloxycarbonylamino)butyl]phenol

$R_f$-Wert: 0,33 (Kieselgel; Cyclohexan/Essigester = 5:2)

Beispiel LX

3-(3-Benzyloxyphenyl)propionsäure-benzylester

16,6 g 3-(3-Hydroxyphenyl)propionsäure, 27,7 g Kaliumcarbonat und 36 ml Benzylbromid in 150 ml Dimethylformamid werden 17 Stunden bei Raumtemperatur gerührt. Danach werden weitere 10 g Kaliumcarbonat und 10 ml Benzylbromid zugegeben und 7 Stunden bei Raumtemperatur, dann einen Tag bei 50°C und später einen Tag bei 100°C gerührt. Nach dem Abkühlen wird der Ansatz in 1 l Wasser gegeben, mit Essigester extrahiert und die organische Phase über Natriumsulfat getrocknet und einrotiert. Nach Chromatographie über eine Kieselgelsäule mit Cyclohexan/Methylenchlorid (5:1) werden 17,7 g (51 % der Theorie) erhalten. $R_f$-Wert: 0,76 (Kieselgel; Methylenchlorid)

Analog wird erhalten:

(1) 3-(4-Benzyloxyphenyl)propionsäure-benzylester

$R_f$-Wert: 0,63 (Kieselgel; Methylenchlorid)

Beispiel LXI

4-[3-(Benzyloxycarbonylamino)propyl]phenol

2,5 g 4-(3-Aminopropyl)phenol werden in 8,3 ml 2N Natronlauge gelöst und dazu bei 0° bis 5°C unter Rühren portionsweise 5 ml 4N Natronlauge und 3,2 g Chlorameisensäure-benzylester in 3 ml Toluol gegeben. Der Ansatz wird 18 Stunden bei Raumtemperatur gerührt, dann mit verdünnter Salzsäure sauer gestellt, mit Essigester extrahiert und die organische Phase über Natriumsulfat getrocknet, über Aktivkohle filtriert und einrotiert. Das Rohprodukt wird über eine Kieselgelsäule mit Methylenchlorid/Methanol (100:1) gereinigt.

Ausbeute: 3,1 g (66 % der Theorie),
$R_f$-Wert: 0,51 (Kieselgel; Methylenchlorid/Methanol = 15:1)

Beispiel LXII

4-(4-Methoxyphenyl)buttersäurenitril

12,4 g Natriumcyanid werden in 60 ml Dimethylsulfoxid unter Erwärmen auf 100°C gelöst und 57,3 g 3-(4-Methoxyphenyl)propyljodid so zugetropft, daß die Temperatur zwischen 100-120°C blieb. Man läßt 16 Stunden bei Raumtemperatur stehen, rührt in 200 ml Wasser ein, extrahiert mit Ether und dampft die organischen Phasen ein.

Ausbeute: 97 g (100 % der Theorie),
$R_f$-Wert: 0,22 (Kieselgel; Cyclohexan/Essigester = 5:1)

Beispiel LXIII

4-(4-Benzyloxyphenyl)buttersäurenitril

29,4 g 3-(4-Benzyloxyphenyl)-1-methansulfonyloxy-propan werden in 300 ml Dimethylformamid gelöst, 6 g Natriumcyanid zugegeben und die Mischung 2 Tage auf 100°C erhitzt. Das Lösungsmittel wird im Hochvakuum abdestilliert, der Rückstand mit Wasser verrührt und mit Essigester extrahiert. Der nach dem Eindampfen des Essigesters verbleibende Rückstand wird chromatographisch über Kieselgel gereinigt (Elutionsmittel: Methylenchlorid).

Ausbeute: 16,6 g (72 % der Theorie),
$R_f$-Wert: 0,72 (Kieselgel; Methylenchlorid)

Analog wird erhalten:

(1) 4-(3-Benzyloxyphenyl)buttersäurenitril

$R_f$-Wert: 0,69 (Kieselgel; Methylenchlorid/Cyclohexan = 1:1)

Beispiel LXIV

(3R,5S)-3-Allyl-5-cyanomethyl-1-(3-phenylpropyl)-2-pyrrolidinon

56,6 g (3R,5S)-3-Allyl-1-(3-phenylpropyl)-5-[(p-toluolsulfonyloxy)methyl]-2-pyrrolidinon werden in 60 ml Dimethylformamid gelöst, mit 7,8 g Natriumcyanid versetzt und 45 Minuten bei 90°C gerührt. Man versetzt mit 500 ml Eiswasser und extrahiert mit Ether. Nach Einengen des Ethers hinterbleibt das Produkt als Öl.

Ausbeute: 36,6 g (98 % der Theorie),
$R_f$-Wert: 0,41 (Kieselgel; Ether/Petrolether = 10:1 (nach zweifacher Entwicklung)

Analog wird erhalten:

(1) 5-(4-Methoxyphenyl)valeriansäurenitril
Hergestellt aus 4-[4-(Methansulfonyloxy)-butyl]anisol und Natriumcyanid.

$R_f$-Wert: 0,44 (Kieselgel; Cyclohexan/Essigester = 5:1)

Beispiel LXV

2-Cyano-5-hydroxyindan

19,6 g 5-Acetoxy-2-cyanoindan werden in 200 ml Methanol gelöst, 20 g Pottasche dazugegeben und eine Stunde unter Rückfluß erhitzt. Die Feststoffe werden abfiltriert, das Filtrat eingeengt, mit verdünnter Salzsäure versetzt und mit Essigester extrahiert. Die Essigesterphase wird mit Aktivkohle behandelt, über Kieselgel filtriert und eingedampft.

$R_f$-Wert: 0,47 (Kieselgel; Methylenchlorid/Methanol = 15:1)

Beispiel LXVI

5-Acetyl-2-cyanoindan

40 g Aluminiumchlorid werden in 180 ml 1,2-Dichlorethan suspendiert und unter Rühren 13,2 ml Acetylchlorid zugegeben. Nun trägt man portionsweise 17,2 g 2-Cyanoindan ein, wobei man die Temperatur unter 40°C hält. Man rührt noch 3 Stunden bei Raumtemperatur nach, gießt auf Eis, stellt mit Salzsäure kräftig sauer und extrahiert mit Methylenchlorid, dem Methanol zugefügt wurde. Die organischen Phasen werden mit gesättigter Kochsalzlösung und Wasser gewaschen und nach dem Trocknen mit Natriumsulfat eingeengt. Beim Verreiben mit Diisopropylether kristallisiert das Produkt.

Ausbeute: 19,3 g (87 % der Theorie),
$R_f$-Wert: 0,34 (Kieselgel; Cyclohexan/Essigester = 2:1)

Analog wird erhalten:

(1) 5-Acetyl-2-(acetylaminomethyl)indan

Schmelzpunkt: 90-92°C

Beispiel LXVII

5-Acetoxy-2-cyanoindan

Eine Mischung aus 18,4 g 5-Acetyl-2-cyanoindan, 34 g m-Chlorperoxybenzoesäure und 300 ml Chloroform wird unter Lichtausschluß eine Woche bei Raumtemperatur gerührt. Man filtriert die festen Bestandteile ab, verdünnt das Filtrat mit 300 ml Methylenchlorid und schüttelt nacheinander mit NatriumsulfitLösung, Natriumbicarbonat-Lösung und gesättigter Kochsalzlösung aus. Das beim Einengen der organischen Phase hinterbleibende Rohprodukt wird in dieser Form weiterverarbeitet.

Ausbeute: 19,6 g (98 % der Theorie),
$R_f$-Wert: 0,44 (Kieselgel; Cyclohexan/Essigester = 2:1)

Beispiel LXVIII

4-(3-Cyanophenyl)buttersäure

12,7 g 4-(3-Aminophenyl)buttersäure-hydrochlorid werden in 50 ml Wasser und 5 ml konzentrierter Salzsäure gelöst. Zu dieser Lösung tropft man bei 0 bis -5°C eine Lösung von 4,07 g Natriumnitrit in 20 ml Wasser im Verlaufe von 30 Minuten. Diese Lösung wird bei 70°C zu einer Mischung aus 40 ml Wasser, 17,3 g Kaliumcyanid und 5,9 g Kupfer(I)cyanid getropft. Man hält noch 15 Minuten bei dieser Temperatur, kühlt auf Raumtemperatur ab und stellt mit Eisessig ein pH von 5-6 ein. Man extrahiert mit Essigester und reinigt das nach dem Eindampfen hinterbleibende Rohprodukt durch Chromatographie über Kieselgel (Elutionsmittel: Methylenchlorid/Methanol = 50:1).

Ausbeute: 4,5 g (40 % der Theorie),
$R_f$-Wert: 0,27 (Kieselgel; Methylenchlorid/Methanol = 19:1)

Analog wird erhalten:

(1) 2-Cyano-7-methoxy-9,10-dihydrophenanthren

$R_f$-Wert: 0,52 (Kieselgel; Methylenchlorid/Cyclohexan 3:1)

Beispiel LXIX

2-(Aminomethyl)indan-5-carbonsäure-hydrochlorid

7,5 g 2-(Acetylaminomethyl)indan-5-carbonsäure werden in 180 ml halbkonzentrierter Salzsäure 2 Tage unter Rückfluß gekocht. Das beim Eindampfen zurückbleibende Produkt wird unmittelbar weiterverwendet.

Ausbeute: 6,6 g (90 % der Theorie),
$R_f$-Wert: 0,34 (Kieselgel; Methanol mit 2 % wäßrigem Ammoniak)

Analog wird erhalten:

(1) 2-Aminoindan-5-carbonsäure-hydrochlorid

$R_f$-Wert: 0,67 (Kieselgel; Methanol/konz. wäßriges Ammoniak = 1:0,02)

Beispiel LXX

2-(Acetylaminomethyl)indan-5-carbonsäure

18 g 5-Acetyl-2-(acetylaminomethyl)indan werden zusammen mit einer Lösung von 47 g Natriumhydroxid in 500 ml Wasser und 15 ml Brom 64 Stunden bei Raumtemperatur gerührt. Man setzt 7,5 ml 40-prozentige Natriumbisulfitlösung zu und extrahiert mit Ether. Die wäßrige Phase wird mit Schwefelsäure auf pH 4 gebracht und im Vakuum auf 250 ml eingeengt. Der ausgefallene Niederschlag wird abfiltriert und das Filtrat mit Methylenchlorid extrahiert. Die organische Phase wird eingedampft und der Rückstand mit Essigester kristallin gerieben.

Ausbeute: 7,5 g (41 % der Theorie),
Schmelzpunkt: 184-185°C

Analog wird erhalten:

(1) 2-Acetylaminoindan-5-carbonsäure

$R_f$-Wert: 0,31 (Kieselgel; Methylenchlorid/Ethanol = 15:1)

Beispiel LXXI

2-(Acetylaminomethyl)indan

29,6 g 2-Aminomethylindan werden in 200 ml Essigester gelöst, 35 ml Triethylamin hinzugefügt und unter Eiskühlung 14,3 ml Acetylchlorid zugetropft. Man rührt eine Stunde bei Raumtemperatur nach, filtriert vom Niederschlag ab, wäscht das Filtrat mit verdünnter Salzsäure und dampft ein.

Ausbeute: 35,5 g (96 % der Theorie),
$R_f$-Wert: 0,56 (Kieselgel; Methylenchlorid/Cyclohexan/Methanol/konz. wäßriges Ammoniak = 68:15:15:2)

Beispiel LXXII

2-(N-Phthalimidomethyl)indan-5-sulfochlorid

7,4 g 2-(N-Phthalimidomethyl)indan werden portionsweise in 15 ml auf 0°C gekühlte Chlorsulfonsäure eingerührt. Man rührt noch 30 Minuten bei Raumtemperatur nach, gießt auf 200 ml Eiswasser und filtriert das ausgefallene Festprodukt ab.

Ausbeute: 9,4 g (94 % der Theorie),
$R_f$-Wert: 0,39 (Kieselgel; Cyclohexan/Essigester = 2:1)

Analog wird erhalten:

(1) 2-(N-Phthalimido)indan-5-sulfochlorid

$R_f$-Wert: 0,41 (Kieselgel; Cyclohexan/Essigester = 2:1)

Beispiel LXXIII

2-(N-Phthalimidomethyl)indan

Eine Mischung aus 7,4 g 2-Aminomethylindan, 8,9 g Phthalsäureanhydrid und 40 ml Dioxan wird 4 Stunden zum Rückfluß erhitzt. Nach dem Erkalten gießt man in Wasser, filtriert den Niederschlag ab und verreibt ihn mit Methylenchlorid. Die Methylenchloridphase wird mit Natriumsulfat getrocknet und eingedampft.

Ausbeute: 7,4 g (53 % der Theorie),
$R_f$-Wert: 0,91 (Kieselgel; Methylenchlorid/Cyclohexan/Methanol/konz. wäßriges Ammoniak = 68:15:15:2)

Beispiel LXXIV

2-Aminomethylindan-5-essigsäure

Eine Mischung aus 25,5 g des gemäß Beispiel LXXV erhaltenen Reaktionsproduktes (enthält etwa 17 g 2-(Acetylaminomethyl)indan-5-thioessigsäure-morpholid), 20 g in 30 ml Wasser gelöstem Kaliumhydroxid und 20 ml Ethanol wird einen Tag unter Rückfluß erhitzt. Man engt im Vakuum weitgehend ein, versetzt den Rückstand mit 200 ml Eiswasser und extrahiert mit Methylenchlorid. Die wäßrige Phase wird mit Salzsäure neutralisiert, wobei das Produkt ausfällt. Eine weitere Fraktion erhält man durch Einengen der Mutterlauge.

Ausbeute: 7,0 g (64 % der Theorie),
Schmelzpunkt: 249°C (Zers.)

Beispiel LXXV

2-(Acetylaminomethyl)indan-5-thioessigsäure-morpholid

Eine Mischung aus 12,3 g 5-Acetyl-2-(acetylaminomethyl)indan, 2,6 g Schwefel und 10,8 ml Morpholin wird einen Tag lang auf 140°C erhitzt. Das erhaltene Reaktionsgemisch wird direkt in Beispiel LXXIV eingesetzt.

Beispiel LXXVI

4-(4-Hydroxybutyl)anisol

Zu einer zum Sieden erhitzten Lösung von 30,3 g Lithiumalanat in 1000 ml Tetrahydrofuran werden 100 g 4-(4-Methoxyphenyl)buttersäure, gelöst in 500 ml Tetrahydrofuran, getropft. Man erhitzt noch weitere 4 Stunden zum Rückfluß, tropft nach dem Erkalten nacheinander 30 ml Wasser, 30 ml 20-prozentige Natronlauge und schließlich weitere 90 ml Wasser zu, filtriert den Niederschlag ab und dampft ein.

Ausbeute: 89,8 g (97 % der Theorie),
$R_f$-Wert: 0,71 (Kieselgel; Methylenchlorid/Cyclohexan/Methanol/konz. wäßriges Ammoniak = 68:15:15:2)

Beispiel LXXVII

3-Cyano-4'-methoxy-diphenylsulfid

Zu einer Lösung von 5 g 4-Amino-3-cyano-4'-methoxy-diphenylsulfid in 5 ml Wasser und 15 ml konz. Salzsäure wird bei 0°C und unter Rühren langsam eine Lösung von 1,6 g Natriumnitrit in 10 ml Wasser getropft. Nach beendeter Zugabe rührt man weitere 15 Minuten bei 0°C, gibt dann 40 ml Äthanol zu und wärmt auf 40°C. Man erwärmt langsam weiter auf 80°C und hält die Temperatur bei bis keine Stickstoffentwicklung mehr zu beobachten ist. Anschließend gießt man auf Eis und extrahiert mit Essigester. Die vereinigten Extrakte werden getrocknet und zur Trockne eingeengt. Der Rückstand wird über eine Kieselgelsäule gereinigt, wobei Dichlormethan als Elutionsmittel dient. Das so gewonnene 3-Cyano-4'-methoxy-diphenylsulfid wird mit Petroläther verrieben und abgesaugt.

Ausbeute: 2,7 g (58 % der Theorie),
$R_f$-Wert: 0,65 (Kieselgel; Dichlormethan)

Beispiel LXXVIII

4-Amino-3-cyano-4'-methoxy-diphenylsulfid

Zu einer Lösung von 5 g 3-Cyano-4'-methoxy-4-nitrodiphenylsulfid in 25 ml konz. Salzsäure gibt man portionsweise und unter Rühren 13,2 g Zinndichlorid-monohydrat. Nach 2-stündigem Rühren gießt man auf eine Mischung aus Eis und 10N Natronlauge und extrahiert mit Dichlormethan. Die vereinigten Extrakte werden getrocknet, mit Aktivkohle geschüttelt und im Vakuum zur Trockne eingedampft. Der Rückstand wird mit Äther/Petroläther zur Kristallisation gebracht.

Ausbeute: 3,7 g (82 % der Theorie),
Schmelzpunkt: 95-98°C

| Ber.: | C 65,38 | H 5,76 | N 11,44 |
|-------|---------|--------|---------|
| Gef.: | 65,20 | 5,80 | 11,54 |

Beispiel LXXIX

3-Cyano-4'-methoxy-4-nitro-diphenylsulfid

Zu einer Lösung von 23 ml 4-Methoxythiophenol und 20,2 g Natriumcarbonat in 250 ml Wasser tropft man bei Raumtemperatur und unter Rühren eine heiße Lösung von 34 g 5-Chlor-2-nitrobenzonitril in Ethanol. Nach beendeter Zugabe wird während 4 Stunden auf Rückflußtemperatur erhitzt. Das ausgefallene gelbe Produkt wird abgesaugt und in Dichlormethan gelöst. Diese Lösung wird getrocknet und im Vakuum zur Trockne eingedampft. Der Rückstand wird aus Toluol/Petroläther kristallisiert.

Ausbeute: 45,2 g (85 % der Theorie),
Schmelzpunkt: 99-101°C

Beispiel LXXX

(3R,5S)-3-Allyl-5-aminomethyl-1-(3-phenylpropyl)-2-pyrrolidinon

73 g (3R,5S)-3-Allyl-1-(3-phenylpropyl)-5-(N-phtalimidomethyl)-2-pyrrolidinon, 600 ml 40%ige wäßrige Methylamin-Lösung und 1000 ml Toluol werden 4 1/2 Tage bei Raumtemperatur kräftig gerührt. Die Toluolphase wird abgetrennt und die wäßrige Phase nochmals mit Toluol ausgeschüttelt. Die vereinigten Toluolphasen werden eingeengt und der Rückstand über eine Kieselgelsäule mit Methylenchlorid/Methanol (10:1) gereinigt. Es werden 41,6 g (89 % der Theorie) eines Öls erhalten.

$R_f$-Wert: 0,40 (Kieselgel; Methylenchlorid/Methanol = 10:1)

Analog werden erhalten:

(1) (3R,S;4S,R)-3-Allyl-4-aminomethyl-1-(4-methoxybenzyl)-2-pyrrolidinon

$R_f$-Wert: 0,28 (Kieselgel; Essigester/Methanol = 9:1)

| Ber.: | C 70,04 | H 8,08 | N 10,21 |
|-------|---------|--------|---------|
| Gef.: | 69,98 | 8,25 | 10,00 |

(2) (3S,5S)-5-Aminomethyl-3-carboxymethyl-1-(3-phenylpropyl)-2-pyrrolidinon
Durch Aufarbeitung und chromatographische Reinigung der wäßrigen Phase.

Schmelzpunkt: 188-192°C
$R_f$-Wert: 0,44 (Kieselgel; Methanol/Wasser = 95:5)

(3) (3R,5S)-3-Allyl-5-aminomethyl-1-(4-phenoxybutyl)-2-pyrrolidinon

$R_f$-Wert: 0,46 (Kieselgel; Methylenchlorid/Methanol = 10:1)

(4) (3R,5S)-3-Allyl-5-aminomethyl-1-(2-phenylethyl)-2-pyrrolidinon

$R_f$-Wert: 0,37 (Kieselgel; Methylenchlorid/Methanol = 10:1)

(5) (3R,5S)-3-Allyl-5-aminomethyl-1-benzyl-2-pyrrolidinon

$R_f$-Wert: 0,43 (Kieselgel; Methylenchlorid/Methanol = 10:1)

(6) (3R,5S)-3-Allyl-5-aminomethyl-1-isobutyl-2-pyrrolidinon

$R_f$-Wert: 0,62 (Kieselgel; Methylenchlorid/Methanol = 4:1)

(7) (3R,5S)-5-Aminomethyl-3-(1-buten-4-yl)-1-(3-phenylpropyl)-2-pyrrolidinon

$R_f$-Wert: 0,40 (Kieselgel; Methylenchlorid/Methanol = 9:1)

(8) (3R,S;4S,R)-4-Aminomethyl-3-carboxymethyl-1-(3-phenylpropyl)-2-pyrrolidinon
Durch Aufarbeitung und chromatographische Reinigung der wäßrigen Phase.

$R_f$-Wert: 0,38 (Kieselgel; Ethanol/Wasser = 10:1)

(9) 3-Amino-3'-nitro-biphenyl

$R_f$-Wert: 0,35 (Kieselgel; Methylenchlorid/Cyclohexan = 2,5:1) (nach zweifacher Entwicklung)

Beispiel LXXXI

(3R,5S)-3-Allyl-5-hydroxymethyl-pyrrolidin

Zu 13,3 g Lithiumaluminiumhydrid in 600 ml trockenem Tetrahydrofuran werden bei Raumtemperatur 40 g (3R,5S)-3-Allyl-5-hydroxymethyl-2-pyrrolidinon gelöst in 500 ml trockenem Tetrahydrofuran zugetropft. Dann wird 2 Stunden bei Raumtemperatur und 4 Stunden bei 60°C gerührt und anschließend 18 Stunden bei Raumtemperatur stehen gelassen. Nach Zugabe von weiteren 5,7 g Lithiumaluminiumhydrid wird nochmals 4 Stunden bei 60°C gerührt und 18 Stunden bei Raumtemperatur stehen gelassen. Der Ansatz wird mit Eiswasser gekühlt und unter Rühren tropfenweise vorsichtig mit Wasser versetzt. Der Niederschlag wird abgesaugt, mit Ether nachgewaschen und die vereinigten Filtrate im Vakuum einrotiert.

Ausbeute: 35,4 g (97,2 % der Theorie),
$R_f$-Wert: 0,22 (Kieselgel; Methylenchlorid/Methanol = 3:1)

Beispiel LXXXII

(S)-1-(Benzyloxycarbonyl)-5-[(trityloxy)methyl]-2-pyrrolidinon

Eine Lösung von 160 g (S)-5-[(Trityloxy)methyl]-2-pyrrolidinon in 1600 ml trockenem Tetrahydrofuran wird innerhalb von 35 Minuten bei -65°C mit 179 ml einer 2,5 M Lösung von Butyllithium in Hexan versetzt. Nach 10 Minuten wird bei -65°C eine Lösung von 66,8 ml Chlorameisensäure-benzylester in 100 ml trockenem Tetrahydrofuran zugetropft und eine Stunde gerührt. Dann wird mit 200 ml gesättigter Kochsalzlösung versetzt und das Tetrahydrofuran abrotiert. Der Rückstand wird zwischen 3,5 l Essigester und 200 ml Wasser verteilt, die organische Phase abgetrennt und je zweimal mit Wasser und Kochsalzlösung gewaschen. Die organische Phase wird abgetrennt, getrocknet und einrotiert. Das Rohprodukt wird aus wenig Ethanol umkristallisiert.

Ausbeute: 181 g (82 % der Theorie),
Schmelzpunkt: 103-105°C
$R_f$-Wert: 0,53 (Kieselgel; Cyclohexan/Essigester = 2:1)

| Ber.: | C 78,19 | H 5,95 | N 2,85 |
|-------|---------|--------|--------|
| Gef.: | 78,34   | 6,00   | 3,10   |

Beispiel LXXXIII

(3S,5S)-3-[(tert.Butyloxycarbonyl)methyl]-5-hydroxymethyl-2-pyrrolidinon

246 g (3S,5S)-1-(Benzyloxycarbonyl)-3-[(tert.butyloxycarbonyl)methyl]-5-[(trityloxy)methyl]-2-pyrrolidinon in 1,6 l tert.Butanol werden 1 1/2 Tage bei 50°C unter einem Wasserstoffdruck von 5 bar mit 50 g Palladium (10 % auf Aktivkohle) hydriert. Anschließend wird mit Aceton verdünnt, der Katalysator abfiltriert und das Filtrat eingeengt. Der Rückstand wird in 3 Portionen mit insgesamt 2 l Petrolether ausgerührt. Das verbleibende Öl wird im Vakuum getrocknet.

Ausbeute: 77,8 g (84 % der Theorie),
$R_f$-Wert: 0,43 (Kieselgel; Essigester/Methanol = 15:1)

Analog wird erhalten:

(1) (3S,5S)-5-Hydroxymethyl-3-[(methoxycarbonyl)methyl]-2-pyrrolidinon

$R_f$-Wert: 0,36 (Kieselgel; Methylenchlorid/Methanol = 10:1)

Beispiel LXXXIV

(3R,5S)-3-(3-Cyanopropyl)-1-isobutyl-5-iodmethyl-2-pyrrolidinon

2,5 g (3R,5S)-3-(3-Cyanopropyl)-1-isobutyl-5-[(methansulfonyloxy)methyl]-2-pyrrolidinon, 2 g Natriumjodid und 80 ml trockenes Aceton werden einen Tag am Rückfluß gekocht. Nach dem Abkühlen wird filtriert und das Filtrat einrotiert, in Methylenchlorid aufgenommen und mit Wasser, Natriumdisulfit-Lösung und nochmals mit Wasser gewaschen. Die organische Phase wird getrocknet, filtriert und einrotiert. Der Rückstand wird durch Kieselgelchromatographie mit Toluol/Aceton = 4:1 gereinigt.

Ausbeute: 1,95 g (71 % der Theorie),
Schmelzpunkt: 45-50°C
$R_f$-Wert: 0,35 (Kieselgel; Toluol/Aceton = 4:1)

Analog wird erhalten:

(1) 1-(4'-Cyano-4-biphenylyl)-4-iodmethyl-2-pyrrolidinon

Schmelzpunkt: 178-179°C
$R_f$-Wert: 0,75 (Kieselgel; Essigester)

Beispiel LXXXV

(3S,5S)-5-(2-Hydroxyethyl)-3-[(methoxycarbonyl)methyl]-1-(3-phenylpropyl)-2-pyrrolidinon

36,5 g (3S,5S)-5-(2-Aminoethyl)-3-[(methoxycarbonyl)methyl]-1-(3-phenylpropyl)-2-pyrrolidinon werden in 750 ml Wasser unter Zusatz von 13 ml Eisessig gelöst und bei 0°C über 10 Minuten mit einer Lösung von 15,8 g Natriumnitrit in 50 ml Wasser versetzt. Man rührt 30 Minuten nach und erhitzt 3 Stunden auf dem Dampfbad. Man extrahiert mit Methylenchlorid und reinigt den nach Einengen der organischen Phase verbleibenden Rückstand durch Chromatographie an Kieselgel (Elutionsmittel: Aceton/Methanol = 40:3).

Ausbeute: 19 g (52 % der Theorie),
$R_f$-Wert: 0,70 (Kieselgel; Aceton/Methanol = 7,5:1)

Beispiel LXXXVI

(3S,5S)-5-[2-[N-(3'-Nitro-3-biphenylyl)benzylamino]ethyl]-3-[(methoxycarbonyl)methyl]-1-(3-phenylpropyl)-2-pyrrolidinon

3,2 g (3S,5S)-3-[(Methoxycarbonyl)methyl]-5-[2-[(3'-nitro-3-biphenylyl)amino]ethyl]-1-(3-phenylpropyl)-2-pyrrolidinon werden mit 1,5 ml Ethyl-diisopropylamin und 0,75 ml Benzylchlorid 7 Stunden unter Rühren auf dem Dampfbad erhitzt. Das Reaktionsgemisch wird mit Ether und Wasser verrührt, die Etherphase eingeengt und der verbleibende Rückstand durch Chromatographie an Kieselgel (Elutionsmittel: Ether) gereinigt.

Ausbeute: 3,1 g (83 % der Theorie),
$R_f$-Wert: 0,43 (Kieselgel; Essigester/Cyclohexan = 1:1)

Beispiel LXXXVII

4-[cis/trans-4-Aminocyclohexyl]benzoesäure

Eine Lösung von 23,5 g 4-[4-(Dibenzylamino)cyclohex-1-enyl]benzoesäure-hydrochlorid in 1,2 l Eisessig und 23,5 g Palladium-dihydroxid auf Kohle wird unter einem Wasserstoffdruck von 5 bar bei 50°C hydriert. Nach beendeter Umsetzung wird der Katalysator abfiltriert und mehrmals mit Eisessig nachgewaschen. Das Filtrat dampft man ein, verreibt den festen Rückstand mit Ethanol, saugt ab und trocknet im Vakuum.

Ausbeute: 9,8 g (86 % der Theorie),
$R_f$-Wert: 0,19 (Kieselgel; Methylenchlorid/Methanol/konz. wäßriges Ammoniak = 4:1:0,25)

Analog werden erhalten:

(1) 4-[cis-4-(Aminomethyl)cyclohexyl]phenol
Hergestellt durch Hydrierung des cis/trans-Gemisches, Verreiben des Rohproduktes mit Ethanol und Isolierung des ungelösten Materials.

$R_f$-Wert: 0,59 (Kieselgel; Methylenchlorid/Methanol/konz. wäßriges Ammoniak = 4:1:0,25)

(2) 4-[trans-4-(Aminomethyl)cyclohexyl]phenol
Hergestellt durch Hydrierung des cis/trans-Gemisches und Aufarbeitung der Ethanol-Mutterlauge.

$R_f$-Wert: 0,59 (Kieselgel; Methylenchlorid/Methanol/konz. wäßriges Ammoniak = 4:1:0,25)

(3) cis/trans-4-(4-Aminocyclohexyl)phenol

$R_f$-Wert: 0,52 (Kieselgel; Methylenchlorid/Methanol/konz. wäßriges Ammoniak = 4:1:0,25)

Beispiel LXXXVIII

4-[4-(Dibenzylamino)cyclohex-1-enyl]benzoesäure-hydrochlorid

Eine Lösung von 1,0 g 2-[4-[4-(Dibenzylamino)-1-hydroxycyclohexyl]phenyl]-4,4-dimethyl-oxazolin in 40 ml 3N Salzsäure wird 30 Minuten zum Rückfluß erhitzt. Man läßt abkühlen und saugt den kristallinen Niederschlag ab. Der Feststoff wird in 40 ml Methanol gelöst, mit 10 ml 10N Natronlauge versetzt und die Suspension erneut 30 Minuten zum Rückfluß erhitzt. Man läßt abkühlen, saugt ab und engt das Filtrat am Rotationsverdampfer ein. Die verbleibende wäßrige Phase wird unter Kühlung mit 32%iger Salzsäure angesäuert, der Niederschlag abgesaugt und mit Wasser gewaschen. Der Feststoff wird anschließend aus Methanol umkristallisiert.

Ausbeute: 0,65 g (68 % der Theorie),
$R_f$-Wert: 0,71 (Kieselgel; Methylenchlorid/Methanol = 8:2)

Beispiel LXXXIX

4-(Dibenzylamino)cyclohexanon

Zu einer Lösung von 7,4 g Oxalylchlorid in 150 ml absolutem Methylenchlorid tropft man unter Inertgas bei -78°C langsam 9,4 g absolutes Dimethylsulfoxid. Es wird 10 Minuten gerührt und nachfolgend eine Lösung von 15,0 g trans-4-(Dibenzylamino)cyclohexanol in 60 ml absolutem Methylenchlorid zugetropft. Man rührt eine Stunde bei -78°C und gibt anschließend 25,3 g absolutes Triethylamin zu. Unter Rühren läßt man das Kühlbad über Nacht auf Raumtemperatur kommen und gießt die Reaktionslösung auf ein 500 ml Eis/Wasser-Gemisch. Die wäßrige Phase wird 3 x mit Methylenchlorid extrahiert, die vereinigten organischen Phasen 1 x mit Wasser gewaschen, die organische Phase über Natriumsulfat getrocknet und eingeengt. Der erhaltene Rückstand wird durch Chromatographie über Kieselgel mit Cyclohexan/Essigester = 4:1 gereinigt.

Ausbeute: 13,9 g (95 % der Theorie),
$R_f$-Wert: 0,45 (Kieselgel; Cyclohexan/Essigester = 4:1)

Analog wird erhalten:

(1) 4-(Dibenzylaminomethyl)cyclohexanon

$R_f$-Wert: 0,45 (Kieselgel; Cyclohexan/Essigester = 4:1)

Beispiel XC

trans-4-(Dibenzylamino)cyclohexanol

Zu einer gut gerührten Lösung von 60,65 g trans-4-Aminocyclohexanol-hydrochlorid, 110,6 g Kaliumcarbonat in 700 ml Wasser/Methanol = 1:1 tropft man 154 g Benzylbromid und rührt 16 Stunden bei Raumtemperatur. Anschließend wird eine Stunde zum Rückfluß erhitzt, abgekühlt und mit wenig Wasser versetzt. Der ausfallende Feststoff wird abgesaugt, mit Wasser nachgewaschen, getrocknet und aus ca. 1,5 l Cyclohexan umkristallisiert.

Ausbeute: 109 g (92 % der Theorie),
$R_f$-Wert: 0,57 (Kieselgel; Methylenchlorid/Methanol = 9:1)

Analog wird erhalten:

(1) cis/trans-4-(Dibenzylaminomethyl)cyclohexanol

$R_f$-Wert des trans-Produktes: 0,30 (Kieselgel; Cyclohexan/Essigester = 6:1)
$R_f$-Wert des cis-Produktes: 0,25 (Kieselgel; Cyclohexan/Essigester = 6:1)

Beispiel XCI

4'-Cyano-4-methoxy-3-methylsulfenyl-biphenyl

Eine Lösung von 17 g rohem 4'-Aminocarbonyl-4-methoxy-3-methylsulfenyl-biphenyl in 70 ml Phosphoroxychlorid wird 1 Stunde über dem Dampfbad erhitzt. Die Reaktionslösung wird abgekühlt und mit Wasser versetzt und mit Essigester extrahiert. Die organische Phase wird mit Wasser gewaschen, getrocknet, über Aktivkohle filtriert und einrotiert. Das erhaltene Rohprodukt wird über Kieselgel mit Cyclohexan/Essigester = 2:1 chromatographisch gereinigt.

Ausbeute: 4,6 g (29 % der Theorie),
$R_f$-Wert: 0,63 (Kieselgel; Cyclohexan/Essigester = 2:1)

Beispiel XCII

4'-Aminocarbonyl-4-methoxy-3-methylsulfenyl-biphenyl

Eine Suspension von 18 g rohem 4'-Aminocarbonyl-4-methoxy-3-mercapto-biphenyl in 200 ml 5%iger methanolischer Kalilauge wird 3 Stunden bei Raumtemperatur gerührt. Es werden 10 ml Dimethylsulfat zugegeben und 30 Minuten bei Raumtemperatur gerührt. Anschließend wird mit Wasser verdünnt, der Niederschlag abgesaugt und im Vakuum getrocknet.

Ausbeute: 17 g (90 % der Theorie),
$R_f$-Wert: 0,50 (Kieselgel; Methylenchlorid/Cyclohexan/Methanol/konz. wäßriges Ammoniak = 7:1,5:1,5:0,2)

Beispiel XCIII

4'-Aminocarbonyl-4-methoxy-3-mercapto-biphenyl

8,3 g roter Phosphor und 0,4 g Jod in 35 ml Eisessig werden unter Rückfluß erwärmt und portionsweise mit 30,8 g 4'-Cyano-3-chlorsulfonyl-4-methoxy-biphenyl versetzt. Es wird 5 Stunden unter Rückfluß erwärmt, anschließend mit Wasser versetzt und weitere 30 Minuten unter Rückfluß erhitzt. Man läßt abkühlen, überführt die Reaktionslösung in Wasser und saugt den Niederschlag ab. Der Niederschlag wird in Essigester gelöst, über Natriumsulfat getrocknet, über Aktivkohle filtriert und einrotiert.

Ausbeute: 42,5 g Rohprodukt,
$R_f$-Wert: 0,16 (Kieselgel; Cyclohexan/Essigester = 2:1)

Analog wird erhalten:

(1) 4'-Cyano-4-mercapto-biphenyl
Die Reaktionslösung wird nach Wasserzugabe nicht mehr weiter erhitzt.

$R_f$-Wert: 0,61 (Kieselgel; Cyclohexan/Essigester = 2:1)

Beispiel XCIV

4'-Cyano-3-chlorsulfonyl-4-methoxy-biphenyl

31 g 4'-Cyano-4-methoxy-biphenyl-3-natriumsulfonat werden in 150 ml Phosphoroxychlorid 3,5 Stunden zum Rückfluß erwärmt. Man läßt abkühlen und überführt die Reaktionslösung in Wasser. Der Niederschlag wird abgesaugt und im Vakuum getrocknet.

Ausbeute: 30,8 g (100 % der Theorie),
$R_f$-Wert: 0,28 (Kieselgel; Cyclohexan/Essigester = 2:1)

Analog wird erhalten:

(1) 4-Chlorsulfonyl-4'-cyano-biphenyl

$R_f$-Wert: 0,61 (Kieselgel; Cyclohexan/Essigester = 2:1)

Beispiel XCV

4'-Cyano-biphenyl-4-natriumsulfonat

Zu einer Lösung von 50 g 4-Cyanobiphenyl in 400 ml absolutem Methylenchlorid tropft man bei -10°C 75 ml Chlorsulfonsäure. Es wird 15 Minuten bei -10°C gerührt, anschließend weitere 2 Stunden bei Raumtemperatur. Die Reaktionslösung wird in 1,5 l Wasser überführt und das Methylenchlorid abrotiert. Nach Zugabe von 60 g Natriumhydroxid wird der feine Niederschlag abgesaugt und im Vakuum getrocknet.

Ausbeute: 78,6 g (100 % der Theorie),
$R_f$-Wert: 0,33 (Kieselgel; Methylenchlorid/Methanol/konz. wäßriges Ammoniak = 4:1:0,25)

Analog wird erhalten:

(1) 4'-Cyano-4-methoxy-biphenyl-3-natriumsulfonat

$R_f$-Wert: 0,31 (Kieselgel; Methylenchlorid/Methanol/konz. wäßriges Ammoniak = 4:1:0,25)

Beispiel XCVI

(3S,5S)-5-Aminocarbonylmethyl-3-[(methoxycarbonyl)methyl]-1-(3-phenylpropyl)-2-pyrrolidinon

Hergestellt durch Veresterung von (3S,5S)-5-Aminocarbonylmethyl-3-carboxymethyl-1-(3-phenylpropyl)-2-pyrrolidinon mit Methanol.

$R_f$-Wert: 0,58 (Kieselgel; Methylenchlorid/Methanol = 9:1)

Beispiel XCVII

(3R,5S)-3-Allyl-5-aminocarbonylmethyl-1-(3-phenylpropyl)-2-pyrrolidinon

In eine Lösung von 9 g (3R,5S)-3-Allyl-5-cyanomethyl-1-(3-phenylpropyl)-2-pyrrolidinon in 5 ml Ameisensäure wird während 2 1/2 Stunden Salzsäuregas eingeleitet und über Nacht gerührt. Dann werden nochmals 5 ml Ameisensäure zugegebenen und 2 Stunden bei 50°C, 2 1/2 Tage bei Raumtemperatur und dann nochmals 2 Stunden bei 50°C gerührt. Die Reaktionslösung wird auf Wasser gegossen, 2 x mit Essigester extrahiert, mit Wasser gewaschen, getrocknet und eingeengt. Der Rückstand wird durch Chromatographie über eine Kieselgelsäule gereinigt.

Ausbeute: 6,1 g (64 % der Theorie),
$R_f$-Wert: 0,16 (Kieselgel; Essigester/Methanol = 40:1)

Beispiel XCVIII

4'-Cyano-3-biphenylylcarbonsäure

Eine Lösung von 1,7 g 4'-Cyano-3-biphenylylcarbonsäure-methylester und 1,28 g Lithiumhydroxid-hydrat in 12,5 ml Tetrahydrofuran und 10 ml Wasser wird 16 Stunden bei Raumtemperatur gerührt. Anschließend wird das Lösungsmittel abrotiert, der Rückstand mit 1N Salzsäure angesäuert und der Niederschlag abgesaugt.

Ausbeute: 1,0 g (59 % der Theorie),
$R_f$-Wert: 0,23 (Kieselgel; Methylenchlorid/Methanol/konz. wäßriges Ammoniak = 4:1:0,25)

Beispiel XCIX

3-Chlor-1-(4-cyanophenyl)-1-propen

Bei -10°C tropft man 8,6 g Mesylchlorid zu einer gut gerührten Lösung von 7,5 g 1-(4-Cyanophenyl)-3-hydroxy-1-propen und 6,4 g Pyridin in 50 ml absolutem Methylenchlorid. Anschließend wird das Kühlbad entfernt und 2 Tage bei Raumtemperatur gerührt. Man überführt die Reaktionslösung in 200 ml 1N Salzsäure und extrahiert mit Methylenchlorid. Die organische Phase wird getrocknet, einrotiert und das erhaltene Öl mit Cyclohexan/Essigester = 4:1 über Kieselgel chromatographiert.

Ausbeute: 4,8 g (58 % der Theorie),
$R_f$-Wert: 0,48 (Kieselgel; Cyclohexan/Essigester = 4:1)

Beispiel C

4-Hydroxymethyl-1-(4-methoxybenzyl)-2-pyrrolidinon

Zu 151 g 1-(4-Methoxybenzyl)-4-[(methoxycarbonyl)methyl]-2-pyrrolidinon in 1 l Methanol werden 47,7 g Natriumborhydrid portionsweise eingetragen. Nach 1 Stunde werden nochmals 9,3 g Natriumborhydrid und später noch 9,3 g und 5,2 g Natriumborhydrid zugegeben und dann 2 1/2 Tage bei Raumtemperatur gerührt. Nach dem Eindampfen wird der erhaltene Rückstand zwischen Essigester und Wasser verteilt, die organische Phase abgetrennt, getrocknet, filtriert und einrotiert.

Ausbeute: 118,8 g (94 % der Theorie),
$R_f$-Wert: 0,25 (Kieselgel; Essigester)

Beispiel CI

2-Methoxy-7-nitro-9,10-dihydro-phenanthren

8 g 2-Hydroxy-7-nitro-9,10-dihydro-phenanthren werden in 250 ml Aceton und 35 ml 1 N Natronlauge aufgenommen und bei Raumtemperatur tropfenweise mit 3,3 ml Dimethylsulfat versetzt. Nach 30 Minuten werden nochmals 3,5 ml 1 N Natronlauge und 0,35 ml Dimethylsulfat zugesetzt. Anschließend wird 45 Minuten auf dem Dampfbad erhitzt und nach dem Erkalten mit 300 ml Essigester extrahiert. Nach Waschen mit 1 N Natronlauge und Wasser wird die Essigesterphase eingeengt und der Rückstand durch Chromatographie an Kieselgel (Elutionsmittel: Methylenchlorid/Cyclohexan = 1:2) gereinigt.

Ausbeute: 7,4 g (88 % der Theorie),
$R_f$-Wert: 0,96 (Kieselgel; Methylenchlorid)

Beispiel CII

2-Hydroxy-7-nitro-9,10-dihydro-phenanthren

In eine Lösung von 16 g 2-Amino-7-nitro-9,10-dihydro-phenanthren in 50 ml konzentrierter Schwefelsäure werden unter Rühren bei -5°C im Laufe von 30 Minuten 5,6 g Natriumnitrit eingetragen. Man rührt bei -5°C bis 0°C 1,5 Stunden nach, gießt auf 500 ml Eis und erhitzt für 45 Minuten auf 75°C. Man extrahiert mit Essigester und reinigt das nach dem Einengen der Essigesterphase erhaltene Produkt chromatographisch über Kieselgel (Eutionsmittel: Methylenchlorid/Methanol/konz. wäßriges Ammoniak = 75:1:0,1).

Ausbeute: 8,1 g (50 % der Theorie),
$R_f$-Wert: 0,39 (Kieselgel; Methylenchlorid/Methanol/konz. wäßriges Ammoniak = 30:1:0,1)

Beispiel CIII

exo-5-(4-Cyanophenyl)bicyclo[2.2.1]heptan-exo-2-carbonsäure

Unter Inertgasatmosphäre und starkem Rühren wird eine Lösung von 3,38 g exo-2-Bicyclo[2.2.1]hepten-carbonsäure, 5,5 g 4-Iodbenzonitril, 6,8 g Piperidin und 1,0 g Bis(triphenylphosphin)palladium-(II)-acetat in 30 ml Dimethylformamid mit 2,8 g Ameisensäure versetzt. Die Reaktionslösung wird 4 Stunden auf 6o°C erwärmt, anschließend abgekühlt, mit Wasser versetzt und mehrmals mit Essigester extrahiert. Die organische Phase wird über Natriumsulfat getrocknet und eingedampft. Man erhält ein Öl, welches mit Methylenschlorid/Methanol (20:1) über Kieselgel chromatographiert wird. Die erhaltenen Fraktionen mit einem $R_f$-Wert von 0,58 (Kieselgel; Methylenchlorid/Methanol = 9:1) werden einrotiert und der erhaltene Feststoff aus Essigester umkristallisiert.

Ausbeute: 470 mg (8 % der Theorie),
Schmelzpunkt: 210-220°C
$R_f$-Wert: 0,58 (Kieselgel; Methylenchlorid/Methanol = 9:1)

Beispiel CIV

1-(4'-Cyano-4-biphenylyl)-4-(O,O'-dimethyl-phosphonomethyl)-2-pyrrolidinon

2,2 g Phosphorigsäuredimethylester in 100 ml Dimethylformamid werden mit 830 mg Natriumhydrid (55 % in Paraffinöl) versetzt. Nach 15 Minuten Rühren bei Raumtemperatur werden 7,5 g 1-(4'-Cyano-4-biphenylyl)-4-iodmethyl-2-pyrrolidinon zugegeben und 3 Stunden bei Raumtemperatur und noch eine Stunde bei 40°C gerührt. Nach dem Abkühlen wird mit Eisessig leicht angesäuert, mit Eiswasser versetzt und mit Essigester extrahiert. Die organische Phase wird mit Wasser und Kochsalzlösung gewaschen, getrocknet und einrotiert. Nach Reinigung über eine Kieselgelsäule mit Methylenchlorid/Methanol (97,5:2,5 und 96:4) werden 1,5 g (21 % der Theorie) erhalten.

Schmelzpunkt: 154-157°C
$R_f$-Wert: 0,30 (Kieselgel; Toluol/Dioxan/Ethanol/Eisessig = 90:10:10:6)

| Ber.: | C 62,50 | H 5,51 | N 7,29 |
|-------|---------|--------|--------|
| Gef.: | 62,68 | 5,61 | 7,13 |

Beispiel CV

(3S,5S)-5-Aminomethyl-3-[(methoxycarbonyl)methyl]-1-(3-phenylpropyl)-2-pyrrolidinon-hydrochlorid

Eine Mischung aus 77 g (3S,5S)-5-[(Methansulfonyloxy)methyl]-3-[(methoxycarbonyl)methyl]-1-(3-phenylpropyl)-2-pyrrolidinon, 17 g Natriumazid, 500 ml Methanol und 50 ml Wasser wird 6 Stunden bei 100°C in einem Druckgefäß geschüttelt. Nach dem Abkühlen werden 10 g 10%ige Palladiumkohle zugesetzt und 3 Stunden bei Raumtemperatur und 5 bar Wasserstoffdruck hydriert. Nach Abfiltrieren des Katalysators wird im Vakuum eingeengt, der Rückstand mit Essigester aufgenommen, die organische Phase mit Natriumbicarbonatlösung gewaschen, mit Aktivkohle behandelt und eingedampft. Der Rückstand wird mit Methanol aufgenommen, mit etherischer Salzsäure versetzt und das Lösungsmittel abdestilliert.

Ausbeute: 53,2 g (78 % der Theorie),
$R_f$-Wert: 0,25 (Kieselgel; Methylenchlorid/Ethanol = 15:1)

Beispiel CVI

(3S,5S)-5-(2-Aminoethyl)-3-[(methoxycarbonyl)methyl]-1-(3-phenylpropyl)-2-pyrrolidinon-hydrochlorid

Hergestellt analog Beispiel 26 durch Umsetzung von (3S,5S)-5-(2-aminoethyl)-3-carboxymethyl-1-(3-phenylpropyl)-2-pyrrolidinon mit methanolischer Salzsäure.

$R_f$-Wert: 0,42 (Kieselgel; Toluol/Dioxan/Methanol/konz. wäßriges Ammoniak (20:50:20:5)

Beispiel CVII

(3R,5S)-3-Allyl-5-[(4'-cyano-4-biphenylyl)oxymethyl]-1-methyl-2-pyrrolidinon

Hergestellt analog Beispiel I aus (3R,5S)-3-Allyl-5-[(4'-cyano-4-biphenylyl)oxymethyl]-2-pyrrolidinon und Methyljodid.

$R_f$-Wert: 0,31 (Kieselgel; Cyclohexan/Essigester = 4:6)

| Ber.: | C 76,28 | H 6,40 | N 8,08 |
|-------|---------|--------|--------|
| Gef.: | 75,99 | 6,77 | 7,87 |

Analog werden erhalten:

(1) (3R,5S)-3-Allyl-1-[(aminocarbonyl)methyl]-5-[(4'-cyano-4-biphenylyl)oxymethyl]-2-pyrrolidinon

$R_f$-Wert: 0,60 (Kieselgel; Essigester/Methanol= 9:1)

| Ber.: | C 70,93 | H 5,95 | N 10,79 |
|-------|---------|--------|---------|
| Gef.: | 70,96 | 5,90 | 10,64 |

(2) (3R,5S)-3-Allyl-5-[(4'-cyano-4-biphenylyl)oxymethyl]-1-[(methoxycarbonyl)methyl]-2-pyrrolidinon

$R_f$-Wert: 0,70 (Kieselgel; Essigester/Cyclohexan = 4:1)

| Ber.: | C 71,27 | H 5,98 | N 6,93 |
|-------|---------|--------|--------|
| Gef.: | 71,19 | 6,18 | 6,81 |

(3) (3S,5S)-5-[(4'-Cyano-4-biphenylyl)oxymethyl]-1-[3-(3,4-dimethoxyphenyl)propyl]-3-[(methoxycarbonyl)methyl]-2-pyrrolidinon

$R_f$-Wert: 0,47 (Kieselgel; Essigester/Cyclohexan = 4:1)

(4) (3S,5S)-5-[(4'-Cyano-4-biphenylyl)oxymethyl]-3-[(methoxycarbonyl)methyl]-1-[3-(4-hexyloxyphenyl)propyl]-2-pyrrolidinon

$R_f$-Wert: 0,83 (Kieselgel; Essigester)

(5) (3S,5S)-1-[3-(4-tert.Butylphenyl)propyl]-5-[(4'-cyano-4-biphenylyl)oxymethyl]-3-[(methoxycarbonyl)methyl]-2-pyrrolidinon

$R_f$-Wert: 0,78 (Kieselgel; Essigester)

(6) (3S,5S)-5-[(4'-Cyano-4-biphenylyl)oxymethyl]-3-[(methoxycarbonyl)methyl]-1-[3-(3-trifluormethylphenyl)propyl]-2-pyrrolidinon

$R_f$-Wert: 0,78 (Kieselgel; Essigester)

(7) (3S,5S)-5-[(4'-Cyano-4-biphenylyl)oxymethyl]-1-[3-(2,4-dichlorphenyl)propyl]-3-[(methoxycarbonyl)methyl]-2-pyrrolidinon

$R_f$-Wert: 0,71 (Kieselgel; Essigester)

(8) (3S,5S)-5-[(4'-Cyano-4-biphenylyl)oxymethyl]-3-[(methoxycarbonyl)methyl]-1-[3-(3-benzylphenyl)propyl]-2-pyrrolidinon

$R_f$-Wert: 0,81 (Kieselgel; Essigester)

(9) (3S,5S)-5-[(4'-Cyano-4-biphenylyl)oxymethyl]-1-(4,4-diphenylbutyl)-3-[(methoxycarbonyl)methyl]-2-pyrrolidinon

$R_f$-Wert: 0,81 (Kieselgel; Essigester)

(10)  (3S,5S)-5-[(4'-Cyano-4-biphenylyl)oxymethyl]-3-[(methoxycarbonyl)methyl]-1-[3-(4-methylsulfenylphenyl)propyl]-2-pyrrolidinon

$R_f$-Wert: 0,78 (Kieselgel; Essigester)

(11)  (3S,5S)-1-(4-Biphenylylmethyl)-5-[(4'-cyano-4-biphenylyl)oxymethyl]-3-[(methoxycarbonyl)methyl]-2-pyrrolidinon

$R_f$-Wert: 0,81 (Kieselgel; Essigester)

(12)  (3S,5S)-3-[(tert.Butyloxycarbonyl)methyl]-5-[(4'-cyano-4-biphenylyl)oxymethyl]-1-[(pyrrolidin-N-carbonyl)methyl]-2-pyrrolidinon

$R_f$-Wert: 0,35 (Kieselgel; Essigester)

(13)  (3S,5S)-3-[(tert.Butyloxycarbonyl)methyl]-5-[(4'-cyano-4-biphenylyl)oxymethyl]-1-[(morpholin-N-carbonyl)methyl]-2-pyrrolidinon

$R_f$-Wert: 0,44 (Kieselgel; Essigester)

Beispiel CVIII

(3R,5S)-3-Allyl-5-[(4'-cyano-4-biphenylyl)oxymethyl]-1-(3-phenylpropyl)-2-pyrrolidinon

Zu einer Mischung aus 132 g 4-Cyano-4'-hydroxy-biphenyl, 500 ml Dimethylformamid und 112,2 g Pottasche wird unter Rühren eine Lösung von 246 g (3R,5S)-3-Allyl-5-[(methansulfonyloxy)methyl]-1-(3-phenylpropyl)-2-pyrrolidinon gegeben. Man rührt 20 Stunden bei 60°C weiter und dampft dann das Dimethylformamid im Vakuum weitgehend ab. Der Rückstand wird mit 750 ml Essigester und 500 ml Wasser verrührt. Die Essigesterphase wird mit verdünnter Kochsalzlösung gewaschen und die wäßrigen Phasen nochmals mit Essigester extrahiert. Die organischen Phasen werden eingeengt und mit 1000 ml Ether und Impfkristallen verrührt. Das angefallene Rohprodukt wird abfiltriert und mit Ether gewaschen. Aus den Mutterlaugen erhält man durch Chromatographie an Kieselgel (Elutionsmittel: Ether, konzentrierter wäßriger Ammoniak = 10:0,05) eine weitere Fraktion. Die Rohprodukte werden aus Methylenchlorid/Ether umkristallisiert.

Ausbeute: 257 g (85 % der Theorie),
Schmelzpunkt: 74-76°C

Analog werden erhalten:

(1) (3R,5S)-3-Allyl-5-[(4'-cyano-4-biphenylyl)oxymethyl]-1-(3-cyclohexylpropyl)-2-pyrrolidinon

Schmelzpunkt: 157-159°C
$R_f$-Wert: 0,37 (Kieselgel; Cyclohexan/Essigester = 7:3

| Ber.: | C 78,91 | H 7,99 | N 6,13 |
|-------|---------|--------|--------|
| Gef.: | 79,00 | 8,11 | 5,94 |

(2) (3R,5S)-3-Allyl-5-[(4'-cyano-4-biphenylyl)oxymethyl]-1-(4-methoxybenzyl)-2-pyrrolidinon

Schmelzpunkt: 120-122°C
$R_f$-Wert: 0,56 (Kieselgel; Cyclohexan/Essigester = 1:1)

| Ber.: | C 76,97 | H 6,24 | N 6,19 |
|-------|---------|--------|--------|
| Gef.: | 76,95 | 6,38 | 6,26 |

(3) (3S,5R)-3-Allyl-5-[(4'-cyano-4-biphenylyl)oxymethyl]-1-(3-phenylpropyl)-2-pyrrolidinon

$R_f$-Wert: 0,60 (Kieselgel; Cyclohexan/Essigester = 1:1)

| Ber.: | C 79,97 | H 6,71 | N 6,22 |
|-------|---------|--------|--------|
| Gef.: | 80,10 | 7,00 | 6,00 |

(4) (3R,5S)-3-Allyl-5-[(4'-cyano-4-biphenylyl)oxymethyl]-2-pyrrolidinon

Schmelzpunkt: 99-101°C
$R_f$-Wert: 0,38 (Kieselgel; Cyclohexan/Essigester = 1:4)

| Ber.: | C 75,88 | H 6,06 | N 8,43 |
|-------|---------|--------|--------|
| Gef.: | 75,86 | 6,26 | 8,36 |

(5) (3R,5S)-3-Allyl-5-[[4-[(4-cyanophenyl)aminocarbonyl]phenyl]oxymethyl]-1-(3-phenylpropyl)-2-pyrrolidinon

$R_f$-Wert: 0,65 (Kieselgel; Methylenchlorid/Ethanol = 9:1)

(6) (3S,5S)-3-Allyl-5-[(4'-cyano-4-biphenylyl)oxymethyl]-2-pyrrolidinon

Schmelzpunkt: 169-171°C
$R_f$-Wert: 0,27 (Kieselgel; Essigester/Cyclohexan = 8:2)

| Ber.: | C 75,88 | H 6,06 | N 8,43 |
|-------|---------|--------|--------|
| Gef.: | 75,72 | 6,14 | 8,50 |

(7) (3R,5S)-3-Allyl-1-[2-(benzyloxy)ethyl]-5-[(4'-cyano-4-biphenylyl)oxymethyl]-2-pyrrolidinon

$R_f$-Wert: 0,39 (Kieselgel; Cyclohexan/Essigester = 1:1)

| Ber.: | C 77,23 | H 6,48 | N 6,00 |
|-------|---------|--------|--------|
| Gef.: | 76,97 | 6,57 | 5,97 |

(8) (3S,5R)-3-Allyl-5-[(4'-cyano-4-biphenylyl)oxymethyl]-2-pyrrolidinon

Schmelzpunkt: 99-101°C,
$R_f$-Wert: 0,42 (Kieselgel; Cyclohexan/Essigester = 1:4)

| Ber.: | C 75,88 | H 6,06 | N 8,43 |
|-------|---------|--------|--------|
| Gef.: | 75,75 | 6,09 | 8,17 |

(9) (3S,5S)-3-[(tert.Butyloxycarbonyl)methyl]-5-[(4'-cyano-4-biphenylyl)oxymethyl]-2-pyrrolidinon

Als Base wurde Kalium-tert.butylat verwendet.

Schmelzpunkt: 158-159°C,
$R_f$-Wert: 0,50 (Kieselgel; Essigester/Cyclohexan = 1:9)

| Ber.: | C 70,91 | H 6,45 | N 6,89 |
|-------|---------|--------|--------|
| Gef.: | 71,00 | 6,69 | 7,04 |

(10) (S)-5-[(4'-Cyano-4-biphenylyl)oxymethyl]-3,3-diallyl-2-pyrrolidinon

$R_f$-Wert: 0,44 (Kieselgel; Cyclohexan/Essigester = 1:1)

(11) (3R,S;4R,S)-3-Allyl-4-[(4'-cyano-4-biphenylyl)oxymethyl]-1-(4-methoxybenzyl)-1-pyrrolidinon

$R_f$-Wert: 0,22 (Kieselgel; Cyclohexan/Essigester = 2:1)

| Ber.: | C 76,97 | H 6,24 | N 6,19 |
|-------|---------|--------|--------|
| Gef.: | 77,10 | 6,47 | 5,98 |

(12) (3S,5S)-5-[(7-Cyano-9,10-dihydro-2-phenanthrenyl)oxymethyl]-3-[(methoxycarbonyl)methyl]-2-pyrrolidinon

$R_f$-Wert: 0,52 (Kieselgel; Methylenchlorid/Methanol/konz. wäßriges Ammoniak = 30:1:0,1)

(13) (3R,S;4R,S)-3-Allyl-4-[(4'-cyano-4-biphenylyl]oxymethyl]-1-(4-methoxybenzyl)-3-methyl-2-pyrrolidinon

$R_f$-Wert: 0,32 (Kieselgel; Cyclohexan/Essigester = 2:1)

<u>Beispiel CIX</u>

<u>(3R,5S)-3-Allyl-5-[(4'-cyano-4-biphenylyl)oxymethyl]-1-(3-phenylpropyl)-2-pyrrolidinon</u>

3,9 g 4-Cyano-4'-hydroxybiphenyl in 30 ml trockenem Dimethylformamid werden mit 9,8 g Cäsiumcarbonat versetzt und 2 Stunden bei Raumtemperatur kräftig gerührt. Dann werden 7,03 g (3R,5S)-3-Allyl-5-[(methansulfonyloxy)methyl]-1-(3-phenylpropyl)-2-pyrrolidinon in 20 ml Dimethylformamid dazugegeben und 18 Stunden bei 55-60°C gerührt. Danach wird abgekühlt, in 200 ml Wasser gegossen und dreimal mit Essigester ausgeschüttelt. Die vereinigten organischen Phasen werden mit Wasser gewaschen, über Natriumsulfat getrocknet und eingeengt. Nach Reinigung über eine Kieselgelsäule mit Cyclohexan/Essigester = 1:1 werden 6,9 g (76 % der Theorie) erhalten.

Schmelzpunkt: 74-76°C,
$R_f$-Wert: 0,47 (Kieselgel; Cyclohexan/Essigester = 1:1)

Analog werden erhalten:

(1) (3R,5S)-3-Allyl-5-[(3-brom-4'-cyano-4-biphenylyl)oxymethyl]-1-(3-phenylpropyl)-2-pyrrolidinon

$R_f$-Wert: 0,55 (Kieselgel; 1,2-Dichlorethan/Essigester = 3:1)

| Ber.: | C 68,05 | H 5,52 | N 5,29 | Br 15,09 |
|-------|---------|--------|--------|----------|
| Gef.: | 68,18 | 5,61 | 5,34 | 15,38 |

(2) (3R,5S)-3-Allyl-5-[(4'-cyano-3-nitro-4-biphenylyl)oxymethyl]-1-(3-phenylpropyl)-2-pyrrolidinon

$R_f$-Wert: 0,54 (Kieselgel; 1,2-Dichlorethan/Essigester = 3:1)

| Ber.: | C 72,71 | H 5,90 | N 8,48 |
|-------|---------|--------|--------|
| Gef.: | 72,45 | 5,79 | 8,24 |

(3) (3R,5S)-3-Allyl-5-[(4'-cyano-3-trifluormethyl-4-biphenylyl)oxymethyl]-1-(3-phenylpropyl)-2-pyrrolidinon

$R_f$-Wert: 0,49 (Kieselgel; Cyclohexan/Essigester = 1:1)

| Ber.: | C 71,80 | H 5,64 | N 5,40 |
|-------|---------|--------|--------|
| Gef.: | 71,51 | 5,79 | 5,06 |

(4) (3R,5S)-3-Allyl-5-[(4'-cyano-3-fluor-4-biphenylyl)oxymethyl]-1-(3-phenylpropyl)-2-pyrrolidinon

$R_f$-Wert: 0,51 (Kieselgel; Cyclohexan/Essigester = 1:1)

| Ber.: | C 76,90 | H 6,24 | N 5,98 |
|-------|---------|--------|--------|
| Gef.: | 77,10 | 6,59 | 5,94 |

(5) (3R,5S)-3-Allyl-5-[(4'-cyano-2,3-dimethyl-4-biphenylyl)oxymethyl]-1-(3-phenylpropyl)-2-pyrrolidinon

$R_f$-Wert: 0,65 (Kieselgel; Cyclohexan/Essigester = 2:1)

| Ber.: | C 80,30 | H 7,16 | N 5,85 |
|-------|---------|--------|--------|
| Gef.: | 79,98 | 7,32 | 5,59 |

(6) (3S,5S)-5-[(4'-Cyano-2'-methyl-4-biphenylyl)oxymethyl]-3-[(methoxycarbonyl)methyl]-1-(3-phenylpropyl)-2-pyrrolidinon

$R_f$-Wert: 0,39 (Kieselgel; Cyclohexan/Essigester = 1:1)

| Ber.: | C 74,97 | H 6,50 | N 5,64 |
|-------|---------|--------|--------|
| Gef.: | 74,70 | 6,51 | 5,64 |

(7) (3R,3S)-3-Allyl-5-[[4-[4-(tert.butyloxycarbonylamino)butyl]phenyl]oxymethyl]-1-(4-phenylbutyl)-2-pyrrolidinon

$R_f$-Wert: 0,58 (Kieselgel; Cyclohexan/Essigester = 1:1)

(8) (3R,5S)-3-Allyl-5-[(6-cyano-2-naphthyl)oxymethyl]-1-(3-phenylpropyl)-2-pyrrolidinon

$R_f$-Wert: 0,55 (Kieselgel; Cyclohexan/Essigester = 1:1)

(9) (3R,5S)-3-Allyl-1-benzyl-5-[[4-[4-(tert.butyloxycarbonylamino)butyl]phenyl]oxymethyl]-2-pyrrolidinon

$R_f$-Wert: 0,35 (Kieselgel; Cyclohexan/Essigester = 2:1)

(10) (3R,5S)-3-Allyl-5-[[4-[4-(tert.butyloxycarbonylamino)butyl]phenyl]oxymethyl]-1-(4-phenoxybutyl)-2-pyrrolidinon

$R_f$-Wert: 0,86 (Kieselgel; Essigester)

(11) (3R,5S)-3-Allyl-5-[[4-[4-(tert.butyloxycarbonylamino)butyl]phenyl]oxymethyl]-1-(2-phenylethyl)-2-pyrrolidinon

$R_f$-Wert: 0,26 (Kieselgel; Cyclohexan/Essigester = 2:1)

(12) (3R,5S)-3-Allyl-5-[[4-[3-(benzyloxycarbonylamino)propyl]phenyl]oxymethyl]-1-(3-phenylpropyl)-2-pyrrolidinon

$R_f$-Wert: 0,54 (Kieselgel; tert.Butyl-methylether/Petrolether = 4:1)

(13) (3R,5S)-3-Allyl-5-[[4-[(tert.butyloxycarbonylamino)methyl]phenyl)oxymethyl]-1-(3-phenylpropyl)-2-pyrrolidinon

$R_f$-Wert: 0,37 (Kieselgel; Methylenchlorid/Methanol = 20:1)

(14) (3R,5S)-3-Allyl-5-[[3-[(tert.butyloxycarbonylamino)methyl]phenyl]oxymethyl]-1-(3-phenylpropyl)-2-pyrrolidinon

$R_f$-Wert: 0,34 (Kieselgel; Methylenchlorid/Methanol = 20:1)

(15) (3R,5S)-3-Allyl-5-[[3-[4-(tert.butyloxycarbonylamino)butyl]phenyl]oxymethyl]-1-(3-phenylpropyl)-2-pyrrolidinon

$R_f$-Wert: 0,45 (Kieselgel; Methylenchlorid/Methanol = 20:1)

(16) (3R,5S)-3-Allyl-5-[[3-[2-(tert.butyloxycarbonylamino)ethyl]phenyl]oxymethyl]-1-(3-phenylpropyl)-2-pyrrolidinon

$R_f$-Wert: 0,39 (Kieselgel; Methylenchlorid/Methanol = 20:1)

(17) (3R,5S)-3-Allyl-5-[[4-[2-(tert.butyloxycarbonylamino)ethyl]phenyl]oxymethyl]-1-(3-phenylpropyl)-2-pyrrolidinon

$R_f$-Wert: 0,22 (Kieselgel; Methylenchlorid/Methanol = 50:1)

(18) (3R,5S)-3-Allyl-5-[[4-[2-(tert.butyloxycarbonylamino)ethyl]phenyl]oxymethyl]-1-isobutyl-2-pyrrolidinon

$R_f$-Wert: 0,33 (Kieselgel; Methylenchlorid/Methanol = 20:1)

(19) (3R,5S)-3-Allyl-5-[[4-[cis-4-(tert.butyloxycarbonylamino)cyclohexyl]phenyl]oxymethyl]-1-(3-phenylpropyl)-2-pyrrolidinon

$R_f$-Wert: 0,77 (Kieselgel; Methylenchlorid/Methanol = 9:1)

(20) (3R,5S)-3-Allyl-5-[[4-[trans-4-(tert.butyloxycarbonylamino)cyclohexyl]phenyl]oxymethyl]-1-(3-phenylpropyl)-2-pyrrolidinon

$R_f$-Wert: 0,79 (Kieselgel; Methylenchlorid/Methanol = 9:1)

(21) (3R,5S)-3-Allyl-5-[(4'-cyano-4-biphenylyl)oxymethyl]-3-methyl-1-(3-phenylpropyl)-2-pyrrolidinon

$R_f$-Wert: 0,38 (Kieselgel; Cyclohexan/Essigester = 2:1)

(22) (3R,5S)-3-Allyl-1-benzyl-5-[(4'-cyano-4-biphenylyl)oxymethyl]-2-pyrrolidinon

$R_f$-Wert: 0,41 (Kieselgel; Cyclohexan/Essigester = 2:1)

(23) (3R,5S)-3-Allyl-5-[(4'-cyano-4-biphenylyl)oxymethyl]-1-(2-phenylethyl)-2-pyrrolidinon

$R_f$-Wert: 0,39 (Kieselgel; Cyclohexan/Essigester = 2:1)

(24) (3R,5S)-3-Allyl-5-[(4'-cyano-4-biphenylyl)oxymethyl]-1-(4-phenylbutyl)-2-pyrrolidinon

$R_f$-Wert: 0,35 (Kieselgel; Cyclohexan/Essigester = 2:1)

(25) (3R,5S)-3-Allyl-5-[(4'-cyano-4-biphenylyl)oxymethyl]-1-(4-phenyloxybutyl)-2-pyrrolidinon

$R_f$-Wert: 0,38 (Kieselgel; Cyclohexan/Essigester = 2:1)

(26) (3R,5S)-3-Allyl-5-[(4'-cyano-4-biphenylyl)oxymethyl]-1,3-bis-(3-phenylpropyl)-2-pyrrolidinon

$R_f$-Wert: 0,60 (Kieselgel; Cyclohexan/Essigester = 2:1)

(27) (3R,5S)-3-Allyl-3-(n-butyl)-5-[(4'-cyano-4-biphenylyl)oxymethyl]-1-(3-phenylpropyl)-2-pyrrolidinon

$R_f$-Wert: 0,66 (Kieselgel; Cyclohexan/Essigester = 2:1)

(28) (3R,5S)-3-Allyl-5-[[4-(3-cyanopropyl)phenyl]oxymethyl]-3-methyl-1-(3-phenylpropyl)-2-pyrrolidinon

$R_f$-Wert: 0,36 (Kieselgel; Cyclohexan/Essigester = 2:1)

(29) (3R,5S)-3-Allyl-5-[[4-(3-cyanopropyl)phenyl]oxymethyl]-1-(3-phenylpropyl)-2-pyrrolidinon

$R_f$-Wert: 0,39 (Kieselgel; Cyclohexan/Essigester = 2:1)

(30) (3R,5S)-3-Allyl-1-[3-(4-benzyloxyphenyl)propyl]-5-[[4-[4-(tert.butyloxycarbonylamino)butyl]phnoxy]oxymethyl]-2-pyrrolidinon

$R_f$-Wert: 0,55 (Kieselgel; Toluol/Aceton = 2:1)

(31) (3R,5S)-3-Allyl-5-[(2-cyano-5-indanyl)oxymethyl]-1-(3-phenylpropyl)-2-pyrrolidinon

$R_f$-Wert: 0,32 (Kieselgel; Cyclohexan/Essigester = 2:1)

(32) (3S,5S)-5-[(7-Cyano-9-keto-2-fluorenyl)oxymethyl]-3-[(methoxycarbonyl)methyl]-1-(3-phenylpropyl)-2-pyrrolidinon

$R_f$-Wert: 0,55 (Kieselgel; Essigester/Cyclohexan = 1:1) (nach zweimaligem Lauf)

(33) (3S,5S)-3-Allyl-5-[(4'-cyano-4-biphenylyl)oxymethyl]-1-3-phenylpropyl)-2-pyrrolidinon
Hergestellt aus den Mutterlaugen der Herstellung des (3R,5S)Isomeren.

$R_f$-Wert: 0,55 (Kieselgel; Ether/konz. wäßriges Ammoniak = 10:0,1)

(34) (3R,5S)-3-Allyl-5-[[3-[3-(tert.butyloxycarbonylamino)propyl]phenyl]oxymethyl]-1-(3-phenylpropyl)-2-pyrrolidinon

$R_f$-Wert: 0,36 (Kieselgel; Cyclohexan/Essigester = 2:1)

(35) (3R,5S)-3-Allyl-5-[[4-[5-(tert.butyloxycarbonylamino)pentyl]phenyl]oxymethyl]-(3-phenylpropyl)-2-pyrrolidinon

$R_f$-Wert: 0,50 (Kieselgel; Cyclohexan/Essigester = 2:1)

(36) (3R,S;5R,S)-3-Allyl-5-[[4-[(tert.butyloxycarbonylamino)methyl]phenyl]oxymethyl]-3-methyl-1-(3-phenylpropyl)-2-pyrrolidinon

$R_f$-Wert: 0,44 (Kieselgel; Cyclohexan/Essigester = 2:1)

(37) (3R,S;5R,S)-3-Allyl-5-[[4-[2-(tert.butyloxycarbonylamino)ethyl]phenyl]oxymethyl]-3-methyl-1-(3-phenylpropyl)-2-pyrrolidinon

R$_f$-Wert: 0,39 (Kieselgel; Cyclohexan/Essigester = 2:1)

(38) (3R,S;5R,S)-3-Allyl-5-[[4-[4-(tert.butyloxycarbonylamino)butyl]phenyl]oxymethyl]-3-methyl-1-(3-phenylpropyl)-2-pyrrolidinon

R$_f$-Wert: 0,35 (Kieselgel; Cyclohexan/Essigester = 2:1)

(39) (3R,5S)-3-Allyl-5-[[4-[(4-cyanophenyl)carbonylamino]phenyl]oxymethyl]-1-(3-phenylpropyl)-2-pyrrolidinon

R$_f$-Wert: 0,63 (Kieselgel; Essigester/Cyclohexan = 3:1)

(40) (3R,5S)-3-Allyl-5-[[4-[(4-cyanophenyl)aminosulfonyl]phenyl]oxymethyl]-1-(3-phenylpropyl)-2-pyrrolidinon

R$_f$-Wert: 0,84 (Kieselgel; Essigester/Cyclohexan = 4:1)

(41) (3S,5S)-5-[[4-[(3-Cyanophenyl)sulfenyl]phenyl]oxymethyl]-3-[(methoxycarbonyl)methyl]-1-(3-phenylpropyl)2-pyrrolidinon

R$_f$-Wert: 0,53 (Kieselgel; Essigester/Cyclohexan = 1:1)

(42) (3S,5S)-5-[[4-[(3-Cyanophenyl)carbonyl]phenyl]oxymethyl]-3-[(methoxycarbonyl)methyl]-1-(3-phenylpropyl)2-pyrrolidinon

R$_f$-Wert: 0,36 (Kieselgel; Essigester/Cyclohexan = 1:1)

(43) (3S,5S)-5-[(4'-Cyano-3-methylsulfenyl-4-biphenylyl)oxymethyl]-3-[(methoxycarbonyl)methyl]-1-(3-phenylpropyl)-2-pyrrolidinon

R$_f$-Wert: 0,75 (Kieselgel; Methylenchlorid/Methanol = 15:1)

(44) (3R,5S)-5-[(4'-Cyano-4-biphenylyl)oxymethyl]-3-[(methoxycarbonyl)propyl]-1-(3-phenylpropyl)-2-pyrrolidinon

R$_f$-Wert: 0,40 (Kieselgel; Methylenchlorid/Methanol = 19:1)

(45) (3S,5S)-3-[(tert.Butyloxycarbonyl)methyl]-5-[(4'-cyano-3'-fluor-4-biphenylyl]oxymethyl]-2-pyrrolidinon

Schmelzpunkt: 142-144°C
R$_f$-Wert: 0,29 (Kieselgel; Methylenchlorid/Essigester = 8:2)

| Ber.: | C 67,91 | H 5,94 | N 6,60 |
|-------|---------|--------|--------|
| Gef.: | 67,79   | 5,94   | 6,64   |

(46) (3S,5S)-3-[(tert.Butyloxycarbonyl)methyl]-5-[(3'-chlor-4'-cyano-4-biphenylyl]oxymethyl]-2-pyrrolidinon

Schmelzpunkt: 140-142°C
R$_f$-Wert: 0,36 (Kieselgel; Cyclohexan/Essigester = 3:7)

| Ber.: | C 65,38 | H 5,72 | N 6,35 | Cl 8,04 |
|-------|---------|--------|--------|---------|
| Gef.: | 65,32   | 5,75   | 6,41   | 8,11    |

(47) (3S,5S)-5-[(3-Brom-4'-cyano-4-biphenylyl]oxymethyl]-3-[(methoxycarbonyl)methyl]-2-pyrrolidinon

R$_f$-Wert: 0,52 (Kieselgel; Methylenchlorid/Methanol = 15:1)

| Ber.: | C 56,90 | H 4,32 | N 6,32 | Br 18,03 |
|-------|---------|--------|--------|----------|
| Gef.: | 56,78   | 4,41   | 6,17   | 17,92    |

Beispiel CX

(3R,S;5S,R)-3-Allyl-5-[4-[(4-cyanobutyl)oxy]phenyl]-1-(3-phenylpropyl)-2-pyrrolidinon

Eine Mischung aus 3,6 g (3R,S;5S,R)-3-Allyl-5-(4-hydroxyphenyl)-1-(3-phenylpropyl)-2-pyrrolidinon, 1,8 g 5-Bromvaleriansäurenitril, 7,6 g Kaliumcarbonat und 100 ml Aceton wird 24 Stunden unter Rückfluß gekocht. Nach dem Abkühlen wird der Ansatz filtriert und das Filtrat im Vakuum einrotiert. Der ölige Rückstand wird über eine Kieselgelsäule mit Chloroform chromatographiert.

Ausbeute: 3,9 g (88,3 % der Theorie),
$R_f$-Wert: 0,10 (Kieselgel; Chloroform)

| Ber.: | C 77,85 | H 7,74 | N 6,73 |
|-------|---------|--------|--------|
| Gef.: | 77,56   | 7,84   | 6,66   |

Beispiel CXI

(3S,5S)-5-[(4'-Cyano-4-biphenylyl)oxymethyl]-3-[(methoxycarbonyl)methyl]-1-(3-phenylpropyl)-2-pyrrolidinon

Zu einer Mischung aus 203 g (3R,5S)-3-Allyl-5-[(4'-cyano-4-biphenylyl)oxymethyl]-1-(3-phenylpropyl)-2-pyrrolidinon, 1800 ml Methylenchlorid, 1800 ml Acetonitril und 2 g Rutheniumtrichlorid-trihydrat wird innerhalb von 1,75 Stunden eine Lösung von 580 g Natriummetaperjodat in 3200 ml Wasser unter gutem Rühren zugetropft, wobei die Temperatur zwischen 25 und 31°C gehalten wird. Man rührt noch 4 Stunden nach und versetzt mit weiteren 1800 ml Methylenchlorid. Die organische Phase wird abgetrennt und nacheinander mit 1000 ml Wasser, 1000 ml 10%iger Natriumdisulfitlösung und 1000 ml Wasser gewaschen. Die wäßrigen Phasen werden noch zweimal mit je 350 ml Methylenchlorid extrahiert. Die vereinigten Methylenchloridphasen werden mit Magnesiumsulfat getrocknet, mit Aktivkohle behandelt und eingeengt. Der verbleibende schaumige Rückstand von (3S,5S)-3-Carboxymethyl-5-[(4'-cyano-4-biphenylyl)oxymethyl]-1-(3-phenylpropyl)-2-pyrrolidinon wird in dieser Form weiter verwendet.

Ausbeute: 208 g (84 % der Theorie),
$R_f$-Wert: 0,66 (Kieselgel; Methylenchlorid/Methanol = 15:1) (nach zweimaliger Entwicklung)

Das oben erhaltene Rohprodukt wird in 1200 ml Methanol gelöst, mit 10 ml gesättigter methanolischer Salzsäure versetzt und 16 Stunden bei Raumtemperatur gerührt. Man engt ein und reinigt den Rückstand durch Chromatographie über Kieselgel (Elutionsmittel: Cyclohexan/Essigester = 1,5:1). Das nach dem Abdampfen des Elutionsmittels verbleibende ölige Produkt wird mit 750 ml Ether und Impfkristallen verrührt. Das erhaltene Kristallisat wird abfiltriert und zweimal mit je 150 ml Ether gewaschen. Aus den Mutterlaugen erhält man eine weitere Fraktion.

Ausbeute: 147 g (67 % der Theorie über beide Stufen),
Schmelzpunkt: 75-76°C

Analog werden erhalten:

(1)   (3S,5S)-5-[(4'-Cyano-4-biphenylyl)oxymethyl]-1-(3-cyclohexylpropyl)-3-[(methoxycarbonyl)methyl]-2-pyrrolidinon

$R_f$-Wert: 0,59 (Kieselgel; Cyclohexan/Essigester = 4:6)

| Ber.: | C 73,74 | H 7,43 | N 5,73 |
|---|---|---|---|
| Gef.: | 73,52 | 7,60 | 5,46 |

Zwischenprodukt:

(3S,5S)-3-Carboxymethyl-5-[(4'-cyano-4-biphenylyl)oxymethyl]-1-(3-cyclohexylpropyl)-2-pyrrolidinon
$R_f$-Wert: 0,24 (Kieselgel; Cyclohexan/Essigester = 4:6)

(2) (3S,5S)-5-[(4'-Cyano-4-biphenylyl)oxymethyl]-3-[(methoxycarbonyl)methyl]-2-pyrrolidinon

Schmelzpunkt: 140-142°C
$R_f$-Wert: 0,42 (Kieselgel; Methylenchlorid/Methanol = 100:2)

| Ber.: | C 69,22 | H 5,53 | N 7,69 |
|---|---|---|---|
| Gef.: | 68,95 | 5,59 | 7,50 |

Zwischenprodukt:

(3S,5S)-3-Carboxymethyl-5-[(4'-cyano-4-biphenylyl)oxymethyl]-2-pyrrolidinon
$R_f$-Wert: 0,35 (Kieselgel; 1,2-Dichloräthan/Essigester/Eisessig = 100:30:5)

(3) (3S,5S)-5-[(4'-Cyano-4-biphenylyl)oxymethyl]-3-[(methoxycarbonyl)methyl]-1-methyl-2-pyrrolidinon

$R_f$-Wert: 0,41 (Kieselgel; Methylenchlorid/Methanol = 100:2)

| Ber.: | C 69,83 | H 5,86 | N 7,40 |
|---|---|---|---|
| Gef.: | 69,60 | 6,10 | 7,20 |

(4) (3S,5S)-5-[(3-Brom-4'-cyano-4-biphenylyl)oxymethyl]-3-[(methoxycarbonyl)methyl]-1-(3-phenylpropyl)-2-pyrrolidinon

$R_f$-Wert: 0,62 (Kieselgel; Methylenchlorid/Essigester = 4:1)

| Ber.: | C 64,17 | H 5,21 | N 4,99 |
|---|---|---|---|
| Gef.: | 64,20 | 5,43 | 5,06 |

Zwischenprodukt:

(3S,5S)-5-[(3-Brom-4'-cyano-4-biphenylyl)oxymethyl]-3-carboxymethyl-1-(3-phenylpropyl)-2-pyrrolidinon
$R_f$-Wert: 0,75 (Kieselgel; Essigester/Methanol = 9:1)

(5) (3S,5S)-5-[(4'-Cyano-3-nitro-4-biphenylyl)oxymethyl]-3-[(methoxycarbonyl)methyl]-1-(3-phenylpropyl)-2-pyrrolidinon

$R_f$-Wert: 0,53 (Kieselgel; 1,2-Dichlorethan/Essigester = 3:1)

| Ber.: | C 68,30 | H 5,54 | N 7,97 |
|---|---|---|---|
| Gef.: | 68,28 | 5,34 | 7,75 |

Zwischenprodukt:

(3S,5S)-3-Carboxymethyl-5-[(4'-cyano-3-nitro-4-biphenylyl)oxymethyl]-1-(3-phenylpropyl)-2-pyrrolidinon
$R_f$-Wert: 0,51 (Kieselgel; Essigester/Methanol = 10:1)

(6) (3R,5R)-5-[(4'-Cyano-4-biphenylyl)oxymethyl]-3-[(methoxycarbonyl)methyl]-1-(3-phenylpropyl)-2-pyrrolidinon

$R_f$-Wert: 0,70 (Kieselgel; Cyclohexan/Essigester = 2:3)

| Ber.: | C 74,66 | H 6,27 | N 5,81 |
|---|---|---|---|
| Gef.: | 74,51 | 6,47 | 5,63 |

(7) (3S,5S)-5-[(4'-Cyano-3-trifluormethyl-4-biphenylyl)oxymethyl]-3-[(methoxycarbonyl)methyl]-1-(3-phenylpropyl)-2-pyrrolidinon

$R_f$-Wert: 0,45 (Kieselgel; Cyclohexan/Essigester = 1:1)

| Ber.: | C 67,62 | H 5,31 | N 5,09 |
|---|---|---|---|
| Gef.: | 67,73 | 5,48 | 5,08 |

Zwischenprodukt:

(3S,5S)-3-Carboxymethyl-5-[(4'-cyano-3-trifluormethyl-4-biphenylyl)oxymethyl]-1-(3-phenylpropyl)-2-pyrrolidinon
$R_f$-Wert: 0,58 (Kieselgel; Essigester/Methanol = 20:1)

(8) (3S,5S)-5-[(4'-Cyano-3-fluor-4-biphenylyl)oxymethyl]-3-[(methoxycarbonyl)methyl]-1-(3-phenylpropyl)-2-pyrrolidinon

$R_f$-Wert: 0,34 (Kieselgel; Cyclohexan/Essigester = 1:1)

Zwischenprodukt:

(3S,5S)-3-Carboxymethyl-5-[(4'-cyano-3-fluor-4-biphenylyl)oxymethyl]-1-(3-phenylpropyl)-2-pyrrolidinon
$R_f$-Wert: 0,61 (Kieselgel; Essigester/Methanol = 20:1)

(9) (3S,5S)-5-[(4'-Cyano-2,3-dimethyl-4-biphenylyl)oxymethyl]-3-[(methoxycarbonyl)methyl]-1-(3-phenylpropyl)2-pyrrolidinon

$R_f$-Wert: 0,50 (Kieselgel; Cyclohexan/Essigester = 4:1)

| Ber.: | C 75,27 | H 6,71 | N 5,49 |
|---|---|---|---|
| Gef.: | 74,96 | 7,00 | 5,37 |

Zwischenprodukt:

(3S,5S)-3-Carboxymethyl-5-[(4'-cyano-2,3-dimethyl-4-biphenylyl)oxymethyl]-1-(3-phenylpropyl)-2-pyrrolidinon
$R_f$-Wert: 0,73 (Kieselgel; Essigester/Methanol = 19:1)

(10) (3S,5S)-5-[[trans-4-(4-Cyanophenyl)cyclohexyl]oxymethyl]-3-[(methoxycarbonyl)methyl]-1-(3-phenylpropyl)2-pyrrolidinon-semihydrat

$R_f$-Wert: 0,52 (Kieselgel; Cyclohexan/Essigester = 1:1)

| Ber.: | C 72,41 | H 7,50 | N 5,63 |
|---|---|---|---|
| Gef.: | 72,35 | 7,61 | 5,62 |

(11) (3S,5S)-5-[(4'-Cyano-4-biphenylyl)oxymethyl]-3-[(methoxycarbonyl)methyl]-3-methyl-1-(3-phenylpropyl)-2-pyrrolidinon

$R_f$-Wert: 0,74 (Kieselgel; Methylenchlorid/Methanol = 15:1)

Zwischenprodukt:

(3S,5S)-3-Carboxymethyl-5-[(4'-cyano-4-biphenylyl)oxymethyl]-3-methyl-1-(3-phenylpropyl)-2-pyrrolidinon
$R_f$-Wert: 0,40 (Kieselgel; Methylenchlorid/Cyclohexan/Metha nol/konz. wäßriges Ammoniak = 68:15:15:2)

(12) (3S,5S)-1-Benzyl-5-[(4'-cyano-4-biphenylyl)oxymethyl]-3-[(methoxycarbonyl)methyl]-2-pyrrolidinon

$R_f$-Wert: 0,69 (Kieselgel; Methylenchlorid/Methanol = 15:1)

Zwischenprodukt:

(3S,5S)-1-Benzyl-3-carboxymethyl-5-[(4'-cyano-4-biphenylyl)oxymethyl]-2-pyrrolidinon
$R_f$-Wert: 0,38 (Kieselgel; Methylenchlorid/Cyclohexan/Metha nol/konz. wäßriges Ammoniak = 68:15:15:2)

(13) (3S,5S)-5-[(4'-Cyano-4-biphenylyl)oxymethyl]-3-[(methoxycarbonyl)methyl]-1-(2-phenylethyl)-2-pyrrolidinon

$R_f$-Wert: 0,44 (Kieselgel; Cyclohexan/Essigester/Methanol = 64:32:4)

Zwischenprodukt:

(3S,5S)-3-Carboxymethyl-5-[(4'-cyano-4-biphenylyl)oxymethyl]-1-(2-phenylethyl)-2-pyrrolidinon
$R_f$-Wert: 0,41 (Kieselgel; Methylenchlorid/Cyclohexan/Metha nol/konz. wäßriges Ammoniak = 68:15:15:2)

(14) (3S,5S)-5-[(4'-Cyano-4-biphenylyl)oxymethyl]-3-[(methoxycarbonyl)methyl]-1-(4-phenylbutyl)-2-pyrrolidinon

$R_f$-Wert: 0,41 (Kieselgel; Cyclohexan/Essigester/Methanol = 64:32:4)

Zwischenprodukt:

(3S,5S)-3-Carboxymethyl-5-[(4'-cyano-4-biphenylyl)oxymethyl]-1-(4-phenylbutyl)-2-pyrrolidinon
$R_f$-Wert: 0,38 (Kieselgel; Methylenchlorid/Cyclohexan/Metha nol/konz. wäßriges Ammoniak = 68:15:15:2)

(15) (3S,5S)-5-[(4'-Cyano-4-biphenylyl)oxymethyl]-3-[(methoxycarbonyl)methyl]-1-(4-phenyloxybutyl)-2-pyrrolidinon

$R_f$-Wert: 0,41 (Kieselgel; Cyclohexan/Essigester/Methanol = 64:32:4)

Zwischenprodukt:

(3S,5S)-3-Carboxymethyl-5-[(4'-cyano-4-biphenylyl)oxymethyl]-1-(4-phenyloxybutyl)-2-pyrrolidinon

$R_f$-Wert: 0,34 (Kieselgel; Methylenchlorid/Cyclohexan/Metha nol/konz. wäßriges Ammoniak = 68:15:15:2)

(16) (3S,5S)-5-[(4'-Cyano-4-biphenylyl)oxymethyl]-3-[(methoxycarbonyl)methyl]-1,3-bis-(3-phenylpropyl)-2-pyrrolidinon

$R_f$-Wert: 0,41 (Kieselgel; Cyclohexan/Essigester = 2:1)

Zwischenprodukt:

(3S,5S)-3-Carboxymethyl-5-[(4'-cyano-4-biphenylyl)oxymethyl]-1,3-bis-(3-phenylpropyl)-2-pyrrolidinon
$R_f$-Wert: 0,41 (Kieselgel; Methylenchlorid/Cyclohexan/Metha nol/konz. wäßriges Ammoniak = 68:15:15:2)

(17) (3S,5S)-3-(n-Butyl)-5-[(4'-cyano-4-biphenylyl)oxymethyl]-3-[(methoxycarbonyl)methyl]-1-(3-phenylpropyl)2-pyrrolidinon

$R_f$-Wert: 0,42 (Kieselgel; Cyclohexan/Essigester = 2:1)

Zwischenprodukt:

(3S,5S)-3-(n-Butyl)-3-carboxymethyl-5-[(4'-cyano-4-biphenylyl)oxymethyl]-1-(3-phenylpropyl)-2-pyrrolidinon
$R_f$-Wert: 0,41 (Kieselgel; Methylenchlorid/Cyclohexan/Metha nol/konz. wäßriges Ammoniak = 68:15:15:2)

(18) (3R,5S)-5-[(4'-Cyano-4-biphenylyl)oxymethyl]-3-[(methoxycarbonyl)methyl]-1-(3-phenylpropyl)-2-pyrrolidinon

$R_f$-Wert: 0,59 (Kieselgel; Methylenchlorid/Methanol = 19:1)

Zwischenprodukt:

(3R,5S)-3-Carboxymethyl-5-[(4'-cyano-4-biphenylyl)oxymethyl]-1-(3-phenylpropyl)-2-pyrrolidinon
$R_f$-Wert: 0,52 (Kieselgel; Methylenchlorid/Methanol/konz. wäßriges Ammoniak = 8:2:0,1

(19) (3S,5S)-5-[[4-[(4-Cyanophenyl)carbonylamino]phenyl]oxymethyl]-3-[(methoxycarbonyl)methyl]-1-(3-phenylpropyl)-2-pyrrolidinon

$R_f$-Wert: 0,35 (Kieselgel; Essigester/Cyclohexan = 1:1)

(20) (3S,5S)-5-[[4-[(4-Cyanophenyl)aminosulfonyl]phenyl]oxymethyl]-3-[(methoxycarbonyl)methyl]-1-(3-phenylpropyl)-2-pyrrolidinon

$R_f$-Wert: 0,28 (Kieselgel; Essigester/Cyclohexan = 3:1)

(21) (3S,5S)-5-[(4'-Cyano-4-biphenylyl)oxymethyl]-3-[(methoxycarbonyl)methyl]-3-methyl-2-pyrrolidinon

Schmelzpunkt: 105-107,5°C,
$R_f$-Wert: 0,27 (Kieselgel; Cyclohexan/Essigester = 1:4)

| Ber.: | C 69,83 | H 5,86 | N 7,40 |
|---|---|---|---|
| Gef.: | 69,64 | 5,73 | 7,70 |

Zwischenprodukt:

(3S,5S)-3-Carboxymethyl-5-[(4'-cyano-4-biphenylyl)oxymethyl]-3-methyl-2-pyrrolidinon
Schmelzpunkt: 178-181°C,
$R_f$-Wert: 0,41 (Kieselgel; Toluol/Dioxan/Ethanol/Eisessig = 90:10:10:6)

(22) (3S,5S)-5-[(4'-Cyano-4-biphenylyl)oxymethyl]-3-[(methoxycarbonyl)methyl]-1-(2-methoxyethyl)-2-pyrrolidinon

$R_f$-Wert: 0,55 (Kieselgel; Toluol/Dioxan/Ethanol/Eisessig = 90:10:10:6)

| Ber.: | C 68,23 | H 6,20 | N 6,63 |
|-------|---------|--------|--------|
| Gef.: | 68,02 | 6,22 | 6,55 |

Zwischenprodukt:

(3S,5S)-3-Carboxymethyl-5-[(4'-cyano-4-biphenylyl)oxymethyl]-1-(2-methoxyethyl)-2-pyrrolidinon
$R_f$-Wert: 0,43 (Kieselgel; Toluol/Dioxan/Ethanol/Eisessig = 90:10:10:6)

(23) (3S,5S)-1-(2-Acetoxyethyl)-5-[(4'-cyano-4-biphenylyl)oxymethyl]-3-[(methoxycarbonyl)methyl]-2-pyrrolidinon

$R_f$-Wert: 0,26 (Kieselgel; Essigester)

(24) (3R,5R)-5-[(4'-Cyano-4-biphenylyl)oxymethyl]-3-[(methoxycarbonyl)methyl]-2-pyrrolidinon

Schmelzpunkt: 138-140°C,
$R_f$-Wert: 0,51 (Kieselgel; Methylenchlorid/Methanol = 100:2)

| Ber.: | C 69,22 | H 5,53 | N 7,69 |
|-------|---------|--------|--------|
| Gef.: | 68,98 | 5,51 | 7,62 |

(25) (3R,S;4R,S)-4-[(4'-Cyano-4-biphenylyl)oxymethyl]-3-[(methoxycarbonyl)methyl]-2-pyrrolidinon

$R_f$-Wert: 0,29 (Kieselgel; Methylenchlorid/Methanol = 98:2)

(26) (3R,S;4R,S)-4-[[(4'-Cyano-4-biphenylyl)carbonylamino]methyl]-3-[(methoxycarbonyl)methyl]-2-pyrrolidinon

$R_f$-Wert: 0,16 (Kieselgel; Essigester/Methanol = 97:3)

(27) (3R,S;4R,S)-4-[(4'-Cyano-4-biphenylyl)oxymethyl]-3-[(methoxycarbonyl)methyl]-3-methyl-2-pyrrolidinon

Schmelzpunkt: 163-164°C
$R_f$-Wert: 0,39 (Kieselgel; Essigester)

| Ber.: | C 69,82 | H 5,86 | N 7,40 |
|-------|---------|--------|--------|
| Gef.: | 70,00 | 6,10 | 7,55 |

Beispiel CXII

(3S,R;5S,R)-3-Carboxymethyl-5-[4-[(4-cyanobutyl)oxy]phenyl]-1-(3-phenylpropyl)-2-pyrrolidinon

Hergestellt analog Beispiel XI durch Oxidation von (3R,S;5S,R)-3-Allyl-5-[4-[(4-cyanobutyl)oxy]phenyl]-1(3-phenylpropyl)-2-pyrrolidinon.

$R_f$-Wert: 0,78 (Kieselgel; Methylenchlorid/Methanol = 10:1)

| Ber.: | C 71,86 | H 6,96 | N 6,45 |
|-------|---------|--------|--------|
| Gef.: | 71,62 | 6,68 | 6,42 |

Analog werden erhalten:

(1) (3S,5S)-3-Carboxymethyl-5-[(6-cyano-2-naphthyl)oxymethyl]-1-(3-phenylpropyl)-2-pyrrolidinon

$R_f$-Wert: 0,49 (Kieselgel; Methylenchlorid/Methanol = 9:1)

(2) (3S,5S)-3-Carboxymethyl-5-[(4'-cyano-4-biphenylyl)oxymethyl]-1-(3-phenylpropyl)-2-pyrrolidinon

$R_f$-Wert: 0,44 (Kieselgel; Methylenchlorid/Methanol = 9:1)

(3) (3S,5S)-3-Carboxymethyl-5-[[(3-cyanophenyl)carbonylamino]methyl]-1-(3-phenylpropyl)-2-pyrrolidinon

$R_f$-Wert: 0,55 (Kieselgel; Methylenchlorid/Methanol = 4:1)

(4) (3S,5S)-3-Carboxymethyl-5-[[(4-cyanophenyl)carbonylamino]methyl]-1-(3-phenylpropyl)-2-pyrrolidinon

Schmelzpunkt: 184-188°C
$R_f$-Wert: 0,67 (Kieselgel; Methylenchlorid/Methanol = 4:1)

(5)    (3S,5S)-3-Carboxymethyl-5-[[[3-(4-cyanophenyl)propyl]carbonylamino]methyl]-1-(3-phenylpropyl)-2-pyrrolidinon

$R_f$-Wert: 0,50 (Kieselgel; Methylenchlorid/Methanol/konz. wäßriges Ammoniak = 4:1:0,1)

(6)    (3S,5S)-3-Carboxymethyl-5-[[[3-(3-cyanophenyl)propyl]carbonylamino]methyl]-1-(3-phenylpropyl)-2-pyrrolidinon

$R_f$-Wert: 0,24 (Kieselgel; Methylenchlorid/Methanol/konz. wäßriges Ammoniak = 4:1:0,1)

(7) (3S,5S)-3-Carboxymethyl-5-[[4-(3-cyanopropyl)phenyl]oxymethyl]-3-methyl-1-(3-phenylpropyl)-2-pyrrolidinon

$R_f$-Wert: 0,22 (Kieselgel; Methylenchlorid/Cyclohexan/Methanol/konz. wäßriges Ammoniak = 68:15:15:2)

(8) (3S,5S)-3-Carboxymethyl-5-[[4-(3-cyanopropyl)phenyl]oxymethyl]-1-(3-phenylpropyl)-2-pyrrolidinon

$R_f$-Wert: 0,25 (Kieselgel; Methylenchlorid/Cyclohexan/Methanol/konz. wäßriges Ammoniak = 68:15:15:2)

(9) (3S,5S)-3-Carboxymethyl-5-[(2-cyano-5-indanyl)oxymethyl]-1-(3-phenylpropyl)-2-pyrrolidinon

$R_f$-Wert: 0,31 (Kieselgel; Methylenchlorid/Cyclohexan/Methanol/konz. wäßriges Ammoniak = 68:15:15:2)

(10)    (3S,5S)-3-Carboxymethyl-5-[[4-[(4-cyanophenyl)aminocarbonyl]phenyl]oxymethyl]-1-(3-phenylpropyl)-2-pyrrolidinon

$R_f$-Wert: 0,50 (Kieselgel; Methylenchlorid/Ethanol = 9:1)

(11) (3S,5S)-1-[(Aminocarbonyl)methyl]-3-carboxymethyl-5-[(4'-cyano-4-biphenylyl)oxymethyl]-2-pyrrolidinon

$R_f$-Wert: 0,28 (Kieselgel; Essigester/Cyclohexan/Eisessig = 40:5:2)

(12) (3S,5S)-3-Carboxymethyl-5-[(4'-cyano-4-biphenylyl)oxymethyl]-1-[(ethylaminocarbonyl)methyl]-2-pyrrolidinon

Schmelzpunkt: 194-198°C,
$R_f$-Wert: 0,23 (Kieselgel; Essigester/Eisessig = 50:1)

(13) (3S,5S)-3-Carboxymethyl-5-[(4'-cyano-4-biphenylyl)oxymethyl]-1-[(dimethylaminocarbonyl)methyl]-2-pyrrolidinon

$R_f$-Wert: 0,16 (Kieselgel; Essigester/Eisessig = 50:1)

(14) (3S,5S)-1-[(Benzylaminocarbonyl)methyl]-3-carboxymethyl-5-[(4'-cyano-4-biphenylyl)oxymethyl]-2-pyrrolidinon

$R_f$-Wert: 0,34 (Kieselgel; Cyclohexan/Essigester = 8:3)

(15) (3S,5S)-1-(tert.Butyloxycarbonyl)-3-carboxymethyl-5-[(4'-cyano-4-biphenylyl)oxymethyl]-pyrrolidin

Schmelzpunkt: 132-135°C,

$R_f$-Wert: 0,52 (Kieselgel; Cyclohexan/Essigester = 1:1)

| Ber.: | C 68,79 | H 6,47 | N 6,42 |
|---|---|---|---|
| Gef.: | 68,72 | 6,58 | 6,47 |

(16) (3R,5S)-3-Carboxymethyl-5-[(4'-cyano-4-biphenylyl)oxymethyl]-2-pyrrolidinon

Schmelzpunkt: 223-227°C (Zers.),
$R_f$-Wert: 0,41 (Kieselgel; Toluol/Dioxan/Ethanol/Eisessig = 90:10:10:6)

| Ber.: | C 68,56 | H 5,18 | N 8,00 |
|---|---|---|---|
| Gef.: | 68,30 | 5,19 | 7,89 |

Beispiel CXIII

(3R,5S)-3-Allyl-5-[(4'-cyano-4-biphenylyl)oxymethyl]-2-pyrrolidinon

4,2 g (3R,5S)-3-Allyl-5-[(4'-cyano-4-biphenylyl)oxymethyl]-1-(4-methoxybenzyl)-2-pyrrolidinon werden in 30 ml Acetonitril und 10 ml Wasser suspendiert. Dazu wird eine Mischung aus 15,3 g pulverisiertem Cer(IV)-ammoniumnitrat und 15,3 g Kieselgel (Korngröße: 0,03-0,06 mm) gegeben. Nachdem 30 Minuten bei Raumtemperatur gerührt wurde, wird mit Methylenchlorid verdünnt und von den ungelösten Bestandteilen abgenutscht. Das Filtrat wird mit Wasser verdünnt, über Magnesiumsulfat getrocknet und am Rotationsverdampfer eingeengt. Das verbleibende orangefarbene Öl wird mit Cyclohexan/Essigester (4:6) über 330 g Kieselgel chromatographiert.

Ausbeute: 1,4 g (45 % der Theorie),
Schmelzpunkt: 97-99°C
$R_f$-Wert: 0,24 (Kieselgel; Cyclohexan/Essigester = 4:6)

Analog werden erhalten:

(1) (3R,S;4R,S)-3-Allyl-4-[(4'-cyano-4-biphenylyl)oxymethyl]-2-pyrrolidinon

Schmelzpunkt: 101-105°C
$R_f$-Wert: 0,37 (Kieselgel; Essigester)

(2) (3R,S;4R,S)-3-Allyl-4-[[(4'-cyano-4-biphenylyl)carbonylamino]-methyl]-2-pyrrolidinon

$R_f$-Wert: 0,12 (Kieselgel; Essigester)

(3) (3R,S;4R,S)-3-Allyl-4-[(4'-cyano-4-biphenylyl)oxymethyl]-3-methyl-2-pyrrolidinon

Schmelzpunkt: 137-138°C
$R_f$-Wert: 0,46 (Kieselgel; Essigester)

| Ber.: | C 76,27 | H 6,40 | N 8,09 |
|-------|---------|--------|--------|
| Gef.: | 76,09 | 6,31 | 7,97 |

Beispiel CXIV

(3S,5S)-5-[(3-Amino-4'-cyano-4-biphenylyl)oxymethyl]-3-[(methoxycarbonyl)methyl]-1-(3-phenylpropyl)-2-pyrrolidinon

4 g (3S,5S)-5-[(4'-Cyano-3-nitro-4-biphenylyl)oxymethyl]-3-[(methoxycarbonyl)methyl]-1-(3-phenylpropyl)-2-pyrrolidinon werden in 40 ml Ethanol und 10 ml Dimethylformamid mit 1,5 g Palladium (10%ig auf Aktivkohle) unter einem Wasserstoffdruck von 3 bar bei Raumtemperatur hydriert. Nach 40 Minuten wird vom Katalysator abgesaugt und das Filtrat eingeengt. Der Rückstand wird zwischen Wasser und Methylenchlorid verteilt, die organische Phase abgetrennt, mit Magnesiumsulfat getrocknet und einrotiert. Säulenchromatographie an Kieselgel mit Methylenchlorid/Essigester (4:1) ergeben 2,9 g (77 % der Theorie) der gewünschten Verbindung.

Schmelzpunkt: 111-112°C,
$R_f$-Wert: 0,37 (Kieselgel; 1,2-Dichlorethan/Essigester = 3:1)

| Ber.: | C 72,41 | H 6,28 | N 8,45 |
|-------|---------|--------|--------|
| Gef.: | 72,31 | 6,54 | 8,27 |

Beispiel CXV

(3S,5S)-5-[(4'-Cyano-3-methansulfonylamino-4-biphenylyl)oxymethyl]-3-[(methoxycarbonyl)methyl]-1-(3-phenylpropyl)-2-pyrrolidinon

0,5 g (3S,5S)-5-[(3-Amino-4'-cyano-4-biphenylyl)oxymethyl]-3-[(methoxycarbonyl)methyl]-1-(3-phenylpropyl)-2-pyrrolidinon und 0,09 ml Pyridin gelöst in 5 ml trockenem Methylenchlorid werden mit 0,09 ml Methansulfonsäurechlorid versetzt. Nach zweistündigem Rühren bei Raumtemperatur wird Wasser und verdünnte Salzsäure zugegeben und die organische Phase abgetrennt. Die wäßrige Phase wird mit Methylenchlorid extrahiert und die vereinigten organischen Phasen mit Natriumsulfat getrocknet. Der Eindampfrückstand wird über eine Kieselgelsäule mit Methylenchlorid/Essigester (2:1) chromatographiert. Dabei erhält man 0,39 g (68,4 % der Theorie) eines weißen Feststoffes.

Schmelzpunkt: 140-141°C,
$R_f$-Wert: 0,55 (Kieselgel; 1,2-Dichlorethan/Essigester = 3:1)

| Ber.: | C 64,68 | H 5,78 | N 7,30 |
|-------|---------|--------|--------|
| Gef.: | 64,52 | 5,73 | 7,25 |

Analog wird erhalten:

(1) (3S,5S)-5-[[N-(4'-Cyano-4-biphenylyl)-N-methansulfonyl]aminomethyl]-3-[(methoxycarbonyl)methyl]-1-(3-phenylpropyl)-2-pyrrolidinon

$R_f$-Wert: 0,38 (Kieselgel; Cyclohexan/Essigester = 1:3)

Beispiel CXVI

(3S,5S)-5-[(3-Acetylamino-4'-cyano-4-biphenylyl)oxymethyl]-3-[(methoxycarbonyl)methyl]-1-(3-phenylpropyl)-2-pyrrolidinon

0,5 g (3S,5S)-5-[(3-Amino-4'-cyano-4-biphenylyl)oxymethyl]-3-[(methoxycarbonyl)methyl]-1-(3-phenylpropyl)-2-pyrrolidinon und 0,14 ml Triethylamin werden in 5 ml trockenem Methylenchlorid gelöst. Dazu wird 0,07 ml Acetylchlorid gegeben. Nach 2 Stunden Rühren bei Raumtemperatur wird mit verdünnter Salzsäure versetzt und die organische Phase abgetrennt. Die wäßrige Phase wird mit Methylenchlorid extrahiert und die vereinigten organischen Phasen über Natriumsulfat getrocknet. Nach dem Abrotieren des Lösungsmittels erhält man 0,45 g (83 % der Theorie) an gewünschtem Produkt.

Schmelzpunkt: 177-179°C,
$R_f$-Wert: 0,34 (Kieselgel; 1,2-Dichlorethan/Essigester = 3:1)

| Ber.: | C 71,22 | H 6,16 | N 7,79 |
|-------|---------|--------|--------|
| Gef.: | 70,97 | 6,39 | 7,53 |

Analog wird erhalten:

(1) (3S,5S)-5-[[N-(4'-Cyano-4-biphenylyl)-N-acetyl]aminomethyl]-3-[(methoxycarbonyl)methyl]-1-(3-phenylpropyl)-2-pyrrolidinon

$R_f$-Wert: 0,43 (Kieselgel; Cyclohexan/Essigester = 1:3)

Beispiel CXVII

(3R,5S)-3-Allyl-5-[[[3-(3-cyanophenyl)propyl]carbonylamino]methyl]-1-(3-phenylpropyl)-2-pyrrolidinon

4,5 g 4-(3-Cyanophenyl)buttersäure und 4,6 g Carbonyldiimidazol werden in 50 ml Tetrahydrofuran gelöst und eine Stunde bei Raumtemperatur gerührt. Man fügt 7,66 g (3R,5S)-3-Allyl-5-aminomethyl-1-(3-phenylpropyl)-2-pyrrolidinon zu und rührt weitere 16 Stunden bei Raumtemperatur. Die Lösung wird eingedampft, der Rückstand mit Essigester aufgenommen. Die Essigesterphase wird nach Waschen mit verdünnter Salzsäure, Bicarbonatlösung und Wasser eingedampft und das zurückbleibende Rohprodukt direkt weiter verarbeitet.

Ausbeute: 4,5 g (40 % der Theorie),
$R_f$-Wert: 0,30 (Kieselgel; Methylenchlorid/Methanol = 19:1)

Analog werden erhalten:

(1) (3R,5S)-3-Allyl-5-[[(3-cyanophenyl)carbonyl]aminomethyl]-1-(3-phenylpropyl)-2-pyrrolidinon

$R_f$-Wert: 0,72 (Kieselgel; Essigester)

(2) (3R,5S)-3-Allyl-5-[[[4-[2-(tert.butyloxycarbonyl)ethyl]phenyl]carbonyl]aminomethyl]-1-(3-phenylpropyl)-2-pyrrolidinon

$R_f$-Wert: 0,53 (Kieselgel; Methylenchlorid/Methanol = 10:1)

(3) (3R,5S)-3-Allyl-5-[[(4-cyanophenyl)carbonyl]aminomethyl]-1-(3-phenylpropyl)-2-pyrrolidinon

$R_f$-Wert: 0,70 (Kieselgel; Methylenchlorid/Methanol = 20:1)

(4) (3R,5S)-3-Allyl-5-[[[3-[2-(tert.butyloxycarbonylamino)ethyl]phenyl]carbonyl]aminomethyl]-1-(3-phenylpropyl)-2-pyrrolidinon

$R_f$-Wert: 0,48 (Kieselgel; Methylenchlorid/Methanol = 10:1)

(5) (3R,5S)-3-Allyl-5-[[[5-(tert.butyloxycarbonylamino)pentyl]carbonyl]aminomethyl]-1-(4-phenoxybutyl)-2-pyrrolidinon

$R_f$-Wert: 0,44 (Kieselgel; Methylenchlorid/Methanol = 10:1)

(6) (3R,5S)-3-Allyl-5-[[[5-(tert.butyloxycarbonylamino)pentyl]carbonyl]aminomethyl]-1-(2-phenylethyl)-2-pyrrolidinon

$R_f$-Wert: 0,68 (Kieselgel; Methylenchlorid/Methanol = 9:1)

(7) (3R,5S)-3-Allyl-1-benzyl-5-[[[5-(tert.butyloxycarbonylamino)pentyl]carbonyl]aminomethyl]-2-pyrrolidinon

$R_f$-Wert: 0,49 (Kieselgel; Methylenchlorid/Methanol = 10:1)

(8) (3R,5S)-3-Allyl-5-[[[2-(tert.butyloxycarbonylamino)-5-indanyl]methylcarbonyl]aminomethyl]-1-(3-phenylpropyl)-2-pyrrolidinon

$R_f$-Wert: 0,57 (Kieselgel; Essigester/Methanol = 20:1)

(9) (3R,5S)-3-Allyl-5-[[[5-(tert.butyloxycarbonylamino)pentyl]carbonyl]aminomethyl]-1-isobutyl-2-pyrrolidinon

$R_f$-Wert: 0,44 (Kieselgel; Essigester/Methanol = 9:1)

(10) (3R,5S)-3-Allyl-5-[[[3-(4-cyanophenyl)propyl]carbonylamino]methyl]-1-(3-phenylpropyl)-2-pyrrolidinon

$R_f$-Wert: 0,30 (Kieselgel; Methylenchlorid/Methanol = 19:1)

(11) (3R,5S)-3-Allyl-5-[[[3-[3-(tert.butyloxycarbonylamino)phenyl]propyl]carbonylamino]methyl]-1-(3-phenylpropyl)-2-pyrrolidinon

$R_f$-Wert: 0,30 (Kieselgel; Methylenchlorid/Methanol = 19:1)

(12) (3R,5S)-3-Allyl-5-[[[2-(tert.butyloxycarbonylamino)-5-indanyl]carbonyl]aminomethyl]-1-(3-phenylpropyl)-2-pyrrolidinon

$R_f$-Wert: 0,47 (Kieselgel; Methylenchlorid/Methanol = 15:1)

(13) (3R,5S)-3-Allyl-5-[[[2-[(tert.butyloxycarbonylamino)methyl]-5-indanyl]carbonyl]aminomethyl]-1-(3-phenylpropyl)-2-pyrrolidinon

$R_f$-Wert: 0,20 (Kieselgel; Cyclohexan/Essigester = 1:1)

(14) (3R,5S)-3-Allyl-5-[[[2-[(tert.butyloxycarbonylamino)methyl]-5-indanyl]methyl]carbonyl]aminomethyl]-1-(3-phenylpropyl)-2-pyrrolidinon

$R_f$-Wert: 0,47 (Kieselgel; Essigester/Ethanol = 40:1)

(15) (3R,5S)-3-(1-Buten-4-yl)-5-[[[4-(tert.butyloxycarbonylamino)butyl]carbonylamino]methyl]-1-(3-phenylpropyl)-2-pyrrolidinon

$R_f$-Wert: 0,50 (Kieselgel; Methylenchlorid/Methanol = 9:1)

(16) (3R,5S)-3-(1-Buten-4-yl)-5-[[[5-(tert.butyloxycarbonylamino)pentyl]carbonylamino]methyl]-1-(3-phenylpropyl)-2-pyrrolidinon

$R_f$-Wert: 0,54 (Kieselgel; Methylenchlorid/Methanol = 9:1)

(17) (3R,5S)-3-Allyl-5-[[[2-[(4-tert.butyloxycarbonylaminomethyl)phenyl]ethyl]carbonylamino]methyl]-1-(3-phenylpropyl)-2-pyrrolidinon

$R_f$-Wert: 0,50 (Kieselgel; Methylenchlorid/Methanol = 19:1)

(18) (3R,5S)-3-Allyl-5-[[[2-[3-(tert.butyloxycarbonylamino)phenyl]ethyl]carbonylamino]methyl]-1-(3-phenylpropyl)-

2-pyrrolidinon

$R_f$-Wert: 0,60 (Kieselgel; Methylenchlorid/Methanol = 9:1)

(19) (3R,5S)-3-Allyl-5-[(4'-cyano-4-biphenylyl)oxymethyl]-1-[(ethylaminocarbonyl)methyl]-2-pyrrolidinon

$R_f$-Wert: 0,55 (Kieselgel; Essigester/Methanol = 20:1)

| Ber.: | C 71,92 | H 6,52 | N 10,06 |
|-------|---------|--------|---------|
| Gef.: | 71,75 | 6,64 | 10,26 |

(20) (3R,5S)-3-Allyl-5-[(4'-cyano-4-biphenylyl)oxymethyl]-1-[(dimethylaminocarbonyl)methyl]-2-pyrrolidinon x 0,25 Wasser

$R_f$-Wert: 0,55 (Kieselgel; Essigester/Methanol = 20:1)

| Ber.: | C 71,16 | H 6,57 | N 9,96 |
|-------|---------|--------|--------|
| Gef.: | 70,98 | 6,74 | 9,93 |

(21) (3S,5S)-5-[2-[(4-Cyanocinnamoyl)amino]ethyl]-3-[(methoxycarbonyl)methyl]-1-(3-phenylpropyl)-2-pyrrolidinon

$R_f$-Wert: 0,43 (Kieselgel; Methylenchlorid/Methanol = 15:1)

<u>Beispiel CXVIII</u>

<u>(3S,5S)-5-[[(4'-Cyano-4-biphenylyl)carbonyl]aminomethyl]-3-[(methoxycarbonyl)methyl]-1-(3-phenylpropyl)-2-pyrrolidinon</u>

0,3 g 4'-Cyano-4-biphenylcarbonsäure, 0,45 g (3S,5S)-5-Aminomethyl-3-[(methoxycarbonyl)methyl]-1-(3-phenylpropyl)-2-pyrrolidinon-hydrochlorid, 0,18 g 1-Hydroxybenztriazol und 0,4 ml Triethylamin werden in 10 ml trockenem Dimethylformamid vorgelegt. Dann wird unter Eiskühlung 0,3 g N,N'-Dicyclohexylcarbodiimid zugegeben und 20 Stunden bei Raumtemperatur gerührt. Nach dieser Zeit wird der Ansatz mit Wasser versetzt, mit Essigester extrahiert, die organische Phase abgetrennt, getrocknet und eingeengt. Der Rückstand wird über eine Kieselgelsäule mit Essigester gereinigt. Das resultierende Produkt wird in wenig Essigester aufgenommen, es wird vom Niederschlag abfiltriert und das Filtrat eingeengt.

Ausbeute: 390 mg (57 % der Theorie),
$R_f$-Wert: 0,46 (Kieselgel; Essigester)

Analog werden erhalten:

(1) (3S,5S)-5-[(7-Cyano-2-naphthylcarbonyl)aminomethyl]-3-[(methoxycarbonyl)methyl]-1-(3-phenylpropyl)-2-pyrrolidinon

$R_f$-Wert: 0,51 (Kieselgel; Essigester)

(2) (3S,5S)-5-[2-[(7-Cyano-2-naphthylcarbonyl)amino]ethyl]-3-[(methoxycarbonyl)methyl]-1-(3-phenylpropyl)-2-pyrrolidinon

$R_f$-Wert: 0,31 (Kieselgel; Essigester)

| Ber.: | C 72,41 | H 6,28 | N 8,44 |
|-------|---------|--------|--------|
| Gef.: | 72,22 | 6,58 | 8,29 |

(3)   (3S,5S)-5-[(6-Cyano-5,6,7,8-tetrahydro-2-naphthylcarbonyl)aminomethyl]-3-[(methoxycarbonyl)methyl]-1-(3-phenylpropyl)-2-pyrrolidinon

$R_f$-Wert: 0,43 (Kieselgel; Essigester)

(4)   (3S,5S)-5-[2-[(6-Cyano-5,6,7,8-tetrahydro-2-naphthylcarbonyl)amino]ethyl]-3-[(methoxycarbonyl)methyl]-1-(3-phenylpropyl)-2-pyrrolidinon

$R_f$-Wert: 0,25 (Kieselgel; Essigester)

| Ber.: | C 70,57 | H 7,11 | N 8,23 |
|-------|---------|--------|--------|
| Gef.: | 70,61 | 7,33 | 8,33 |

(5)   (3S,5S)-5-[[trans-3-(4-Cyanophenyl)cyclobutyl]carbonylaminomethyl]-3-[(methoxycarbonyl)methyl]-1-(3-phenylpropyl)-2-pyrrolidinon-hydrat
Durch chromatographische Trennung des cis/trans-Gemisches

$R_f$-Wert: 0,60 (Kieselgel; Essigester/Methanol = 15:1)

| Ber.: | C 68,89 | H 6,98 | N 8,31 |
|-------|---------|--------|--------|
| Gef.: | 69,21 | 7,11 | 8,67 |

(6)   (3S,5S)-5-[[cis-3-(4-Cyanophenyl)cyclobutyl]carbonylaminomethyl]-3-[(methoxycarbonyl)methyl]-1-(3-phenylpropyl)-2-pyrrolidinon-hydrat
Durch chromatographische Trennung des cis/trans-Gemisches

$R_f$-Wert: 0,51 (Kieselgel; Essigester/Methanol = 15:1)

| Ber.: | C 68,89 | H 6,98 | N 8,31 |
|-------|---------|--------|--------|
| Gef.: | 68,85 | 7,26 | 8,41 |

(7)   (3S,5S)-5-[2-[[cis-3-(4-Cyanophenyl)cyclobutyl]carbonylamino]ethyl]-3-[(methoxycarbonyl)methyl]-1-(3-phenylpropyl)-2-pyrrolidinon
Durch chromatographische Trennung des cis/trans-Gemisches

$R_f$-Wert: 0,49 (Kieselgel; Essigester/Methanol = 15:1)

(8)   (3S,5S)-5-[2-[[trans-3-(4-Cyanophenyl)cyclobutyl]carbonylamino]ethyl]-3-[(methoxycarbonyl)methyl]-1-(3-phenylpropyl)-2-pyrrolidinon-hydrat
Durch chromatographische Trennung des cis/trans-Gemisches

$R_f$-Wert: 0,54 (Kieselgel; Essigester/Methanol = 15:1)

| Ber.: | C 69,34 | H 7,18 | N 8,09 |
|-------|---------|--------|--------|
| Gef.: | 69,59   | 7,26   | 7,92   |

(9) (3R,S;4S,R)-3-Allyl-4-[[(4'-cyano-4-biphenylyl)carbonylamino]methyl]-1-(4-methoxybenzyl)-2-pyrrolidinon

$R_f$-Wert: 0,59 (Kieselgel; Essigester)

Beispiel CXIX

(3S,5S)-5-[2-[(6-Cyano-2-naphthylcarbonyl)amino]ethyl]-3-[(methoxycarbonyl)methyl]-1-(3-phenylpropyl)-2-pyrrolidinon

Zu 2,1 g (3S,5S)-5-(2-Aminoethyl)-3-[(methoxycarbonyl)methyl]-1-(3-phenylpropyl)-2-pyrrolidinon-hydrochlorid in 20 ml Chloroform werden bei Raumtemperatur 1,35 g 6-Cyano-2-naphthylcarbonylchlorid in 20 ml Chloroform gegeben. Anschließend werden 4,2 ml Triethylamin unter Rühren zugetropft. Nach 18 Stunden Rühren bei Raumtemperatur wird eingeengt, in Essigester aufgenommen und mit verdünnter Salzsäure, Wasser und Kochsalzlösung gewaschen. Die organische Phase wird mit Natriumsulfat getrocknet, filtriert und einrotiert. Nach Reinigung über eine Kieselgelsäule mit Cyclohexan/Essigester (3:7) werden 1 g (32 % der Theorie) reines Produkt erhalten.

Schmelzpunkt: 93-99°C
$R_f$-Wert: 0,30 (Kieselgel; Essigester)

Analog werden erhalten:

(1) (3S,5S)-5-[(6-Cyano-2-naphthylcarbonyl)aminomethyl]-3-[(methoxycarbonyl)methyl]-1-(3-phenylpropyl)-2-pyrrolidinon

$R_f$-Wert: 0,57 (Kieselgel; Essigester)

(2) (3S,5S)-5-[[[2-[(4-Cyanophenyl)amino]phenyl]carbonylamino]methyl]-3-[(methoxycarbonyl)methyl]-1-(3-phenylpropyl)-2-pyrrolidinon

Schmelzpunkt: 135°C
$R_f$-Wert: 0,42 (Kieselgel; Cyclohexan/Essigester/Methanol = 6:3:0,5)

Beispiel CXX

(3R,5S)-3-Allyl-5-[[[2-[4-(tert.butyloxycarbonylamino)phenyl]ethyl]carbonylamino]methyl]-1-(3-phenylpropyl)-2-pyrrolidinon

2 g 3-[4-(tert.Butyloxycarbonylamino)phenyl]propionsäure werden in 30 ml Tetrahydrofuran gelöst und bei -15°C mit 0,82 ml N-Methyl-morpholin versetzt und 1,0 g Chlorameisensäureisobutylester zugegeben. Nach 30 Minuten tropft man eine Lösung von 2,5 g (3R,5S)-3-Allyl-5-aminomethyl-1-(3-phenylpropyl)-2-pyrrolidinon in 10 ml Dimethylformamid zu. Man rührt 2 Tage bei Raumtemperatur nach, filtriert vom ausgefallenen N-Methyl-morpholin-hydrochlorid ab, dampft ein und nimmt den Rückstand in Essigester auf. Nach Waschen mit 2N Salzsäure und Wasser und Behandeln mit Aktivkohle wird die Lösung eingedampft und der Rückstand säulenchromatographisch über Kieselgel (Elutionsmittel: Methylenchlorid/Methanol = 40:1) gereinigt.

Ausbeute: 1,7 g (47 % der Theorie),
$R_f$-Wert: 0,46 (Kieselgel; Methylenchlorid/Methanol = 19:1)

Analog werden erhalten:

(1) (3R,5S)-3-Allyl-5-[[[3-[4-(tert.butyloxycarbonylamino)phenyl]propyl]carbonylamino]methyl]-1-(3-phenylpropyl)-2-pyrrolidinon

$R_f$-Wert: 0,86 (Kieselgel; Methylenchlorid/Methanol = 9:1)

(2)   (3S,5S)-5-[[(4'-Cyano-3-biphenylyl)carbonyl]aminomethyl]-3-[(methoxycarboxyl)methyl]-1-(3-phenylpropyl)-2-pyrrolidinon

$R_f$-Wert: 0,27 (Kieselgel; Methylenchlorid/Methanol = 40:1)

(3) (3S,5S)-5-[(3-Cyanobenzoyl)aminomethyl]-3-[(methoxycarbonyl)methyl]-1-(3-phenylpropyl)-2-pyrrolidinon

$R_f$-Wert: 0,22 (Kieselgel; Methylenchlorid/Methanol = 40:1)
Massenspektrum: $M^+$ = 433

(4)       (3S,5S)-5-[exo-5-(4-Cyanophenyl)bicyclo[2.2.1]heptyl-exo-2-carbonyl]aminomethyl]-3-[(methoxycarbonyl)methyl]-1-(3-phenylpropyl)-2-pyrrolidinon

$R_f$-Wert: 0,52 (Kieselgel; Methylenchlorid/Methanol = 9:1)
Massenspektrum: $M^+$ = 527

## Beispiel CXXI

(3R,5S)-3-Allyl-5-[[trans-4-(4-cyanophenyl)cyclohexyl)oxymethyl]-1-(3-phenylpropyl)-2-pyrrolidinon

0,73 g trans-4-(4-Cyanophenyl)cyclohexanol, 2,8 g (3R,5S)-3-Allyl-5-[(methansulfonyl)oxymethyl]-1-(3-phenylpropyl)-2-pyrrolidinon, 2,5 ml 60%ige wäßrige Kalilauge und 0,3 g Polyethylenglykol-750-monomethylether (gebunden an Polystyrol-1% Divinylbenzol) werden 24 Stunden bei 45°C gerührt. Der Ansatz wird mit Eis und Wasser versetzt, mit Salzsäure angesäuert und mit Essigester extrahiert. Die organische Phase wird mit Natriumsulfat getrocknet, eingeengt und der Rückstand über eine Kieselgelsäule mit Petrolether/Essigester (2:1) chromatographiert. Es werden 0,4 g (24 % der Theorie) Produkt erhalten.

$R_f$-Wert: 0,61 (Kieselgel; Petrolether/Essigester = 2:1)

| Ber.: | C 78,91 | H 7,95 | N 6,14 |
|-------|---------|--------|--------|
| Gef.: | 78,88 | 8,13 | 6,10 |

## Beispiel CXXII

1-(4'-Cyano-4-biphenylyl)-4-methoxycarbonyl-2-pyrrolidinon

28 g 4-Carboxy-1-(4'-cyano-4-biphenylyl)-2-pyrrolidinon in 100 ml Dioxan werden bei 100°C mit 15,5 g Dimethylformamiddimethylacetal versetzt und weitere 20 Minuten bei dieser Temperatur gerührt. Nach dem Abkühlen wird eingeengt, in Essigester aufgenommen, mit Aktivkohle behandelt, filtriert und einrotiert. Nach Reinigung über eine Kieselgelsäule mit Cyclohexan/Essigester (1:1) werden 19 g (65 % der Theorie) erhalten.

Schmelzpunkt: 153-155°C
$R_f$-Wert: 0,34 (Kieselgel; Cyclohexan/Essigester = 1:1)

## Beispiel CXXIII

(3S,5S)-5-[(4'-Cyano-4-biphenylyl)aminomethyl]-3-[(methoxycarbonyl)methyl]-1-(3-phenylpropyl)-2-pyrrolidinon

Eine Mischung aus 9,7 g 4-Amino-4'-cyanobiphenyl, 19,2 g (3S,5S)-5-[(Methansulfonyloxy)methyl]-3-[(methoxycarbonyl)methyl]-1-(3-phenylpropyl)-2-pyrrolidinon und 6 g Ethyl-diisopropylamin wird 10 Stunden auf 160°C erhitzt. Man nimmt mit Essigester auf, wäscht mit 1N Salzsäure, dampft die organische Phase ein und reinigt durch Chromatographie an Kieselgel (Elutionsmittel: Cyclohexan/Essigester = 1:1).

Ausbeute: 18 g (74 % der Theorie),

$R_f$-Wert: 0,26 (Kieselgel; Cyclohexan/Essigester = 1:1)

Analog werden erhalten:

(1) (3S,5S)-5-[2-[(4'-Cyano-3-biphenylyl)amino]ethyl]-3-[(methoxycarbonyl)methyl]-1-(3-phenylpropyl)-2-pyrrolidinon

$R_f$-Wert: 0,35 (Kieselgel; Methylenchlorid/Methanol = 100:1)

(2) (3S,5S)-3-[(Methoxycarbonyl)methyl]-5-[2-[(3'-nitro-3-biphenylyl)amino]ethyl]-1-(3-phenylpropyl)-2-pyrrolidinon

Lösungsmittel: Dimethylacetamid
$R_f$-Wert: 0,26 (Kieselgel; Cyclohexan/Essigester = 1:1)

Beispiel CXXIV

(3S,5S)-5-[(4'-Cyano-4-biphenylyl)oxymethyl]-1,3-bis[(methoxycarbonyl)methyl]-2-pyrrolidinon

Hergestellt analog Beispiel 26 mittels Methanol/Salzsäure

Schmelzpunkt: 121-123°C,
$R_f$-Wert: 0,60 (Kieselgel; Essigester/Cyclohexan/Eisessig = 20:5:1)

| Ber.: | C 65,40 | H 5,25 | N 6,63 |
|-------|---------|--------|--------|
| Gef.: | 65,54 | 5,56 | 6,40 |

Analog werden erhalten:

(1) (3S,5S)-5-[(4'-Cyano-4-biphenylyl)oxymethyl]-1-[(ethylaminocarbonyl)methyl]-3-[(methoxycarbonyl)methyl]-2-pyrrolidinon
Durchführung mit Methanol/Salzsäure

Schmelzpunkt: 171-173°C
$R_f$-Wert: 0,58 (Kieselgel; Essigester/Methanol = 20:1)

| Ber.: | C 66,80 | H 6,05 | N 9,35 |
|-------|---------|--------|--------|
| Gef.: | 66,67 | 6,02 | 9,10 |

(2) (3S,5S)-5-[(4'-Cyano-4-biphenylyl)oxymethyl]-1-[(dimethylaminocarbonyl)methyl]-3-[(methoxycarbonyl)methyl]-2-pyrrolidinon
Durchführung mit Methanol/Salzsäure

Schmelzpunkt: 139-140°C
$R_f$-Wert: 0,40 (Kieselgel; Essigester/Methanol = 20:1)

| Ber.: | C 66,80 | H 6,05 | N 9,35 |
|-------|---------|--------|--------|
| Gef.: | 66,99 | 6,21 | 9,07 |

(3) (3S,5S)-1-[(Benzylaminocarbonyl)methyl]-5-[(4'-cyano-4-biphenylyl)oxymethyl]-3-[(methoxycarbonyl)methyl]-2-pyrrolidinon
Durchführung mit Methanol/Salzsäure

Schmelzpunkt: 108-110°C
$R_f$-Wert: 0,43 (Kieselgel; Essigester)

| Ber.: | C 70,57 | H 5,53 | N 8,23 |
|---|---|---|---|
| Gef.: | 70,73 | 5,78 | 8,12 |

(4) (3S,5S)-5-[(4'-Cyano-4-biphenylyl)oxymethyl]-3-[(methoxycarbonyl)methyl]-pyrrolidin-hydrochlorid
Durchführung mit Methanol/Salzsäure

Schmelzpunkt: 188-190°C
$R_f$-Wert: 0,76 (Kieselgel; Methylenchlorid/Methanol/konz. wäßriges Ammoniak = 40:4:1)

| Ber.: | C 65,19 | H 5,99 | N 7,24 | Cl 9,16 |
|---|---|---|---|---|
| Gef.: | 65,39 | 5,92 | 7,12 | 9,12 |

(5) (3R,5S)-5-[(4'-Cyano-4-biphenylyl)oxymethyl]-3-[(methoxycarbonyl)methyl]-2-pyrrolidinon
Durchführung mit Methanol/Salzsäure

Schmelzpunkt: 138-140,5°C
$R_f$-Wert: 0,35 (Kieselgel; Methylenchlorid/Methanol = 98:2)

| Ber.: | C 69,22 | H 5,53 | N 7,69 |
|---|---|---|---|
| Gef.: | 69,09 | 5,46 | 7,67 |

<u>Beispiel CXXV</u>

<u>(3S,5S)-5-[[4-[(3-Cyanophenyl)sulfonyl]phenyl]oxymethyl]-3-[(methoxycarbonyl)methyl]-1-(3-phenylpropyl)-2-pyrrolidinon</u>

Zu 3 g (3S,5S)-5-[[4-[(3-Cyanophenyl)sulfenyl]phenyl]oxymethyl]-3-[(methoxycarbonyl)methyl]-1-(3-phenylpropyl)-2-pyrrolidinon in 10 ml Essigsäureanhydrid und 6 ml Eisessig werden bei 90°C unter starkem Rühren 3 ml wäßrige 30%ige Wasserstoffperoxid-Lösung gegeben. Nach einer Stunde Reaktionszeit bei 90°C gießt man auf Eis, neutralisiert mit festem Natriumhydrogencarbonat und extrahiert mit Essigester. Nach Reinigung über eine Kieselgelsäule (Elutionsmittel: Essigester/Cyclohexan = 3:1) wird ein farbloser Festkörper erhalten.

Ausbeute: 2,0 g (46 % der Theorie),
$R_f$-Wert: 0,23 (Kieselgel; Essigester/Cyclohexan = 1:1)
$R_f$-Wert: 0,37 (Kieselgel; Essigester/Cyclohexan = 3:2)

Analog werden erhalten:

(1)    (3S,5S)-5-[(4'-Cyano-3-methylsulfonyl-4-biphenylyl)oxymethyl]-3-[(methoxycarbonyl)methyl]-1-(3-phenylpropyl)-2-pyrrolidinon

$R_f$-Wert: 0,57 (Kieselgel; Methylenchlorid/Ethanol = 15:1)

(2)    (3S,5S)-5-[(4'-Cyano-4-biphenylyl)oxymethyl]-3-[(methoxycarbonyl)methyl]-1-[3-(4-methylsulfonylphenyl)propyl]-2-pyrrolidinon

$R_f$-Wert: 0,51 (Kieselgel; Methylenchlorid/Ethanol = 15:1)

(3) (3S,5S)-5-[[(4'-Cyano-4-biphenylyl)sulfonyl]methyl]-3-[(methoxycarbonyl)methyl]-1-(3-phenylpropyl)-2-pyrrolidi-

EP 0 483 667 B1

non

$R_f$-Wert: 0,53 (Kieselgel; Methylenchlorid/Ethanol = 15:1)

Beispiel CXXVI

(3R,5S)-3-Allyl-1-(tert.butyloxycarbonyl)-5-[(4'-cyano4-biphenylyl)oxymethyl]-pyrrolidin

Zu 35,1 g 4-Cyano-4'-hydroxybiphenyl in 250 ml trockenem Dimethylformamid werden portionsweise 9,5 g Natriumhydrid (55%ig in Paraffinöl) eingetragen. Nach eineinhalb Stunden Rühren bei Raumtemperatur werden 58,1 g (3R,5S)-3-Allyl-1(tert.butyloxycarbonyl)-5-[(methansulfonyloxy)methyl]pyrrolidin zugegeben und 7 Tage bei 40°C gerührt. Danach wird das Lösungsmittel im Vakuum entfernt, der Rückstand zwischen Essigester und Wasser verteilt, die organische Phase abgetrennt, getrocknet und eingedampft. Der Rückstand wird über eine Kieselgelsäule mit Cyclohexan/Essigester = 5:1 gereinigt.

Ausbeute: 37,5 g (49 % der Theorie),
$R_f$-Wert: 0,54 (Kieselgel; Cyclohexan/Essigester = 2:1)

Analog wird erhalten:

(1) (3R,5S)-3-Allyl-5-[(4'-cyano-4-biphenylyl)oxymethyl]-3-methyl-2-pyrrolidinon

Rf-Wert: 0,39 (Kieselgel; Cyclohexan/Essigester = 3:7)

| Ber.: | C 76,28 | H 6,40 | N 8,09 |
|-------|---------|--------|--------|
| Gef.: | 75,98 | 6,69 | 8,13 |

Beispiel CXXVII

(3S,5S)-5-[2-[[(3'-Cyano-4-biphenylyl)carbonyl]amino]ethyl]-3-[(methoxycarbonyl)methyl]-1-(3-phenylpropyl)-2-pyrrolidinon

Hergestellt analog Beispiel LXVIII durch Diazotierung von (3S,5S)-5-[2-[[(3'-Amino-4-biphenylyl)carbonyl]amino]ethyl]-3-[(methoxycarbonyl)methyl]-1-(3-phenylpropyl)-2-pyrrolidinon und Umsetzung mit Kaliumcyanid in Gegenwart von Kupfer(I)cyanid.

$R_f$-Wert: 0,45 (Kieselgel; Methylenchlorid/Methanol = 9:1)

Analog wird erhalten:

(1) (3S,5S)-5-[[(3'-Cyano-4-biphenylyl)carbonyl]aminomethyl]-3-[(methoxycarbonyl)methyl]-1-(3-phenylpropyl)-2-pyrrolidinon

$R_f$-Wert: 0,33 (Kieselgel; Cyclohexan/Essigester = 1:2)

Beispiel CXXVIII

(3R,5S)-3-Allyl-1-[(benzylaminocarbonyl)methyl]-5-[(4'-cyano-4-biphenylyl)oxymethyl]-2-pyrrolidinon

5 g (3R,5S)-3-Allyl-5-[(4'-cyano-4-biphenylyl)oxymethyl]-1-[(methoxycarbonyl)methyl]-2-pyrrolidinon, 4 ml Benzylamin und 5 ml trockenes Methanol werden 3 Tage bei Raumtemperatur gerührt. Das Gemisch wird eingeengt, der Rückstand in Essigester aufgenommen und zweimal mit 2N Salzsäure ausgeschüttelt. Die organische Phase wird mit Kochsalzlösung gewaschen, über Magnesiumsulfat getrocknet, filtriert und einrotiert. Der Rückstand wird über eine Kieselgelsäule mit Essigester/Cyclohexan = 7:1 gereinigt.

Ausbeute: 3,4 g (57 % der Theorie),

94

R$_f$-Wert: 0,60 (Kieselgel; Essigester/Cyclohexan = 9:1)

| Ber.: | C 75,29 | H 5,90 | N 8,78 |
|-------|---------|--------|--------|
| Gef.: | 75,07   | 5,99   | 8,70   |

Beispiel CXXIX

(3S,5S)-1-Benzoyl-5-[(4'-cyano-4-biphenylyl)oxymethyl]-3-[(methoxycarbonyl)methyl]-pyrrolidin

1,16 g (3S,5S)-5-[(4'-Cyano-4-biphenylyl)oxymethyl]-3-[(methoxycarbonyl)methyl]-pyrrolidin-hydrochlorid in 50 ml Methylenchlorid werden mit 2 ml Triethylamin versetzt. Dann wird unter Eiskühlung 0,46 ml Benzoylchlorid zugegeben und 3 Tage bei Raumtemperatur gerührt. Danach wird eingeengt, der Rückstand zwischen Wasser und Essigester verteilt und die organische Phase abgetrennt, getrocknet und einrotiert. Der Rückstand wird über eine Kieselgelsäule mit Cyclohexan/Essigester = 2:1 gereinigt.

Ausbeute: 1,07 g (78,7 % der Theorie),
R$_f$-Wert: 0,37 (Kieselgel; Cyclohexan/Essigester = 1:1)

| Ber.: | C 73,99 | H 5,77 | N 6,16 |
|-------|---------|--------|--------|
| Gef.: | 74,16   | 5,78   | 6,02   |

Analog werden erhalten:

(1) (3S,5S)-5-[(4'-Cyano-4-biphenylyl)oxymethyl]-1-methansulfonyl-3-[(methoxycarbonyl)methyl]-pyrrolidin

Acylierungsmittel: Methansulfochlorid
Schmelzpunkt: 146-148°C
R$_f$-Wert: 0,40 (Kieselgel; Cyclohexan/Essigester = 1:1)

| Ber.: | C 61,66 | H 5,65 | N 6,54 | S 7,48 |
|-------|---------|--------|--------|--------|
| Gef.: | 61,39   | 5,74   | 6,70   | 7,31   |

(2) (3S,5S)-1-Acetyl-5-[(4'-cyano-4-biphenylyl)oxymethyl]-3-[(methoxycarbonyl)methyl]-pyrrolidin x 0,25 Wasser

Acylierungsmittel: Acetanhydrid
R$_f$-Wert: 0,17 (Kieselgel; Cyclohexan/Essigester = 1:1)

| Ber.: | C 69,59 | H 6,22 | N 7,06 |
|-------|---------|--------|--------|
| Gef.: | 69,38   | 6,48   | 6,95   |

(3) (3S,5S)-5-[(4'-Cyano-4-biphenylyl)oxymethyl]-1-(ethylaminocarbonyl)-3-[(methoxycarbonyl)methyl]-pyrrolidin

Acylierungsmittel: Ethylisocyanat
Schmelzpunkt: 134-136°C
R$_f$-Wert: 0,12 (Kieselgel; Cyclohexan/Essigester = 1:1)

| Ber.: | C 68,39 | H 6,46 | N 9,97 |
|-------|---------|--------|--------|
| Gef.: | 68,11   | 6,47   | 10,00  |

(4)  (3S,5S)-5-[(4'-Cyano-4-biphenylyl)oxymethyl]-1-(dimethylaminosulfonyl)-3-[(methoxycarbonyl)methyl]-pyrrolidin

Acylierungsmittel: Dimethylaminosulfonylchlorid
Schmelzpunkt: 89-90°C
$R_f$-Wert: 0,51 (Kieselgel; Cyclohexan/Essigester = 1:1)

| Ber.: | C 60,38 | H 5,95 | N 9,18 | S 7,01 |
|-------|---------|--------|--------|--------|
| Gef.: | 60,34   | 5,99   | 9,33   | 6,81   |

(5) (3S,5S)-5-[(4'-Cyano-4-biphenylyl)oxymethyl]-1-methoxyacetyl-3-[(methoxycarbonyl)methyl]-pyrrolidin

Acylierungsmittel: Methoxyacetylchlorid
$R_f$-Wert: 0,13 (Kieselgel; Cyclohexan/Essigester = 1:1)

| Ber.: | C 68,23 | H 6,20 | N 6,63 |
|-------|---------|--------|--------|
| Gef.: | 68,00   | 6,43   | 6,43   |

(6)  (3S,5S)-5-[(4'-Cyano-4-biphenylyl)oxymethyl]-3-[(methoxycarbonyl)methyl]-1-[(4-methoxyphenyl)sulfonyl]-pyrrolidin

Acylierungsmittel: 4-Methoxyphenylsulfonsäurechlorid
Schmelzpunkt: 105-107°C
$R_f$-Wert: 0,51 (Kieselgel; Cyclohexan/Essigester = 1:1)

| Ber.: | C 64,60 | H 5,42 | N 5,38 | S 6,16 |
|-------|---------|--------|--------|--------|
| Gef.: | 64,66   | 5,54   | 5,11   | 6,18   |

(7)  (3S,5S)-5-[(4'-Cyano-4-biphenylyl)oxymethyl]-1-[(N,N-dimethylamino)carbonyl]-3-[(methoxycarbonyl)methyl]-pyrrolidin

Acylierungsmittel: Phosgen (im Überschuß), dann weitere Umsetzung mit Dimethylamin
$R_f$-Wert: 0,55 (Kieselgel; Essigester)

Beispiel CXXX

(3S,5S)-5-[(4'-Cyano-4-biphenylyl)oxymethyl]-3-[(methoxycarbonyl)methyl]-1-methyl-pyrrolidin x 0,25 Wasser

1,93 g (3S,5S)-5-[(4'-Cyano-4-biphenylyl)oxymethyl]-3-[(methoxycarbonyl)methyl]-pyrrolidin-hydrochlorid, 20 ml Tetrahydrofuran, 1,94 g N-Ethyldiisopropylamin und 0,31 ml Methyljodid werden 4 Stunden bei Raumtemperatur gerührt. Das Lösungsmittel wird im Vakuum einrotiert und der Rückstand mit Wasser verrieben. Der Niederschlag wird abgesaugt und durch Kieselgelchromatographie mit Methylenchlorid/Methanol = 10:1 gereinigt.

Ausbeute: 340 mg (19 % der Theorie),
Schmelzpunkt: 80-82°C
$R_f$-Wert: 0,47 (Kieselgel; Methylenchlorid/Methanol = 10:1)

| Ber.: | C 71,62 | H 6,69 | N 7,59 |
|-------|---------|--------|--------|
| Gef.: | 71,48   | 6,59   | 7,36   |

Beispiel CXXXI

(3R,5S)-3-Allyl-5-[(4'-cyano-4-biphenylyl)oxymethyl]-1-(2-methoxyethyl)-2-pyrrolidinon

Zu 3 g (3R,5S)-3-Allyl-5-[(4'-cyano-4-biphenylyl)oxymethyl]-1-(2-hydroxyethyl)-2-pyrrolidinon in 20 ml Dimethylformamid werden bei 0°C 0,42 g Natriumhydrid (55-60 % in Paraffinöl) zugefügt und eine Stunde gerührt. Dann werden 1,35 g Methyljodid in 1 ml Dimethylformamid zugetropft und eine Stunde bei Raumtemperatur gerührt. Nun werden nochmals 100 mg Natriumhydrid und dann 0,15 ml Methyljodid zugegeben. Nach weiteren 4 Stunden wird mit Wasser und Essigester versetzt, mit Eisessig neutralisiert und die organische Phase abgetrennt. Die wäßrige Phase wird mit Essigester extrahiert und die vereinigten organischen Phasen mit Wasser gewaschen, getrocknet und eingeengt. Der erhaltene Rückstand wird durch Chromatographie über Kieselgel mit Essigester/Cyclohexan = 75:25 gereinigt.

Ausbeute: 2,2 g (71 % der Theorie),
$R_f$-Wert: 0,61 (Kieselgel; Essigester)

Beispiel CXXXII

(3R,5S)-3-Allyl-5-[(4'-cyano-4-biphenylyl)oxymethyl]-1-(2-hydroxyethyl)-2-pyrrolidinon

Zu 15,8 g (3R,5S)-3-Allyl-1-[2-(benzyloxy)ethyl]-5-[(4'-cyano-4-biphenylyl)oxymethyl]-2-pyrrolidinon in 200 ml trokkenem Methylenchlorid werden 7 g Jodtrimethylsilan gegeben und bei Raumtemperatur unter Stickstoff 4 Wochen stehen gelassen. Dann werden 5 ml Methanol zugegeben, mit wäßriger Natriumhydrogensulfit-Lösung und anschließend mit Wasser gewaschen. Nach Trocknen und Einengen der organischen Phase wird das erhaltene Rohprodukt durch Säulenchromatographie über Kieselgel mit Essigester/Cyclohexan = 75:25 gereinigt.

Ausbeute: 5,5 g (43 % der Theorie),
$R_f$-Wert: 0,21 (Kieselgel; Essigester)

Beispiel CXXXIII

(3R,5S)-1-(2-Acetoxyethyl)-3-allyl-5-[(4'-cyano-4-biphenylyl)oxymethyl]-2-pyrrolidinon

Zu 3 g (3R,5S)-3-Allyl-1-(2-hydroxyethyl)-5-[(4'-cyano-4-biphenylyl)oxymethyl]-2-pyrrolidinon in 30 ml Dioxan werden 1,7 ml Acetanhydrid, 1 ml Pyridin und 1 Spatelspitze 4-Dimethylaminopyridin gegeben und 2 Stunden bei Raumtemperatur gerührt. Anschließend wird mit Methanol versetzt und eingeengt. Der Rückstand wird in Essigester aufgenommen, mit verdünnter Salzsäure, Wasser und gesättigter Kochsalzlösung gewaschen, die organische Phase abgetrennt, getrocknet und eingeengt. Der erhaltene Rückstand wird durch Chromatographie über eine Kieselgelsäule mit Essigester/Cyclohexan = 75:25 gereinigt.

Ausbeute: 3,2 g (97 % der Theorie),

Beispiel CXXXIV

(3S,5S)-5-[[(4'-Cyano-4-biphenylyl)sulfonyl]aminomethyl]-3-[(methoxycarbonyl)methyl]-1-(3-phenylpropyl)-2-pyrrolidinon

Hergestellt analog Beispiel XIX aus 4'-Cyano-4-biphenylsulfonsäurechlorid und (3S,5S)-5-Aminomethyl-3-[(methoxycarbonyl)methyl]-1-(3-phenylpropyl)-2-pyrrolidinon-hydrochlorid.

$R_f$-Wert: 0,25 (Kieselgel; Cyclohexan/Essigester/Methanol = 63:32:5)

Beispiel CXXXV

(3S,5S)-5-[(4'-Cyano-4-biphenylyl)sulfenylmethyl]-3-[(methoxycarbonyl)methyl]-1-(3-phenylpropyl)-2-pyrrolidinon

Eine Lösung von 0,6 g 4'-Cyano-4-mercaptobiphenyl und 1,4 g Caesiumcarbonat in 5 ml Dimethylformamid wird eine Stunde bei Raumtemperatur gerührt. Anschließend wird auf 55°C erwärmt, mit einer Lösung von 1,1 g (3S,5S)-5-[(Methansulfonyloxy)methyl]-3-[(methoxycarbonyl)methyl]-1-(3-phenylpropyl)-2-pyrrolidinon in 5 ml Dimethylformamid versetzt und 4 Stunden bei dieser Temperatur gerührt. Danach wird die Lösung in Wasser gegossen und mit Essigester extrahiert. Nach Trocknen der organischen Phase und Einengen erhält man ein Rohprodukt, welches mit Cyclohexan/Essigester = 1:1 über Kieselgel chromatographiert wird.

Ausbeute: 0,18 g (13 % der Theorie),
$R_f$-Wert: 0,41 (Kieselgel; Cyclohexan/Essigester = 1:1)

Beispiel CXXXVI

(3S,5S)-5-[[[1-(4-Cyanophenyl)-1-propen-3-yl]aminocarbonyl]methyl]-3-[(methoxycarbonyl)methyl]-1-(3-phenylpropyl)-2-pyrrolidinon

Unter Inertgas wird eine Lösung von 2,1 g (3S,5S)-5-(Aminocarbonylmethyl)-3-[(methoxycarbonyl)methyl]-1-(3-phenylpropyl)-2-pyrrolidinon in 20 ml absolutem Dimethylformamid portionsweise mit 275 mg einer 55%igen Dispersion von Natriumhydrid in Mineralöl versetzt und eine Stunde gerührt. Anschließend wird diese Lösung unter kräftigem Rühren zu einer Lösung von 1,5 g 3-Chlor-1-(4-cyanophenyl)-1-propen in 10 ml absolutem Dimethylformamid getropft. Die Reaktionslösung wird 2 Tage gerührt, danach in gesättigte Kochsalzlösung gegossen und dreimal mit Essigester extrahiert. Nach Trocknen und Eindampfen der organischen Phase erhält man 3,5 g eines Öls, welches mit Methylenchlorid/Methanol = 9:1 über Kieselgel chromatographiert wird.

Ausbeute: 1,1 g (37 % der Theorie),
$R_f$-Wert: 0,50 (Kieselgel; Methylenchlorid/Methanol = 9:1)

Beispiel CXXXVII

(3S,5S)-5-[(4'-Cyano-4-biphenylyl)oxymethyl]-3-(2-hydroxyethyl)-2-pyrrolidinon

Zu 1,5 g (3S,5S)-5-[(4'-Cyano-4-biphenylyl)oxymethyl]-3-[(methoxycarbonyl)methyl]-2-pyrrolidinon in 30 ml Tetrahydrofuran werden 0,25 ml Methanol und unter Eiskühlung 135 mg Lithiumborhydrid gegeben. Nach 2 Stunden Rühren bei Raumtemperatur wird mit Wasser versetzt, mit 3N Salzsäure angesäuert und viermal mit Methylenchlorid extrahiert. Die organische Phase wird mit Wasser gewaschen, über Magnesiumsulfat getrocknet, filtriert und einrotiert. Der erhaltene Rückstand wird über eine Kieselgelsäule mit Essigester/Methanol (100:1) gereinigt.

Ausbeute: 0,9 g (64 % der Theorie),
Schmelzpunkt: 114-116°C
$R_f$-Wert: 0,25 (Kieselgel; Methylenchlorid/Methanol = 100:2)

Analog wird erhalten:

(1) 1-(4'-Cyano-4-biphenylyl)-4-hydroxymethyl-2-pyrrolidinon

Schmelzpunkt: 159-161°C
$R_f$-Wert: 0,26 (Kieselgel; Methylenchlorid/Methanol = 95:5)

| Ber.: | C 73,96 | H 5,52 | N 9,58 |
|-------|---------|--------|--------|
| Gef.: | 73,70 | 5,67 | 9,72 |

Beispiel CXXXIX

1-(4'-Cyano-4-biphenylyl)-4-phosphonomethyl-2-pyrrolidinon

Zu 1,0 g 1-(4'-Cyano-4-biphenylyl)-4-(O,O'-dimethyl-phosphonomethyl)-2-pyrrolidinon und 0,78 g Natriumjodid in 5 ml Acetonitril werden 0,67 ml Chlortrimethylsilan zugegeben und der Ansatz 30 Minuten bei 40°C gerührt. Der Niederschlag wird abgesaugt und mit Acetonitril gewaschen. Das Filtrat wird eingeengt und der Rückstand 1,5 Stunden mit Wasser verrührt. Anschließend wird mit Natronlauge leicht alkalisch gestellt und zweimal mit Methylenchlorid, dem etwas Methanol zugesetzt ist, extrahiert. Die wäßrige Phase wird etwas eingeengt, filtriert und das Filtrat mit 2N Salzsäure angesäuert. Der ausgefallene Niederschlag wird abgesaugt, mit Wasser gewaschen und getrocknet

Ausbeute: 720 mg (78 % der Theorie),
$R_f$-Wert: 0,38 (reversed phase Kieselgel; Methanol/10%ige wäßrige Kochsalzlösung = 1:1)

| Ber.: | C 60,68 | H 4,81 | N 7,86 |
|-------|---------|--------|--------|
| Gef.: | 60,41   | 4,60   | 7,98   |

Beispiel CXL

1-(4'-Cyano-4-biphenylyl)-4-(O-methyl-phosphonomethyl)-2-pyrrolidinon

500 mg 1-(4'-Cyano-4-biphenylyl)-4-(O,O'-dimethyl-phosphonomethyl)-2-pyrrolidinon, 195 mg Natriumjodid und 5 ml Ethylmethylketon werden 3,5 Stunden unter Rückfluß erhitzt. Das Reaktionsgemisch wird im Eiswasserbad abgekühlt. Das Kristallisat wird isoliert und mit Aceton gewaschen. Das Kristallisat wird in Wasser gelöst, es wird filtriert und das Filtrat mit 2N Salzsäure angesäuert. Der Niederschlag wird abgesaugt, mit Wasser gewaschen und getrocknet.

Ausbeute: 360 mg (75 % der Theorie),
Schmelzpunkt: 198-204°C
$R_f$-Wert: 0,30 (reversed phase Kieselgel; Methanol/10%ige wäß rige Kochsalzlösung = 1:1)

Herstellung der Endprodukte:

Beispiel 1

(3S,5S)-5-[[4'-(Benzyloxycarbonylamidino)-4-biphenylyl]oxymethyl]-3-[(methoxycarbonyl)methyl]-1-phenyl-2-pyrrolidinon

Hergestellt analog Beispiel CVIII.

Schmelzpunkt: 157-159°C
$R_f$-Wert: 0,50 (Kieselgel; Chloroform/Methanol = 95:5)

| Ber.: | C 71,05 | H 5,62 | N 7,10 |
|-------|---------|--------|--------|
| Gef.: | 70,98   | 5,66   | 7,07   |

Analog werden erhalten:

(1)   (3S,5S)-5-[[4'-(N-Benzyloxycarbonylamidino)-4-biphenylyl]oxymethyl]-3-[(tert.butyloxycarbonyl)methyl]-2-pyrrolidinon Schmelzpunkt: 136-140°C

$R_f$-Wert: 0,39 (Kieselgel; Essigester)

(2) (3S,5S)-5-[[4'-(N-Benzyloxycarbonylamidino)-4-biphenylyl]oxymethyl]-3-[(methoxycarbonyl)methyl]-2-pyrrolidinon

Schmelzpunkt: 160-164°C
$R_f$-Wert: 0,43 (Kieselgel; Essigester)

| Ber.: | C 67,56 | H 5,67 | N 8,15 |
|-------|---------|--------|--------|
| Gef.: | 67,31   | 5,63   | 8,09   |

Beispiel 2

(3R,5S)-3-[4-(tert.Butyloxycarbonylamino)butyl]-5-[[4'[(methoxycarbonyl)methyl]-4-biphenylyl]oxymethyl]-1-methyl-2-pyrrolidinon

Hergestellt analog Beispiel CIX.

R$_f$-Wert: 0,28 (Kieselgel; Cyclohexan/Essigester = 1:4)

Analog werden erhalten:

(1)     (3S,5S)-5-[[4-[cis-4-(tert.Butyloxycarbonylaminomethyl)cyclohexyl]phenyl]oxymethyl]-3-[(methoxycarbonyl)methyl]-1-(3-phenylpropyl)-2-pyrrolidinon

R$_f$-Wert: 0,45 (Kieselgel; Cyclohexan/Essigester = 1:1)

(2)     (3S,5S)-5-[[4-[trans-4-(tert.Butyloxycarbonylaminomethyl)cyclohexyl]phenyl]oxymethyl]-3-[(methoxycarbonyl)methyl]-1-(3-phenylpropyl)-2-pyrrolidinon

R$_f$-Wert: 0,45 (Kieselgel; Cyclohexan/Essigester = 1:1)

Beispiel 3

(3S,5S)-5-[(4'-Amidino-4-biphenylyl)oxymethyl]-3-[(methoxycarbonyl)methyl]-1-(3-phenylpropyl)-2-pyrrolidinon-hydrochlorid-semihydrat

140 g (3S,5S)-5-[(4'-Cyano-4-biphenylyl)oxymethyl]-3-[(methoxycarbonyl)methyl]-1-(3-phenylpropyl)-2-pyrrolidinon werden in 1100 ml Methanol gelöst und auf -20°C abgekühlt. Man leitet bei dieser Temperatur 4 Stunden lang Salzsäuregas unter Rühren ein und rührt weitere 16 Stunden bei Raumtemperatur nach. Man dampft das Lösungsmittel im Vakuum ab, wobei das rohe Iminoester-hydrochlorid als zähes Öl zurück bleibt (172 g). Dieses Rohprodukt wird in 1500 ml Methanol gelöst und nach Zugabe von 144 g Ammoniumcarbonat 2 Stunden bei Raumtemperatur gerührt. Danach fügt man weitere 48 g Ammoniumcarbonat zu und rührt für weitere 1 1/2 Stunden. Das Reaktionsgemisch wird unter Rühren mit methanolischer Salzsäure auf pH 3,5 gebracht. Man engt nun im Vakuum auf etwa 800 ml ein und filtriert das ausgefallene Ammoniumchlorid ab. Das Filtrat wird nun bis zur beginnenden Kristallisation weiter eingeengt (auf etwa 350 ml). Nach beendeter Kristallisation filtriert man den Niederschlag ab und wäscht mit 75 ml eiskaltem Methanol und schließlich mit Aceton und Ether nach. Durch Einengen der Filtrate erhält man eine weitere Fraktion. Beide Kristallisate werden vereinigt und aus Methanol umkristallisiert.

Ausbeute: 128,7 g (83 % der Theorie),
Schmelzpunkt: 184-187°C (Zers.)

| Ber.: | C 66,11 | H 6,47 | N 7,71 | Cl 6,50 |
|-------|---------|--------|--------|---------|
| Gef.: | 65,98 | 6,41 | 7,67 | 6,67 |

Als Nebenprodukt wird erhalten:

(3S,5S)-5-[(4'-Aminocarbonyl-4-biphenylyl)oxymethyl]-3-[(aminocarbonyl)methyl]-1-(3-phenylpropyl)-2-pyrrolidinon

Schmelzpunkt: 197-198°C

Analog werden erhalten:

(1) (3S,5S)-5-[(4'-Amidino-4-biphenylyl)oxymethyl]-1-(3-cyclohexylpropyl)-3-[(methoxycarbonyl)methyl]-2-pyrrolidinonhydrochlorid

$R_f$-Wert: 0,36 (Kieselgel; Methylenchlorid/Methanol/konz. wäßriges Ammoniak = 80:20:5)

(2) (3S,5S)-5-[(4'-Amidino-4-biphenylyl)oxymethyl]-3-[(methoxycarbonyl)methyl]-2-pyrrolidinon x 1,25 HCl

Schmelzpunkt: ab 141°C (Zers.)
$R_f$-Wert: 0,30 (Kieselgel; Methylenchlorid/Methanol = 85:15)

| Ber.: | C 59,07 | H 5,72 | N 9,84 | Cl 10,38 |
|-------|---------|--------|--------|----------|
| Gef.: | 58,96 | 5,96 | 9,68 | 10,10 |

(3) (3S,5S)-5-[(4'-Amidino-4-biphenylyl)oxymethyl]-3-[(methoxycarbonyl)methyl]-1-methyl-2-pyrrolidinon-hydrochlorid

Schmelzpunkt: ab 138°C (Zers.)
$R_f$-Wert: 0,31 (Kieselgel; Methylenchlorid/Methanol/konz. wäßriges Ammoniak = 80:20:5)

(4) (3S,5S)-5-[(4'-Amidino-3-brom-4-biphenylyl)oxymethyl]-3-[(methoxycarbonyl)methyl]-1-(3-phenylpropyl)-2-pyrrolidinonhydrochlorid

Schmelzpunkt: 104-106°C (Zers.)
$R_f$-Wert: 0,46 (Kieselgel; Methylenchlorid/Methanol/konz. wäßriges Ammoniak = 40:10:2)

| Ber.: | C 58,59 | H 5,41 | N 6,83 |
|-------|---------|--------|--------|
| Gef.: | 58,70 | 5,66 | 6,64 |

(5) (3S,5S)-5-[(4'-Amidino-3-nitro-4-biphenylyl)oxymethyl]-3-[(methoxycarbonyl)methyl]-1-(3-phenylpropyl)-2-pyrrolidinon-hydrochlorid

$R_f$-Wert: 0,63 (Kieselgel; Methylenchlorid/Methanol/konz. wäßriges Ammoniak = 80:20:5)

(6) (3R,5R)-5-[(4'-Amidino-4-biphenylyl)oxymethyl]-3-[(methoxycarbonyl)methyl]-1-(3-phenylpropyl)-2-pyrrolidinon-semihydrochlorid

$R_f$-Wert: 0,43 (Kieselgel; Methylenchlorid/Methanol/konz. wäßriges Ammoniak = 80:20:5)

| Ber.: | C 69,58 | H 6,52 | N 8,11 | Cl 3,42 |
|-------|---------|--------|--------|---------|
| Gef.: | 69,69 | 6,73 | 7,97 | 2,86 |

(7) (3S,5S)-5-[(4'-Amidino-3-trifluormethyl-4-biphenylyl)oxymethyl]-3-[(methoxycarbonyl)methyl]-1-(3-phenylpropyl)-2-pyrrolidinon-hydrochlorid

$R_f$-Wert: 0,37 (Kieselgel; Methylenchlorid/Methanol/konz. wäßriges Ammoniak = 80:20:5)

(8) (3S,5S)-5-[(4'-Amidino-3-fluor-4-biphenylyl)oxymethyl]-3-[(methoxycarbonyl)methyl]-1-(3-phenylpropyl)-2-pyrrolidinon-hydrochlorid

$R_f$-Wert: 0,36 (Kieselgel; Methylenchlorid/Methanol/konz. wäßriges Ammoniak = 80:20:5)

(9) (3S,5S)-5-[(4'-Amidino-2,3-dimethyl-4-biphenylyl)oxymethyl]-3-[(methoxycarbonyl)methyl]-1-(3-phenylpropyl)-2-pyrrolidinon-hydrochlorid

$R_f$-Wert: 0,42 (Kieselgel; Methylenchlorid/Methanol/konz. wäßriges Ammoniak = 80:20:5)

(10) (3S,5S)-5-[(3-Acetylamino-4'-amidino-4-biphenylyl)oxymethyl]-3-[(methoxycarbonyl)methyl]-1-(3-phenylpropyl)-2-pyrrolidinon-hydrochlorid

$R_f$-Wert: 0,38 (Kieselgel; Methylenchlorid/Methanol/konz. wäßriges Ammoniak = 80:20:5)

(11) (3S,5S)-5-[(4'-Amidino-3-methansulfonylamino-4-biphenylyl)oxymethyl]-3-[(methoxycarbonyl)methyl]-1-(3-phenylpropyl)-2-pyrrolidinon-hydrochlorid

$R_f$-Wert: 0,42 (Kieselgel; Methylenchlorid/Methanol/konz. wäßriges Ammoniak = 80:20:5)

(12) (3S,5S)-5-[(4'-Amidino-2'-methyl-4-biphenylyl)oxymethyl]-3-[(methoxycarbonyl)methyl]-1-(3-phenylpropyl)-2-pyrrolidinon-hydrochlorid

$R_f$-Wert: 0,44 (Kieselgel; Methylenchlorid/Methanol/konz. wäßriges Ammoniak = 80:20:5)

(13) (3S,5S)-5-[[(4'-Amidino-4-biphenylyl)carbonyl]aminomethyl]-3-[(methoxycarbonyl)methyl]-1-(3-phenylpropyl)-2-pyrrolidinon-hydrochlorid

$R_f$-Wert: 0,34 (Kieselgel; Methylenchlorid/Methanol/konz. wäßriges Ammoniak = 40:10:2)

(14) (3S,5S)-5-[[trans-4-(4-Amidinophenyl)cyclohexyl]oxymethyl]-3-[(methoxycarbonyl)methyl]-1-(3-phenylpropyl)-2-pyrrolidinon-hydrochlorid

$R_f$-Wert: 0,25 (Kieselgel; Methylenchlorid/Methanol/konz. wäßriges Ammoniak = 80:20:5)

(15) (3S,5S)-5-[2-[(6-Amidino-2-naphthylcarbonyl)amino]ethyl]-3-[(methoxycarbonyl)methyl]-1-(3-phenylpropyl)-2-pyrrolidinon-hydrochlorid

$R_f$-Wert: 0,26 (Kieselgel; Methylenchlorid/Methanol/konz. wäßriges Ammoniak = 80:20:3)

(16) (3S,5S)-5-[(6-Amidino-2-naphthylcarbonyl)aminomethyl]-3-[(methoxycarbonyl)methyl]-1-(3-phenylpropyl)-2-pyrrolidinon-hydrochlorid

$R_f$-Wert: 0,26 (Kieselgel; Methylenchlorid/Methanol/konz. wäßriges Ammoniak = 80:20:3)

(17) (3S,5S)-5-[(7-Amidino-2-naphthylcarbonyl)aminomethyl]-3-[(methoxycarbonyl)methyl]-1-(3-phenylpropyl)-2-pyrrolidinon-hydrochlorid

$R_f$-Wert: 0,19 (Kieselgel; Methylenchlorid/Methanol = 9:1)

(18) (3S,5S)-5-[2-[(7-Amidino-2-naphthylcarbonyl)amino]ethyl]-3-[(methoxycarbonyl)methyl]-1-(3-phenylpropyl)-2-pyrrolidinon-hydrochlorid

$R_f$-Wert: 0,25 (Kieselgel; Methylenchlorid/Methanol = 9:1)

(19) (3S,5S)-5-[[trans-3-(4-Amidinophenyl)cyclobutyl]carbonylaminomethyl]-3-[(methoxycarbonyl)methyl]-1-(3-phenylpropyl)-2-pyrrolidinon-hydrochlorid x 1,5 Wasser

$R_f$-Wert: 0,42 (Kieselgel; Methylenchlorid/Methanol/konz. wäßriges Ammoniak = 35:15:4)

| Ber.: | C 61,31 | H 7,10 | N 9,86 |
|---|---|---|---|
| Gef.: | 61,45 | 7,18 | 9,64 |

(20) (3S,5S)-5-[[cis-3-(4-Amidinophenyl)cyclobutyl]carbonylaminomethyl]-3-[(methoxycarbonyl)methyl]-1-(3-phenylpropyl)-2-pyrrolidinon-hydrochlorid x 1,5 Wasser

R$_f$-Wert: 0,42 (Kieselgel; Methylenchlorid/Methanol/konz. wäßriges Ammoniak = 35:15:4)

| Ber.: | C 61,31 | H 7,10 | N 9,86 |
|-------|---------|--------|--------|
| Gef.: | 61,67 | 6,98 | 9,80 |

(21) (3S,5S)-5-[2-[[cis-3-(4-Amidinophenyl)cyclobutyl]carbonylamino]ethyl]-3-[(methoxycarbonyl)methyl]-1-(3-phenylpropyl)-2-pyrrolidinon-hydrochlorid

R$_f$-Wert: 0,48 (Kieselgel; Methylenchlorid/Methanol/konz. wäßriges Ammoniak = 35:15:4)

(22) (3S,5S)-5-[2-[[trans-3-(4-Amidinophenyl)cyclobutyl]carbonylamino]ethyl]-3-[(methoxycarbonyl)methyl]-1-(3-phenylpropyl)-2-pyrrolidinon-hydrochlorid

R$_f$-Wert: 0,48 (Kieselgel; Methylenchlorid/Methanol/konz. wäßriges Ammoniak = 35:15:4)

(23) (3S,5S)-5-[[3-(4-Amidinophenyl)propyl]carbonylaminomethyl]-3-[(methoxycarbonyl)methyl]-1-(3-phenylpropyl)-2-pyrrolidinon-hydrochlorid

R$_f$-Wert: 0,50 (Kieselgel; Methylenchlorid/Methanol = 4:1)

(24) (3S,5S)-5-[(4'-Amidino-4-biphenylyl)oxymethyl]-3-[(methoxycarbonyl)methyl]-3-methyl-1-(3-phenylpropyl)-2-pyrrolidinon-hydrochlorid

R$_f$-Wert: 0,25 (Kieselgel; Methylenchlorid/Cyclohexan/Metha nol/konz. wäßriges Ammoniak = 68:15:15:2)

(25) (3S,5S)-5-[(4'-Amidino-4-biphenylyl)oxymethyl]-1-benzyl-3-[(methoxycarbonyl)methyl]-2-pyrrolidinon-hydrochlorid

R$_f$-Wert: 0,19 (Kieselgel; Methylenchlorid/Cyclohexan/Methanol/konz. wäßriges Ammoniak = 68:15:15:2)

(26) (3S,5S)-5-[(4'-Amidino-4-biphenylyl)oxymethyl]-3-[(methoxycarbonyl)methyl]-1-(2-phenylethyl)-2-pyrrolidinon-hydrochlorid

R$_f$-Wert: 0,22 (Kieselgel; Methylenchlorid/Cyclohexan/Methanol/konz. wäßriges Ammoniak = 68:15:15:2)

(27) (3S,5S)-5-[(4'-Amidino-4-biphenylyl)oxymethyl]-3-[(methoxycarbonyl)methyl]-1-(4-phenylbutyl)-2-pyrrolidinon-hydrochlorid

R$_f$-Wert: 0,41 (Kieselgel; Methylenchlorid/Cyclohexan/Methanol/konz. wäßriges Ammoniak = 68:15:15:2)

(28) (3S,5S)-5-[(4'-Amidino-4-biphenylyl)oxymethyl]-3-[(methoxycarbonyl)methyl]-1-(4-phenyloxybutyl)-2-pyrrolidinon-hydrochlorid

R$_f$-Wert: 0,38 (Kieselgel; Methylenchlorid/Cyclohexan/Methanol/konz. wäßriges Ammoniak = 68:15:15:2)

(29) (3S,5S)-5-[[4'-(n-Butylamidino)-4-biphenylyl]oxymethyl]-3-[(methoxycarbonyl)methyl]-1-(3-phenylpropyl)-2-pyrrolidinon-hydrochlorid

Der Iminoester wird in absolutem Methanol aufgenommen und zur Lösung die 20-fache molare Menge an n-Butylamin zugesetzt.

R$_f$-Wert: 0,38 (Kieselgel; Methylenchlorid/Cyclohexan/Methanol/konz. wäßriges Ammoniak = 68:15:15:2)

Als Nebenprodukt wird (3S,5S)-5-[[4'-(n-Butylamidino)-4-biphenylyl]oxymethyl]-3-[(n-butylaminocarbonyl)methyl]-1-(3-phenylpropyl)-2-pyrrolidinon-hydrochlorid erhalten.

R$_f$-Wert: 0,60 (Kieselgel; Methylenchlorid/Methanol/konz. wäßriges Ammoniak = 4:1:0,2)

(30) (3S,5S)-3-[(Methoxycarbonyl)methyl]-5-[(4'-methylamidino-4-biphenylyl)oxymethyl]-1-(3-phenylpropyl)-2-pyrrolidinon-hydrochlorid

Die Umsetzung wird analog Beispiel 6 (31) durchgeführt.

R$_f$-Wert: 0,22 (Kieselgel; Methylenchlorid/Cyclohexan/Methanol/konz. wäßriges Ammoniak = 68:15:15:2)

Als Nebenprodukt wird (3S,5S)-5-[(4'-Methylamidino-4-biphenylyl)oxymethyl]-3-[(methylaminocarbonyl)methyl]-1-(3-phenylpropyl)-2-pyrrolidinon-hydrochlorid erhalten.

| Ber.: | C 67,80 | H 6,79 | N 10,20 | Cl 6,46 |
|-------|---------|--------|---------|---------|
| Gef.: | 67,35 | 6,68 | 10,23 | 6,43 |

(31) (3S,5S)-5-[(4'-Isopropylamidino-4-biphenylyl)oxymethyl]-3-[(methoxycarbonyl)methyl]-1-(3-phenylpropyl)-2-pyrrolidinon-hydrochlorid

Die Umsetzung wird analog Beispiel 6 (31) durchgeführt.

R$_f$-Wert: 0,47 (Kieselgel; Methylenchlorid/Cyclohexan/Methanol/konz. wäßriges Ammoniak = 68:15:15:2)

(32) (3S,5S)-5-[(4'-Amidino-4-biphenylyl)oxymethyl]-3-[(methoxycarbonyl)methyl]-1,3-bis-(3-phenylpropyl)-2-pyrrolidinon-hydrochlorid

R$_f$-Wert: 0,31 (Kieselgel; Methylenchlorid/Cyclohexan/Methanol/konz. wäßriges Ammoniak = 68:15:15:2)

(33) (3S,5S)-5-[(4'-Amidino-4-biphenylyl)oxymethyl]-3-(n-butyl)-3-[(methoxycarbonyl)methyl]-1-(3-phenylpropyl)-2-pyrrolidinon-hydrochlorid

R$_f$-Wert: 0,22 (Kieselgel; Methylenchlorid/Cyclohexan/Methanol/konz. wäßriges Ammoniak = 68:15:15:2)

(34) (3S,5S)-5-[(7-Amidino-9-keto-2-fluorenyl)oxymethyl]-3-[(methoxycarbonyl)methyl]-1-(3-phenylpropyl)-2-pyrrolidinon-hydrochlorid-hydrat

| Ber.: | C 64,13 | H 5,69 | N 7,24 | Cl 6,12 |
|-------|---------|--------|--------|---------|
| Gef.: | 64,90 | 5,86 | 7,39 | 6,76 |

(35) (3R,5S)-5-[(4'-Amidino-4-biphenylyl)oxymethyl]-3-[(methoxycarbonyl)methyl]-1-(3-phenylpropyl)-2-pyrrolidinon-hydrochlorid

R$_f$-Wert: 0,33 (Kieselgel; Methylenchlorid/Methanol/methanolische Salzsäure = 9:1:0,05)

(36) (3S,5S)-5-[(4'-Amidino-4-biphenylyl)aminomethyl]-3-[(methoxycarbonyl)methyl]-1-(3-phenylpropyl)-2-pyrrolidinon x 1,25 HCl

| Ber.: | C 66,15 | H 6,48 | N 10,29 | Cl 8,15 |
|-------|---------|--------|---------|---------|
| Gef.: | 65,96 | 6,66 | 10,38 | 7,96 |

(37) (3S,5S)-5-[[N-(4'-Amidino-4-biphenylyl)-N-methansulfonyl]aminomethyl]-3-[(methoxycarbonyl)methyl]-1-(3-phenylpropyl)-2-pyrrolidinon-hydrochlorid

| Ber.: | C 60,62 | H 6,25 | N 9,12 | Cl 5,77 |
|-------|---------|--------|--------|---------|
| Gef.: | 60,87 | 6,14 | 8,93 | 5,66 |

(38)  (3S,5S)-5-[[N-(4'-Amidino-4-biphenylyl)-N-acetyl]aminomethyl]-3-[(methoxycarbonyl)methyl]-1-(3-phenylpropyl)-2-pyrrolidinon-hydrochlorid

| Ber.: | C 66,59 | H 6,47 | N 9,71 | Cl 6,14 |
|-------|---------|--------|--------|---------|
| Gef.: | 66,61   | 6,85   | 9,52   | 5,94    |

(39)  (3S,5S)-5-[[2-[(4-Amidinophenyl)amino]phenyl]carbonylaminomethyl]-3-[(methoxycarbonyl)methyl]-1-(3-phenylpropyl)-2-pyrrolidinon-hydrochlorid

Schmelzpunkt: ab 105°C (Zers.)
$R_f$-Wert: 0,27 (Kieselgel; Cyclohexan/Essigester/Methanol = 2:1:0,6)

(40)  (3S,5S)-5-[[4-[(4-Amidinophenyl)aminocarbonyl]phenyl]oxymethyl]-3-[(methoxycarbonyl)methyl]-1-(3-phenylpropyl)-2-pyrrolidinon-hydrochlorid

$R_f$-Wert: 0,63 (Kieselgel; Methylenchlorid/Ethanol = 4:1)
Massenspektrum: $(M + H)^+ = 543$

(41)  (3S,5S)-5-[[4-[(4-Amidinophenyl)carbonylamino]phenyl]oxymethyl]-3-[(methoxycarbonyl)methyl]-1-(3-phenylpropyl)-2-pyrrolidinon-hydrochlorid

$R_f$-Wert: 0,30 (Kieselgel; Methylenchlorid/Methanol/konz. wäßriges Ammoniak = 16:4:1)

(42)  (3S,5S)-5-[[4-[(4-Amidinophenyl)aminosulfonyl]phenyl]oxymethyl]-3-[(methoxycarbonyl)methyl]-1-(3-phenylpropyl)-2-pyrrolidinon-hydrochlorid

$R_f$-Wert: 0,33 (Kieselgel; Methylenchlorid/Methanol/konz. wäßriges Ammoniak = 16:2:1)
Massenspektrum: $(M + H)^+ = 579$

(43)  (3S,5S)-5-[[4-[(3-Amidinophenyl)sulfenyl]phenyl]oxymethyl]-3-[(methoxycarbonyl)methyl]-1-(3-phenylpropyl)-2-pyrrolidinon-hydrochlorid

$R_f$-Wert: 0,42 (Kieselgel; Essigester/Cyclohexan = 3:2)

(44)  (3S,5S)-5-[[4-[(3-Amidinophenyl)carbonyl]phenyl]oxymethyl]-3-[(methoxycarbonyl)methyl]-1-(3-phenylpropyl)-2-pyrrolidinon-hydrochlorid

$R_f$-Wert: 0,21 (Kieselgel; Methylenchlorid/Methanol/konz. wäßriges Ammoniak = 16:4:1)

(45)  (3S,5S)-5-[[4-[(3-Amidinophenyl)sulfonyl]phenyl]oxymethyl]-3-[(methoxycarbonyl)methyl]-1-(3-phenylpropyl)-2-pyrrolidinon-hydrochlorid

$R_f$-Wert: 0,37 (Kieselgel; Methylenchlorid/Ethanol/konz. wäßriges Ammoniak = 16:4:1)
Massenspektrum: $(M + H)^+ = 564$

(46)  (3S,5S)-5-[(4'-Amidino-4-biphenylyl)oxymethyl]-1,3-bis[(methoxycarbonyl)methyl]-2-pyrrolidinon-hydrochlorid-semihydrat

$R_f$-Wert: 0,40 (Kieselgel; Methylenchlorid/Methanol/konz. wäßriges Ammoniak = 15:5:1)

| Ber.: | C 57,77 | H 5,86 | N 8,42 | Cl 7,11 |
|-------|---------|--------|--------|---------|
| Gef.: | 57,97   | 5,95   | 8,38   | 7,37    |

Massenspektrum: $(M + H)^+ = 454$

(47)  (3S,5S)-5-[(4'-Amidino-4-biphenylyl)oxymethyl]-1-[(ethylaminocarbonyl)methyl]-3-[(methoxycarbonyl)methyl]-2-pyrrolidinon-hydrochlorid x 0,25 Wasser

$R_f$-Wert: 0,27 (Kieselgel; Methylenchlorid/Methanol/konz. wäßriges Ammoniak = 30:10:2)

| Ber.: | C 59,17 | H 6,26 | N 11,04 | Cl 6,99 |
|-------|---------|--------|---------|---------|
| Gef.: | 59,13   | 6,47   | 10,91   | 6,78    |

(48)  (3S,5S)-5-[(4'-Amidino-4-biphenylyl)oxymethyl]-1-[(dimethylaminocarbonyl)methyl]-3-[(methoxycarbonyl)methyl]-2-pyrrolidinon-hydrochlorid-semihydrat

$R_f$-Wert: 0,33 (Kieselgel; Methylenchlorid/Methanol/konz. wäßriges Ammoniak = 30:10:2)

| Ber.: | C 58,65 | H 6,30 | N 10,94 | Cl 6,92 |
|-------|---------|--------|---------|---------|
| Gef.: | 58,50   | 6,55   | 10,79   | 6,80    |

(49)  (3S,5S)-5-[(4'-Amidino-4-biphenylyl)oxymethyl]-1-[(benzylaminocarbonyl)methyl]-3-[(methoxycarbonyl)methyl]-2-pyrrolidinon-hydrochlorid

$R_f$-Wert: 0,50 (Kieselgel; Methylenchlorid/Methanol/konz. wäßriges Ammoniak = 30:10:2)

| Ber.: | C 63,88 | H 5,72 | N 9,93 | Cl 6,29 |
|-------|---------|--------|--------|---------|
| Gef.: | 64,06   | 6,00   | 9,92   | 6,31    |

(50)  (3S,5S)-5-[(4'-Amidino-4-biphenylyl)oxymethyl]-1-benzoyl-3-[(methoxycarbonyl)methyl]-pyrrolidin-hydrochloridsemihydrat

$R_f$-Wert: 0,18 (Kieselgel; Methylenchlorid/Methanol = 10:1)

| Ber.: | C 65,05 | H 6,04 | N 8,13 | Cl 6,86 |
|-------|---------|--------|--------|---------|
| Gef.: | 65,00   | 6,30   | 7,99   | 6,61    |

(51)  (3S,5S)-5-[(4'-Amidino-4-biphenylyl)oxymethyl]-1-methansulfonyl-3-[(methoxycarbonyl)methyl]-pyrrolidin-hydrochlorid

$R_f$-Wert: 0,24 (Kieselgel; Methylenchlorid/Methanol = 10:1)

| Ber.: | C 54,82 | H 5,85 | N 8,72 | Cl 7,36 |
|-------|---------|--------|--------|---------|
| Gef.: | 54,68   | 5,82   | 8,47   | 7,20    |

(52) (3S,5S)-1-Acetyl-5-[(4'-amidino-4-biphenylyl)oxymethyl]-3-[(methoxycarbonyl)methyl]-pyrrolidin-hydrochlorid

$R_f$-Wert: 0,16 (Kieselgel; Methylenchlorid/Methanol = 10:1)

| Ber.: | C 61,95 | H 6,33 | N 9,42 | Cl 7,95 |
|-------|---------|--------|--------|---------|
| Gef.: | 61,76   | 6,31   | 9,11   | 7,84    |

(53)  (3S,5S)-5-[(4'-Amidino-4-biphenylyl)oxymethyl]-1-(ethylaminocarbonyl)-3-[(methoxycarbonyl)methyl]-pyrrolidin x 1,25 HCl

$R_f$-Wert: 0,13 (Kieselgel; Methylenchlorid/Methanol = 10:1)

| Ber.: | C 59,58 | H 6,51 | N 11,58 | Cl 9,16 |
|---|---|---|---|---|
| Gef.: | 59,73 | 6,56 | 11,33 | 9,07 |

(54) (3S,5S)-5-[(4'-Amidino-4-biphenylyl)oxymethyl]-1-(dimethylaminosulfonyl)-3-[(methoxycarbonyl)methyl]-pyrrolidin-hydrochlorid

Schmelzpunkt: 196-198°C,
$R_f$-Wert: 0,18 (Kieselgel; Methylenchlorid/Methanol = 10:1)

| Ber.: | C 54,06 | H 6,11 | N 10,96 | Cl 6,94 | S 6,27 |
|---|---|---|---|---|---|
| Gef.: | 53,92 | 6,14 | 10,77 | 7,02 | 6,32 |

(55) (3S,5S)-5-[(4'-Amidino-4-biphenylyl)oxymethyl]-3-[(methoxycarbonyl)methyl]-1-methyl-pyrrolidin-hydrochlorid

$R_f$-Wert: 0,18 (Kieselgel; Methylenchlorid/Methanol = 10:1)
Massenspektrum: $(M + H)^+ = 382$

(56) (3R,5S)-5-[(4'-Amidino-4-biphenylyl)oxymethyl]-3-[(methoxycarbonyl)methyl]-2-pyrrolidinon-hydrochlorid

$R_f$-Wert: 0,20 (Kieselgel; Methylenchlorid/Methanol = 9:1)

| Ber.: | C 57,86 | H 6,01 | N 9,64 | Cl 8,13 |
|---|---|---|---|---|
| Gef.: | 58,06 | 5,93 | 9,66 | 8,25 |

(57)  (3S,5S)-5-[(4'-Amidino-4-biphenylyl)oxymethyl]-3-[(methoxycarbonyl)methyl]-3-methyl-2-pyrrolidinon-hydrochlorid-semihydrat

$R_f$-Wert: 0,28 (Kieselgel; Methylenchlorid/Methanol/konz. wäßriges Ammoniak = 80:20:2)

| Ber.: | C 59,93 | H 6,17 | N 9,53 | Cl 8,04 |
|---|---|---|---|---|
| Gef.: | 59,70 | 6,08 | 9,37 | 8,21 |

(58)  (3S,5S)-5-[(4'-Amidino-4-biphenylyl)oxymethyl]-3-[(methoxycarbonyl)methyl]-1-(2-methoxyethyl)-2-pyrrolidinon-hydrochlorid
Als Base wurde konz. wäßriges Ammoniak verwendet.

$R_f$-Wert: 0,39 (reversed phase Kieselgel (RP8); Methanol/10 % wäßrige Kochsalzlösung = 6:4)

(59)  (3S,5S)-5-[(4'-Amidino-4-biphenylyl)oxymethyl]-1-(2-hydroxyethyl)-3-[(methoxycarbonyl)methyl]-2-pyrrolidinon-hydrochlorid
Als Base wurde konz. wäßriges Ammoniak verwendet.

$R_f$-Wert: 0,55 (reversed phase Kieselgel (RP8); Methanol/10 % wäßrige Kochsalzlösung = 6:4)
und
(3S,5S)-5-[(4'-Amidino-4-biphenylyl)oxymethyl]-3-carboxymethyl-1-(2-hydroxyethyl)-2-pyrrolidinon

R$_f$-Wert: 0,70 (reversed phase Kieselgel (RP8); Methanol/10 % wäßrige Kochsalzlösung = 6:4)

(60) (3S,5S)-5-[(4'-Amidino-4-biphenylyl)oxymethyl]-3-[(ethoxycarbonyl)methyl]-2-pyrrolidinon-hydrochlorid
Als Säure wurde ethanolische Salzsäure verwendet.

R$_f$-Wert: 0,44 (reversed phase Kieselgel (RP8); Methanol/10 % wäßrige Kochsalzlösung = 6:4)

| Ber. x 0,25 H$_2$0: | C 60,55 | H 6,12 | N 9,63 | Cl 8,12 |
|---|---|---|---|---|
| Gef.: | 60,49 | 6,23 | 9,65 | 8,21 |

(61)  (3R,5R)-5-[(4'-Amidino-4-biphenylyl)oxymethyl]-3-[(methoxycarbonyl)methyl]-2-pyrrolidinon-hydrochlorid-semihydrat

Schmelzpunkt: 138°C (Zers.)
R$_f$-Wert: 0,52 (reversed phase Kieselgel (RP8); Methanol/10 % wäßrige Kochsalzlösung = 6:4)

| Ber.: | C 59,08 | H 5,90 | N 9,84 | Cl 8,31 |
|---|---|---|---|---|
| Gef.: | 58,96 | 6,19 | 9,68 | 8,93 |

(62) (3S,5S)-5-[2-[(4'-Amidino-3-biphenylyl)amino]ethyl]-3-[(methoxycarbonyl)methyl]-1-(3-phenylpropyl)-2-pyrrolidinon-hydrochlorid

R$_f$-Wert: 0,39 (Kieselgel; Chloroform/Methanol = 3:1)

(63)  (3S,5S)-5-[2-[[(3'-Amidino-4-biphenylyl)carbonyl]amino]ethyl]-3-[(methoxycarbonyl)methyl]-1-(3-phenylpropyl)-2-pyrrolidinon-hydrochlorid

R$_f$-Wert: 0,39 (Kieselgel; Methylenchlorid/Methanol = 3:1)

(64)  (3S,5S)-5-[(4'-Amidino-3-methylsulfonyl-4-biphenylyl)oxymethyl]-3-[(methoxycarbonyl)methyl]-1-(3-phenylpropyl)-2-pyrrolidinon-hydrochlorid

R$_f$-Wert: 0,24 (Kieselgel; Methylenchlorid/Cyclohexan/Methanol/wäßriges Ammoniak = 7:1,5:1,5:0,2)
Massenspektrum: (M + H)$^+$ = 578

(65)  (3S,5S)-5-[(4'-Amidino-3-methylsulfenyl-4-biphenylyl)oxymethyl]-3-[(methoxycarbonyl)methyl]-1-(3-phenylpropyl)-2-pyrrolidinon-hydrochlorid

R$_f$-Wert: 0,25 (Kieselgel; Methylenchlorid/Cyclohexan/Methanol/wäßriges Ammoniak = 7:1,5:1,5:0,2)

| Ber.: | C 63,96 | H 6,23 | N 7,22 | S 5,51 | Cl 6,09 |
|---|---|---|---|---|---|
| Gef.: | 64,12 | 6,50 | 7,03 | 5,36 | 5,88 |

(66)  (3S,5S)-5-[[(4'-Amidino-4-biphenylyl)sulfonyl]aminomethyl]-3-[(methoxycarbonyl)methyl]-1-(3-phenylpropyl)-2-pyrrolidinon-hydrochlorid

R$_f$-Wert: 0,19 (Kieselgel; Methylenchlorid/Cyclohexan/Methanol/konz. wäßriges Ammoniak = 7:1,5:1,5:0,2)

(67)  (3S,5S)-5-[2-[(4-Amidinocinnamoyl)amino]ethyl]-3-[(methoxycarbonyl)methyl]-1-(3-phenylpropyl)-2-pyrrolidinon-hydrochlorid

R$_f$-Wert: 0,29 (Kieselgel; Methylenchlorid/Methanol/Cyclohexan/konz. wäßriges Ammoniak = 68:15:15:2)

(68)    (3S,5S)-5-[(4'-Amidino-4-biphenylyl)oxymethyl]-1-[3-(3,4-dimethoxyphenyl)propyl]-3-[(methoxycarbonyl)methyl]-2-pyrrolidinon-semihydrochlorid

$R_f$-Wert: 0,24 (Kieselgel; Methylenchlorid/Methanol/konz. wäßriges Ammoniak = 4:1:0,25)

| Ber.: | C 66,51 | H 6,54 | N 7,27 | Cl 3,07 |
|---|---|---|---|---|
| Gef.: | 66,34 | 6,31 | 7,07 | 2,92 |

(69)  (3S,5S)-5-[(4'-Amidino-4-biphenylyl)oxymethyl]-3-[(methoxycarbonyl)methyl]-1-[3-(4-hexyloxyphenyl)propyl]-2-pyrrolidinon-hydrochlorid

$R_f$-Wert: 0,23 (Kieselgel; Methylenchlorid/Methanol/konz. wäßriges Ammoniak = 4:1:0,25)

(70)  (3S,5S)-5-[(4'-Amidino-4-biphenylyl)oxymethyl]-1-[3-(4-tert.butylphenyl)propyl]-3-[(methoxycarbonyl)methyl]-2-pyrrolidinon-hydrochlorid

$R_f$-Wert: 0,22 (Kieselgel; Methylenchlorid/Methanol/konz. wäßriges Ammoniak = 4:1:0,25)

(71)  (3S,5S)-5-[(4'-Amidino-4-biphenylyl)oxymethyl]-3-[(methoxycarbonyl)methyl]-1-[3-(3-trifluormethylphenyl)propyl]-2-pyrrolidinon-hydrochlorid

$R_f$-Wert: 0,22 (Kieselgel; Methylenchlorid/Methanol/konz. wäßriges Ammoniak = 4:1:0,25)

(72) (3S,5S)-5-[(4'-Amidino-4-biphenylyl)oxymethyl]-1-[3-2,4-dichlorphenyl)propyl]-3-[(methoxycarbonyl)methyl]-2-pyrrolidinon-hydrochlorid

$R_f$-Wert: 0,28 (Kieselgel; Methylenchlorid/Methanol/konz. wäßriges Ammoniak = 4:1:0,25)

(73)  (3S,5S)-5-[(4'-Amidino-4-biphenylyl)oxymethyl]-3-[(methoxycarbonyl)methyl]-1-[3-(3-benzylphenyl)propyl]-2-pyrrolidinon-hydrochlorid

$R_f$-Wert: 0,34 (Kieselgel; Methylenchlorid/Methanol/konz. wäßriges Ammoniak = 4:1:0,25)

(74) (3S,5S)-5-[(4'-Amidino-4-biphenylyl)oxymethyl]-1-(4,4-diphenylbutyl)-3-[(methoxycarbonyl)methyl]-2-pyrrolidinonhydrochlorid

$R_f$-Wert: 0,29 (Kieselgel; Methylenchlorid/Methanol/konz. wäßriges Ammoniak = 4:1:0,25)

(75)    (3S,5S)-5-[(4'-Amidino-4-biphenylyl)oxymethyl]-3-[(methoxycarbonyl)methyl]-1-[3-(4-methylsulfenylphenyl)propyl]-2-pyrrolidinon-hydrochlorid

$R_f$-Wert: 0,23 (Kieselgel; Methylenchlorid/Methanol/konz. wäßriges Ammoniak = 4:1:0,25)

(76)    (3S,5S)-5-[(4'-Amidino-4-biphenylyl)oxymethyl]-3-[(methoxycarbonyl)methyl]-1-[3-(4-methylsulfonylphenyl)propyl]-2-pyrrolidinon-hydrochlorid

$R_f$-Wert: 0,25 (Kieselgel; Methylenchlorid/Methanol/konz. wäßriges Ammoniak = 4:2:0,25)

(77) (3S,5S)-5-[(4'-Amidino-4-biphenylyl)oxymethyl]-1-(4-biphenylylmethyl)-3-[(methoxycarbonyl)methyl]-2-pyrrolidinonhydrochlorid

$R_f$-Wert: 0,28 (Kieselgel; Methylenchlorid/Methanol/konz. wäßriges Ammoniak = 4:2:0,25)

| Ber.: | C 69,91 | H 5,87 | N 7,19 | Cl 6,07 |
|-------|---------|--------|--------|---------|
| Gef.: | 69,82 | 5,86 | 7,39 | 6,17 |

(78)  (3R,5S)-5-[(4'-Amidino-4-biphenylyl)oxymethyl]-3-[3-(methoxycarbonyl)propyl]-1-(3-phenylpropyl)-2-pyrrolidinonhydrochlorid

$R_f$-Wert: 0,51 (Kieselgel; Methylenchlorid/Methanol/Wasser = 8:2:0,10)

| Ber.: | C 68,13 | H 6,79 | N 7,45 |
|-------|---------|--------|--------|
| Gef.: | 67,94 | 7,03 | 7,26 |

(79)  (3S,5S)-5-[[[(3'-Amidino-4-biphenylyl)carbonyl]aminomethyl]-3-[(methoxycarbonyl)methyl]-1-(3-phenylpropyl)-2-pyrrolidinon-hydrochlorid

$R_f$-Wert: 0,37 (Kieselgel; Methylenchlorid/Methanol/konz. wäßriges Ammoniak = 4:1:0,25)
Massenspektrum: $(M + H)^+ = 527$

(80)  (3S,5S)-5-[[[(4'-Amidino-3-biphenylyl)carbonyl]aminomethyl]-3-[(methoxycarbonyl)methyl]-1-(3-phenylpropyl)-2-pyrrolidinon-hydrochlorid

$R_f$-Wert: 0,25 (Kieselgel; Methylenchlorid/Methanol/konz. wäßriges Ammoniak = 4:1:0,25)
Massenspektrum: $(M + H)^+ = 527$

(81) (3S,5S)-5-[(4'-Amidino-4-biphenylyl)sulfonylmethyl]-3-[(methoxycarbonyl)methyl]-1-(3-phenylpropyl)-2-pyrrolidinon-hydrochlorid

$R_f$-Wert: 0,16 (Kieselgel; Methylenchlorid/Methanol/Cyclohexan/konz. wäßriges Ammoniak = 68:15:15:2)

(82) (3S,5S)-5-[(4'-Amidino-4-biphenylyl)sulfenylmethyl]-3-[(methoxycarbonyl)methyl]-1-(3-phenylpropyl)-2-pyrrolidinon-hydrochlorid

$R_f$-Wert: 0,81 (Kieselgel; Methylenchlorid/Methanol/konz. wäßriges Ammoniak = 4:1:0,25)

(83)  (3S,5S)-5-[[[1-(4-Amidinophenyl)-1-propen-3-yl]aminocarbonyl]methyl]-3-[(methoxycarbonyl)methyl]-1-(3-phenylpropyl)-2-pyrrolidinon-hydrochlorid

$R_f$-Wert: 0,15 (Kieselgel; Methylenchlorid/Methanol/konz. wäßriges Ammoniak = 4:1:0,25)

Als Nebenprodukt wird erhalten:

(3S,5S)-5-[[[1-(4-Amidinophenyl)-1-propen-3-yl]aminocarbonyl]methyl]-3-[(aminocarbonyl)methyl]-1-(3-phenylpropyl)-2-pyrrolidinon-hydrochlorid

$R_f$-Wert: 0,13 (Kieselgel; Methylenchlorid/Methanol/konz. wäßriges Ammoniak = 4:1:0,25)
Massenspektrum: $(M + H)^+ = 476$

(84) (3R,S;4R,S)-4-[(4'-Amidino-4-biphenylyl)oxymethyl]-3-[(methoxycarbonyl)methyl]-2-pyrrolidinon-hydrochlorid

$R_f$-Wert: 0,30 (Kieselgel; Methylenchlorid/Methanol/wäßriges Ammoniak = 80:20:5)

(85) (3S,5S)-5-[(4'-Amidino-4-biphenylyl)oxymethyl]-3-(2-hydroxyethyl)-2-pyrrolidinon x 1,25 HCl x 1 Wasser

$R_f$-Wert: 0,29 (Kieselgel; Methylenchlorid/Methanol = 85:15)

110

| Ber.: | C 57,61 | H 6,34 | N 10,07 | Cl 10,63 |
|---|---|---|---|---|
| Gef.: | 57,89 | 6,22 | 9,94 | 10,46 |

(86) (3S,5S)-5-[(7-Amidino-9,10-dihydro-2-phenanthrenyl)oxymethyl]-3-[(methoxycarbonyl)methyl]-2-pyrrolidinon-hydrochlorid

$R_f$-Wert: 0,39 (Kieselgel; Methylenchlorid/Methanol/Wasser = 8:2:0,1)

(87) (3S,5S)-5-[(3-Amidinobenzoyl)aminomethyl]-3-[(methoxycarbonyl)methyl]-1-(3-phenylpropyl)-2-pyrrolidinon-hydrochlorid

$R_f$-Wert: 0,27 (Kieselgel; Methylenchlorid/Methanol/konz. wäßriges Ammoniak = 4:1:0,25)
Massenspektrum: $(M + H)^+ = 451$

(88) (3S,5S)-5-[[exo-5-(4-Amidinophenyl)bicyclo[2.2.1]heptyl-exo-2-carbonyl]aminomethyl]-3-[(methoxycarbonyl)methyl]-1-(3-phenylpropyl)-2-pyrrolidinon-hydrochlorid

$R_f$-Wert: 0,27 (Kieselgel; Methylenchlorid/Methanol/konz. wäßriges Ammoniak = 4:1:0,25)

(89) (3S,5S)-5-[(4'-Amidino-3'-fluor-4-biphenylyl)oxymethyl]-3-[(methoxycarbonyl)methyl]-2-pyrrolidinon-hydrochlorid-semihydrat

$R_f$-Wert: 0,56 (reversed phase Kieselgel; Methanol/10%ige wäßrige Kochsalzlösung = 6:4)

| Ber.: | C 56,70 | H 5,43 | N 9,45 | Cl 7,97 |
|---|---|---|---|---|
| Gef.: | 56,61 | 5,45 | 9,49 | 8,01 |

(90) (3S,5S)-5-[(4'-Amidino-4-biphenylyl)oxymethyl]-3-[(methoxycarbonyl)methyl]-1-[(4-methoxyphenyl)sulfonyl]-2-pyrrolidin-hydrochlorid

$R_f$-Wert: 0,55 (Kieselgel; Methylenchlorid/Methanol = 9:1)

(91) (3S,5S)-5-[(4'-Amidino-3'-chlor-4-biphenylyl)oxymethyl]-3-[(methoxycarbonyl)methyl]-2-pyrrolidinon-hydrochlorid

$R_f$-Wert: 0,33 (Kieselgel; Methylenchlorid/Methanol = 4:1)
Massenspektrum: $(M + H)^+ = 416$ und 418

(92) (3R,S;4R,S)-4-[(4'-Amidino-4-biphenylyl)oxymethyl]-3-[(methoxycarbonyl)methyl]-3-methyl-2-pyrrolidinon-hydrochlorid

$R_f$-Wert: 0,53 (reversed phase Kieselgel; Methanol/10%ige wäßrige Kochsalzlösung = 6:4)

(93) (3R,S;4R,S)-4-[[(4'-Amidino-4-biphenylyl)carbonylamino]methyl]-3-[(methoxycarbonyl)methyl]-2-pyrrolidinon-hydrochlorid

$R_f$-Wert: 0,23 (Kieselgel; Methylenchlorid/Methanol/Eisessig = 80:20:2)

(94) (3R,S;4R,S)-4-[[(4'-Amidino-4-biphenylyl)carbonylamino]methyl]-3-[(methoxycarbonyl)methyl]-3-methyl-2-pyrrolidinon-hydrochlorid

(95) (3S,5S)-5-[(4'-Amidino-4-biphenylyl)oxymethyl]-3-[(methoxycarbonyl)methyl]-1-[2-(methoxycarbonyl)phenyl]-2-pyrrolidinon-hydrochlorid

(96) (3S,5S)-5-[(4'-Amidino-4-biphenylyl)oxymethyl]-1-[2-(aminocarbonyl)phenyl]-3-[(methoxycarbonyl)methyl]-2-pyrrolidinon-hydrochlorid

(97) (3S,5S)-5-[(4'-Amidino-4-biphenylyl)oxymethyl]-1-[2-(ethylaminocarbonyl)phenyl]-3-[(methoxycarbonyl)methyl]-2-pyrrolidinon-hydrochlorid

(98) (3S,5S)-5-[(4'-Amidino-4-biphenylyl)oxymethyl]-1-[2-(dimethylaminocarbonyl)phenyl]-3-[(methoxycarbonyl)methyl]-2-pyrrolidinon-hydrochlorid

(99) (3S,5S)-5-[(4'-Amidino-4-biphenylyl)oxymethyl]-3-[(methoxycarbonyl)methyl]-1-[3-(methoxycarbonyl)phenyl]-2-pyrrolidinon-hydrochlorid

(100) (3S,5S)-5-[(4'-Amidino-4-biphenylyl)oxymethyl]-1-[3-(aminocarbonyl)phenyl]-3-[(methoxycarbonyl)methyl]-2-pyrrolidinon-hydrochlorid

(101) (3S,5S)-5-[(4'-Amidino-4-biphenylyl)oxymethyl]-1-[3-ethylaminocarbonyl)phenyl]-3-[(methoxycarbonyl)methyl]-2-pyrrolidinon-hydrochlorid

(102) (3S,5S)-5-[(4'-Amidino-4-biphenylyl)oxymethyl]-1-[3-(dimethylaminocarbonyl)phenyl]-3-[(methoxycarbonyl)methyl]-2-pyrrolidinon-hydrochlorid

(103) (3S,5S)-5-[(4'-Amidino-4-biphenylyl)oxymethyl]-3-[(methoxycarbonyl)methyl]-1-[4-(methoxycarbonyl)phenyl]-2-pyrrolidinon-hydrochlorid

(104) (3S,5S)-5-[(4'-Amidino-4-biphenylyl)oxymethyl]-1-[4-(aminocarbonyl)phenyl]-3-[(methoxycarbonyl)methyl]-2-pyrrolidinon-hydrochlorid

(105) (3S,5S)-5-[(4'-Amidino-4-biphenylyl)oxymethyl]-1-[4-(ethylaminocarbonyl)phenyl]-3-[(methoxycarbonyl)methyl]-2-pyrrolidinon-hydrochlorid

(106) (3S,5S)-5-[(4'-Amidino-4-biphenylyl)oxymethyl]-1-[4-(dimethylaminocarbonyl)phenyl]-3-[(methoxycarbonyl)methyl]-2-pyrrolidinon-hydrochlorid

(107) (3S,5S)-5-[(4'-Amidino-4-biphenylyl)oxymethyl]-3-[(methoxycarbonyl)methyl]-1-[3-(methansulfonylamino)phenyl]-2-pyrrolidinon-hydrochlorid

(108) (3S,5S)-1-[3-(Acetaminophenyl)-5-[(4'-amidino-4-biphenylyl)oxymethyl]-3-[(methoxycarbonyl)methyl]-2-pyrrolidinon-hydrochlorid

(109) (3S,5S)-5-[[(6-Amidino-1,2,3,4-tetrahydro-2-naphthyl)aminocarbonyl]aminomethyl]-3-[(methoxycarbonyl)methyl]-2-pyrrolidinon-hydrochlorid

(110) (3S,5S)-5-[[N-Acetyl-N-[trans-4-(4-amidinophenyl)cyclohexyl]]aminomethyl]-3-[(methoxycarbonyl)methyl]-2-pyrrolidinon-hydrochlorid

(111) (3S,5S)-5-[[N-[trans-4-(4-Amidinophenyl)cyclohexyl]-N-methansulfonyl]aminomethyl]-3-[(methoxycarbonyl)methyl]-2-pyrrolidinon-hydrochlorid

(112) (3S,5S)-5-[[trans-4-(4-Amidinophenyl)cyclohexyl]oxymethyl-3-[(methoxycarbonyl)methyl]-2-pyrrolidinon-hydrochlorid

(113) (3S,5S)-5-[(4'-Amidino-3-ethoxy-4-biphenylyl)oxymethyl]-3-[(methoxycarbonyl)methyl]-2-pyrrolidinon-hydrochlorid

(114) (3S;5S)-5-[(4'-Amidino-3-brom-4-biphenylyl)oxymethyl]-3-[(methoxycarbonyl)methyl]-2-pyrrolidinon-hydrochlorid

$R_f$-Wert: 0,25 (Kieselgel; Methylenchlorid/Methanol/konz. wäßriges Ammoniak = 4:1:0,25)

(115) (3S,5S)-5-[(4'-Amidino-4-biphenylyl)oxymethyl]-3-[(methoxycarbonyl)methyl]-1-[2-(phenylsulfonyl)ethyl]-2-pyrrolidinon-hydrochlorid

(116) (3S,5S)-5-[(4'-Amidino-4-biphenylyl)oxymethyl]-3-[(methoxycarbonyl)methyl]-1-[2-(phenylsulfenyl)ethyl]-2-pyrrolidinon-hydrochlorid

(117) (3S,5S)-5-[[[1-(4-Amidinophenyl)-1-propin-3-yl]aminocarbonyl]methyl]-3-[(methoxycarbonyl)methyl]-2-pyrrolidinonhydrochlorid

(118) (3S,5S)-5-[(7-Amidino-2-phenanthrenyl)oxymethyl]-3-[(methoxycarbonyl)methyl]-2-pyrrolidinon-hydrochlorid

(119) (3R,5S)-3-[(4'-Amidino-4-biphenylyl)methyl]-5-[(methoxycarbonyl)methyl]-2-pyrrolidinon-hydrochlorid

(120) (3S,5S)-3-[(4'-Amidino-4-biphenylyl)oxymethyl]-5-[(methoxycarbonyl)methyl]-2-pyrrolidinon-hydrochlorid

(121) 3-[(4'-Amidino-4-biphenylyl)methyl]-1-[2-(methoxycarbonyl)ethyl]-2-pyrrolidinon-hydrochlorid

(122) 1-[2-(4'-Amidino-4-biphenylyl)ethyl]-3-[(methoxycarbonyl)methyl]-2-pyrrolidinon-hydrochlorid

(123) (3R,5S)-3-[2-(4'-Amidino-4-biphenylyl)ethyl]-5-[(methoxycarbonyl)methyl]-2-pyrrolidinon-hydrochlorid

(124) (2S,5S)-5-[(4'-Amidino-4-biphenylyl)oxymethyl]-2-[(methoxycarbonyl)methyl]-1-methansulfonyl-pyrrolidin-hydrochlorid

(125) (2S,5S)-1-Acetyl-5-[(4'-amidino-4-biphenylyl)oxymethyl]-2-[(methoxycarbonyl)methyl]-pyrrolidin-hydrochlorid

(126) (3S,5S)-5-[[(4'-Amidino-3-biphenylyl)sulfonylamino]methyl]-3-[(methoxycarbonyl)methyl]-2-pyrrolidinon-hydrochlorid

(127) (3S,5S)-5-[[(3'-Amidino-4-biphenylyl)carbonylamino]methyl]-3-[(methoxycarbonyl)methyl]-2-pyrrolidinon-hydrochlorid

(128) (3S,5S)-5-[(4'-Amidino-4-biphenylyl)oxymethyl]-1-(2-aminoethyl)-3-[(methoxycarbonyl)methyl]-2-pyrrolidinondihydrochlorid

(129) (3S,5S)-1-[2-(Acetylamino)ethyl]-5-(4'-amidino-4-biphenylyl)oxymethyl]-3-[(methoxycarbonyl)methyl]-2-pyrrolidinon-hydrochlorid

(130) (3S,5S)-5-[(4'-Amidino-4-biphenylyl)oxymethyl]-1-[2-(methansulfonylamino)ethyl]-3-[(methoxycarbonyl)methyl]-2-pyrrolidinon-hydrochlorid

(131) (3S,5S)-5-[(4'-Amidino-4-biphenylyl)oxymethyl]-1-[2-(benzoylamino)ethyl]-3-[(methoxycarbonyl)methyl]-2-pyrrolidinon-hydrochlorid

(132) (3S,5S)-5-[(4'-Amidino-4-biphenylyl)oxymethyl]-3-[(methoxycarbonyl)methyl]-1-[2-(phenylsulfonylamino)ethyl]-2-pyrrolidinon-hydrochlorid

(133) (3S,5S)-1-[2-(N-Acetyl-methylamino)ethyl]-5-(4'-amidino-4-biphenylyl)oxymethyl]-3-[(methoxycarbonyl)methyl]-2-pyrrolidinon-hydrochlorid

(134) (3S,5S)-5-[[4-(4-Amidinophenyl)naphthyl]oxymethyl]-3-[(methoxycarbonyl)methyl]-2-pyrrolidinon-hydrochlorid

(135) (3S,5S)-5-[[[2-[(4-Amidinophenyl)oxy]phenyl]carbonylamino]methyl]-3-[(methoxycarbonyl)methyl]-2-pyrrolidinonhydrochlorid

(136) (3S,5S)-5-[(4'-Amidino-4-biphenylyl)oxymethyl]-3-[1-(methoxycarbonyl)-1-ethyl]-2-pyrrolidinon-hydrochlorid

(137)     (3S,5S)-5-[(4'-Amidino-4-biphenylyl)oxymethyl)-1-[(N,N-dimethylamino)carbonyl]-3-[(methoxycarbonyl)methyl]pyrrolidin-hydrochlorid

$R_f$-Wert: 0,42 (Kieselgel; Methylenchlorid/Methanol = 4:1)

(138) (3S,5S)-5-[(4'-Amidino-4-biphenylyl)oxymethyl]-1-(dimethylaminooxalyl)-3-[(methoxycarbonyl)methyl]-pyrrolidinhydrochlorid

(139)     (3S,5S)-5-[(4'-Amidino-4-biphenylyl)oxymethyl]-3-[(methoxycarbonyl)methyl]-1-methoxyoxalyl-pyrrolidin-hydrochlorid

(140)   (3S,5S)-5-[(4'-Amidino-4-biphenylyl)oxymethyl]-1-aminoacetyl-3-[(methoxycarbonyl)methyl]-pyrrolidin-dihydrochlorid

(141) (3S,5S)-5-[2-[[N-(6-Amidino-2-naphthylcarbonyl)-N-ethyl]amino]ethyl]-3-[(methoxycarbonyl)methyl]-2-pyrrolidinon-hydrochlorid

(142)     (3S,5S)-5-[(4'-Amidino-4-biphenylyl)oxymethyl]-3-[(methoxycarbonyl)methyl]-1-[(4-methoxyphenyl)sulfonyl]pyrrolidin-hydrochlorid

(143)     (3S,5S)-5-[(4'-Amidino-4-biphenylyl)oxymethyl]-1-aminocarbonyl-3-[(methoxycarbonyl)methyl]pyrrolidin-hydrochlorid

(144)     (2S,4R)-4-[(4'-Amidino-4-biphenylyl)oxymethyl]-1-methansulfonyl-2-[(methoxycarbonyl)methyl]-pyrrolidin-hydrochlorid

(145)   (3R,5S)-5-[(4'-Amidino-4-biphenylyl)oxymethyl]-3-[(methansulfonyl)methyl]-3-[(methoxycarbonyl)methyl]-1-(3-phenylpropyl)-2-pyrrolidinon-hydrochlorid

(146)     (3S,5S)-5-[(4'-Amidino-4-biphenylyl)oxymethyl]-3-[(methoxycarbonyl)methyl]-1-[(pyrrolidin-N-carbonyl)methyl]-2-pyrrolidinon-hydrochlorid

$R_f$-Wert: 0,46 (Kieselgel; Methylenchlorid/Methanol/konz. wäßriges Ammoniak = 30:10:2)

(147)     (3S,5S)-5-[(4'-Amidino-4-biphenylyl)oxymethyl]-3-[(methoxycarbonyl)methyl]-1-[(piperidin-N-carbonyl)methyl]-2-pyrrolidinon-hydrochlorid

(148)     (3S,5S)-5-[(4'-Amidino-4-biphenylyl)oxymethyl]-3-[(methoxycarbonyl)methyl]-1-[(morpholin-N-carbonyl)methyl]-2-pyrrolidinon-hydrochlorid

$R_f$-Wert: 0,46 (Kieselgel; Methylenchlorid/Methanol/konz. wäßriges Ammoniak = 30:10:2)

(149)   (3S,5S)-5-[(4'-Amidino-4-biphenylyl)oxymethyl]-3-[(methoxycarbonyl)methyl]-1-[(N-methylpiperazin-N'-carbonyl)methyl]-2-pyrrolidinon-hydrochlorid

(150) (3S,5S)-1-[(N-Acetylpiperazin-N'-carbonyl)methyl]-5-[(4'-amidino-4-biphenylyl)oxymethyl]-3-[(methoxycarbonyl)methyl]-2-pyrrolidinon-hydrochlorid

(151)     (3S,5S)-5-[(4'-Amidino-4-biphenylyl)oxymethyl]-1-[(N-methansulfonylpiperazin-N'-carbonyl)methyl]-3-[(methoxycarbonyl)methyl]-2-pyrrolidinon-hydrochlorid

(152)     (3S,5S)-5-[(4'-Amidino-4-biphenylyl)oxymethyl]-3-[(methoxycarbonyl)methyl]-1-[(thiomorpholin-N-carbonyl)methyl]-2-pyrrolidinon-hydrochlorid

(153)  (3S,5S)-5-[(4'-Amidino-4-biphenylyl)oxymethyl]-3-[(methoxycarbonyl)methyl]-1-[[(thiomorpholin-S-dioxid)-N-carbonyl]methyl]-2-pyrrolidinon-hydrochlorid

(154)     (3S,5S)-5-[(4'-Amidino-4-biphenylyl)oxymethyl]-3-[(methoxycarbonyl)methyl]-1-[(1-piperazinylcarbonyl)methyl]-2-pyrrolidinon-hydrochlorid

(155)    (3S,5S)-5-[(4'-Amidino-4-biphenylyl)oxymethyl]-1-(4-brombenzyl)-3-[(methoxycarbonyl)methyl]-2-pyrrolidinon-hydrochlorid

(156)  (3S,5S)-5-[(4'-Amidino-4-biphenylyl)oxymethyl]-3-[(methoxycarbonyl)methyl]-1-(4-methylbenzyl)-2-pyrrolidinonhydrochlorid

(157)  (3S,5S)-5-[(4'-Amidino-4-biphenylyl)oxymethyl]-3-[(methoxycarbonyl)methyl]-1-(4-methoxybenzyl)-2-pyrrolidinonhydrochlorid

(158)  (3S,5S)-5-[(4'-Amidino-4-biphenylyl)oxymethyl]-3-[(methoxycarbonyl)methyl]-1-[3-(methylsulfenyl)propyl]-2-pyrrolidinon-hydrochlorid

(159)  (3S,5S)-5-[(4'-Amidino-4-biphenylyl)oxymethyl]-3-[(methoxycarbonyl)methyl]-1-[3-(methylsulfonyl)propyl]-2-pyrrolidinon-hydrochlorid

(160)    (3S,5S)-5-[(4'-Amidino-4-biphenylyl)oxymethyl]-3-[(methoxycarbonyl)methyl]-1-phenylsulfonyl-pyrrolidin-hydrochlorid

(161)     (3S,5S)-5-[(4'-Amidino-3-methoxy-4-biphenylyl)oxymethyl]-3-[(methoxycarbonyl)methyl]-2-pyrrolidinon-hydrochlorid

(162)     (3S,5S)-5-[(4'-Amidino-4-biphenylyl)oxymethyl]-1-methoxyacetyl-3-[(methoxycarbonyl)methyl]-pyrrolidin-hydrochlorid $R_f$-Wert: 0,10 (Kieselgel; Methylenchlorid/Methanol = 10:1)

(163) 1-(4'-Amidino-4-biphenylyl)-4-[(methoxycarbonyl)methyl]-2-pyrrolidinon-hydrochlorid

(164) 1-(4'-Amidino-4-biphenylyl)-4-phosphonomethyl-2-pyrrolidinon

$R_f$-Wert: 0,73 (reversed phase Kieselgel; Methanol/10%ige wäßrige Kochsalzlösung = 1:1)

(165) 1-(4'-Amidino-4-biphenylyl)-4-(O-methyl-phosphonomethyl)-2-pyrrolidinon

$R_f$-Wert: 0,55 (reversed phase Kieselgel; Methanol/10%ige wäßrige Kochsalzlösung = 1:1)

<u>Beispiel 4</u>

<u>(3S,5S)-5-[(4'-Amidino-4-biphenylyl)oxymethyl)-3-carboxymethyl-1-(3-phenylpropyl)-2-pyrrolidinon-hydrat</u>

125,7 g (3S,5S)-5-[(4'-Amidino-4-biphenylyl)oxymethyl]-3-[(methoxycarbonyl)methyl]-1-(3-phenylpropyl)-2-pyrrolidinonhydrochlorid werden in 1000 ml Methanol unter Erwärmen gelöst. Die Lösung wird auf 20°C abgekühlt und unter Rühren mit 355 ml 1N Natronlauge versetzt. Nach einer Stunde fügt man 120 ml und nach weiteren 3 Stunden noch 40 ml 1N Natronlauge hinzu und rührt weitere 16 Stunden bei Raumtemperatur. Dem Reaktionsgemisch wird eine Lösung von 32 g Ammoniumchlorid in 200 ml Wasser zugesetzt, wonach das Produkt auszukristallisieren beginnt. Nach beendeter Kristallisation filtriert man das Produkt ab, wäscht noch zweimal mit je 100 ml eines Gemisches aus 4 Teilen Methanol und einem Teil Wasser und digeriert schließlich zweimal mit 250 ml Aceton. Das erhaltene Rohprodukt wird zweimal aus 400 ml eines Gemisches aus Methanol, Wasser und konzentriertem wäßrigen Ammoniak (40:1:1,5) umkristallisiert.

Ausbeute: 74,7 g (63 % der Theorie),
Schmelzpunkt: 168°C (Zers.)

| Ber.: | C 69,17 | H 6,61 | N 8,48 |
|-------|---------|--------|--------|
| Gef.: | 69,45 | 6,47 | 8,48 |

Aus den bei der Reinigung des Rohproduktes anfallenden Mutterlaugen gewinnt man nach Einengen und Chromatographie an Kieselgel (Elutionsmittel: Methylenchlorid/Methanol/konzentrierter wäßriger Ammoniak = 3:1:0,2) (3S,5S)-5-[(4'-Aminocarbonyl-4-biphenylyl)oxymethyl)-3-carboxymethyl-1-(3-phenylpropyl)-2-pyrrolidinon.

Ausbeute: 10,5 g (9 % der Theorie),
Schmelzpunkt: 183-186°C

Analog werden erhalten:

(1) (3S,5S)-5-[(4'-Amidino-4-biphenylyl)oxymethyl)-3-carboxymethyl-1-phenyl-2-pyrrolidinon-dihydrat

Schmelzpunkt: ab 215°C (Zers.)
$R_f$-Wert: 0,45 (Reversed-phase-Kieselgel (RP8); Methanol/10 %ige wäßrige Kochsalzlösung = 6:4)

| Ber.: | C 65,12 | H 6,09 | N 8,76 |
|---|---|---|---|
| Gef.: | 65,32 | 5,94 | 8,75 |

(2) (3S,5S)-5-[(4'-Amidino-4-biphenylyl)oxymethyl]-1-(3-cyclohexylpropyl)-3-carboxymethyl-2-pyrrolidinon-hydrat

Schmelzpunkt: 182-190°C (Zers.)
$R_f$-Wert: 0,13 (Reversed-phase-Kieselgel (RP8); Methanol/10 %ige wäßrige Kochsalzlösung = 6:4)

| Ber.: | C 68,34 | H 7,71 | N 8,24 |
|---|---|---|---|
| Gef.: | 68,24 | 7,87 | 8,13 |

(3) (3S,5S)-5-[(4'-Amidino-4-biphenylyl)oxymethyl]-3-carboxymethyl-2-pyrrolidinon

Schmelzpunkt: 292-294°C (Zers.)
$R_f$-Wert: 0,63 (Reversed-phase-Kieselgel (RP8); Methanol/10 %ige wäßrige Kochsalzlösung = 6:4)

| Ber.: | C 65,38 | H 5,76 | N 11,44 |
|---|---|---|---|
| Gef.: | 65,20 | 5,80 | 11,54 |

(4) (3S,5S)-5-[(4'-Amidino-4-biphenylyl)oxymethyl]-3-carboxymethyl-1-methyl-2-pyrrolidinon x 0,25 Wasser

Schmelzpunkt: 278-281°C (Zers.)
$R_f$-Wert: 0,20 (Kieselgel; Methylenchlorid/Methanol/konz. wäßriges Ammoniak = 80:20:5)

| Ber.: | C 65,36 | H 6,14 | N 10,89 |
|---|---|---|---|
| Gef.: | 65,32 | 5,97 | 10,84 |

(5)     (3S,5S)-5-[(4'-Amidino-3-brom-4-biphenylyl)oxymethyl]-3-carboxymethyl-1-(3-phenylpropyl)-2-pyrrolidinon-hydrat

Schmelzpunkt: 236-238°C (Zers.)
$R_f$-Wert: 0,45 (Kieselgel; Methylenchlorid/Methanol/konz. wäßriges Ammoniak = 40:40:5)

| Ber.: | C 59,80 | H 5,54 | N 7,21 | Br 13,72 |
|-------|---------|--------|--------|----------|
| Gef.: | 60,00 | 5,73 | 6,97 | 13,56 |

(6)     (3S,5S)-5-[(4'-Amidino-3-nitro-4-biphenylyl)oxymethyl]-3-carboxymethyl-1-(3-phenylpropyl)-2-pyrrolidinon-hydrat

Schmelzpunkt: ab 192°C (Zers.), ab 180°C Sintern
$R_f$-Wert: 0,18 (Kieselgel; Methylenchlorid/Methanol/konz. wäßriges Ammoniak = 80:30:5)

| Ber.: | C 63,49 | H 5,88 | N 10,21 |
|-------|---------|--------|---------|
| Gef.: | 63,64 | 5,93 | 10,33 |

(7) (3R,5R)-5-[(4'-Amidino-4-biphenylyl)oxymethyl]-3-carboxymethyl-1-(3-phenylpropyl)-2-pyrrolidinon-semihydrat

Schmelzpunkt: 183-185°C (Zers.)
$R_f$-Wert: 0,23 (Kieselgel; Methylenchlorid/Methanol/konz. wäßriges Ammoniak = 80:20:5)

| Ber.: | C 70,43 | H 6,52 | N 8,50 |
|-------|---------|--------|--------|
| Gef.: | 70,82 | 6,78 | 8,54 |

$[\alpha]^{20}_{D}$ = - 39,8 c = 1, Eisessig

(8) (3S,5S)-5-[(4'-Amidino-3-trifluormethyl-4-biphenylyl)oxymethyl]-3-carboxymethyl-1-(3-phenylpropyl)-2-pyrrolidinon-hydrat

Schmelzpunkt: 238-240°C (Zers.)
$R_f$-Wert: 0,33 (Kieselgel; Methylenchlorid/Methanol/konz. wäßriges Ammoniak = 80:20:6)

| Ber.: | C 63,00 | H 5,64 | N 7,35 |
|-------|---------|--------|--------|
| Gef.: | 63,16 | 5,98 | 7,37 |

(9)     (3S,5S)-5-[(4'-Amidino-3-fluor-4-biphenylyl)oxymethyl]-3-carboxymethyl-1-(3-phenylpropyl)-2-pyrrolidinon-hydrat

Schmelzpunkt: 195-197°C (Zers.)
$R_f$-Wert: 0,33 (Kieselgel; Methylenchlorid/Methanol/konz. wäßriges Ammoniak = 80:20:5)

| Ber.: | C 66,78 | H 6,19 | N 8,06 |
|-------|---------|--------|--------|
| Gef.: | 66,90 | 6,20 | 8,06 |

(10)   (3S,5S)-5-[(4'-Amidino-2,3-dimethyl-4-biphenylyl)oxymethyl]-3-carboxymethyl-1-(3-phenylpropyl)-2-pyrrolidinonhydrat

Schmelzpunkt: 211-213°C (Zers.)
$R_f$-Wert: 0,14 (Kieselgel; Methylenchlorid/Methanol/konz. wäßriges Ammoniak = 14:5:1)

| Ber.: | C 70,04 | H 7,02 | N 7,90 |
|-------|---------|--------|--------|
| Gef.: | 70,22 | 6,79 | 8,06 |

(11)   (3S,5S)-5-[(3-Acetylamino-4'-amidino-4-biphenylyl)oxymethyl]-3-carboxymethyl-1-(3-phenylpropyl)-2-pyrrolidinon-hydrochlorid-semihydrat

Schmelzpunkt: 183-185°C (Zers.)
$R_f$-Wert: 0,17 (Kieselgel; Methylenchlorid/Methanol/konz. wäßriges Ammoniak = 40:10:3)

| Ber.: | C 63,31 | H 6,17 | N 9,53 |
|-------|---------|--------|--------|
| Gef.: | 63,59 | 6,31 | 9,65 |

(12)   (3S,5S)-5-[(4'-Amidino-3-methansulfonylamino-4-biphenylyl)oxymethyl]-3-carboxymethyl-1-(3-phenylpropyl)-2-pyrrolidinon x 0,25 Wasser

Schmelzpunkt: 219-220°C (Zers.)
$R_f$-Wert: 0,23 (Kieselgel; Methylenchlorid/Methanol/konz. wäßriges Ammoniak = 80:20:5)

| Ber.: | C 61,78 | H 5,96 | N 9,61 |
|-------|---------|--------|--------|
| Gef.: | 61,82 | 6,22 | 9,48 |

(13)   (3S,5S)-5-[(4'-Amidino-2'-methyl-4-biphenylyl)oxymethyl]-3-carboxymethyl-1-(3-phenylpropyl)-2-pyrrolidinon-semihydrat

Schmelzpunkt: ab 187°C (Zers.)
$R_f$-Wert: 0,27 (Kieselgel; Methylenchlorid/Methanol/konz. wäßriges Ammoniak = 12:6:1)

| Ber.: | C 70,85 | H 6,74 | N 8,26 |
|-------|---------|--------|--------|
| Gef.: | 71,08 | 6,77 | 8,20 |

(14) (3S,5S)-5-[(4'-Amidino-3'-chlor-4-biphenylyl)oxymethyl]-3-carboxymethyl-1-(3-phenylpropyl)-2-pyrrolidinon

Schmelzpunkt: ab 166°C (Zers.)
$R_f$-Wert: 0,13 (Kieselgel; Methylenchlorid/Methanol/konz. wäßriges Ammoniak = 35:15:2)

| Ber.: | C 66,98 | H 5,82 | N 8,08 |
|-------|---------|--------|--------|
| Gef.: | 66,63 | 5,87 | 8,07 |

(15)   (3S,5S)-5-[[(4'-Amidino-4-biphenylyl)carbonyl]aminomethyl]-3-carboxymethyl-1-(3-phenylpropyl)-2-pyrrolidinon-dihydrat

Schmelzpunkt: 208-214°C
$R_f$-Wert: 0,40 (Kieselgel; Methylenchlorid/Methanol/konz. wäßriges Ammoniak = 6:3:1)

| Ber.: | C 65,68 | H 6,61 | N 10,21 |
|-------|---------|--------|---------|
| Gef.: | 65,48 | 6,20 | 9,99 |

(16)  (3S,5S)-5-[[trans-4-(4-Amidinophenyl)cyclohexyl]oxymethyl]-3-carboxymethyl-1-(3-phenylpropyl)-2-pyrrolidinonsemihydrat

Schmelzpunkt: 186-198°C
$R_f$-Wert: 0,25 (Kieselgel; Methylenchlorid/Methanol/konz. wäßriges Ammoniak = 30:15:3)

| Ber.: | C 69,57 | H 7,65 | N 8,39 |
|-------|---------|--------|--------|
| Gef.: | 69,60 | 7,58 | 8,38 |

(17)  (3S,5S)-5-[2-[(6-Amidino-2-naphthylcarbonyl)amino]ethyl]-3-carboxymethyl-1-(3-phenylpropyl)-2-pyrrolidinon x 2,5 Wasser

Schmelzpunkt: Sintern ab 166°C, 205°C (Zers.)
$R_f$-Wert: 0,09 (Kieselgel; Methylenchlorid/Methanol/konz. wäßriges Ammoniak = 70:30:2)

| Ber.: | C 63,84 | H 6,84 | N 10,27 |
|-------|---------|--------|---------|
| Gef.: | 64,00 | 6,77 | 10,13 |

(18)  (3S,5S)-5-[(6-Amidino-2-naphthylcarbonyl)aminomethyl]-3-carboxymethyl-1-(3-phenylpropyl)-2-pyrrolidinondihydrat

Schmelzpunkt: 189-202°C (Zers.)
$R_f$-Wert: 0,10 (Kieselgel; Methylenchlorid/Methanol/konz. wäßriges Ammoniak = 70:30:2)

| Ber.: | C 64,35 | H 6,56 | N 10,72 |
|-------|---------|--------|---------|
| Gef.: | 64,55 | 6,53 | 10,55 |

(19)  (3S,5S)-5-[(7-Amidino-2-naphthylcarbonyl)aminomethyl]-3-carboxymethyl-1-(3-phenylpropyl)-2-pyrrolidinontrihydrat

Schmelzpunkt: 195-207°C (Zers.)
$R_f$-Wert: 0,38 (Reversed-phase-Kieselgel (RP8); Methanol/10 %ige wäßrige Kochsalzlösung = 6:4)

| Ber.: | C 62,21 | H 6,71 | N 10,36 |
|-------|---------|--------|---------|
| Gef.: | 61,98 | 6,75 | 10,55 |

(20)  (3S,5S)-5-[2-[(7-Amidino-2-naphthylcarbonyl)amino]ethyl]-3-carboxymethyl-1-(3-phenylpropyl)-2-pyrrolidinon x 2,5 Wasser

Schmelzpunkt: 185-200°C (Zers.)
$R_f$-Wert: 0,41 (Reversed-phase-Kieselgel (RP8); Methanol/10 %ige wäßrige Kochsalzlösung = 6:4)

| Ber.: | C 63,84 | H 6,83 | N 10,27 |
|-------|---------|--------|---------|
| Gef.: | 63,73 | 6,51 | 10,18 |

(21)  (3S,5S)-5-[[trans-3-(4-Amidinophenyl)cyclobutyl]carbonylaminomethyl]-3-carboxymethyl-1-(3-phenylpropyl)-2-pyrrolidinon x 1,5 Wasser

$R_f$-Wert: 0,31 (Kieselgel; Methylenchlorid/Methanol/konz. wäßriges Ammoniak = 35:15:4)

| Ber.: | C 65,35 | H 7,25 | N 10,89 |
|-------|---------|--------|---------|
| Gef.: | 65,60 | 7,26 | 10,61 |

(22)  (3S,5S)-5-[[cis-3-(4-Amidinophenyl)cyclobutyl]carbonylaminomethyl]-3-carboxymethyl-1-(3-phenylpropyl)-2-pyrrolidinon

$R_f$-Wert: 0,38 (Kieselgel; Methylenchlorid/Methanol/konz. wäßriges Ammoniak = 35:15:4)

(23)  (3S,5S)-5-[2-[[cis-3-(4-Amidinophenyl)cyclobutyl]carbonylamino]ethyl]-3-carboxymethyl-1-(3-phenylpropyl)-2-pyrrolidinon x 1,5 Wasser

$R_f$-Wert: 0,34 (Kieselgel; Methylenchlorid/Methanol/konz. wäßriges Ammoniak = 35:15:4)

| Ber.: | C 65,51 | H 7,39 | N 10,54 |
|-------|---------|--------|---------|
| Gef.: | 65,04 | 7,24 | 10,26 |

(24)  (3S,5S)-5-[2-[[trans-3-(4-Amidinophenyl)cyclobutyl]carbonylamino]ethyl]-3-carboxymethyl-1-(3-phenylpropyl)-2-pyrrolidinon-dihydrat

$R_f$-Wert: 0,30 (Kieselgel; Methylenchlorid/Methanol/konz. wäßriges Ammoniak = 35:15:4)

| Ber.: | C 64,43 | H 7,45 | N 10,36 |
|-------|---------|--------|---------|
| Gef.: | 64,24 | 7,28 | 9,98 |

(25)  (3S,5S)-5-[[3-(4-Amidinophenyl)propyl]carbonylaminomethyl]-3-carboxymethyl-1-(3-phenylpropyl)-2-pyrrolidinon

$R_f$-Wert: 0,16 (Kieselgel; Methylenchlorid/Methanol/wäßriges Ammoniak = 4:1:0,2)

(26)  (3S,5S)-5-[4'-Amidino-4-biphenylyl)oxymethyl]-3-carboxymethyl-3-methyl-1-(3-phenylpropyl)-2-pyrrolidinon-dihydrat

| Ber.: | C 67,27 | H 6,96 | N 7,85 |
|-------|---------|--------|--------|
| Gef.: | 67,46 | 6,85 | 8,13 |

Als Nebenprodukt entsteht (3S,5S)-5-[4'-Aminocarbonyl-4-biphenylyl)oxymethyl]-3-carboxymethyl-3-methyl-1-(3-phenylpropyl)-2-pyrrolidinon, das durch Chromatographie an Kieselgel (Elutionsmittel: Methylenchlorid/Methanol/konz. wäßriges Ammoniak = 4:1:0,25) abgetrennt wird.

$R_f$-Wert: 0,81 (Kieselgel; Methylenchlorid/Methanol/wäßriges Ammoniak = 4:1:0,25)

(27) (3S,5S)-5-[4'-Amidino-4-biphenylyl)oxymethyl]-1-benzyl-3-carboxymethyl-2-pyrrolidinon-dihydrat

| Ber.: | C 65,71 | H 6,33 | N 8,51 |
|-------|---------|--------|--------|
| Gef.: | 66,08 | 6,13 | 8,48 |

(28) (3S,5S)-5-[4'-Amidino-4-biphenylyl)oxymethyl]-3-carboxymethyl-1-(2-phenylethyl)-2-pyrrolidinon-hydrat

| Ber.: | C 68,69 | H 6,38 | N 8,58 |
|-------|---------|--------|--------|
| Gef.: | 68,43 | 6,08 | 8,41 |

(29) (3S,5S)-5-[4'-Amidino-4-biphenylyl)oxymethyl]-3-carboxymethyl-1-(4-phenylbutyl)-2-pyrrolidinon-hydrat

| Ber.: | C 69,61 | H 6,81 | N 8,12 |
|-------|---------|--------|--------|
| Gef.: | 69,29 | 6,57 | 8,35 |

(30) (3S,5S)-5-[4'-Amidino-4-biphenylyl)oxymethyl]-3-carboxymethyl-1-(4-phenyloxybutyl)-2-pyrrolidinon-dihydrat

| Ber.: | C 65,32 | H 6,76 | N 7,62 |
|-------|---------|--------|--------|
| Gef.: | 65,67 | 6,48 | 8,17 |

(31) (3S,5S)-5-[[4'-(n-Butylamidino)-4-biphenylyl]oxymethyl]-3-carboxymethyl-1-(3-phenylpropyl)-2-pyrrolidinon x 1,5 Wasser

| Ber.: | C 69,69 | H 7,44 | N 7,39 |
|-------|---------|--------|--------|
| Gef.: | 69,42 | 7,37 | 7,35 |

(32) (3S,5S)-3-Carboxymethyl-5-[(4'-methylamidino-4-biphenylyl)oxymethyl]-1-(3-phenylpropyl)-2-pyrrolidinon-hydrat

| Ber.: | C 69,61 | H 6,81 | N 8,12 |
|-------|---------|--------|--------|
| Gef.: | 69,22 | 6,88 | 7,98 |

(33) (3S,5S)-3-Carboxymethyl-5-[(4'-isopropylamidino-4-biphenylyl)oxymethyl]-1-(3-phenylpropyl)-2-pyrrolidinon-dihydrat

| Ber.: | C 68,18 | H 7,33 | N 7,45 |
|-------|---------|--------|--------|
| Gef.: | 67,87 | 7,47 | 7,91 |

(34) (3S,5S)-5-[(4'-Amidino-4-biphenylyl)oxymethyl]-3-carboxymethyl-1,3-bis-(3-phenylpropyl)-2-pyrrolidinon-semihydrat

| Ber.: | C 74,48 | H 6,91 | N 6,86 |
|-------|---------|--------|--------|
| Gef.: | 74,67   | 7,02   | 6,78   |

(35)    (3S,5S)-5-[(4'-Amidino-4-biphenylyl)oxymethyl]-3-(n-butyl)-3-carboxymethyl-1-(3-phenylpropyl)-2-pyrrolidinon-hydrat

| Ber.: | C 70,81 | H 7,38 | N 7,51 |
|-------|---------|--------|--------|
| Gef.: | 70,54   | 7,33   | 7,47   |

(36) (3S,5S)-5-[(7-Amidino-9-keto-2-fluorenyl)oxymethyl]-3-carboxymethyl-1-(3-phenylpropyl)-2-pyrrolidinon

Schmelzpunkt: 226°C (Zers.)

(37) (3R,5S)-5-[(4'-Amidino-4-biphenylyl)oxymethyl]-3-carboxymethyl-1-(3-phenylpropyl)-2-pyrrolidinon

Schmelzpunkt: 225-227°C (Zers.)

(38) (3S,5S)-5-[(4'-Amidino-4-biphenylyl)aminomethyl]-3-carboxymethyl-1-(3-phenylpropyl)-2-pyrrolidinon

Schmelzpunkt: 208°C (Zers.)

(39) (3S,5S)-5-[[N-(4'-Amidino-4-biphenylyl)-N-methansulfonyl]aminomethyl]-3-carboxymethyl-1-(3-phenylpropyl)-2-pyrrolidinon

Schmelzpunkt: über 200°C

| Ber.: | C 64,03 | H 6,10 | N 9,96 | S 5,70 |
|-------|---------|--------|--------|--------|
| Gef.: | 63,96   | 5,91   | 9,73   | 5,60   |

(40) (3S,5S)-5-[[N-(4'-Amidino-4-biphenylyl)-N-acetyl]aminomethyl]-3-carboxymethyl-1-(3-phenylpropyl)-2-pyrrolidinonhydrat

| Ber.: | C 68,29 | H 6,61 | N 10,28 |
|-------|---------|--------|---------|
| Gef.: | 68,57   | 6,62   | 10,15   |

(41) (3S,5S)-5-[[2-[(4-Amidinophenyl)amino]phenyl]carbonylaminomethyl]-3-carboxylmethyl-1-(3-phenylpropyl)-2-pyrrolidinon

Schmelzpunkt: ab 172°C (Zers.)
$R_f$-Wert: 0,30 (Kieselgel; Cyclohexan/Essigester/Methanol = 2:1:0,6)

(42) (3S,5S)-5-[[4-[(4-Amidinophenyl)aminocarbonyl]phenyl]oxymethyl]-3-carboxymethyl-1-(3-phenylpropyl)-2-pyrrolidinon

$R_f$-Wert: 0,45 (Kieselgel; Methylenchlorid/Methanol/konz. wäßriges Ammoniak = 4:2:1)

| Ber.: | C 68,16 | H 6,10 | N 10,60 |
|-------|---------|--------|---------|
| Gef.: | 68,46   | 6,19   | 10,44   |

Massenspektrum: $(M + H)^+ = 529$

(43) (3S,5S)-5-[[4-[(4-Amidinophenyl)carbonylamino]phenyl]oxymethyl]-3-carboxymethyl-1-(3-phenylpropyl)-2-pyrrolidinon

Schmelzpunkt: >250°C
$R_f$-Wert: 0,43 (Kieselgel; Methylenchlorid/Methanol/konz. wäßriges Ammoniak = 4:2:1)
Massenspektrum: $(M + H)^+ = 529$

(44) (3S,5S)-5-[[4-[(4-Amidinophenyl)aminosulfonyl]phenyl]oxymethyl]-3-carboxymethyl-1-(3-phenylpropyl)-2-pyrrolidinon

Schmelzpunkt: >250°C
$R_f$-Wert: 0,40 (Kieselgel; Methylenchlorid/Methanol/konz. wäßriges Ammoniak = 4:2:1)
Massenspektrum: $(M + H)^+ = 565$

(45) (3S,5S)-5-[[4-[(3-Amidinophenyl)sulfenyl]phenyl]oxymethyl]-3-carboxymethyl-1-(3-phenylpropyl)-2-pyrrolidinon

$R_f$-Wert: 0,14 (Kieselgel; Methylenchlorid/Methanol/konz. wäßriges Ammoniak = 16:4:1)
Massenspektrum: $(M + H)^+ = 518$

(46) (3S,5S)-5-[[4-[(3-Amidinophenyl)carbonyl]phenyl]oxymethyl]-3-carboxymethyl-1-(3-phenylpropyl)-2-pyrrolidinon

$R_f$-Wert: 0,21 (Kieselgel; Methylenchlorid/Ethanol/konz. wäßriges Ammoniak = 8:2:1)
Massenspektrum: $(M + H)^+ = 514$

(47) (3S,5S)-5-[[4-[(3-Amidinophenyl)sulfonyl]phenyl]oxymethyl]-3-carboxymethyl-1-(3-phenylpropyl)-2-pyrrolidinon Schmelzpunkt: 205-207°C

$R_f$-Wert: 0,20 (Kieselgel; Methylenchlorid/Ethanol/konz. wäßriges Ammoniak = 4:4:1)

| Ber.: | C 63,37 | H 5,69 | N 7,63 | S 5,83 |
|-------|---------|--------|--------|--------|
| Gef.: | 63,10   | 5,90   | 7,59   | 5,92   |

Massenspektrum: $(M + H)^+ = 550$

(48) (3S,5S)-5-[[4-[(3-Amidinophenyl)sulfinyl]phenyl]oxymethyl]-3-carboxymethyl-1-(3-phenylpropyl)-2-pyrrolidinon

$R_f$-Wert: 0,25 (Kieselgel; Methylenchlorid/Ethanol/konz. wäßriges Ammoniak = 4:4:1)
Massenspektrum: $(M + H)^+ = 534$

(49) (3S,5S)-5-[(4'-Amidino-4-biphenylyl)oxymethyl]-1,3-bis(carboxymethyl)-2-pyrrolidinon x 0,25 Wasser

$R_f$-Wert: 0,56 (Kieselgel; Methanol/konz. wäßriges Ammoniak = 25:1)

| Ber.: | C 61,46 | H 5,51 | N 9,77 |
|-------|---------|--------|--------|
| Gef.: | 61,46 | 5,55 | 9,57 |

Massenspektrum: $(M + H)^+ = 426$

(50)   (3S,5S)-5-[(4'-Amidino-4-biphenylyl)oxymethyl]-3-carboxymethyl-1-[(ethylaminocarbonyl)methyl]-2-pyrrolidinonhydrochlorid-hydrat

$R_f$-Wert: 0,36 (Kieselgel; Methanol/konz. wäßriges Ammoniak = 50:3)

| Ber.: | C 56,86 | H 6,16 | N 11,05 | Cl 6,99 |
|-------|---------|--------|---------|---------|
| Gef.: | 56,87 | 6,39 | 11,26 | 6,95 |

Massenspektrum: $(M + H)^+ = 426$

(51) (3S,5S)-5-[(4'-Amidino-4-biphenylyl)oxymethyl]-3-carboxymethyl-1-[(dimethylaminocarbonyl)methyl]-2-pyrrolidinonhydrochlorid-semihydrat

$R_f$-Wert: 0,24 (Kieselgel; Methanol/konz. wäßriges Ammoniak = 50:3)

| Ber.: | C 57,89 | H 6,07 | N 11,25 | Cl 7,11 |
|-------|---------|--------|---------|---------|
| Gef.: | 57,76 | 6,08 | 11,34 | 7,23 |

(52) (3S,5S)-5-[(4'-Amidino-4-biphenylyl)oxymethyl]-1-[(benzylaminocarbonyl)methyl]-3-carboxymethyl-2-pyrrolidinon

Schmelzpunkt: > 200°C,
$R_f$-Wert: 0,37 (Kieselgel; Methanol/konz. wäßriges Ammoniak = 50:2)
Massenspektrum: $(M + H)^+ = 515$

(53)   (3S,5S)-5-[(4'-Amidino-4-biphenylyl)oxymethyl]-1-[(aminocarbonyl)methyl]-3-carboxymethyl-2-pyrrolidinon-hydrochlorid-hydrat

Schmelzpunkt: > 200°C,
$R_f$-Wert: 0,30 (Kieselgel; Methanol/konz. wäßriges Ammoniak = 50:2)

| Ber.: | C 55,17 | H 5,68 | N 11,70 | Cl 7,40 |
|-------|---------|--------|---------|---------|
| Gef.: | 57,35 | 5,68 | 11,88 | 7,20 |

Massenspektrum: $(M + H)^+ = 425$

(54) (3S,5S)-5-[(4'-Amidino-4-biphenylyl)oxymethyl]-1-benzoyl-3-carboxymethyl-pyrrolidin-semihydrat

Schmelzpunkt: 234-237°C (Zers.),
$R_f$-Wert: 0,51 (Kieselgel; Methylenchlorid/Methanol/konz. wäßriges Ammoniak = 70:30:8)

| Ber.: | C 69,51 | H 6,05 | N 9,01 |
|-------|---------|--------|--------|
| Gef.: | 69,34 | 6,22 | 8,90 |

(55) (3S,5S)-5-[(4'-Amidino-4-biphenylyl)oxymethyl]-3-carboxymethyl-1-methansulfonyl-pyrrolidin-hydrat

Schmelzpunkt: 266-268°C,
$R_f$-Wert: 0,31 (Kieselgel; Methylenchlorid/Methanol/konz. wäßriges Ammoniak = 100:10:4)

| Ber.: | C 56,23 | H 5,84 | N 9,37 | S 7,15 |
|-------|---------|--------|--------|--------|
| Gef.: | 56,41 | 5,78 | 9,16 | 7,64 |

(56) (3S,5S)-1-Acetyl-5-[(4'-amidino-4-biphenylyl)oxymethyl]-3-carboxymethyl-pyrrolidin-semihydrat

Schmelzpunkt: 265-268°C (Zers.),
$R_f$-Wert: 0,05 (Kieselgel; Methylenchlorid/Methanol/konz. wäßriges Ammoniak = 100:30:4)

| Ber.: | C 65,33 | H 6,48 | N 10,39 |
|-------|---------|--------|---------|
| Gef.: | 65,58 | 6,55 | 10,20 |

(57) (3S,5S)-5-[(4'-Amidino-4-biphenylyl)oxymethyl]-3-carboxymethyl-1-(ethylaminocarbonyl)-pyrrolidin-dihydrat

Schmelzpunkt: 195-200°C (Zers.),
$R_f$-Wert: 0,30 (Kieselgel; Methylenchlorid/Methanol/konz. wäßriges Ammoniak = 100:30:4)

| Ber.: | C 60,00 | H 7,00 | N 12,17 |
|-------|---------|--------|---------|
| Gef.: | 60,18 | 6,98 | 12,34 |

(58) (3S,5S)-5-[(4'-Amidino-4-biphenylyl)oxymethyl]-3-carboxymethyl-1-(dimethylaminosulfonyl)-pyrrolidin x 0,25 Wasser

Schmelzpunkt: 278°C (Zers.),
$R_f$-Wert: 0,37 (Kieselgel; Methylenchlorid/Methanol/konz. wäßriges Ammoniak = 100:30:4)

| Ber.: | C 56,81 | H 6,18 | N 12,05 | S 6,89 |
|-------|---------|--------|---------|--------|
| Gef.: | 56,69 | 6,16 | 11,76 | 7,17 |

(59) (3S,5S)-5-[(4'-Amidino-4-biphenylyl)oxymethyl]-3-carboxymethyl-1-methyl-pyrrolidin

$R_f$-Wert: 0,57 (reversed phase Kieselgel; Methanol/10 % wäßrige Kochsalzlösung = 6:4)
Massenspektrum: $(M + H)^+ = 368$

(60) (3R,5S)-5-[(4'-Amidino-4-biphenylyl)oxymethyl]-3-carboxymethyl-2-pyrrolidinon-hydrat

Schmelzpunkt: 288°C (Zers.),
$R_f$-Wert: 0,80 (reversed phase Kieselgel; Methanol/10 % wäßrige Kochsalzlösung = 6:4)

| Ber.: | C 62,33 | H 6,01 | N 10,90 |
|---|---|---|---|
| Gef.: | 62,53 | 5,90 | 11,03 |

(61) (3S,5S)-5-[(4'-Amidino-4-biphenylyl)oxymethyl]-3-carboxymethyl-3-methyl-2-pyrrolidinon-hydrat

Schmelzpunkt: 204°C (Zers.),
$R_f$-Wert: 0,74 (reversed phase Kieselgel; Methanol/10 % wäßrige Kochsalzlösung = 6:4)

| Ber.: | C 63,14 | H 6,31 | N 10,52 |
|---|---|---|---|
| Gef.: | 63,31 | 6,53 | 10,41 |

(62) (3S,5S)-5-[(4'-Amidino-4-biphenylyl)oxymethyl]-3-carboxymethyl-1-(2-methoxyethyl)-2-pyrrolidinon

Schmelzpunkt: 230°C (Zers.),
$R_f$-Wert: 0,75 (reversed phase Kieselgel; Methanol/10 % wäßrige Kochsalzlösung = 6:4)

(63) (3R,5R)-5-[(4'-Amidino-4-biphenylyl)oxymethyl]-3-carboxymethyl-2-pyrrolidinon-semihydrat

Schmelzpunkt: 286-288°C (Zers.),
$R_f$-Wert: 0,63 (reversed phase Kieselgel; Methanol/10 % wäßrige Kochsalzlösung = 6:4)

| Ber.: | C 63,82 | H 5,89 | N 11,16 |
|---|---|---|---|
| Gef.: | 64,04 | 5,82 | 10,89 |

(64) (3S,5S)-5-[2-[(4'-Amidino-3-biphenylyl)amino]ethyl]-3-carboxymethyl-1-(3-phenylpropyl)-2-pyrrolidinon-semihydrat

| Ber.: | C 70,98 | H 6,90 | N 11,04 |
|---|---|---|---|
| Gef.: | 70,72 | 6,99 | 10,86 |

(65) (3S,5S)-3-Carboxymethyl-5-[2-[(3'-guanidino-3-biphenylyl)amino]ethyl]-1-(3-phenylpropyl)-2-pyrrolidinon x $NH_3$ x 2 $H_2O$ x 0,75 HCl

$R_f$-Wert: 0,43 (Kieselgel; Methylenchlorid/Methanol/konz. wäßriges Ammoniak = 8:2:0,1)

| Ber. : | C 60,65 | H 7,25 | N 14,15 | Cl 4,48 |
|---|---|---|---|---|
| Gef.: | 60,49 | 7,60 | 14,07 | 4,29 |

(66) (3S,5S)-5-[2-[[(3'-Amidino-4-biphenylyl)carbonyl]amino]ethyl]-3-carboxymethyl-1-(3-phenylpropyl)-2-pyrrolidinon-semihydrochlorid-hydrat

Schmelzpunkt: 214°C (Zers.),

| Ber.: | C 66,20 | H 6,54 | N 9,96 | Cl 3,15 |
|---|---|---|---|---|
| Gef.: | 66,41 | 6,53 | 9,91 | 3,22 |

(67) (3S,5S)-5-[(4'-Amidino-3-methylsulfonyl-4-biphenylyl)oxymethyl]-3-carboxymethyl-1-(3-phenylpropyl)-2-pyrrolidinon

$R_f$-Wert: 0,28 (Kieselgel; Methylenchlorid/Methanol/wäßriges Ammoniak = 2:1:0,25)
Massenspektrum: $(M + H)^+ = 564$

(68) (3S,5S)-5-[(4'-Amidino-3-methylsulfenyl-4-biphenylyl)oxymethyl]-3-carboxymethyl-1-(3-phenylpropyl)-2-pyrrolidinonhydrat

$R_f$-Wert: 0,28 (Kieselgel; Methylenchlorid/Methanol/wäßriges Ammoniak = 2:1:0,25)

| Ber.: | C 65,55 | H 6,42 | N 7,64 | S 5,83 |
|-------|---------|--------|--------|--------|
| Gef.: | 65,70 | 6,58 | 7,64 | 5,50 |

(69) (3S,5S)-5-[(4'-Amidino-3-methylsulfinyl-4-biphenylyl)oxymethyl]-3-carboxymethyl-1-(3-phenylpropyl)-2-pyrrolidinonhydrat

$R_f$-Wert: 0,31 (Kieselgel; Methylenchlorid/Methanol/konz. wäßriges Ammoniak = 2:1:0,25)

| Ber.: | C 63,70 | H 6,07 | N 7,43 | S 5,67 |
|-------|---------|--------|--------|--------|
| Gef.: | 63,55 | 6,31 | 7,34 | 5,69 |

(70) (3S,5S)-5-[(4'-Amidino-4-biphenylyl)sulfonyl]aminomethyl]-3-carboxymethyl-1-(3-phenylpropyl)-2-pyrrolidinonsemihydrochlorid

$R_f$-Wert: 0,22 (Kieselgel; Methylenchlorid/Methanol/konz. wäßriges Ammoniak = 2:1:0,25)

| Ber.: | C 61,44 | H 5,78 | N 9,88 | S 5,66 | Cl 3,13 |
|-------|---------|--------|--------|--------|---------|
| Gef.: | 61,27 | 5,98 | 9,65 | 5,58 | 2,92 |

(71) (3S,5S)-5-[2-[(4-Amidinocinnamoyl)amino]ethyl]-3-carboxymethyl-1-(3-phenylpropyl)-2-pyrrolidinon

$R_f$-Wert: 0,29 (Kieselgel; Methylenchlorid/Methanol/konz. wäßriges Ammoniak = 2:1:0,25)
Massenspektrum: $(M + H)^+ = 477$

(72) (3S,5S)-5-[(4'-Amidino-4-biphenylyl)oxymethyl]-3-carboxymethyl-1-[3-(3,4-dimethoxyphenyl)propyl]-2-pyrrolidinon x 1,5 Wasser

$R_f$-Wert: 0,38 (Kieselgel; Methylenchlorid/Methanol/konz. wäßriges Ammoniak = 2:1:0,25)

| Ber.: | C 65,02 | H 6,69 | N 7,34 |
|-------|---------|--------|--------|
| Gef.: | 65,20 | 6,73 | 7,42 |

(73) (3S,5S)-5-[(4'-Amidino-4-biphenylyl)oxymethyl]-3-carboxymethyl-1-[3-(4-hexyloxyphenyl)propyl]-2-pyrrolidinon-hydrat

$R_f$-Wert: 0,31 (Kieselgel; Methylenchlorid/Methanol/konz. wäßriges Ammoniak = 2:1:0,25)

| Ber.: | C 69,63 | H 7,51 | N 6,96 |
|-------|---------|--------|--------|
| Gef.: | 69,54   | 7,67   | 6,92   |

(74)   (3S,5S)-5-[(4'-Amidino-4-biphenylyl)oxymethyl]-1-[3-(4-tert.butylphenyl)propyl]-3-carboxymethyl-2-pyrrolidinonsemihydrat

$R_f$-Wert: 0,25 (Kieselgel; Methylenchlorid/Methanol/konz. wäßriges Ammoniak = 2:1:0,25)

| Ber.: | C 71,97 | H 7,32 | N 7,63 |
|-------|---------|--------|--------|
| Gef.: | 72,22   | 7,24   | 7,70   |

(75) (3S,5S)-5-[(4'-Amidino-4-biphenylyl)oxymethyl]-3-carboxymethyl-1-[3-(3-trifluormethylphenyl)propyl]-2-pyrrolidinon-hydrat

$R_f$-Wert: 0,31 (Kieselgel; Methylenchlorid/Methanol/konz. wäßriges Ammoniak = 2:1:0,25)

| Ber.: | C 63,03 | H 5,64 | N 7,35 |
|-------|---------|--------|--------|
| Gef.: | 63,16   | 5,53   | 7,41   |

(76)   (3S,5S)-5-[(4'-Amidino-4-biphenylyl)oxymethyl]-3-carboxymethyl-1-[3-(2,4-dichlorphenyl)propyl]-2-pyrrolidinonhydrat

$R_f$-Wert: 0,41 (Kieselgel; Methylenchlorid/Methanol/konz. wäßriges Ammoniak = 2:1:0,25)

| Ber.: | C 60,84 | H 5,46 | N 7,34 | Cl 12,39 |
|-------|---------|--------|--------|----------|
| Gef.: | 61,09   | 5,59   | 7,26   | 12,71    |

(77)  (3S,5S)-5-[(4'-Amidino-4-biphenylyl)oxymethyl]-3-carboxymethyl-1-[3-(3-benzylphenyl)propyl]-2-pyrrolidinon-semihydrat

$R_f$-Wert: 0,50 (Kieselgel; Methylenchlorid/Methanol/konz. wäßriges Ammoniak = 2:1:0,25)

| Ber.: | C 73,95 | H 6,55 | N 7,19 |
|-------|---------|--------|--------|
| Gef.: | 73,85   | 6,79   | 6,90   |

(78) (3S,5S)-5-[(4'-Amidino-4-biphenylyl)oxymethyl]-1-(4,4-diphenylbutyl)-3-carboxymethyl-2-pyrrolidinon-hydrat

$R_f$-Wert: 0,22 (Kieselgel; Methylenchlorid/Methanol/konz. wäßriges Ammoniak = 2:1:0,25)

| Ber.: | C 72,51 | H 6,43 | N 7,25 |
|-------|---------|--------|--------|
| Gef.: | 72,73   | 6,61   | 7,29   |

(79)   (3S,5S)-5-[(4'-Amidino-4-biphenylyl)oxymethyl]-3-carboxymethyl-1-[3-(4-methylsulfenylphenyl)propyl]-2-pyrrolidinon x 1,5 Wasser

R$_f$-Wert: 0,28 (Kieselgel; Methylenchlorid/Methanol/konz. wäßriges Ammoniak = 2:1:0,25)

| Ber.: | C 64,49 | H 6,50 | N 7,52 | S 5,74 |
|-------|---------|--------|--------|--------|
| Gef.: | 64,62   | 6,34   | 7,35   | 5,84   |

(80) (3S,5S)-5-[(4'-Amidino-4-biphenylyl)oxymethyl]-3-carboxymethyl-1-[3-(4-methylsulfonylphenyl)propyl]-2-pyrrolidinon

R$_f$-Wert: 0,30 (Kieselgel; Methylenchlorid/Methanol/konz. wäßriges Ammoniak = 2:1:0,25)
Massenspektrum: (M + H)$^+$ = 564

(81) (3S,5S)-5-[(4'-Amidino-4-biphenylyl)oxymethyl]-1-(4-biphenylylmethyl)-3-carboxymethyl-2-pyrrolidinon x 1,5 Wasser

R$_f$-Wert: 0,33 (Kieselgel; Methylenchlorid/Methanol/konz. wäßriges Ammoniak = 2:1:0,25)

| Ber.: | C 70,70 | H 6,11 | N 7,50 |
|-------|---------|--------|--------|
| Gef.: | 70,58   | 6,09   | 7,39   |

(82) (3R,5S)-5-[(4'-Amidino-4-biphenylyl)oxymethyl]-3-(3-carboxypropyl)-1-(3-phenylpropyl)-2-pyrrolidinon

R$_f$-Wert: 0,26 (Kieselgel; Methylenchlorid/Methanol/konz. wäßriges Ammoniak = 8:2:0,10)

| Ber.: | C 72,49 | H 6,87 | N 8,18 |
|-------|---------|--------|--------|
| Gef.: | 72,29   | 7,02   | 8,34   |

(83) (3S,5S)-5-[[(3'-Amidino-4-biphenylyl)carbonyl]aminomethyl]-3-carboxymethyl-1-(3-phenylpropyl)-2-pyrrolidinonhydrochlorid-hydrat
Durchführung mit Lithiumhydroxid und Aufarbeitung mit Salzsäure.

R$_f$-Wert: 0,14 (Kieselgel; Methylenchlorid/Methanol/konz. wäßriges Ammoniak = 4:1:0,25)

| Ber.: | C 63,54 | H 6,22 | N 9,88 | Cl 6,25 |
|-------|---------|--------|--------|---------|
| Gef.: | 63,79   | 6,08   | 9,61   | 6,40    |

(84) (3S,5S)-5-[[(4'-Amidino-3-biphenylyl)carbonyl]aminomethyl]-3-carboxymethyl-1-(3-phenylpropyl)-2-pyrrolidinon

R$_f$-Wert: 0,13 (Kieselgel; Methylenchlorid/Methanol/konz. wäßriges Ammoniak = 4:1:0,25)
Massenspektrum: (M + H)$^+$ = 513

(85) (3S,5S)-5-[(4'-Amidino-4-biphenylyl)sulfonylmethyl]-3-carboxymethyl-1-(3-phenylpropyl)-2-pyrrolidinon

R$_f$-Wert: 0,25 (Kieselgel; Methylenchlorid/Methanol/konz. wäßriges Ammoniak = 2:1:0,25)
Massenspektrum: (M + H)$^+$ = 534

(86) (3S,5S)-5-[(4'-Amidino-4-biphenylyl)sulfenylmethyl]-3-carboxymethyl-1-(3-phenylpropyl)-2-pyrrolidinon

R$_f$-Wert: 0,41 (Kieselgel; Methylenchlorid/Methanol/konz. wäßriges Ammoniak = 4:1:0,25)
Massenspektrum: (M + H)$^+$ = 502

(87) (3S,5S)-5-[[[1-(4-Amidinophenyl)-1-propen-3-yl]aminocarbonyl]methyl]-3-carboxymethyl-1-(3-phenylpropyl)-2-pyrrolidinon

$R_f$-Wert: 0,14 (Kieselgel; Methylenchlorid/Methanol/konz. wäßriges Ammoniak = 4:1:0,25)
Massenspektrum: $(M + H)^+$ = 477

(88) (3R,S;4R,S)-4-[(4'-Amidino-4-biphenylyl)oxymethyl]-3-carboxymethyl-2-pyrrolidinon

Schmelzpunkt: > 260°C
$R_f$-Wert: 0,67 (reversed phase Kieselgel RP 18; Methanol/10 %ige wäßrige Kochsalzlösung = 6:4)

(89) (3S,5S)-5-[(7-Amidino-9,10-dihydro-2-phenanthrenyl)oxymethyl]-3-carboxymethyl-3-pyrrolidinon x 1,25 $H_2O$ x 0,2 HCl

$R_f$-Wert: 0,57 (Kieselgel; Methylenchlorid/Methanol/konz. wäßriges Ammoniak = 1:1:0,2)

| Ber.: | C 62,43 | H 6,12 | N 9,93 | Cl 1,68 |
|-------|---------|--------|--------|---------|
| Gef.: | 62,46 | 6,13 | 9,96 | 1,64 |

(90) (3S,5S)-5-[(3-Amidinobenzoyl)aminomethyl]-3-carboxymethyl-1-(3-phenylpropyl)-2-pyrrolidinon

$R_f$-Wert: 0,13 (Kieselgel; Methylenchlorid/Methanol/konz. wäßriges Ammoniak = 4:1:0,25)
Massenspektrum: $(M + H)^+$ = 437

(91) (3S,5S)-5-[[exo-5-(4-Amidinophenyl)bicyclo[2.2.1]heptyl-exo-2-carbonyl]aminomethyl]-3-carboxymethyl-1-(3-phenylpropyl)-2-pyrrolidinon

$R_f$-Wert: 0,45 (Kieselgel; Methylenchlorid/Methanol/konz. wäßriges Ammoniak = 4:1:0,25)
Massenspektrum: $(M + H)^+$ = 531

(92) (3S,5S)-5-[(4'-Amidino-3'-fluor-4-biphenylyl)oxymethyl]-3-carboxymethyl-2-pyrrolidinon

Schmelzpunkt: 265-267°C (Zers.)
$R_f$-Wert: 0,47 (reversed phase Kieselgel; Methanol/10%ige wäßrige Kochsalzlösung 6:4)

(93) (3R,S;4R,S)-4-[(4'-Amidino-4-biphenylyl)oxymethyl]-3-carboxymethyl-3-methyl-2-pyrrolidinon

Schmelzpunkt: 278°C (Zers.)
$R_f$-Wert: 0,59 (reversed phase Kieselgel; Methanol/10%ige wäßrige Kochsalzlösung 6:4)

(94) (3R,S;4R,S)-4-[[(4'-Amidino-4-biphenylyl)carbonylamino]methyl]-3-carboxymethyl-2-pyrrolidinon

Schmelzpunkt: 278-280°C (Zers.)
$R_f$-Wert: 0,64 (reversed phase Kieselgel; Methanol/10%ige wäßrige Kochsalzlösung 6:4)

(95) (3R,S;4R,S)-4-[[(4'-Amidino-4-biphenylyl)carbonylamino]methyl]-3-carboxymethyl-3-methyl-2-pyrrolidinon

(96) (3S,5S)-5-[(4'-Amidino-4-biphenylyl)oxymethyl]-3-carboxymethyl-1-(2-carboxyphenyl)-2-pyrrolidinon

(97) (3S,5S)-5-[(4'-Amidino-4-biphenylyl)oxymethyl]-1-[2-(aminocarbonyl)phenyl]-3-carboxymethyl-2-pyrrolidinon

(98) (3S,5S)-5-[(4'-Amidino-4-biphenylyl)oxymethyl]-3-carboxymethyl)-1-[2-(ethylaminocarbonyl)phenyl]-2-pyrrolidinon

(99) (3S,5S)-5-[(4'-Amidino-4-biphenylyl)oxymethyl]-3-carboxymethyl-1-[2-(dimethylaminocarbonyl)phenyl]-2-pyrrolidinon

(100) (3S,5S)-5-[(4'-Amidino-4-biphenylyl)oxymethyl]-3-carboxymethyl-1-(3-carboxyphenyl)-2-pyrrolidinon

(101) (3S,5S)-5-[(4'-Amidino-4-biphenylyl)oxymethyl]-1-[3-(aminocarbonyl)phenyl]-3-carboxymethyl-2-pyrrolidinon

(102) (3S,5S)-5-[(4'-Amidino-4-biphenylyl)oxymethyl]-3-carboxymethyl-1-[3-(ethylaminocarbonyl)phenyl]-2-pyrrolidinon

(103) (3S,5S)-5-[(4'-Amidino-4-biphenylyl)oxymethyl]-3-carboxymethyl-1-[3-(dimethylaminocarbonyl)phenyl]-2-pyrrolidinon

(104) (3S,5S)-5-[(4'-Amidino-4-biphenylyl)oxymethyl]-3-carboxymethyl-1-(4-carboxyphenyl)-2-pyrrolidinon

(105) (3S,5S)-5-[(4'-Amidino-4-biphenylyl)oxymethyl]-1-[4-(aminocarbonyl)phenyl]-3-carboxymethyl-2-pyrrolidinon

(106) (3S,5S)-5-[(4'-Amidino-4-biphenylyl)oxymethyl]-3-carboxymethyl-1-[4-(ethylaminocarbonyl)phenyl]-2-pyrrolidinon

(107) (3S,5S)-5-[(4'-Amidino-4-biphenylyl)oxymethyl]-3-carboxymethyl-1-[4-(dimethylaminocarbonyl)phenyl]-2-pyrrolidinon

(108) (3S,5S)-5-[(4'-Amidino-4-biphenylyl)oxymethyl]-3-carboxymethyl-1-[3-(methansulfonylamino)phenyl]-2-pyrrolidinon

(109) (3S,5S)-1-[3-(Acetamino)phenyl]-5-[(4'-amidino-4-biphenylyl)oxymethyl]-3-carboxymethyl-2-pyrrolidinon

(110) (3S,5S)-5-[[(6-Amidino-1,2,3,4-tetrahydro-2-naphthyl)aminocarbonyl]aminomethyl]-3-carboxymethyl-2-pyrrolidinon

(111) (3S,5S)-5-[[N-Acetyl-N-[trans-4-(4-amidinophenyl)cyclohexyl)]aminomethyl]-3-carboxymethyl-2-pyrrolidinon

(112) (3S,5S)-5-[[N-[trans-4-(4-Amidinophenyl)cyclohexyl-N-methansulfonyl]aminomethyl]-3-carboxymethyl-2-pyrrolidinon

(113) (3S,5S)-5-[(trans-4-(4-Amidinophenyl)cyclohexyl]oxymethyl]-3-carboxymethyl-2-pyrrolidinon

(114) (3S,5S)-5-[(4'-Amidino-3-ethoxy-4-biphenylyl)oxymethyl]-3-carboxymethyl-2-pyrrolidinon

(115) (3S,5S)-5-[(4'-Amidino-3'-chlor-4-biphenylyl)oxymethyl]-3-carboxymethyl-2-pyrrolidinon x 1,5 Wasser

Schmelzpunkt: 258-260°C
$R_f$-Wert: 0,66 (reversed phase Kieselgel; Methanol/5%ige wäßrige Kochsalzlösung = 6:4)

| Ber.: | C 56,01 | H 5,40 | N 9,80 |
|-------|---------|--------|--------|
| Gef.: | 56,24 | 5,18 | 9,58 |

(116) (3S;5S)-5-[(4'-Amidino-3-brom-4-biphenylyl)oxymethyl]-3-carboxymethyl-2-pyrrolidinon

$R_f$-Wert: 0,20 (Kieselgel; Methylenchlorid/Methanol/konz. wäßriges Ammoniak = 2:1:0,25)

(117) (3S,5S)-5-[(4'-Amidino-4-biphenylyl)oxymethyl]-3-carboxymethyl-1-[2-(phenylsulfonyl)ethyl]-2-pyrrolidinon

(118) (3S,5S)-5-[(4'-Amidino-4-biphenylyl)oxymethyl]-3-carboxymethyl-1-[2-(phenylsulfenyl)ethyl]-2-pyrrolidinon

(119) (3S,5S)-5-[(4'-Amidino-4-biphenylyl)oxymethyl]-3-carboxymethyl-1-[2-(phenylsulfinyl)ethyl]-2-pyrrolidinon

(120) (3S,5S)-5-[[[1-(4-Amidinophenyl)-1-propin-3-yl]aminocarbonyl]methyl]-3-carboxymethyl-2-pyrrolidinon

(121) (3S,5S)-5-[(7-Amidino-2-phenanthrenyl)oxymethyl]-3-carboxymethyl-2-pyrrolidinon

(122) (3R,5S)-3-[(4'-Amidino-4-biphenylyl)methyl]-5-carboxymethyl-2-pyrrolidinon

(123) (3S,5S)-3-[(4'-Amidino-4-biphenylyl)oxymethyl]-5-carboxymethyl-2-pyrrolidinon

(124) 3-[(4'-Amidino-4-biphenylyl)methyl]-1-(2-carboxyethyl)-2-pyrrolidinon

(125) 1-[2-(4'-Amidino-4-biphenylyl)ethyl]-3-carboxymethyl-2-pyrrolidinon

(126) (3R,5S)-3-[2-(4'-Amidino-4-biphenylyl)ethyl]-5-carboxymethyl-2-pyrrolidinon

(127) (2S,5S)-5-[(4'-Amidino-4-biphenylyl)oxymethyl]-2-carboxymethyl-1-methansulfonyl-pyrrolidin

(128) (2S,5S)-1-Acetyl-5-[(4'-amidino-4-biphenylyl)oxymethyl]-2-carboxymethyl-pyrrolidin

(129) (3S,5S)-5-[[(4'-Amidino-3-biphenylyl)sulfonylamino]methyl]-3-carboxymethyl-2-pyrrolidinon

(130) (3S,5S)-5-[[(3'-Amidino-4-biphenylyl)carbonylamino]methyl]-3-carboxymethyl-2-pyrrolidinon

(131) (3S,5S)-5-[(4'-Amidino-4-biphenylyl)oxymethyl]-1-(2-aminoethyl)-3-carboxymethyl-2-pyrrolidinon

(132) (3S,5S)-1-[2-(Acetylamino)ethyl]-5-[(4'-amidino-4-biphenylyl)oxymethyl]-3-carboxymethyl-2-pyrrolidinon

(133) (3S,5S)-5-[(4'-Amidino-4-biphenylyl)oxymethyl]-3-carboxymethyl-1-[2-(methansulfonylamino)ethyl]-2-pyrrolidinon

(134) (3S,5S)-5-[(4'-Amidino-4-biphenylyl)oxymethyl]-1-[2-(benzoylamino)ethyl]-3-carboxymethyl-2-pyrrolidinon

(135) (3S,5S)-5-[(4'-Amidino-4-biphenylyl)oxymethyl]-3-carboxymethyl-1-[2-(phenylsulfonylamino)ethyl]-2-pyrrolidinon

(136) (3S,5S)-1-[2-[(N-Acetyl-N-methyl)amino]ethyl]-5-[(4'-amidino-4-biphenylyl)oxymethyl]-3-carboxymethyl-2-pyrrolidinon

(137) (3S,5S)-5-[[4-(4-Amidinophenyl)naphthyl]oxymethyl]-3-carboxymethyl-2-pyrrolidinon

(138) (3S,5S)-5-[[[2-[(4-Amidinophenyl)oxy]phenyl]carbonylamino]methyl]-3-carboxymethyl-2-pyrrolidinon

(139) (3S,5S)-5-[(4'-Amidino-4-biphenylyl)oxymethyl]-3-(1-carboxy-1-ethyl)-2-pyrrolidinon

(140) (3S,5S)-5-[(4'-Amidino-4-biphenylyl)oxymethyl]-3-carboxymethyl-1-[(N,N-dimethylamino)carbonyl]-pyrrolidin x 1,3 $H_2O$

$R_f$-Wert: 0,56 (reversed phase Kieselgel; Methanol/5%ige wäßrige Kochsalzlösung = 6:4)

| Ber.: | C 61,67 | H 6,89 | N 12,51 |
|---|---|---|---|
| Gef.: | 61,70 | 6,66 | 12,50 |

Massenspektrum: $(M + H)^+ = 425$

(141) (3S,5S)-5-[(4'-Amidino-4-biphenylyl)oxymethyl]-3-carboxymethyl-1-(dimethylamino-oxalyl)-pyrrolidin

(142) (3S,5S)-5-[(4'-Amidino-4-biphenylyl)oxymethyl]-3-carboxymethyl-1-hydroxyoxalyl-pyrrolidin

(143) (3S,5S)-5-[(4'-Amidino-4-biphenylyl)oxymethyl]-1-aminoacetyl-3-carboxymethyl-pyrrolidin

(144) (3S,5S)-5-[2-[[N-(6-Amidino-2-naphthylcarbonyl)-N-ethyl]amino]ethyl]-3-carboxymethyl-2-pyrrolidinon

(145) (3S,5S)-5-[(4'-Amidino-4-biphenylyl)oxymethyl]-3-carboxymethyl-1-[(4-methoxyphenyl)sulfonyl]-pyrrolidin x 0,5 $H_2O$

$R_f$-Wert: 0,39 (reversed phase Kieselgel; Methanol/10%ige wäßrige Kochsalzlösung = 6:4)

| Ber.: | C 60,88 | H 5,68 | N 7,89 |
|-------|---------|--------|--------|
| Gef.: | 61,00 | 5,87 | 7,63 |

(146) (3S,5S)-5-[(4'-Amidino-4-biphenylyl)oxymethyl]-1-aminocarbonyl-3-carboxymethyl-pyrrolidin

(147) (2S,4R)-4-[(4'-Amidino-4-biphenylyl)oxymethyl]-2-carboxymethyl-1-methansulfonyl-pyrrolidin

(148) (3R,5S)-5-[(4'-Amidino-4-biphenylyl)oxymethyl]-3-carboxymethyl-3-(methansulfonyl)methyl-1-(3-phenylpropyl)-2-pyrrolidinon

(149) (3S,5S)-5-[(4'-Amidino-4-biphenylyl)oxymethyl]-3-carboxymethyl-1-[(pyrrolidin-N-carbonyl)methyl]-2-pyrrolidinon

$R_f$-Wert: 0,64 (reversed phase Kieselgel; Methanol/10%ige wäßrige Kochsalzlösung = 6:4)

(150) (3S,5S)-5-[(4'-Amidino-4-biphenylyl)oxymethyl]-3-carboxymethyl-1-[(piperidin-N-carbonyl)methyl]-2-pyrrolidinon

(151) (3S,5S)-5-[(4'-Amidino-4-biphenylyl)oxymethyl]-3-carboxymethyl-1-[(morpholin-N-carbonyl)methyl]-2-pyrrolidinon

$R_f$-Wert: 0,55 (reversed phase Kieselgel; Methanol/10%ige wäßrige Kochsalzlösung = 6:4)

(152) (3S,5S)-5-[(4'-Amidino-4-biphenylyl)oxymethyl]-3-carboxymethyl-1-[(N-methylpiperazin-N'-carbonyl)methyl]-2-pyrrolidinon

(153) (3S,5S)-1-[(N-Acetylpiperazin-N'-carbonyl)methyl]-5-[(4'-amidino-4-biphenylyl)oxymethyl]-3-carboxymethyl-2-pyrrolidinon

(154) (3S,5S)-5-[(4'-Amidino-4-biphenylyl)oxymethyl]-3-carboxymethyl-1-[(N-methansulfonylpiperazin-N'-carbonyl)methyl]-2-pyrrolidinon

(155) (3S,5S)-5-[(4'-Amidino-4-biphenylyl)oxymethyl]-3-carboxymethyl-1-[(thiomorpholin-N-carbonyl)methyl]-2-pyrrolidinon

(156) (3S,5S)-5-[(4'-Amidino-4-biphenylyl)oxymethyl]-3-carboxymethyl-1-[[(thiomorpholin-S-oxid)-N-carbonyl]methyl]-2-pyrrolidinon

(157) (3S,5S)-5-[(4'-Amidino-4-biphenylyl)oxymethyl]-3-carboxymethyl-1-[[(thiomorpholin-S-dioxid)-N-carbonyl]methyl]-2-pyrrolidinon

(158) (3S,5S)-5-[(4'-Amidino-4-biphenylyl)oxymethyl]-3-carboxymethyl-1-[(1-piperazinylcarbonyl)methyl]-2-pyrrolidinon

(159) (3S,5S)-5-[(4'-Amidino-4-biphenylyl)oxymethyl]-1-(4-brombenzyl)-3-carboxymethyl-2-pyrrolidinon

(160) (3S,5S)-5-[(4'-Amidino-4-biphenylyl)oxymethyl]-3-carboxymethyl-1-(4-methylbenzyl)-2-pyrrolidinon

(161) (3S,5S)-5-[(4'-Amidino-4-biphenylyl)oxymethyl]-3-carboxymethyl-1-(4-methoxybenzyl)-2-pyrrolidinon

(162) (3S,5S)-5-[(4'-Amidino-4-biphenylyl)oxymethyl]-3-carboxymethyl-1-[3-(methylsulfenyl)propyl]-2-pyrrolidinon

(163) (3S,5S)-5-[(4'-Amidino-4-biphenylyl)oxymethyl]-3-carboxymethyl-1-[3-(methylsulfinyl)propyl]-2-pyrrolidinon

(164) (3S,5S)-5-[(4'-Amidino-4-biphenylyl)oxymethyl]-3-carboxymethyl-1-[3-(methylsulfonyl)propyl]-2-pyrrolidinon

(165) (3S,5S)-5-[(4'-Amidino-4-biphenylyl)oxymethyl]-3-carboxymethyl-1-phenylsulfonyl-pyrrolidin

(166) (3S,5S)-5-[(4'-Amidino-3-methoxy-4-biphenylyl)oxymethyl]-3-carboxymethyl-2-pyrrolidinon

(167) (3S,5S)-5-[(4'-Amidino-4-biphenylyl)oxymethyl]-3-carboxymethyl-1-methoxyacetyl-pyrrolidin

Schmelzpunkt: 230-233°C
$R_f$-Wert: 0,06 (Kieselgel; Methylenchlorid/Methanol/konz. wäßriges Ammoniak = 10:2:0,4)

(168) 1-(4'-Amidino-4-biphenylyl)-4-carboxymethyl-2-pyrrolidinon

Beispiel 5

(3S,5S)-5-[[4-[4-(tert.Butyloxycarbonylamino)butyl]phenyl]oxymethyl]-3-carboxymethyl-1-(4-phenylbutyl)-2-pyrrolidinon

Hergestellt analog Beispiel CXII.

$R_f$-Wert: 0,47 (Kieselgel; Methylenchlorid/Methanol = 9:1)

Analog werden erhalten:

(1) (3S,5S)-1-Benzyl-5-[[4-[4-(tert.butyloxycarbonylamino)butyl]phenyl]oxymethyl]-3-carboxymethyl-2-pyrrolidinon

$R_f$-Wert: 0,47 (Kieselgel; Methylenchlorid/Methanol = 9:1)

(2) (3S,5S)-5-[[4-[4-(tert.Butyloxycarbonylamino)butyl]phenyl]oxymethyl]-3-carboxymethyl-1-(4-phenoxybutyl)-2-pyrrolidinon

$R_f$-Wert: 0,48 (Kieselgel; Methylenchlorid/Methanol = 9:1)

(3) (3S,5S)-5-[[4-(4-tert.Butyloxycarbonylaminobutyl)phenyl]oxymethyl]-3-carboxymethyl-1-(2-phenylethyl)-2-pyrrolidinon

$R_f$-Wert: 0,46 (Kieselgel; Methylenchlorid/Methanol = 9:1)

(4) (3S,5S)-5-[[[4-[2-(tert.Butyloxycarbonylamino)ethyl]phenyl]carbonylamino]methyl]-3-carboxymethyl-1-(3-phenylpropyl)-2-pyrrolidinon

$R_f$-Wert: 0,65 (Kieselgel; Methylenchlorid/Methanol = 4:1)

(5) (3S,5S)-5-[[[3-[2-(tert.Butyloxycarbonylamino)ethyl]phenyl]carbonylamino]methyl]-3-carboxymethyl-1-(3-phenylpropyl)-2-pyrrolidinon

$R_f$-Wert: 0,30 (Kieselgel; Methylenchlorid/Methanol = 10:1)

(6) (3S,5S)-5-[[[5-(tert.Butyloxycarbonylamino)pentyl]carbonylamino]methyl]-3-carboxymethyl-1-(4-phenoxybutyl)-2-pyrrolidinon

$R_f$-Wert: 0,58 (Kieselgel; Methylenchlorid/Methanol = 4:1)

(7) (3S,5S)-5-[[[5-(tert.Butyloxycarbonylamino)pentyl]carbonylamino]methyl]-3-carboxymethyl-1-(2-phenylethyl)-2-pyrrolidinon

$R_f$-Wert: 0,53 (Kieselgel; Methylenchlorid/Methanol = 4:1)

(8) (3S,5S)-1-Benzyl-5-[[[5-(tert.butyloxycarbonylamino)pentyl]carbonylamino]methyl]-3-carboxymethyl-2-pyrrolidinon

$R_f$-Wert: 0,58 (Kieselgel; Methylenchlorid/Methanol = 4:1)

(9) (3S,5S)-5-[[(2-tert.Butyloxycarbonylamino-5-indanyl)methylcarbonyl)aminomethyl]-3-carboxymethyl-1-(3-phenylpropyl)-2-pyrrolidinon

$R_f$-Wert: 0,65 (Kieselgel; Methylenchlorid/Methanol = 4:1)
Massenspektrum: $(M + H)^+ = 564$

(10) (3S,5S)-5-[[4-[3-(Benzyloxycarbonylamino)propyl]phenyl]oxymethyl]-3-carboxymethyl-1-(3-phenylpropyl)-2-pyrrolidinon

$R_f$-Wert: 0,44 (Kieselgel; Methylenchlorid/Methanol = 8:1)
Massenspektrum: $M^+ = 558$

(11) (3S,5S)-5-[[4-(tert.Butyloxycarbonylaminomethyl)phenyl]oxymethyl]-3-carboxymethyl-1-(3-phenylpropyl)-2-pyrrolidinon

$R_f$-Wert: 0,36 (Kieselgel; Methylenchlorid/Methanol = 12:1)

(12) (3S,5S)-5-[[3-(tert.Butyloxycarbonylaminomethyl)phenyl]oxymethyl]-3-carboxymethyl-1-(3-phenylpropyl)-2-pyrrolidinon

$R_f$-Wert: 0,25 (Kieselgel; Methylenchlorid/Methanol = 20:1)

(13) (3S,5S)-5-[[3-[4-(tert.Butyloxycarbonylamino)butyl]phenyl]oxymethyl]-3-carboxymethyl-1-(3-phenylpropyl)-2-pyrrolidinon

$R_f$-Wert: 0,24 (Kieselgel; Methylenchlorid/Methanol = 20:1)

(14) (3S,5S)-5-[[[5-(tert.Butyloxycarbonylamino)pentyl]carbonylamino]methyl]-3-carboxymethyl-1-isobutyl-2-pyrrolidinon

$R_f$-Wert: 0,60 (Kieselgel; Methylenchlorid/Methanol = 4:1)

(15) (3S,5S)-5-[[3-[2-(tert.Butyloxycarbonylamino)ethyl]phenyl]oxymethyl]-3-carboxymethyl-1-(3-phenylpropyl)-2-pyrrolidinon

$R_f$-Wert: 0,26 (Kieselgel; Methylenchlorid/Methanol = 20:1)

(16) (3S,5S)-5-[[4-[2-(tert.Butyloxycarbonylamino)ethyl]phenyl]oxymethyl]-3-carboxymethyl-1-(3-phenylpropyl)-2-pyrrolidinon

$R_f$-Wert: 0,54 (Kieselgel; Essigester/Methanol = 9:1)
Massenspektrum: $(M + H)^+ = 511$

(17) (3S,5S)-3-Carboxymethyl-5-[[4-[2-(tert.butyloxycarbonylamino)ethyl]phenyl]oxymethyl]-1-isobutyl-2-pyrrolidinon

$R_f$-Wert: 0,57 (Kieselgel; Essigester/Methanol = 9:1)

(18) (3S,5S)-5-[(4'-Amidino-4-biphenylyl)oxymethyl]-3-carboxymethyl-1-(3-phenylpropyl)-2-pyrrolidinon-semihydrat

Schmelzpunkt: sintert ab 170°C

$R_f$-Wert: 0,32 (Kieselgel; Methylenchlorid/Methanol/konz. wäßriges Ammoniak = 2:1:0,25)

| Ber.: | C 70,42 | H 6,53 | N 8,50 |
|---|---|---|---|
| Gef.: | 70,45 | 6,59 | 8,53 |

(19) (3S,5S)-5-[[4-[cis-4-(tert.Butyloxycarbonylamino)cyclohexyl]phenyl]oxymethyl]-3-carboxymethyl-1-(3-phenylpropyl)-2-pyrrolidinon

$R_f$-Wert: 0,45 (Kieselgel; Methylenchlorid/Methanol/konz. wäßriges Ammoniak = 4:1:0,25)

(20) (3S,5S)-5-[[4-[trans-4-(tert.Butyloxycarbonylamino)cyclohexyl]phenyl]oxymethyl]-3-carboxymethyl-1-(3-phenylpropyl)-2-pyrrolidinon

$R_f$-Wert: 0,40 (Kieselgel; Methylenchlorid/Methanol/konz. wäßriges Ammoniak = 4:1:0,25)

(21) (3S,5S)-5-[[[3-(3-Amidinophenyl)propyl]carbonylamino]methyl]-3-carboxymethyl-1-(3-phenylpropyl)-2-pyrrolidinon

$R_f$-Wert: 0,12 (Kieselgel; Methylenchlorid/Methanol/konz. wäßriges Ammoniak = 4:1:0,25)

(22) (3S,5S)-1-[3-(4-Benzyloxyphenyl)propyl]-5-[[4-[4-(tert.butyloxycarbonylamino)butyl]phenyl]oxymethyl]-3-carboxymethyl-2-pyrrolidinon

$R_f$-Wert: 0,40 (Kieselgel; Methylenchlorid/Methanol = 9:1)

Als weiteres Produkt wird (3S,5S)-1-[3-(4-Benzyloxyphenyl)propyl]-5-[[4-[4-(tert.butyloxycarbonylamino)butyl]phenyl]oxymethyl]-3-(2,3-dihydroxypropyl)-2-pyrrolidinon erhalten.

$R_f$-Wert: 0,43 (Kieselgel; Methylenchlorid/Methanol = 9:1)

(23) (3S,5S)-5-[(6-Amidino-2-naphthyl)oxymethyl]-3-carboxymethyl-1-(3-phenylpropyl)-2-pyrrolidinon

$R_f$-Wert: 0,07 (Kieselgel; Methylenchlorid/Methanol/konz. wäßriges Ammoniak = 4:1:0,25)

(24) (3S,5S)-5-[[[3-[3-(tert.Butyloxycarbonylamino)phenyl]propyl]carbonylamino]methyl]-3-carboxymethyl-1-(3-phenylpropyl)-2-pyrrolidinon

$R_f$-Wert: 0,49 (Kieselgel; Methylenchlorid/Methanol/Eisessig = 19:1:0,1)

(25) (3S,5S)-5-[[[2-(tert.Butyloxycarbonylamino)-5-indanyl]carbonylamino]methyl]-3-carboxymethyl-1-(3-phenylpropyl)-2-pyrrolidinon

$R_f$-Wert: 0,22 (Kieselgel; Methylenchlorid/Cyclohexan/Methanol/konz. wäßriges Ammoniak = 68:15:15:2)

(26) (3S,5S)-5-[[[2-[(tert.Butyloxycarbonylamino)methyl]-5-indanyl]carbonylamino]methyl]-3-carboxymethyl-1-(3-phenylpropyl)-2-pyrrolidinon

$R_f$-Wert: 0,18 (Kieselgel; Methylenchlorid/Cyclohexan/Methanol/konz. wäßriges Ammoniak = 68:15:15:2)

(27) (3S,5S)-5-[[[[2-[(tert.Butyloxycarbonylamino)methyl]-5-indanyl]methyl]carbonylamino]methyl]-3-carboxymethyl-1-(3-phenylpropyl)-2-pyrrolidinon

$R_f$-Wert: 0,31 (Kieselgel; Methylenchlorid/Cyclohexan/Methanol/konz. wäßriges Ammoniak = 68:15:15:2)

(28) (3S,5S)-5-[[[3-(tert.Butyloxycarbonylamino)propyl]phenyl]oxymethyl]-3-carboxymethyl-1-(3-phenylpropyl)-2-pyrrolidinon

$R_f$-Wert: 0,42 (Kieselgel; Methylenchlorid/Cyclohexan/Methanol/konz. wäßriges Ammoniak = 68:15:15:2)

(29) (3S,5S)-5-[[4-[5-(tert.Butyloxycarbonylamino)pentyl]phenyl]oxymethyl]-3-carboxymethyl-1-(3-phenylpropyl)-2-pyrrolidinon

$R_f$-Wert: 0,37 (Kieselgel; Methylenchlorid/Cyclohexan/Methanol/konz. wäßriges Ammoniak = 68:15:15:2)

(30)    (3R,S;5S,R)-5-[[4-[(tert.Butyloxycarbonylamino)methyl]phenyl]oxymethyl]-3-carboxymethyl-3-methyl-1-(3-phenylpropyl)-2-pyrrolidinon

$R_f$-Wert: 0,39 (Kieselgel; Methylenchlorid/Ethanol = 15:1)

(31)    (3R,S;5S,R)-5-[[4-[2-(tert.Butyloxycarbonylamino)ethyl]phenyl]oxymethyl]-3-carboxymethyl-3-methyl-1-(3-phenylpropyl)-2-pyrrolidinon

$R_f$-Wert: 0,42 (Kieselgel; Methylenchlorid/Ethanol = 15:1)

(32)    (3R,S;5S,R)-5-[[4-[4-(tert.Butyloxycarbonylamino)butyl]phenyl]oxymethyl]-3-carboxymethyl-3-methyl-1-(3-phenylpropyl)-2-pyrrolidinon

$R_f$-Wert: 0,36 (Kieselgel; Methylenchlorid/Cyclohexan/Methanol/konz. wäßriges Ammoniak = 68:15:15:2)

(33)    (3R,5S)-5-[[[4-(tert.Butyloxycarbonylamino)butyl]carbonylamino]methyl]-3-(2-carboxyethyl)-1-(3-phenylpropyl)-2-pyrrolidinon

$R_f$-Wert: 0,50 (Kieselgel; Methylenchlorid/Methanol/Essigsäure = 9:1:0,1)

(34)  (3R,5S)-5-[[[(5-(tert.Butyloxycarbonylamino)pentyl]carbonylamino]methyl]-3-(2-carboxyethyl)-1-(3-phenylpropyl)-2-pyrrolidinon

$R_f$-Wert: 0,50 (Kieselgel; Methylenchlorid/Methanol/Essigsäure = 9:1:0,1)

(35) (3S,5S)-5-[[2-[4-[(tert.Butyloxycarbonylamino)methyl]phenyl]ethyl]carbonylamino]methyl]-3-carboxymethyl-1-(3-phenylpropyl)-2-pyrrolidinon

$R_f$-Wert: 0,50 (Kieselgel; Methylenchlorid/Methanol/Essigsäure = 9:1:0,1)

| Ber.: | C 67,49 | H 7,49 | N 7,62 |
|-------|---------|--------|--------|
| Gef.: | 67,27 | 7,36 | 7,60 |

(36) (3S,5S)-5-[[[2-[3-(tert.Butyloxycarbonylamino)phenyl]ethyl]carbonylamino]methyl]-3-carboxymethyl-1-(3-phenylpropyl)-2-pyrrolidinon

$R_f$-Wert: 0,55 (Kieselgel; Methylenchlorid/Methanol/Essigsäure = 9:1:0,1)

(37) (3S,5S)-5-[[[2-[4-(tert.Butyloxycarbonylamino)phenyl]ethyl]carbonylamino]methyl]-3-carboxymethyl-1-(3-phenylpropyl)-2-pyrrolidinon

$R_f$-Wert: 0,30 (Kieselgel; Methylenchlorid/Methanol = 9:1)

(38) (3S,5S)-5-[[3-[4-(tert.Butyloxycarbonylamino)phenyl]propyl]carbonylamino]methyl]-3-carboxymethyl-1-(3-phenylpropyl)-2-pyrrolidinon

$R_f$-Wert: 0,38 (Kieselgel; Methylenchlorid/Methanol = 9:1)

Beispiel 6

(3S,5S)-5-[(4'-Amidino-4-biphenylyl)oxymethyl]-3-[(methoxycarbonyl)methyl]-1-phenyl-2-pyrrolidinon-hydrochlorid-hydrat

900 mg (3S,5S)-5-[[4'-(Benzyloxycarbonylamidino)-4-biphenylyl]oxymethyl]-3-[(methoxycarbonyl)methyl]-1-phenyl-2-pyrrolidinon werden mit 50 ml Methanol, 2 ml methanolischer Salzsäure, 5 ml Dioxan und 200 mg Palladium/Kohle-Katalysator versetzt und 1,5 Stunden bei 5 bar Wasserstoffdruck und Raumtemperatur hydriert. Der Katalysator wird abfiltriert und das Filtrat eingeengt. Der feste farblose Eindampfrückstand wird mit tert.-Butyl-methylether 20 Minuten unter Rückfluß erhitzt. Nach dem Abkühlen wird das Produkt abgesaugt und getrocknet. Ausbeute: 700 mg (93 % der Theorie),

$R_f$-Wert: 0,20 (Kieselgel; Toluol/Dioxan/Methanol/konz. wäßriges Ammoniak = 2:5:2:1)

| Ber.: | C 63,34 | H 5,90 | N 8,21 | Cl 6,92 |
|-------|---------|--------|--------|---------|
| Gef.: | 63,17 | 6,10 | 7,93 | 6,96 |

Analog wird erhalten:

(1) (3S,5S)-5-[[4-(3-Aminopropyl)phenyl]oxymethyl]-3-carboxymethyl-1-(3-phenypropyl)-2-pyrrolidinon-hydrat Hydrierung in Methanol, Säulenchromatographie

$R_f$-Wert: 0,29 (Kieselgel; Methanol)

| Ber.: | C 67,84 | H 7,74 | N 6,33 |
|-------|---------|--------|--------|
| Gef.: | 68,06 | 7,75 | 6,13 |

Massenspektrum: $M^+$ = 424

Beispiel 7

(3S,5S)-5-[[(3'-Amino-3-biphenylyl)carbonyl]aminomethyl]-3-[(methoxycarbonyl)methyl]-1-(3-phenylpropyl)-2-pyrrolidinon

Hergestellt analog Beispiel CXIV in Ethanol als Lösungsmittel. $R_f$-Wert: 0,41 (Kieselgel; Essigester/Methylenchlorid = 2:1)

Analog werden erhalten:

(1) (3S,5S)-5-[(4-Aminophenyl)oxymethyl]-3-[(methoxycarbonyl]methyl]-1-(3-phenylpropyl)-2-pyrrolidinon Lösungsmittel: Methanol

$R_f$-Wert: 0,26 (Kieselgel; Cyclohexan/Essigester = 1:1)

(2) (3S,5S)-5-[2-[[(3'-Amino-4-biphenylyl)carbonyl]amino]ethyl]-3-[(methoxycarbonyl)methyl-1-(3-phenylpropyl)-2-pyrrolidinon
Als Katalysator wurde Raney-Nickel und als Lösungsmittel Methanol verwendet.

$R_f$-Wert: 0,52 (Kieselgel; Methylenchlorid/Methanol/konz. wäßriges Ammoniak = 19:1:0,1) (nach zweifacher Entwicklung)

(3) (3S,5S)-5-[[(3'-Amino-4-biphenylyl)carbonyl]aminomethyl]-3-[(methoxycarbonyl)methyl]-1-(3-phenylpropyl)-2-pyrrolidinon Als Katalysator wurde Raney-Nickel eingesetzt.

$R_f$-Wert: 0,88 (Kieselgel; Methylenchlorid/Methanol/konz. wäßriges Ammoniak = 4:1:0,25)

Beispiel 8

(3S,5S)-5-[(4'-Amidino-3'-chlor-4-biphenylyl)oxymethyl]-3-[(methoxycarbonyl)methyl]-1-(3-phenylpropyl)-2-pyrrolidinon

1,07 g (3S,5S)-5-[(4'-Aminocarbonyl-3'-chlor-4-biphenylyl)oxymethyl]-3-[(methoxycarbonyl)methyl]-1-(3-phenylpropyl)-2-pyrrolidinon werden in 5 ml trockenem Methylenchlorid gelöst und unter Stickstoff mit 2 ml einer 1M Lösung von Triethyloxoniumtetrafluorborat in Methylenchlorid versetzt. Nach 18 Stunden Rühren bei Raumtemperatur werden ca. 2/3 des Lösungsmittels im Vakuum abrotiert und die Mischung mit 15 ml trockenem Diethylether versetzt. Das Lösungsmittel wird abdekantiert und der Rückstand in 17 ml Methanol aufgenommen. Dazu gibt man 0,5 g Ammoniumcarbonat und läßt den Ansatz 18 Stunden bei Raumtemperatur rühren. Das Methanol wird abrotiert und der Rückstand mit Methylenchlorid verrührt. Nachdem vom Ungelösten abfiltriert wurde entfernt man das Lösungsmittel im Vakuum und chromatographiert den Rückstand mit Methylenchlorid/Methanol/konz. wäßriges Ammoniak (80:20:1,25) über eine Kieselgelsäule.

Ausbeute: 0,25 g (23,4 % der Theorie),
$R_f$-Wert: 0,57 (Kieselgel; Methylenchlorid/Methanol/konz. wäßriges Ammoniak = 100:20:5)

Beispiel 9

(3S,5S)-5-[[(2-Aminomethyl-5-indanyl)sulfonyl]aminomethyl]-3-carboxymethyl-1-(3-phenylpropyl)-2-pyrrolidinon-hydrat

Hergestellt analog Beispiel LXXX durch Umsetzung von (3S,5S)-3-Carboxymethyl-1-(3-phenylpropyl)-5-[[2-(N-Phthalimidomethyl)-5-indanyl]sulfonyl]aminomethyl]-2-pyrrolidinon mit Methylamin. Aufarbeitung und chromatographische Reinigung der wäßrigen Phase.

$R_f$-Wert: 0,32 (Kieselgel; Methanol/Essigester/wäßriges Ammoniak = 2:1:0,05

| Ber.: | C 60,33 | H 6,82 | N 8,12 | S 6,19 |
|---|---|---|---|---|
| Gef.: | 60,08 | 6,63 | 8,20 | 6,21 |

Analog wird hergestellt:

(1)     (3S,5S)-5-[[(2-Amino-5-indanyl)sulfonyl]aminomethyl]-3-carboxymethyl-1-(3-phenylpropyl)-2-pyrrolidinon-hydrat Aufarbeitung und chromatographische Reinigung der wäßrigen Phase.

$R_f$-Wert: 0,39 (Kieselgel; Methylenchlorid/Cyclohexan/Methanol/konz. wäßriges Ammoniak = 34:7,5:57,5:1)

| Ber.: | C 59,57 | H 6,55 | N 8,33 | S 6,35 |
|---|---|---|---|---|
| Gef.: | 59,80 | 6,62 | 8,89 | 7,02 |

Beispiel 10

(3S,5S)-5-[[4-[[3-(tert.Butyloxycarbonylamino)propyl]carbonylamino]phenyl]oxymethyl]-3-[(methoxycarbonyl)methyl]-1-(3-phenylpropyl)-2-pyrrolidinon-semihydrat

Hergestellt analog Beispiel CXVIII.

$R_f$-Wert: 0,42 (Kieselgel; Cyclohexan/Essigester = 1:2)

| Ber.: | C 65,07 | H 7,51 | N 7,11 |
|-------|---------|--------|--------|
| Gef.: | 64,95 | 7,57 | 6,94 |

Analog werden erhalten:

(1)   (3S,5S)-5-[2-[(6-tert.Butyloxycarbonylamino-5,6,7,8-tetrahydro-2-naphthylcarbonyl)amino]ethyl]-3-[(methoxycarbonyl)-methyl]-1-(3-phenylpropyl)-2-pyrrolidinon-semihydrat

$R_f$-Wert: 0,39 (Kieselgel; Essigester)

| Ber.: | C 67,98 | H 7,72 | N 6,99 |
|-------|---------|--------|--------|
| Gef.: | 67,89 | 7,77 | 7,02 |

(2) (3S,5S)-5-[(6-tert.Butyloxycarbonylamino-5,6,7,8-tetrahydro-2-naphthylcarbonyl)aminomethyl]-3-[(methoxycarbonyl)methyl]-1-(3-phenylpropyl)-2-pyrrolidinon

$R_f$-Wert: 0,56 (Kieselgel; Essigester)

(3)      (3S,5S)-5-[[4-[3-(tert.Butyloxycarbonylamino)cyclobutyl]phenyl]carbonylaminomethyl]-3-[(methoxycarbonyl)methyl]-1-(3-phenylpropyl)-2-pyrrolidinon-semihydrat

$R_f$-Wert: 0,52 (Kieselgel; Essigester)

| Ber.: | C 67,56 | H 7,56 | N 7,16 |
|-------|---------|--------|--------|
| Gef.: | 67,69 | 7,86 | 7,35 |

(4)      (3S,5S)-5-[2-[[4-[3-(tert.Butyloxycarbonylamino)cyclobutyl]phenyl]carbonylamino]ethyl]-3-[(methoxycarbonyl)methyl]-1-(3-phenylpropyl)-2-pyrrolidinon-semihydrat

$R_f$-Wert: 0,23 (Kieselgel; Cyclohexan/Essigester = 1:3)

| Ber.: | C 67,98 | H 7,72 | N 7,00 |
|-------|---------|--------|--------|
| Gef.: | 68,10 | 8,03 | 6,98 |

<u>Beispiel 11</u>

<u>(3S,5S)-5-[[4-[cis/trans-4-(tert.Butyloxycarbonylamino)cyclohexyl]benzoyl]aminomethyl]-3-[(methoxycarbonyl)methyl]-1-(3-phenylpropyl)-2-pyrrolidinon</u>

Hergestellt analog Beispiel CXX.
Das entsprechende Aminhydrochlorid wurde nach zweistündiger Vorbehandlung mit N-Trimethylsilylimidazol in Tetrahydrofuran zum gemischten Anhydrid gegeben.

$R_f$-Wert: 0,65 (Kieselgel; Methylenchlorid/Methanol = 90:1)

Analog wird erhalten:

(1)   (3S,5S)-5-[[4-(tert.Butyloxycarbonylamino)cinnamoyl]aminomethyl]-3-[(methoxycarboxyl)methyl]-1-(3-phenylpropyl)-2-pyrrolidinon

$R_f$-Wert: 0,47 (Kieselgel; Methylenchlorid/Methanol = 15:1)

Beispiel 12

(3S,5S)-5-[[(3'-Amino-3-biphenylyl)carbonyl]aminomethyl]-3-carboxymethyl-1-(3-phenylpropyl)-2-pyrrolidinon

0,47 g (3S,5S)-5-[[(3'-Amino-3-biphenylyl)carbonyl]aminomethyl]-3-[(methoxycarbonyl)methyl]-1-(3-phenylpropyl)-2-pyrrolidinon werden mit 10 ml Methanol und 3 ml 1N Natronlauge versetzt und 5 Stunden bei Raumtemperatur gerührt. Dann werden 3 ml 1N Salzsäure zugegeben. Der Methanol-Anteil wird am Rotationsverdampfer abgezogen, das Wasser abdekantiert und das verbleibende Öl mit Wasser versetzt und 30 Minuten gerührt. Das Kristallisat wird abgesaugt und bei 80°C im Vakuum getrocknet.

Ausbeute: 0,37 g (81 % der Theorie),
Schmelzpunkt: 98-100°C
$R_f$-Wert: 0,47 (Kieselgel; Essigester/Methanol = 4:1)

Analog werden erhalten:

(1) (3S,5S)-5-[[4-(cis-4-Aminocyclohexyl]benzoyl]aminomethyl]-3-carboxymethyl-1-(3-phenylpropyl)-2-pyrrolidinon

$R_f$-Wert: 0,16 (Kieselgel; Methylenchlorid/Methanol/konz. wäßriges Ammoniak = 4:1:0,25)

(2) (3S,5S)-5-[[4-(trans-4-Aminocyclohexyl]benzoyl]aminomethyl]-3-carboxymethyl-1-(3-phenylpropyl)-2-pyrrolidinon

Schmelzpunkt: 232-236°C,
$R_f$-Wert: 0,15 (Kieselgel; Methylenchlorid/Methanol/konz. wäßriges Ammoniak = 4:1:0,25)

| Ber.: | C 70,85 | H 7,59 | N 8,55 |
|---|---|---|---|
| Gef.: | 70,65 | 7,89 | 8,50 |

(3) (3S,5S)-5-[[4-(cis-4-Aminomethylcyclohexyl)phenyl]oxymethyl]-3-carboxymethyl-1-(3-phenylpropyl)-2-pyrrolidinonhydrat

$R_f$-Wert: 0,45 (Kieselgel; Methylenchlorid/Methanol/konz. wäßriges Ammoniak = 4:1:0,25)

| Ber.: | C 70,28 | H 7,93 | N 5,65 |
|---|---|---|---|
| Gef.: | 70,48 | 8,23 | 5,69 |

(4) (3S,5S)-5-[[4-(trans-4-Aminomethylcyclohexyl]phenyl]oxymethyl]-3-carboxymethyl-1-(3-phenylpropyl)-2-pyrrolidinonhydrat

Schmelzpunkt: 192-196°C,
$R_f$-Wert: 0,45 (Kieselgel; Methylenchlorid/Methanol/konz. wäßriges Ammoniak = 4:1:0,25)

| Ber.: | C 70,28 | H 7,93 | N 5,65 |
|---|---|---|---|
| Gef.: | 70,15 | 8,22 | 5,57 |

(5) (3S,5S)-5-[[(3'-Amino-4-biphenylyl)carbonyl]aminomethyl]-3-carboxymethyl-1-(3-phenylpropyl)-2-pyrrolidinon

$R_f$-Wert: 0,30 (Kieselgel; Methylenchlorid/Methanol/konz. wäßriges Ammoniak = 4:1,0:0,25)
Massenspektrum: $M^+$ = 485

(6) (3S,5S)-5-[(4-Aminocinnamoyl)aminomethyl]-3-carboxymethyl-1-(3-phenylpropyl)-2-pyrrolidinon

$R_f$-Wert: 0,26 (Kieselgel; Methylenchlorid/Methanol/Cyclohexan/konz. wäßriges Ammoniak = 68:15:15:2)

(7) (3S,5S)-3-Carboxymethyl-5-[[4'-(N-ethyloxycarbonylamidino)-4-biphenylyl]oxymethyl]-2-pyrrolidinon

Schmelzpunkt: 190-192°C (Zers.)
$R_f$-Wert: 0,61 (reversed phase Kieselgel RP 8; Methanol/10%ige wäßrige Kochsalzlösung = 6:4)

Beispiel 13

(3S,5S)-3-Carboxymethyl-5-[[(3'-guanidino-3-biphenylyl)carbonyl]aminomethyl]-1-(3-phenylpropyl)-2-pyrrolidinon

Zu 290 mg (3S,5S)-5-[[(3'-Amino-3-biphenylyl)carbonyl]aminomethyl]-3-carboxymethyl-1-(3-phenylpropyl)-2-pyrrolidinon werden 10 ml Dioxan und 0,65 ml 1N Salzsäure gegeben und das Lösungsmittel im Vakuum entfernt. Der Rückstand wird 2 x mit trockenem Toluol im Vakuum eingedampft. Zur Suspension des Rückstandes in 10 ml Dioxan gibt man 38 mg Cyanamid und erhitzt 2,5 Stunden unter Rückfluß. Der Ansatz wird abgekühlt, das Lösungsmittel abdekantiert und das erhaltene Harz über eine Kieselgelsäule mit Methylenchlorid/Methanol/konz. wäßriges Ammoniak (10:5:1) gereinigt.

Ausbeute: 136 mg (40 % der Theorie),
Schmelzpunkt: ab 160°C (Zers.)
$R_f$-Wert: 0,64 (Kieselgel; Methylenchlorid/Methanol/konz. wäßriges Ammoniak = 30:20:3)

Analog werden erhalten:

(1) (3S,5S)-3-Carboxymethyl-5-[[[3-(3-guanidinophenyl)propyl]carbonylamino]methyl]-1-(3-phenylpropyl)-2-pyrrolidinonhydrochlorid

$R_f$-Wert: 0,30 (Kieselgel; Methylenchlorid/Methanol/konz. wäßriges Ammoniak = 8:2:0,2)

(2) (3S,5S)-3-Carboxymethyl-5-[[[2-(3-guanidinophenyl)ethyl]carbonylamino]methyl]-1-(3-phenylpropyl)-2-pyrrolidinon-hydrochlorid

$R_f$-Wert: 0,13 (Kieselgel; Methylenchlorid/Methanol/Essigsäure = 4:1:0,1)

(3) (3S,5S)-3-Carboxymethyl-5-[[[2-(4-guanidinophenyl)ethyl]carbonylamino]methyl]-1-(3-phenylpropyl)-2-pyrrolidinon-hydrochlorid

$R_f$-Wert: 0,10 (Kieselgel; Methylenchlorid/Methanol/Essigsäure = 4:1:0,1)
Massenspektrum: $(M+H^+)$ = 480

(4) (3S,5S)-3-Carboxymethyl-5-[[[3-(4-guanidinophenyl)propyl]carbonylamino]methyl]-1-(3-phenylpropyl)-2-pyrrolidinonsemihydrochlorid-semihydrat

$R_f$-Wert: 0,10 (Kieselgel; Methylenchlorid/Methanol/Essigsäure = 4:1:0,1)

| Ber.: | C 62,26 | H 7,06 | N 13,44 | Cl 3,40 |
|---|---|---|---|---|
| Gef.: | 62,08 | 7,06 | 13,18 | 3,42 |

(5) (3S,5S)-5-[2-[N-(3'-Guanidino-3-biphenylyl)benzylamino]ethyl]-3-[(methoxycarbonyl)methyl]-1-(3-phenylpropyl)-2-pyrrolidinon-dihydrochlorid

$R_f$-Wert: 0,51 (Kieselgel; Methylenchlorid/Methanol = 8:2)

(6) (3S,5S)-3-Carboxymethyl-5-[(4-guanidinocinnamoyl)aminomethyl]-1-(3-phenylpropyl)-2-pyrrolidinon

(7)  (3S,5S)-3-Carboxymethyl-5-[[(3'-guanidino-4-biphenylyl)carbonyl]aminomethyl]-1-(3-phenylpropyl)-2-pyrrolidinon

R$_f$-Wert: 0,10 (Kieselgel; Methylenchlorid/Methanol/konz. wäßriges Ammoniak = 4:1:0,25)

(8)  (3S,5S)-3-Carboxymethyl-5-[(4'-guanidino-4-biphenylyl)oxymethyl]-2-pyrrolidinon

Beispiel 14

(3S,R;5S,R)-5-[4-[(5-Aminopentyl)oxy]phenyl]-3-carboxymethyl-1-(3-phenylpropyl)-2-pyrrolidinon-hydrat

1,4 g (3S,R;5S,R)-3-Carboxymethyl-5-[4-[(4-cyanobutyl)oxy]phenyl]-1-(3-phenylpropyl)-2-pyrrolidinon werden in 50 ml Methanol und 50 ml konz. wäßriges Ammoniak mit 0,5 g Raney-Nickel unter einem Wasserstoffdruck von 5 bar bei Raumtemperatur bis zum Verschwinden des Ausgangsmaterials hydriert. Nach Absaugen des Katalysators wird die Lösung einrotiert, der Rückstand in Methanol aufgenommen, filtriert und erneut einrotiert. Das verbleibende Harz wird über eine Kieselgelsäule mit Methylenchlorid/Methanol/konz.wäßrig. Ammoniak (80:20:5) chromatographiert.

Ausbeute: 0,86 g (62 % der Theorie),
R$_f$-Wert: 0,45 (Kieselgel; Methylenchlorid/Methanol/konz. wäßriges Ammoniak = 80:20:5)

| Ber.: | C 68,40 | H 7,95 | N 6,14 |
|---|---|---|---|
| Gef.: | 68,61 | 7,79 | 6,13 |

Analog werden erhalten:

(1)  (3S,5S)-5-[(6-Aminomethyl-5,6,7,8-tetrahydro-2-naphthylcarbonyl)aminomethyl]-3-carboxymethyl-1-(3-phenylpropyl)-2-pyrrolidinon-semihydrat

Schmelzpunkt: 135-150°C, sintert ab 110°C
R$_f$-Wert: 0,27 (Kieselgel; Methanol)

| Ber.: | C 69,11 | H 7,46 | N 8,64 |
|---|---|---|---|
| Gef.: | 68,92 | 7,27 | 8,66 |

(2)  (3S,5S)-5-[2-[(6-Aminomethyl-5,6,7,8-tetrahydro-2-naphthylcarbonyl)amino]ethyl]-3-carboxymethyl-1-(3-phenylpropyl)-2-pyrrolidinon-semihydrat

Schmelzpunkt: 124-135°C (Zers.)
R$_f$-Wert: 0,22 (Kieselgel; Methanol)

| Ber.: | C 69,57 | H 7,65 | N 8,39 |
|---|---|---|---|
| Gef.: | 69,39 | 7,60 | 8,12 |

(3)  (3S,5S)-5-[(6-Aminomethyl-2-naphthyl)oxymethyl]-3-carboxymethyl-1-(3-phenylpropyl)-2-pyrrolidinon-hydrochlorid

Schmelzpunkt: 245-255°C
R$_f$-Wert: 0,25 (Kieselgel; Methylenchlorid/Methanol/konz. wäßriges Ammoniak = 16:4:1)

| Ber.: | C 67,14 | H 6,47 | N 5,80 |
|-------|---------|--------|--------|
| Gef.: | 66,90   | 6,40   | 5,85   |

(4) 3S,5S)-5-[(4'-Aminomethyl-4-biphenylyl)oxymethyl]-3-carboxymethyl-1-(3-phenylpropyl)-2-pyrrolidinon-hydrat

Schmelzpunkt: 185-187°C
$R_f$-Wert: 0,47 (Kieselgel; Methylenchlorid/Methanol/konz. wäßriges Ammoniak = 16:4:1)

| Ber.: | C 71,00 | H 6,99 | N 5,71 |
|-------|---------|--------|--------|
| Gef.: | 71,27   | 6,97   | 5,71   |

(5) (3S,5S)-5-[[3-(Aminomethyl)phenyl]carbonylaminomethyl]-3-carboxymethyl-1-(3-phenylpropyl)-2-pyrrolidinon

$R_f$-Wert: 0,50 (Kieselgel; n-Butanol/Eisessig/Wasser = 4:1:1)

(6) (3S,5S)-5-[[4-(Aminomethyl)phenyl]carbonylaminomethyl]-3-carboxymethyl-1-(3-phenylpropyl)-2-pyrrolidinon

$R_f$-Wert: 0,54 (Kieselgel; n-Butanol/Eisessig/Wasser = 4:1:1)

Bei der Aufarbeitung des Ansatzes mit Ethanol und etwas Salzsäure bildete sich eine geringe Menge des Ethylesters, der chromatographisch abgetrennt und charakterisiert wurde: (3S,5S)-5-[[4-(Aminomethyl)phenyl]carbonylaminomethyl]-3-[(ethoxycarbonyl)methyl]-1-(3-phenylpropyl)-2-pyrrolidinon

$R_f$-Wert: 0,61 (Kieselgel; n-Butanol/Eisessig/Wasser = 4:1:1)

(7)    (3S,5S)-5-[[4-(4-Aminobutyl)phenyl]oxymethyl]-3-carboxymethyl-3-methyl-1-(3-phenylpropyl)-2-pyrrolidinon-semihydrat

| Ber.: | C 70,25 | H 8,09 | N 6,05 |
|-------|---------|--------|--------|
| Gef.: | 70,42   | 8,08   | 6,07   |

(8)   (3S,5S)-5-[[4-(4-Aminobutyl)phenyl]oxymethyl]-3-carboxymethyl-1-(3-phenylpropyl)-2-pyrrolidinon-semihydrat x 1,25 HCl

| Ber.: | C 63,29 | H 7,15 | N 5,68 | Cl 9,00 |
|-------|---------|--------|--------|---------|
| Gef.: | 63,25   | 7,77   | 6,07   | 9,15    |

$R_f$-Wert: 0,44 (Kieselgel; Methanol)

(9) (3S,5S)-5-[(2-Aminomethyl-5-indanyl)oxymethyl]-3-carboxymethyl-1-(3-phenylpropyl)-2-pyrrolidinon-hydrat

| Ber.: | C 68,70 | H 7,54 | N 6,16 |
|-------|---------|--------|--------|
| Gef.: | 69,07   | 7,47   | 6,29   |

(10)    (3S,5S)-5-[[[3-[4-(Aminomethyl)phenyl]propyl]carbonylamino]methyl]-3-carboxymethyl-1-(3-phenylpropyl)-2-pyrrolidinon-semihydrat

| Ber.: | C 68,32 | H 7,64 | N 8,92 |
|-------|---------|--------|--------|
| Gef.: | 68,27 | 7,64 | 8,85 |

(11) (3S,5S)-5-[(4'-Aminomethyl-4-biphenylyl)oxymethyl]-3-carboxymethyl-2-pyrrolidinon

$R_f$-Wert: 0,82 (reversed phase Kieselgel; Methanol/5%ige wäßrige Kochsalzlösung = 6:4)

(12) (3S,5S)-5-[(4'-Aminomethyl-2'-methyl-4-biphenylyl)oxymethyl]-3-carboxymethyl-2-pyrrolidinon

(13) (3S,5S)-5-[(4'-Aminomethyl-2,3-dimethyl-4-biphenylyl)oxymethyl]-3-carboxymethyl-2-pyrrolidinon

Beispiel 15

(3S,5S)-5-[[[3-[3-(Aminomethyl)phenyl]propyl]carbonylamino]methyl]-3-carboxymethyl-1-(3-phenylpropyl)-2-pyrrolidinon x 1,5 Wasser

3,2 g (3S,5S)-3-Carboxymethyl-5-[[[3-(3-cyanophenyl)propyl]carbonylamino]methyl]-1-(3-phenylpropyl)-2-pyrrolidinon werden in 30 ml Eisessig gelöst und 15 Minuten mit Wasserstoff in Gegenwart von 0,5 g Raney-Nickel bei Raumtemperatur und 5 bar Druck hydriert. Die Lösung wird eingedampft, der Rückstand in 0,2N Natronlauge aufgenommen und mit Essigester extrahiert. Die wäßrige Phase wird mit 2N Salzsäure sauer gestellt und erneut mit Essigester extrahiert. Die zurückbleibende wäßrige Phase wird eingeengt und der verbleibende Rückstand durch Chromatographie an Kieselgel (Elutionsmittel: Methylenchlorid/Methanol/konz. wäßriges Ammoniak = 17:3:0,2) gereinigt.

Ausbeute: 0,85 g (25 % der Theorie),

| Ber.: | C 65,83 | H 7,78 | N 8,53 |
|-------|---------|--------|--------|
| Gef.: | 65,61 | 7,83 | 8,60 |

Beispiel 16

(3S,5S)-5-[[4-[3-(tert.Butyloxycarbonylamino)cyclobutyl]phenyl]carbonylaminomethyl]-3-carboxymethyl-1-(3-phenylpropyl)-2-pyrrolidinon-hydrat

1,3 g (3S,5S)-5-[[4-[3-(tert.Butyloxycarbonylamino)cyclobutyl]phenyl]carbonylaminomethyl]-3-[(methoxycarbonyl)methyl]-1-(3-phenylpropyl)-2-pyrrolidinon werden in 5 ml Methanol gelöst und mit 7 ml 1N Natronlauge versetzt. Nach 3 Stunden Rühren bei Raumtemperatur wird das Methanol abrotiert und der Rückstand unter Eiskühlung mit gesättigter wäßriger Kaliumhydrogensulfat-Lösung auf etwa pH 2 gestellt. Das Gemisch wird mit Essigester extrahiert, die Essigesterlösung über Magnesiumsulfat getrocknet, filtriert und einrotiert.

Ausbeute: 1,2 g (100 % der Theorie) eines weißen Schaumes.
$R_f$-Wert: 0,57 (Kieselgel; Essigester/Eisessig = 50:1)

| Ber.: | C 66,08 | H 7,45 | N 7,22 |
|-------|---------|--------|--------|
| Gef.: | 66,29 | 7,54 | 7,25 |

Analog werden erhalten:

(1) (3S,5S)-5-[[4-[[3-(tert.Butyloxycarbonylamino)propyl]carbonylamino]phenyl]oxymethyl]-3-carboxymethyl-1-(3-phenylpropyl)-2-pyrrolidinon-semihydrat

$R_f$-Wert: 0,10 (Kieselgel; Cyclohexan/Essigester = 1:2)

| Ber.: | C 64,56 | H 7,34 | N 7,29 |
|---|---|---|---|
| Gef.: | 64,74 | 7,69 | 7,00 |

(2)   (3S,5S)-5-[2-[[6-(tert.Butyloxycarbonylamino)-5,6,7,8-tetrahydro-2-naphthylcarbonyl]amino]ethyl]-3-carboxymethyl-1-(3-phenylpropyl)-2-pyrrolidinon

$R_f$-Wert: 0,38 (Kieselgel; Toluol/Dioxan/Ethanol/Eisessig = 90:10:10:6)

| Ber.: | C 68,61 | H 7,50 | N 7,27 |
|---|---|---|---|
| Gef.: | 68,59 | 7,66 | 7,33 |

(3)         (3S,5S)-5-[[6-(tert.Butyloxycarbonylamino)-5,6,7,8-tetrahydro-2-naphthylcarbonyl]aminomethyl]-3-carboxymethyl1-(3-phenylpropyl)-2-pyrrolidinon

$R_f$-Wert: 0,36 (Kieselgel; Toluol/Dioxan/Ethanol/Eisessig = 90:10:10:6)

| Ber.: | C 68,18 | H 7,33 | N 7,45 |
|---|---|---|---|
| Gef.: | 67,99 | 7,55 | 7,26 |

(4)   (3S,5S)-5-[2-[[4-[3-(tert.Butyloxycarbonylamino)cyclobutyl]phenyl]carbonylamino]ethyl]-3-carboxymethyl-1-(3-phenylpropyl)-2-pyrrolidinon-semihydrat

$R_f$-Wert: 0,44 (Kieselgel; Essigester/Eisessig = 50:1)

| Ber.: | C 67,56 | H 7,56 | N 7,16 |
|---|---|---|---|
| Gef.: | 67,34 | 7,69 | 7,03 |

<u>Beispiel 17</u>

<u>(3S,5S)-5-[2-[[2-[(tert.Butyloxycarbonylamino)methyl]-5-indanyl]carbonylamino]ethyl]-3-carboxymethyl-1-(3-phenylpropyl)-2-pyrrolidinon</u>

1,9 g (3S,5S)-5-(2-Aminoethyl)-3-carboxymethyl-1-(3-phenylpropyl)-2-pyrrolidinon werden in einer Mischung aus 20 ml Methylenchlorid und 20 ml Acetonitril gelöst und mit 0,8 ml Trimethylchlorsilan versetzt. Man rührt eine Stunde bei Raumtemperatur und dann nach 1,5 Stunden bei 45°C. Man dampft im Vakuum zur Trockne ein, nimmt mit 50 ml Tetrahydrofuran auf und kühlt auf 0°C ab. Diese Lösung wird bei -30°C zu einem Reaktionsgemisch zugetropft, das man durch Zutropfen von 1,0 ml Chlorameisensäure-isobutylester zu einer Lösung von 2,05 g 2-[(tert.Butyloxycabonylamino)methyl]indan-5-carbonsäure und 0,85 ml N-Methylmorpholin in 40 ml Tetrahydrofuran bei -30°C erhalten hat. Man fügt weitere 0,75 ml N-Methylmorpholin zu und hält zwei Stunden bei -20°C, eine Stunde bei 0°C und 3 Stunden bei Raumtemperatur. Das Reaktionsgemisch wird mit 100 ml Wasser und 200 ml Ether versetzt, mit Zitronensäure sauer gestellt, die organische Phase abgetrennt, mit 10%iger Zitronensäure gewaschen und eingeengt. Der Rückstand wird durch Chromatographie an Kieselgel (Elutionsmittel: Ether/Tetrahydrofuran/Wasser = 2:1:0,05) gereinigt.

Ausbeute: 1,25 g (35 % der Theorie),
$R_f$-Wert: 0,49 (Kieselgel; Ether/Tetrahydrofuran/Wasser = 1:1:0,5

### Beispiel 18

### (3S,5S)-5-[[4-(4-Aminobutyl)phenyl]oxymethyl]-3-carboxymethyl-1-[3-(4-hydroxyphenyl)propyl]-2-pyrrolidinon

0,25 g (3S,5S)-5-[[4-(4-Aminobutyl)phenyl]oxymethyl]-1-[3-(4-benzyloxyphenyl)propyl]-3-carboxymethyl-2-pyrrolidinon werden in 20 ml Eisessig gelöst, 0,2 g 10%ige Palladium/Kohle zugesetzt und 5 Stunden bei Raumtemperatur und 5 bar Wasserstoffdruck hydriert. Anschließend wird der Katalysator abgesaugt, das Filtrat eingeengt und der Rückstand durch Chromatographie an Kieselgel (Elutionsmittel: Methylenchlorid/Methanol/konz. wäßriges Ammoniak = 4:1:0,2) gereinigt.

Ausbeute: 0,2 g (93 % der Theorie),
$R_f$-Wert: 0,30 (Kieselgel; Methylenchlorid/Methanol/konz. wäßriges Ammoniak = 4:1:0,25)

Analog wird erhalten:

(1) (3S,5S)-3-Carboxymethyl-5-[[4'-(N-methoxycarbonylamidino)-4-biphenylyl]oxymethyl]-2-pyrrolidinon Der Benzylester wird in Dioxan/Dimethylformamid hydriert.

$R_f$-Wert: 0,61 (reversed phase Kieselgel; Methanol/10%ige Kochsalzlösung = 6:4)

### Beispiel 19

### (3S,5S)-3-Carboxymethyl-5-[[4-(cis-4-guanidinocyclohexyl)phenyl]oxymethyl]-1-(3-phenylpropyl)-2-pyrrolidinon

0,85 g (3S,5S)-5-[[4-(cis-4-Aminocyclohexyl)phenyl]oxymethyl]-3-carboxymethyl-1-(3-phenylpropyl)-2-pyrrolidinon werden in einer Mischung aus 100 ml Dimethylformamid und 50 ml Wasser suspendiert. Man fügt 1,5 g 1-Amidino-3,5-dimethylpyrazol und 1,3 ml Triethylamin zu und rührt 11 Tage bei Raumtemperatur. Man engt ein und verreibt mit Aceton und Essigester. Das so erhaltene Kristallisat wird mit Methanol verrieben und abgesaugt. Eine zweite Fraktion gewinnt man durch Chromatographie der Essigester und Acetonextrakte an Kieselgel (Elutionsmittel: Methylenchlorid/Methanol/konz.wäßriges Ammoniak = 4:1:0,25).

Ausbeute: 0,35 g (38 % der Theorie),
Schmelzpunkt: 240-245°C

Analog werden erhalten:

(1) (3S,R;5S,R)-3-Carboxymethyl-5-[4-[(5-guanidinopentyl)oxy]phenyl]-1-(3-phenylpropyl)-2-pyrrolidinon

$R_f$-Wert: 0,39 (Kieselgel; Methylenchlorid/Methanol/konz. wäßriges Ammoniak = 80:20:5)
Massenspektrum: $(M + H)^+ = 481$

(2)   (3S,5S)-3-Carboxymethyl-5-[[4-[(3-guanidinopropyl)carbonylamino]phenyl]oxymethyl]-1-(3-phenylpropyl)-2-pyrrolidinon

$R_f$-Wert: 0,19 (Kieselgel; Methylenchlorid/Methanol/konz. wäßriges Ammoniak = 40:4:1)

(3)   (3S,5S)-3-Carboxymethyl-5-[2-[(6-guanidino-5,6,7,8-tetrahydro-2-naphtylcarbonyl)amino]ethyl]-1-(3-phenylpropyl)-2-pyrrolidinon-hydrat

$R_f$-Wert: 0,35 (Kieselgel; Methanol/konz. wäßriges Ammoniak = 98:2)

| Ber.: | C 64,78 | H 7,31 | N 13,02 |
|-------|---------|--------|---------|
| Gef.: | 64,85 | 7,36 | 13,30 |

(4) (3S,5S)-3-Carboxymethyl-5-[(6-guanidino-5,6,7,8-tetrahydro-2-naphtylcarbonyl)aminomethyl]-1-(3-phenylpropyl)-2-pyrrolidinon-hydrat

$R_f$-Wert: 0,42 (Kieselgel; Methanol/konz. wäßriges Ammoniak = 98:2)

| Ber.: | C 64,23 | H 7,12 | N 13,37 |
|-------|---------|--------|---------|
| Gef.: | 63,99 | 7,13 | 13,40 |

(5) (3S,5S)-3-Carboxymethyl-5-[[(6-guanidinomethyl-5,6,7,8-tetrahydro-2-naphtylcarbonyl)aminomethyl]-1-(3-phenylpropyl)-2-pyrrolidinon-hydrat

$R_f$-Wert: 0,27 (Kieselgel; Methanol/konz.wäßriges Ammoniak = 98:2)

| Ber.: | C 64,78 | H 7,31 | N 13,02 |
|-------|---------|--------|---------|
| Gef.: | 64,66 | 7,25 | 13,33 |

(6) (3S,5S)-3-Carboxymethyl-5-[2-[(6-guanidinomethyl-5,6,7,8-tetrahydro-2-naphtylcarbonyl)amino]ethyl]-1-(3-phenylpropyl)-2-pyrrolidinon-hydrat

$R_f$-Wert: 0,29 (Kieselgel; Methanol/konz.wäßriges Ammoniak = 99:1)

| Ber.: | C 65,31 | H 7,49 | N 12,69 |
|-------|---------|--------|---------|
| Gef.: | 65,06 | 7,37 | 13,00 |

(7) (3S,5S)-3-Carboxymethyl-5-[[4-(3-guanidinocyclobutyl)phenyl]carbonylaminomethyl]-1-(3-phenylpropyl)-2-pyrrolidinon

$R_f$-Wert: 0,36 (Kieselgel; Methylenchlorid/Methanol/konz. wäßriges Ammoniak = 35:15:4)

(8) (3S,5S)-3-Carboxymethyl-5-[2-[[4-(3-guanidinocyclobutyl)phenyl]carbonylamino]ethyl]-1-(3-phenylpropyl)-2-pyrrolidinon

$R_f$-Wert: 0,27 (Kieselgel; Methylenchlorid/Methanol/konz. wäßriges Ammoniak = 35:15:4)

(9) (3S,5S)-3-Carboxymethyl-5-[[6-(guanidinomethyl)-2-naphthyl]oxymethyl]-1-(3-phenylpropyl)-2-pyrrolidinon

$R_f$-Wert: 0,28 (Kieselgel; Methylenchlorid/Methanol/konz. wäßriges Ammoniak = 16:8:1)

(10) (3S,5S)-3-Carboxymethyl-5-[[4'-(guanidinomethyl)-4-biphenylyl]oxymethyl]-1-(3-phenylpropyl)-2-pyrrolidinon

$R_f$-Wert: 0,13 (Kieselgel; Methylenchlorid/Methanol/konz. wäßriges Ammoniak = 16:4:1)

(11) (3S,5S)-3-Carboxymethyl-5-[[3-(guanidinomethyl)phenyl]carbonylaminomethyl]-1-(3-phenylpropyl)-2-pyrrolidinon-hydrat Schmelzpunkt: sintert ab 120°C

$R_f$-Wert: 0,30 (Kieselgel; Methylenchlorid/Methanol/konz. wäßriges Ammoniak = 8:4:1)

| Ber.: | C 62,10 | H 6,88 | N 14,48 |
|-------|---------|--------|---------|
| Gef.: | 62,47 | 6,90 | 14,60 |

(12) (3S,5S)-3-Carboxymethyl-5-[[4-(2-guanidinoethyl)phenyl]carbonylaminomethyl]-1-(3-phenylpropyl)-2-pyrrolidinon

$R_f$-Wert: 0,64 (Kieselgel; n-Butanol/Eisessig/Wasser = 4:1:1)

(13)   (3S,5S)-3-Carboxymethyl-5-[[4-(guanidinomethyl)phenyl]carbonylaminomethyl]-1-(3-phenylpropyl)-2-pyrrolidinon

Schmelzpunkt: 250-260°C
$R_f$-Wert: 0,54 (Kieselgel; n-Butanol/Eisessig/Wasser = 4:1:1)

| Ber.: | C 64,50 | H 6,71 | N 15,04 |
|-------|---------|--------|---------|
| Gef.: | 64,44 | 6,95 | 15,06 |

(14)   (3S,5S)-3-Carboxymethyl-5-[[3-(2-guanidinoethyl)phenyl]carbonylaminomethyl]-1-(3-phenylpropyl)-2-pyrrolidinon

$R_f$-Wert: 0,14 (Kieselgel; Methylenchlorid/Methanol/konz. wäßriges Ammoniak = 20:5:1)

(15)   (3S,5S)-3-Carboxymethyl-5-[[(2-guanidino-5-indanyl)methylcarbonyl]aminomethyl]-1-(3-phenylpropyl)-2-pyrrolidinon

$R_f$-Wert: 0,64 (Kieselgel; n-Butanol/Eisessig/Wasser = 4:1:1)
Massenspektrum: $(M + H)^+ = 506$

(16) (3S,5S)-3-Carboxymethyl-5-[[4-(3-guanidinopropyl)phenyl]oxymethyl]-1-(3-phenylpropyl)-2-pyrrolidinon-semihydrat

$R_f$-Wert: 0,58 (Kieselgel; n-Butanol/Eisessig/Wasser = 4:1:1)

| Ber.: | C 65,66 | H 7,42 | N 11,78 |
|-------|---------|--------|---------|
| Gef.: | 65,69 | 7,39 | 11,69 |

Massenspektrum: $(M + H)^+ = 467$

(17) (3S,5S)-3-Carboxymethyl-5-[[4-(guanidinomethyl)phenyl]oxymethyl]-1-(3-phenylpropyl)-2-pyrrolidinon

$R_f$-Wert: 0,49 (Kieselgel; n-Butanol/Eisessig/Wasser = 4:1:1)
Massenspektrum: $(M + H)^+ = 439$

(18) (3S,5S)-3-Carboxymethyl-5-[[3-(guanidinomethyl)phenyl]oxymethyl]-1-(3-phenylpropyl)-2-pyrrolidinon

$R_f$-Wert: 0,54 (Kieselgel; n-Butanol/Eisessig/Wasser = 4:1:1)
Massenspektrum: $(M + H)^+ = 439$

(19) (3S,5S)-3-Carboxymethyl-5-[[3-(4-guanidinobutyl)phenyl]oxymethyl]-1-(3-phenylpropyl)-2-pyrrolidinon

$R_f$-Wert: 0,59 (Kieselgel; n-Butanol/Eisessig/Wasser = 4:1:1)
Massenspektrum: $(M + H)^+ = 481$

(20)   (3S,5S)-3-Carboxymethyl-5-[[3-(2-guanidinoethyl)phenyl]oxymethyl]-1-(3-phenylpropyl)-2-pyrrolidinon-semihydrat

$R_f$-Wert: 0,57 (Kieselgel; n-Butanol/Eisessig/Wasser = 4:1:1)

| Ber.: | C 65,05 | H 6,99 | N 12,13 |
|-------|---------|--------|---------|
| Gef.: | 64,81 | 7,17 | 11,92 |

Massenspektrum: $(M + H)^+ = 453$

(21) (3S,5S)-3-Carboxymethyl-5-[[4-(2-guanidinoethyl)phenyl]oxymethyl]-1-(3-phenylpropyl)-2-pyrrolidinon

$R_f$-Wert: 0,51 (Kieselgel; n-Butanol/Eisessig/Wasser = 4:1:1)
Massenspektrum: $(M + H)^+ = 453$

(22) (3S,5S)-3-Carboxymethyl-5-[[4-(2-guanidinoethyl)phenyl]oxymethyl]-1-isobutyl-2-pyrrolidinon

$R_f$-Wert: 0,31 (Kieselgel; Methanol)
Massenspektrum: $(M - H)^- = 389$

(23) (3S,5S)-3-Carboxymethyl-5-[[4-(4-guanidinobutyl)phenyl]oxymethyl]-1-(3-phenylpropyl)-2-pyrrolidinon

$R_f$-Wert: 0,61 (Kieselgel; n-Butanol/Eisessig/Wasser = 4:1:1)
Massenspektrum: $(M + H)^+ = 481$

(24) (3S,5S)-3-Carboxymethyl-5-[[4-(trans-4-guanidinocyclohexyl)phenyl]oxymethyl]-1-(3-phenylpropyl)-2-pyrrolidinon

Schmelzpunkt: sintert ab 130°C
$R_f$-Wert: 0,14 (Kieselgel; Methylenchlorid/Methanol/konz. wäßriges Ammoniak = 4:1:0,25)

(25) (3S,5S)-3-Carboxymethyl-5-[[3-[3-(guanidinomethyl)phenyl]propyl]carbonylaminomethyl]-1-(3-phenylpropyl)-2-pyrrolidinon-semihydrat

| Ber.: | C 65,09 | H 7,41 | N 13,56 |
|-------|---------|--------|---------|
| Gef.: | 64,90 | 7,95 | 13,32 |

(26) (3S,5S)-3-Carboxymethyl-5-[(2-guanidinomethyl-5-indanyl)oxymethyl]-1-(3-phenylpropyl)-2-pyrrolidinonhydrat
Die Reaktion wird ohne Dimethylformamidzusatz durchgeführt, die Reaktionsdauer beträgt einen Tag.

| Ber.: | C 65,30 | H 7,31 | N 11,29 |
|-------|---------|--------|---------|
| Gef.: | 65,50 | 7,20 | 10,93 |

(27) (3S,5S)-3-Carboxymethyl-5-[[3-[4-(guanidinomethyl)phenyl]propyl]carbonylaminomethyl]-1-(3-phenylpropyl)-2-pyrrolidinon

| Ber.: | C 66,25 | H 7,35 | N 13,80 |
|-------|---------|--------|---------|
| Gef.: | 65,99 | 7,59 | 13,60 |

(29) (3S,5S)-3-Carboxymethyl-5-[(2-guanidinomethyl-5-indanyl)sulfonylaminomethyl]-1-(3-phenylpropyl)-2-pyrrolidinon

$R_f$-Wert: 0,27 (Kieselgel; Methanol)

| Ber.: | C 59,87 | H 6,51 | N 12,93 | S 5,92 |
|-------|---------|--------|---------|--------|
| Gef.: | 59,76 | 6,47 | 12,50 | 5,86 |

(30) (3S,5S)-3-Carboxymethyl-5-[(2-guanidino-5-indanyl)sulfonylaminomethyl]-1-(3-phenylpropyl)-2-pyrrolidinon

$R_f$-Wert: 0,63 (Kieselgel; Methanol)

| Ber.: | C 57,23 | H 6,47 | N 12,84 | S 5,88 |
|-------|---------|--------|---------|--------|
| Gef.: | 57,45 | 6,49 | 13,00 | 5,93 |

(31) (3S,5S)-3-Carboxymethyl-5-[(2-guanidino-5-indanyl)carbonylaminomethyl]-1-(3-phenylpropyl)-2-pyrrolidinon-hydrat

$R_f$-Wert: 0,15 (Kieselgel; Methylenchlorid/Cyclohexan/Methanol/konz. wäßriges Ammoniak = 34:7,5:57,5:1)

| Ber.: | C 63,63 | H 6,92 | N 13,74 |
|-------|---------|--------|---------|
| Gef.: | 63,88 | 6,74 | 13,77 |

(32) (3S,5S)-3-Carboxymethyl-5-[(2-guanidinomethyl-5-indanyl)carbonylaminomethyl]-1-(3-phenylpropyl)-2-pyrrolidinondihydrat

$R_f$-Wert: 0,40 (Kieselgel; Methanol/konz. wäßriges Ammoniak = 1:0,02)

| Ber.: | C 62,09 | H 7,26 | N 12,93 |
|-------|---------|--------|---------|
| Gef.: | 62,38 | 7,11 | 12,91 |

(33) (3S,5S)-3-Carboxymethyl-5-[[(2-guanidinomethyl-5-indanyl)methyl]carbonyl]aminomethyl]-1-(3-phenylpropyl)-2-pyrrolidinon-hydrat

$R_f$-Wert: 0,54 (Kieselgel; Methanol/konz. wäßriges Ammoniak = 1:0,02)

| Ber.: | C 64,78 | H 7,31 | N 13,03 |
|-------|---------|--------|---------|
| Gef.: | 64,65 | 7,31 | 13,37 |

(34) (3S,5S)-3-Carboxymethyl-5-[[3-(3-guanidinopropyl)phenyl]oxymethyl]-1-(3-phenylpropyl)-2-pyrrolidinon-hydrat

$R_f$-Wert: 0,52 (Kieselgel; Methanol/Wasser = 9:1)

| Ber.: | C 64,44 | H 7,49 | N 11,56 |
|-------|---------|--------|---------|
| Gef.: | 64,61 | 7,25 | 11,44 |

(35) (3S,5S)-3-Carboxymethyl-5-[[4-(5-guanidinopentyl)phenyl]oxymethyl]-1-(3-phenylpropyl)-2-pyrrolidinon

$R_f$-Wert: 0,53 (Kieselgel; Methanol/Wasser = 9:1)

(36) (3R,S;5S,R)-3-Carboxymethyl-5-[[4-(guanidinomethyl)phenyl]oxymethyl]-3-methyl-1-(3-phenylpropyl)-2-pyrrolidinonsemihydrat

$R_f$-Wert: 0,44 (Kieselgel; Methanol)

| Ber.: | C 65,05 | H 7,21 | N 12,14 |
|-------|---------|--------|---------|
| Gef.: | 64,85 | 7,41 | 12,18 |

(37) (3R,S;5S,R)-3-Carboxymethyl-5-[[4-(2-guanidinoethyl)phenyl]oxymethyl]-3-methyl-1-(3-phenylpropyl)-2-pyrrolidinon-dihydrat

Schmelzpunkt: 119-121°C
$R_f$-Wert: 0,41 (Kieselgel; Methanol)

| Ber.: | C 62,13 | H 7,62 | N 11,15 |
|-------|---------|--------|---------|
| Gef.: | 61,98 | 7,62 | 10,90 |

(38) (3S,5S)-3-Carboxymethyl-5-[[2-[4-(guanidinomethyl)phenyl]ethyl]carbonylaminomethyl]-1-(3-phenylpropyl)-2-pyrrolidinon-hydrat

| Ber.: | C 63,38 | H 7,29 | N 13,69 |
|-------|---------|--------|---------|
| Gef.: | 63,49 | 7,33 | 13,49 |

(39) (3S,5S)-3-Carboxymethyl-5-[[4-(cis-4-guanidinocyclohexyl)benzoyl]aminomethyl]-1-(3-phenylpropyl)-2-pyrrolidinon

$R_f$-Wert: 0,20 (Kieselgel; Methylenchlorid/Methanol/konz. wäßriges Ammoniak = 4:1:0,25)

(40) (3S,5S)-3-Carboxymethyl-5-[[4-(trans-4-guanidinocyclohexyl)benzoyl]aminomethyl]-1-(3-phenylpropyl)-2-pyrrolidinon

$R_f$-Wert: 0,20 (Kieselgel; Methylenchlorid/Methanol/konz. wäßriges Ammoniak = 4:1:0,25)

(41) (3S,5S)-3-Carboxymethyl-5-[[4-[cis-4-(guanidinomethyl)cyclohexyl]phenyl]oxymethyl]-1-(3-phenylpropyl)-2-pyrrolidinon

$R_f$-Wert: 0,16 (Kieselgel; Methylenchlorid/Methanol/konz. wäßriges Ammoniak = 4:1:0,25)

(42) (3S,5S)-3-Carboxymethyl-5-[[[2-[(4-guanidinobutyl)oxy]phenyl]carbonylamino]methyl]-2-pyrrolidinon

(43) (3S,5S)-3-Carboxymethyl-5-[[[3-[(3-guanidinopropyl)carbonylamino]phenyl]carbonylamino]methyl]-2-pyrrolidinon

## Beispiel 20

### (3S,5S)-5-[2-[(6-Amino-5,6,7,8-tetrahydro-2-naphtylcarbonyl)amino]ethyl]-3-carboxymethyl-1-(3-phenylpropyl)-2-pyrrolidinon-semihydrat

Zu einer Lösung von 2,1 g (3S,5S)-5-[2-[(6-tert.Butyloxycarbonylamino-5,6,7,8-tetrahydro-2-naphtylcarbonyl)amino]ethyl]-3-carboxymethyl-1-(3-phenylpropyl)-2-pyrrolidinon in 7 ml Methylenchlorid wird unter Rühren bei Raumtemperatur eine Mischung von 7 ml Trifluoressigsäure und 7 ml Methylenchlorid zugetropft. Nach 30 Minuten Rühren bei Raumtemperatur wird das Reaktionsgemisch eingeengt, mit Methylenchlorid und mit methanolischem Ammoniak versetzt. Die erhaltene Lösung wird dann über Kieselgel mit Methylenchlorid/Methanol/konz. wäßriges Ammoniak (70:30:2) chromatographiert. Nach Einengen des Eluats wird das erhaltene Rohprodukt mit tert.Butyl-methylether verrieben, abgesaugt, mit tert.Butyl-methylether gewaschen und getrocknet.

Ausbeute: 1,5 g (84 % der Theorie),
Schmelzpunkt: 138-145°C
$R_f$-Wert: 0,12 (Kieselgel; Methylenchlorid/Methanol/konz. wäßriges Ammoniak = 70:30:2)

| Ber.: | C 69,11 | H 7,46 | N 8,64 |
|---|---|---|---|
| Gef.: | 68,92 | 7,48 | 8,60 |

Analog werden erhalten:

(1) (3S,5S)-5-[[4-[(3-Aminopropyl)carbonylamino]phenyl]oxymethyl]-3-carboxymethyl-1-(3-phenylpropyl)-2-pyrrolidinon

Schmelzpunkt: 180-182°C
$R_f$-Wert: 0,27 (Kieselgel; Methylenchlorid/Methanol/konz. wäßriges Ammoniak = 40:4:1)

| Ber.: | C 66,79 | H 7,11 | N 8,99 |
|---|---|---|---|
| Gef.: | 66,55 | 7,04 | 8,73 |

(2) (3S,5S)-5-[[4-(cis-4-Aminocyclohexyl)phenyl]oxymethyl]-3-carboxymethyl-1-(3-phenylpropyl)-2-pyrrolidinon

Schmelzpunkt: 238-245°C

(3) (3S,5S)-5-[(6-Amino-5,6,7,8-tetrahydro-2-naphtylcarbonyl)aminomethyl]-3-carboxymethyl-1-(3-phenylpropyl)-2-pyrrolidinon-semihydrat

Schmelzpunkt: 147-156°C (sintert ab 143°C)
$R_f$-Wert: 0,08 (Kieselgel; Methylenchlorid/Methanol/konz. wäßriges Ammoniak = 70:30:2)

| Ber.: | C 68,62 | H 7,25 | N 8,89 |
|---|---|---|---|
| Gef.: | 68,71 | 7,30 | 8,89 |

(7) (3S,5S)-5-[[4-(4-Aminobutyl)phenyl]oxymethyl]-1-benzyl-3-carboxymethyl-2-pyrrolidinon

$R_f$-Wert: 0,53 (Kieselgel; Methylenchlorid/Methanol/konz. wäßriges Ammoniak = 30:10:3)

(8) (3S,5S)-5-[[4-(4-Aminobutyl)phenyl]oxymethyl]-3-carboxymethyl-1-(4-phenoxybutyl)-2-pyrrolidinon

$R_f$-Wert: 0,19 (Kieselgel; Methylenchlorid/Methanol/konz. wäßriges Ammoniak = 16:4:1)

(9) (3S,5S)-5-[[4-(4-Aminobutyl)phenyl]oxymethyl]-3-carboxymethyl-1-(2-phenylethyl)-2-pyrrolidinon

$R_f$-Wert: 0,26 (Kieselgel; Methylenchlorid/Methanol/konz. wäßriges Ammoniak = 30:10:3)

(10) (3S,5S)-5-[[4-(2-Aminoethyl)phenyl]carbonylaminomethyl]-3-carboxymethyl-1-(3-phenylpropyl)-2-pyrrolidinon

$R_f$-Wert: 0,38 (Kieselgel; n-Butanol/Eisessig/Wasser = 4:1:1)

(11) (3S,5S)-5-[[3-(2-Aminoethyl)phenyl]carbonylaminomethyl]-3-carboxymethyl-1-(3-phenylpropyl)-2-pyrrolidinon

$R_f$-Wert: 0,39 (Kieselgel; n-Butanol/Eisessig/Wasser = 4:1:1)

(12) (3S,5S)-5-[[(2-Amino-5-indanyl)methylcarbonyl]aminomethyl]-3-carboxymethyl-1-(3-phenylpropyl)-2-pyrrolidinon

$R_f$-Wert: 0,61 (Kieselgel; n-Butanol/Eisessig/Wasser = 4:1:1)

(13) (3S,5S)-5-[[4-(Aminomethyl)phenyl]oxymethyl]-3-carboxymethyl-1-(3-phenylpropyl)-2-pyrrolidinon

$R_f$-Wert: 0,45 (Kieselgel; n-Butanol/Eisessig/Wasser = 4:1:1)
Massenspektrum: M+ = 396

(14) (3S,5S)-5-[[3-(Aminomethyl)phenyl]oxymethyl]-3-carboxymethyl-1-(3-phenylpropyl)-2-pyrrolidinon

$R_f$-Wert: 0,55 (Kieselgel; n-Butanol/Eisessig/Wasser = 4:1:1)
Massenspektrum: M+ = 396

(15) (3S,5S)-5-[[3-(4-Aminobutyl)phenyl]oxymethyl]-3-carboxymethyl-1-(3-phenylpropyl)-2-pyrrolidinon

$R_f$-Wert: 0,58 (Kieselgel; n-Butanol/Eisessig/Wasser = 4:1:1)

(16) (3S,5S)-5-[[3-(2-Aminoethyl)phenyl)oxymethyl]-3-carboxymethyl-1-(3-phenylpropyl)-2-pyrrolidinon

$R_f$-Wert: 0,50 (Kieselgel; n-Butanol/Eisessig/Wasser = 4:1:1)
Massenspektrum: (M + H)+ = 411

(17) (3S,5S)-5-[[4-(2-Aminoethyl)phenyl]oxymethyl]-3-carboxymethyl-1-(3-phenylpropyl)-2-pyrrolidinon

$R_f$-Wert: 0,49 (Kieselgel; n-Butanol/Eisessig/Wasser = 4:1:1)
Massenspektrum: (M + H)+ = 411

(18) (3S,5S)-5-[[4-(2-Aminoethyl)phenyl]oxymethyl]-3-carboxymethyl-1-isobutyl-2-pyrrolidinon
Abspaltung in Ameisensäure

$R_f$-Wert: 0,28 (Kieselgel; Methanol)

(19) (3S,5S)-5-[[4-(trans-4-Aminocyclohexyl)phenyl]oxymethyl]-3-carboxymethyl-1-(3-phenylpropyl)-2-pyrrolidinon

Schmelzpunkt: 230-240°C

(20) (3S,5S)-5-[[4-(4-Aminobutyl)phenyl]oxymethyl]-1-[3-(4-benzyloxyphenyl)propyl]-3-carboxymethyl-2-pyrrolidinon

Schmelzpunkt: 190-195°C

(21) (3S,5S)-5-[2-[(2-Aminomethyl-5-indanyl)carbonylamino]ethyl]-3-carboxymethyl-1-(3-phenylpropyl)-2-pyrrolidinon

$R_f$-Wert: 0,43 (Kieselgel; Methylenchlorid/Methanol = 9:1) (nach zweimaliger Entwicklung)

(22) (3S,5S)-5-[(2-Amino-5-indanyl)carbonylaminomethyl]-3-carboxymethyl-1-(3-phenylpropyl)-2-pyrrolidinon x 1 $CH_3COOH$ x 1 $H_2O$

$R_f$-Wert: 0,25 (Kieselgel; Methylenchlorid/Cyclohexan/Methanol/konz. wäßriges Ammoniak = 34:7,5:57,5:1)

| Ber.: | C 63,74 | H 7,07 | N 7,96 |
|---|---|---|---|
| Gef.: | 63,83 | 7,29 | 8,16 |

(23) (3S,5S)-5-[(2-Aminomethyl-5-indanyl)carbonylaminomethyl]-3-carboxymethyl-1-(3-phenylpropyl)-2-pyrrolidinonhydrat

$R_f$-Wert: 0,38 (Kieselgel; Methanol/konz. wäßriges Ammoniak = 1:0,02)

| Ber.: | C 67,34 | H 7,33 | N 8,73 |
|---|---|---|---|
| Gef.: | 67,25 | 7,33 | 8,51 |

(24) (3S,5S)-5-[[(2-Aminomethyl-5-indanyl)methylcarbonyl]aminomethyl]-3-carboxymethyl-1-(3-phenylpropyl)-2-pyrrolidinon x 2,5 $CF_3COOH$ x 1 $H_2O$

$R_f$-Wert: 0,50 (Kieselgel; Methanol/konz. wäßriges Ammoniak = 1:0,02)

| Ber.: | C 55,80 | H 5,78 | N 6,30 |
|---|---|---|---|
| Gef.: | 56,09 | 6,01 | 6,59 |

(25) (3S,5S)-5-[[3-(3-Aminopropyl)phenyl]oxymethyl]-3-carboxymethyl-1-(3-phenylpropyl)-2-pyrrolidinon-hydrat

$R_f$-Wert: 0,32 (Kieselgel; Methanol)

| Ber.: | C 67,85 | H 7,74 | N 6,33 |
|---|---|---|---|
| Gef.: | 67,80 | 8,05 | 5,80 |

(26) (3S,5S)-5-[[4-(5-Aminopentyl)phenyl]oxymethyl]-3-carboxymethyl-1-(3-phenylpropyl)-2-pyrrolidinon x 1 $CF_3COOH$ x 1 $CF_3COONH_4$

$R_f$-Wert: 0,22 (Kieselgel; Methylenchlorid/Methanol = 5:1)

| Ber.: | C 53,33 | H 5,88 | N 6,02 |
|---|---|---|---|
| Gef.: | 53,52 | 5,97 | 5,58 |

Bei der Säulenchromatographie mit Methylenchlorid, Methanol = 40:1 entsteht in größeren Mengen (3S,5S)-5-[[4-(5-Aminopentyl)phenyl]oxymethyl]-3-[(methoxycarbonyl)methyl]-1-(3-phenylpropyl)-2-pyrrolidinon

$R_f$-Wert: 0,43 (Kieselgel; Methylenchlorid/Methanol = 5:1)

(27) (3R,S;5S,R)-5-[[4-(Aminomethyl)phenyl]oxymethyl]-3-carboxymethyl-3-methyl-1-(3-phenylpropyl)-2-pyrrolidinon-dihydrat

$R_f$-Wert: 0,41 (Kieselgel; Methanol)

| Ber.: | C 64,55 | H 7,67 | N 6,27 |
|-------|---------|--------|--------|
| Gef.: | 64,72 | 7,60 | 5,95 |

(28)  (3R,S;5S,R)-5-[[4-(2-Aminoethyl)phenyl]oxymethyl]-3-carboxymethyl-3-methyl-1-(3-phenylpropyl)-2-pyrrolidinonsemihydrat

$R_f$-Wert: 0,46 (Kieselgel; Methanol)
Schmelzpunkt: 225-227°C

| Ber.: | C 69,20 | H 7,61 | N 6,45 |
|-------|---------|--------|--------|
| Gef.: | 69,56 | 7,22 | 6,48 |

(29)  (3R,S;5S,R)-5-[[4-(4-Aminobutyl)phenyl]oxymethyl]-3-carboxymethyl-3-methyl-1-(3-phenylpropyl)-2-pyrrolidinonhydrat

$R_f$-Wert: 0,40 (Kieselgel; Methanol)

| Ber.: | C 68,91 | H 8,14 | N 5,95 |
|-------|---------|--------|--------|
| Gef.: | 68,60 | 8,22 | 5,48 |

(30)  (3S,5S)-5-[[4-(cis-4-Aminocyclohexyl)benzoyl]aminomethyl]-3-[(methoxycarbonyl)methyl]-1-(3-phenylpropyl)-2-pyrrolidinon

$R_f$-Wert: 0,26 (Kieselgel; Methylenchlorid/Methanol/konz. wäßriges Ammoniak = 8:1:0,25)

(31)  (3S,5S)-5-[[4-(trans-4-Aminocyclohexyl)benzoyl]aminomethyl]-3-[(methoxycarbonyl)methyl]-1-(3-phenylpropyl)-2-pyrrolidinon

$R_f$-Wert: 0,21 (Kieselgel; Methylenchlorid/Methanol/konz. wäßriges Ammoniak = 8:1:0,25)

(32)  (3S,5S)-5-[[4-(cis-4-Aminomethylcyclohexyl)benzoyl]aminomethyl]-3-[(methoxycarbonyl)methyl]-1-(3-phenylpropyl)-2-pyrrolidinon

$R_f$-Wert: 0,36 (Kieselgel; Methylenchlorid/Methanol/konz. wäßriges Ammoniak = 10:1:0,20)

(33)  (3S,5S)-5-[[4-(trans-4-Aminomethylcyclohexyl)benzoyl]aminomethyl]-3-[(methoxycarbonyl)methyl]-1-(3-phenylpropyl)-2-pyrrolidinon

$R_f$-Wert: 0,33 (Kieselgel; Methylenchlorid/Methanol/konz. wäßriges Ammoniak = 10:1:0,20)

(34) (3S,5S)-5-[(4-Aminocinnamoyl)aminomethyl]-3-[(methoxycarbonyl)methyl]-1-(3-phenylpropyl)-2-pyrrolidinon

$R_f$-Wert: 0,46 (Kieselgel; Methylenchlorid/Methanol = 15:1)

(35) (3S,5S)-5-[[[2-[(4-Aminobutyl)oxy]phenyl]carbonylamino]methyl]-3-carboxymethyl-2-pyrrolidinon

(36) (3S,5S)-5-[[[3-[(3-Aminopropyl)carbonylamino]phenyl]carbonylamino]methyl]-3-carboxymethyl-2-pyrrolidinon

(37) (3S,5S)-5-[[4'-(2-Aminoethyl)-4-biphenylyl]oxymethyl]-3-carboxymethyl-2-pyrrolidinon

(38) (3S,5S)-5-[2-[(6-Amino-2-trans-decalinyl)carbonylamino]ethyl]-3-carboxymethyl-2-pyrrolidinon

(39) (3S,5S)-5-[2-[(9-Amino-3-spiro[5,5]undecanyl)carbonylamino]ethyl]-3-carboxymethyl-2-pyrrolidinon

Beispiel 21

(3S,5S)-5-[[3-(3-Aminophenyl)propyl]carbonylaminomethyl]-3-carboxymethyl-1-(3-phenylpropyl)-2-pyrrolidinon x 1,25 HCl x 0,5 $H_2O$

1,9 g (3S,5S)-5-[3-[3-(tert.Butyloxycarbonylamino)phenyl]propyl]carbonylaminomethyl]-3-carboxymethyl-1-(3-phenylpropyl)-2-pyrrolidinon werden in 20 ml Dioxan gelöst und mit 20 ml etherischer Salzsäure versetzt. Man läßt eine Stunde bei Raumtemperatur stehen und filtriert das ausgefallene Festprodukt ab.

Ausbeute: 1,8 g (99 % der Theorie),
$R_f$-Wert: 0,35 (Kieselgel; Methylenchlorid/Methanol/konz. wäßriges Ammoniak = 8:2:0,2)

| Ber.: | C 61,66 | H 6,97 | N 8,30 | Cl 8,76 |
|---|---|---|---|---|
| Gef.: | 61,51 | 7,11 | 7,94 | 8,52 |

Analog werden erhalten:

(1)    (3S,5S)-5-[[2-[4-(Aminomethyl)phenyl]ethyl]carbonylaminomethyl]-3-carboxymethyl-1-(3-phenylpropyl)-2-pyrrolidinon x 1,2 HCl x 0,5 $H_2O$

| Ber.: | C 61,93 | H 7,04 | N 8,33 | Cl 8,43 |
|---|---|---|---|---|
| Gef.: | 62,16 | 6,78 | 7,97 | 8,31 |

(2)  (3S;5S)-5-[[2-[3-(Aminophenyl)ethyl]carbonylaminomethyl]-3-carboxymethyl-1-(3-phenylpropyl)-2-pyrrolidinon-hydrochlorid

$R_f$-Wert: 0,60 (Kieselgel; Methylenchlorid/Methanol/Essigsäure = 4:1:0,1)

(3)  (3S,5S)-5-[[2-(4-Aminophenyl)ethyl]carbonylaminomethyl]-3-carboxymethyl-1-(3-phenylpropyl)-2-pyrrolidinon x 1,75 HCl x 1,5 $H_2O$

$R_f$-Wert: 0,48 (Kieselgel; Methylenchlorid/Methanol = 5:1)

| Ber.: | C 56,82 | H 6,82 | N 7,95 | Cl 11,74 |
|---|---|---|---|---|
| Gef.: | 56,70 | 6,67 | 7,66 | 11,57 |

(4)    (3S,5S)-5-[[3-(4-Aminophenyl)propyl]carbonylaminomethyl)-3-carboxymethyl-1-(3-phenylpropyl)-2-pyrrolidinon-hydrochlorid

$R_f$-Wert: 0,29 (Kieselgel; Methylenchlorid/Methanol = 9:1)

Beispiel 22

(3S,5S)-5-[(4'-Amidino-4-biphenylyl)oxymethyl]-3-[(isopropyloxycarbonyl)methyl]-1-(3-phenylpropyl)-2-pyrrolidinon-hydrochlorid

2   g   (3S,5S)-5-[(4'-Amidino-4-biphenylyl)oxymethyl]-3-[(methoxycarbonyl)methyl]-1-(3-phenylpropyl)-2-pyrrolidinon-hydrochlorid werden in 300 ml gesättigter isopropanolischer Salzsäure suspendiert und unter Rühren 6 Stunden bei 50-60°C gerührt. Man läßt noch 2 Tage bei Raumtemperatur stehen, destilliert das Lösungsmittel ab und reinigt über Kieselgel (Elutionsmittel: Methylenchlorid/Methanol/konz. wäßriges Ammoniak = 4:1:0,25).

Ausbeute: 1,4 g (67 % der Theorie),
$R_f$-Wert: 0,27 (Kieselgel; Methylenchlorid/Methanol/konz. wäßriges Ammoniak = 4:1:0,25)

Analog wird erhalten:

(1) (3S,5S)-5-[(4'-Amidino-4-biphenylyl)oxymethyl]-3-[(hexyloxycarbonyl)methyl]-1-(3-phenylpropyl)-2-pyrrolidinon-hydrochlorid

Lösungsmittel: eine Mischung aus 150 ml n-Hexanol und 100 ml ätherischer Salzsäure.
$R_f$-Wert: 0,64 (Kieselgel; Methylenchlorid/Methanol/konz. wäßriges Ammoniak = 4:1:0,25)

## Beispiel 23

(3S,5S)-5-[[4'-(N-Hydroxyamidino)-4-biphenylyl]oxymethyl]-3-[(methoxycarbonyl)methyl]-2-pyrrolidinon

Hergestellt aus der entsprechenden Amidinoverbindung mit Hydroxylamin-hydrochlorid/N-Ethyl-diisopropylamin.

Schmelzpunkt: 232°C (Zers.)

| Ber.: | C 63,46 | H 5,83 | N 10,57 |
|---|---|---|---|
| Gef.: | 63,20 | 5,81 | 10,40 |

## Beispiel 24

(3S,5S)-5-[(7-Amidino-9-hydroxy-2-fluorenyl)oxymethyl]-3-carboxymethyl-1-(3-phenylpropyl)-2-pyrrolidinon

0,77 g (3S,5S)-5-[(7-Amidino-9-keto-2-fluorenyl)oxymethyl]-3-carboxymethyl-1-(3-phenylpropyl)-2-pyrrolidinon werden in 15 ml Eisessig gelöst, mit 0,4 g 10-prozentiger Palladiumkohle versetzt und 24 Stunden bei Raumtemperatur und 3 bar Wasserstoffdruck hydriert. Man filtriert den Katalysator ab, dampft ein und reinigt chromatographisch über Kieselgel (Elutionsmittel: Methylenchlorid/Methanol/Eisessig = 3:1:0,1). Das erhaltene Rohprodukt wird mit Ether verrieben, abfiltriert und in 50 ml Eisessig-Wasser-Gemisch (1:2) aufgenommen. Diese Lösung wird bis zur beginnenden Kristallisation eingeengt (etwa 5 ml). Die ausgefallenen Kristalle werden mit Wasser, Aceton und Ether gewaschen.

Ausbeute: 0,35 g (45 % der Theorie),
Schmelzpunkt: 182-185°C (Zers.).

## Beispiel 25

(3S,5S)-5-[(7-Amidino-2-fluorenyl)oxymethyl]-3-carboxymethyl-1-(3-phenylpropyl)-2-pyrrolidinon

0,3 g (3S,5S)-5-[(7-Amidino-9-keto-2-fluorenyl)oxymethyl]-3-carboxymethyl-1-(3-phenylpropyl)-2-pyrrolidinon werden in 7 ml Eisessig gelöst, 0,05 g 10%ige Palladiumkohle zugesetzt und 6 Stunden bei 70°C und 3 bar Wasserstoffdruck hydriert. Man filtriert vom Katalysator ab, engt ein und reinigt das Produkt durch Chromatographie an Kieselgel (Elutionsmittel: Tetrahydroduran/2N Essigsäure = 10:1).

Ausbeute: 0,15 g (51 % der Theorie),
Schmelzpunkt: 182-200°C (Zers.)

## Beispiel 26

(3S,5S)-5-[(4'-Amidino-4-biphenylyl)oxymethyl]-3-[(benzyloxycarbonyl)methyl)-1-(3-phenylpropyl)-2-pyrrolidinonhydrochlorid

2,1 g (3S,5S)-5-[(4'-Amidino-4-biphenylyl)oxymethyl]-3-carboxymethyl-1-(3-phenylpropyl)-2-pyrrolidinon werden in einer Mischung aus 250 ml Benzylalkohol und 30 ml etherischer Salzsäure gelöst. Der Ether wird im Vakuum abdestilliert und die verbleibende Lösung 4 Stunden bei 50-60°C gerührt. Das Lösungsmittel wird im Vakuum abdestilliert und der Rückstand durch Chromatographie an Kieselgel (Elutionsmittel: Methylenchlorid/Methanol/konz. wäßriges Ammoniak = 4:1:0,25) gereinigt.

Ausbeute: 1,6 g (62 % der Theorie),
Schmelzpunkt: 170-180°C

Analog werden erhalten:

(1) (3S,5S)-5-[[4-(4-Aminobutyl)phenyl]oxymethyl]-3-[(ethoxycarbonyl)methyl]-1-(3-phenylpropyl)-2-pyrrolidinon-hydrochlorid
Durchführung mit Ethanol/Salzsäure

$R_f$-Wert: 0,63 (Kieselgel; Methylenchlorid/Ethanol/konz. wäßriges Ammoniak = 4:1:0,25)

(2) (3S,5S)-5-[(4'-Amidino-4-biphenylyl)oxymethyl]-3-[(isopropyloxycarbonyl)methyl]-2-pyrrolidinon-hydrochlorid-semihydrat
Durchführung mit Isopropanol/Salzsäure

$R_f$-Wert: 0,49 (reversed phase Kieselgel (RP8); Methanol/10 % wäßrige Kochsalzlösung = 6:4)

| Ber.: | C 60,72 | H 6,42 | N 9,24 | Cl 7,79 |
|-------|---------|--------|--------|---------|
| Gef.: | 61,08 | 6,42 | 9,06 | 7,90 |

(3) (3S,5S)-5-[(4'-Amidino-4-biphenylyl)oxymethyl]-3-[(benzyloxycarbonyl)methyl]-2-pyrrolidinon-p-toluolsulfonat
Durchführung mit Benzylalkohol/p-Toluolsulfonsäure

Schmelzpunkt: 182-184°C,
$R_f$-Wert: 0,28 (reversed phase Kieselgel (RP8); Methanol/10 % wäßrige Kochsalzlösung = 6:4)

| Ber.: | C 64,85 | H 5,80 | N 6,67 | S 5,09 |
|-------|---------|--------|--------|--------|
| Gef.: | 64,69 | 5,61 | 6,70 | 5,19 |

(4) (3S,5S)-5-[(4'-Amidino-4-biphenylyl)oxymethyl]-3-[(butyloxycarbonyl)methyl]-2-pyrrolidinon x 1,25 HCl Durchführung mit n-Butanol/Salzsäure

$R_f$-Wert: 0,37 (reversed phase Kieselgel (RP8); Methanol/10 % wäßrige Kochsalzlösung = 6:4)

| Ber.: | C 61,45 | H 6,50 | N 8,96 | Cl 9,45 |
|-------|---------|--------|--------|---------|
| Gef.: | 61,26 | 6,56 | 9,11 | 9,46 |

(5) (3S,5S)-5-[(4'-Amidino-4-biphenylyl)oxymethyl]-3-[[(3-phenylpropyl)oxycarbonyl]methyl]-2-pyrrolidinon
Durchführung mit 3-Phenylpropanol/Salzsäure, Isolierung der Base.

$R_f$-Wert: 0,17 (reversed phase Kieselgel (RP8); Methanol/10 % wäßrige Kochsalzlösung = 6:4)

| Ber.: | C 71,73 | H 6,44 | N 8,65 |
|-------|---------|--------|--------|
| Gef.: | 71,43 | 6,37 | 8,58 |

(6) (3S,5S)-5-[(4'-Amidino-4-biphenylyl)oxymethyl]-3-[[[2-(3,4-dimethoxyphenyl)ethyl)oxycarbonyl]methyl]-2-pyrrolidinon x 1,25 p-Toluolsulfonsäure

Durchführung mit 2-(3,4-Dimethoxyphenyl)ethanol/p-Toluolsulfonsäure.

Schmelzpunkt: 183-186°C
$R_f$-Wert: 0,21 (reversed phase Kieselgel (RP8); Methanol/10 % wäßrige Kochsalzlösung = 6:4)

| Ber. | C 62,32 | H 5,80 | N 5,63 | S 5,37 |
|------|---------|--------|--------|--------|
| Gef.: | 62,10 | 5,68 | 5,77 | 5,69 |

(7)    (3S,5S)-5-[[[2-[(4-Aminobutyl)oxy]phenyl]carbonylamino]methyl]-3-[(methoxycarbonyl)methyl]-2-pyrrolidinon-hydrochlorid

(8)    (3S,5S)-5-[[[2-[(4-Guanidinobutyl)oxy]phenyl]carbonylamino]methyl]-3-[(methoxycarbonyl)methyl]-2-pyrrolidinon-hydrochlorid

(9)    (3S,5S)-5-[[[3-[(3-Aminopropyl)carbonylamino]phenyl]carbonylamino]methyl]-3-[(methoxycarbonyl)methyl]-2-pyrrolidinon-hydrochlorid

(10)    (3S,5S)-5-[[[3-[(3-Guanidinopropyl)carbonylamino]phenyl]carbonylamino]methyl]-3-[(methoxycarbonyl)methyl]-2-pyrrolidinon-hydrochlorid

(11 (3S,5S)-5-[(4'-Guanidino-4-biphenylyl)oxymethyl]-3[(methoxycarbonyl)methyl]-2-pyrrolidinon-hydrochlorid

(12)    (3S,5S)-5-[[4'-(2-Aminoethyl)-4-biphenylyl]oxymethyl]-3-[(methoxycarbonyl)methyl]-2-pyrrolidinon-hydrochlorid

(13)    (3S,5S)-5-[2-[(6-Amino-2-trans-decalinyl)carbonylamino]ethyl]-3-[(methoxycarbonyl)methyl]-2-pyrrolidinon-hydrochlorid

(14)    (3S,5S)-5-[2-[(9-Amino-3-spiro[5,5]undecanyl)carbonylamino]ethyl]-3-[(methoxycarbonyl)methyl]-2-pyrrolidinon-hydrochlorid

(15)  (3S,5S)-5-[(4'-Aminomethyl-4-biphenylyl)oxymethyl]-3-[(methoxycarbonyl)methyl]-2-pyrrolidinon-hydrochlorid
Durchführng mit Methanol/Salzsäure.

Schmelzpunkt: 259-261°C

(16)    (3S,5S)-5-[(4'-Aminomethyl-2'-methyl-4-biphenylyl)oxymethyl]-3-[(methoxycarbonyl)methyl]-2-pyrrolidinon-hydrochlorid Durchführung mit Methanol/Salzsäure.

(17) (3S,5S)-5-[(4'-Aminomethyl-2,3-dimethyl-4-biphenylyl)oxymethyl]-3-[(methoxycarbonyl)methyl]-2-pyrrolidinon-hydrochlorid

Durchführung mit Methanol/Salzsäure.

(18)  (3S,5S)-5-[[4-(2-Guanidinoethyl)phenyl]oxymethyl]-3-[(methoxycarbonyl)methyl]-1-(3-phenylpropyl)-2-pyrrolidinon-hydrochlorid

$R_f$-Wert: 0,63 (Kieselgel; n-Butanol/Eisessig/Wasser = 4:1:1)

Beispiel 27

(3S,5S)-5-[[4-[(3-Amidinophenyl)sulfinyl]phenyl]oxymethyl]-3-(methoxycarbonyl)methyl]-1-(3-phenylpropyl)-2-pyrrolidinon

Zu 0,5 g (3S,5S)-5-[[4-[(3-Amidinophenyl)sulfenyl]phenyl]oxymethyl]-3-[(methoxycarbonyl)methyl]-1-(3-phenylpropyl)-2-pyrrolidinon-hydrochlorid in 30 ml Dichlormethan gibt man unter Rühren und bei -20°C 0,2 g m-Chlorperbenzoe-

EP 0 483 667 B1

säure. Anschließend läßt man über Nacht bei -20°C stehen, rührt in eine Natriumhydrogencarbonat-Lösung ein und extrahiert mit Dichlormethan. Nach Trocknen und Einengen im Vakuum reinigt man den Rückstand über eine Kieselgelsäule (Elutionsmittel: Dichlormethan/Methanol/konz. wäßriges Ammoniak = 4:1:0,25).

Ausbeute: 0,19 g (37 % der Theorie),
$R_f$-Wert: 0,30 (Kieselgel; Dichlormethan/Methanol/konz. wäßriges Ammoniak = 4:1:0,25)
Massenspektrum: (M + H)+ = 562

Analog werden erhalten:

(1) (3S,5S)-5-[(4'-Amidino-3-methylsulfinyl-4-biphenylyl)oxymethyl]-3-[(methoxycarbonyl)methyl]-1-(3-phenylpropyl)-2-pyrrolidinon

$R_f$-Wert: 0,22 (Kieselgel; Methylenchlorid/Cyclohexan/Methanol/konz. wäßriges Ammoniak = 7:1,5:1,5:0,2)

(2) (3S,5S)-5-[(4'-Amidino-4-biphenylyl)oxymethyl]-3-[(methoxycarbonyl)methyl]-1-[2-phenylsulfinyl)ethyl]-2-pyrrolidinon-hydrochlorid

(3) (3S,5S)-5-[(4'-Amidino-4-biphenylyl)oxymethyl]-3-[(methoxycarbonyl)methyl]-1-[[(thiomorpholin-S-oxid)-N-carbonyl]methyl]-2-pyrrolidinon-hydrochlorid

(4) (3S,5S)-5-[(4'-Amidino-4-biphenylyl)oxymethyl]-3-[(methoxycarbonyl)methyl]-1-[3-(methylsulfinyl)propyl]-2-pyrrolidinon

Beispiel 28

(3S,5S)-5-[[4-[(3-Amidinophenyl)hydroxymethyl]phenyl]oxymethyl]-3-carboxymethyl-1-(3-phenylpropyl)-2-pyrrolidinon

Zu 0,4 g (3S,5S)-5-[[4-[(3-Amidinophenyl)carbonyl]phenyl]oxymethyl]-3-[(methoxycarbonyl)methyl]-1-(3-phenylpropyl)-2-pyrrolidinon-hydrochlorid, gelöst in 30 ml Methanol und 3 ml Wasser, gibt man unter Rühren und bei Raumtemperatur 0,06 g Natriumborhydrid. Nach Ablauf von 3 Stunden wird mit 2 ml Aceton versetzt und nach weiteren 30 Minuten gibt man 3 ml 1N Natronlauge zu und läßt 2 Stunden bei Raumtemperatur stehen. Anschließend wird 1N Salzsäure bis zum Neutralpunkt zugegeben und unter Vakuum zur Trockne eingeengt. Der verbleibende Festkörper wird zweimal mit Wasser verrieben und abgesaugt. Der so erhaltene Festkörper wird mit Dioxan/Ethanol = 1:1 und anschließend mit Ether gewaschen und getrocknet.

Ausbeute: 0,23 g (56 % der Theorie),
$R_f$-Wert: 0,13 (Kieselgel; Dichlormethan/Äthanol/konz. wäßriges Ammoniak = 4:1:0,25)
Massenspektrum: (M + H)+ = 516

Beispiel 29

(3S,5S)-5-[(4'-Amidino-4-biphenylyl)oxymethyl]-3-[[[2-(2-oxopyrrolidinyl)ethyloxy]carbonyl]methyl]-2-pyrrolidinon-hydrochlorid

1 g (3S,5S)-5-[(4'-Amidino-4-biphenylyl)oxymethyl]-3-carboxymethyl-2-pyrrolidinon, 10 g 1-(2-Hydroxyethyl)-2-pyrrolidinon und 1,5 ml Trimethylchlorsilan werden 18 Stunden bei 40°C, 8 Stunden bei 50°C und 18 Stunden bei 65°C gerührt. Nach dem Abkühlen wird mit konzentiertem wäßrigem Ammoniak alkalisch gestellt und die Reaktionsmischung direkt durch Kieselgelchromatographie mit Methylenchlorid/Methanol/wäßriges Ammoniak (18:2:0,25) gereinigt. Das erhaltene Produkt wird mit Methylenchlorid/Methanol (9:1) gerührt, filtiert und das Filtrat eingeengt.

Ausbeute: 0,95 g
$R_f$-Wert: 0,54 (reversed phase Kieselgel RP-8; Methanol/5%ige wäßrige Kochsalzlösung = 3:2)
Massenspektrum: (M + H)+ = 479

Analog werden erhalten:

(1) (3S,5S)-5-[(4'-Amidino-4-biphenylyl)oxymethyl]-3-[[[(dimethylaminocarbonyl)methyloxy]carbonyl]methyl]-2-pyr-

161

rolidinon-hydrochlorid
Durchführung mit Glycolsäuredimethylamid

$R_f$-Wert: 0,61 (reversed phase Kieselgel RP-8; Methanol/5 %ige wäßrige Kochsalzlösung = 3:2)
Massenspektrum: (M + H)+ = 453

(2)   (3S,5S)-5-[(4'-Amidino-4-biphenylyl)oxymethyl]-3-[[[(diisopropylaminocarbonyl)methyloxy]carbonyl]methyl]-2-pyrrolidinon
Durchführung mit Glycolsäurediisopropylamid.

Beispiel 30

(3S,5S)-5-[(4'-Amidino-4-biphenylyl)oxymethyl]-3-[[[(3-pyridyl)methyloxy]carbonyl]methyl]-2-pyrrolidinon-methansulfonat

Zu 5,5 g 3-Hydroxymethylpyridin in 2 ml trockenem Dimethylformamid werden unter Eiskühlung 6 g Methansulfonsäure gegeben, dann 0,92 g (3S,5S)-5-[(4'-Amidino-4-biphenylyl)oxymethyl]-3-carboxymethyl-2-pyrrolidinon zugesetzt und 3 Tage bei 80°C gerührt. Nach dem Abkühlen wird mit 20 ml Methylenchlorid/Methanol/konz. wäßriges Ammoniak (18:2:0,25) versetzt, mit wäßrigem Ammoniak neutralisiert und direkt durch Kieselgelchromatographie mit Methylenchlorid/Methanol/konz. wäßriges Ammoniak (18:2:0,25) gereinigt. Das erhaltene Produkt wird mit Aceton verrieben, abgesaugt und getrocknet.

Ausbeute: 0,50 g
$R_f$-Wert: 0,58 (reversed phase Kieselgel RP-8; Methanol/5%ige wäßrige Kochsalzlösung = 3:2)
Massenspektrum: (M + H)+ = 459

Analog werden erhalten:

(1) (3S,5S)-5-[(4'-Amidino-4-biphenylyl)oxymethyl]-3[[(2-morpholinoethyl)oxycarbonyl]methyl]-2-pyrrolidinonhydrochlorid Durchführung mit N-(2-Hydroxyethyl)morpholin und Salzsäuregas

$R_f$-Wert: 0,55 (reversed phase Kieselgel RP-8; Methanol/5%ige wäßrige Kochsalzlösung = 3:2)
Massenspektrum: (M + H)+ = 481

Beispiel 31

(3S,5S)-5-[2-[N-(3'-Amino-3-biphenylyl)benzylamino]ethyl]-3-[(methoxycarbonyl)methyl-1-(3-phenylpropyl)-2-pyrrolidinon

3   g   (3S,5S)-5-[2-[N-(3'-Nitro-3-biphenylyl)benzylamino]ethyl]-3-[(methoxycarbonyl)methyl]-1-(3-phenylpropyl)-2-pyrrolidinon werden in einer Mischung aus 3,25 g Zinkstaub, 0,75 g Calciumchlorid, 3 ml Wasser und 30 ml Ethanol 6 Stunden unter Rückfluß erhitzt. Man filtriert heiß, wäscht mehrfach mit heißem Methanol nach und dampft das Filtrat ein. Der Rückstand wird durch Chromatographie an Kieselgel (Elutionsmittel: Methylenchlorid/Methanol/konz. wäßriges Ammoniak = 30:1:0,1) gereinigt.

Ausbeute: 1,0 (35 % der Theorie),
$R_f$-Wert: 0,66 (Kieselgel; Methylenchlorid/Methanol/wäßriges Ammoniak = 8:2:0,1)

Beispiel 32

(3S,5S)-5-[2-[(3'-Guanidino-3-biphenylyl)amino]ethyl]-3-[(methoxycarbonyl)methyl]-1-(3-phenylpropyl)-2-pyrrolidinon

1,3 g (3S,5S)-5-[2-[N-(3'-Guanidino-3-biphenylyl)benzylamino]ethyl]-3-[(methoxycarbonyl)methyl]-1-(3-phenylpropyl)-2-pyrro lidinon-dihydrochlorid werden in 30 ml Methanol in Gegenwart von 1,5 g Palladiumhydroxyd mit Wasserstoff von 5 bar Druck bei Raumtemperatur 48 Stunden hydriert. Nach Abfiltrieren der Festprodukte wird das Filtrat eingeengt und der Rückstand säulenchromatographisch über Kieselgel (Elutionsmittel: Methylenchlorid/Methanol/Eisessig = 9:1,5:0,1) gereinigt.

162

EP 0 483 667 B1

Ausbeute: 0,63 g (55 % der Theorie),
$R_f$-Wert: 0,43 (Kieselgel; Methylenchlorid/Methanol/Eisessig = 9;1,5:0,1)

Beispiel 33

(3S,5S)-5-[[4'-(N-Benzyloxycarbonylamidino)-4-biphenylyl]oxymethyl]-3-carboxymethyl-2-pyrrolidinon

Zu 360 mg (3S,5S)-5-[[4'-(N-Benzyloxycarbonylamidino)-4-biphenylyl]oxymethyl]-3-[(tert.butyloxycarbonyl)methyl]-2-pyrrolidinon in 1 ml Methylenchlorid werden 1 ml Trifluoressigsäure zugetropft. Nach 18 Stunden bei Raumtemperatur wird eingeengt, mit Eiswasser versetzt und mit Essigester extrahiert. Die organische Phase wird mit Wasser gewaschen, wobei ein Teil des Produkts ausfällt, getrocknet und einrotiert. Der Rückstand wird in Chloroform aufgenommen und zusammen mit dem beim Waschen ausgefallenen Produkt noch fünfmal mit Wasser gewaschen. Die organische Phase wird abgetrennt, eingeengt und der erhaltene Rückstand zusammen mit dem ausgefallenen Produkt mit wenig Aceton verrührt. Anschließend wird abgesaugt und getrocknet.

Ausbeute: 170 mg (53 % der Theorie),
Schmelzpunkt: 197-199°C
$R_f$-Wert: 0,35 (reversed phase Kieselgel RP 8; Methanol/10%ige wäßrige Kochsalzlösung = 6:4)

Beispiel 34

(3S,5S)-5-[[4'-(N-Ethyloxycarbonylamidino)-4-biphenylyl]oxymethyl]-3-[(methoxycarbonyl)methyl]-2-pyrrolidinon

Zu 1,0 g (3S,5S)-5-[(4'-Amidino-4-biphenylyl)oxymethyl]-3-[(methoxycarbonyl)methyl]-2-pyrrolidinon-hydrochlorid und 0,28 g Chlorameisensäure-ethylester in 100 ml Methylenchlorid werden unter kräftigem Rühren 50 ml 0,1 N Natronlauge langsam zugetropft. Danach wird die organische Phase abgetrennt, mit Wasser gewaschen, getrocknet und einrotiert. Der erhaltene Rückstand wird durch Chromatographie über Kieselgel mit Essigester gereinigt. Das erhaltene Produkt wird mit Essigester aufgekocht, abgekühlt, abgesaugt und getrocknet.

Ausbeute: 450 mg (43 % der Theorie),
Schmelzpunkt: 165-167°C
$R_f$-Wert: 0,29 (Kieselgel; Essigester)

Analog werden erhalten:

(1) (3S,5S)-5-[[4'-(N-Methoxycarbonylamidino)-4-biphenylyl]oxymethyl]-3-[(methoxycarbonyl)methyl]-2-pyrrolidinon

Schmelzpunkt: 183-184°C (Zers.)
$R_f$-Wert: 0,47 (Kieselgel; Essigester/Methanol = 97:3)

(2) (3S,5S)-3-[(Benzyloxycarbonyl)methyl]-5-[[4'-(N-methoxycarbonylamidino)-4-biphenylyl]oxymethyl]-2-pyrrolidinon

Schmelzpunkt: 203-204°C

| Ber.: | C 67,56 | H 5,67 | N 8,15 |
|-------|---------|--------|--------|
| Gef.: | 67,39 | 5,67 | 8,19 |

(3) (3S,5S)-5-[[4'-(N-Isobutoxycarbonylamidino)-4-biphenylyl]oxymethyl]-3-[(methoxycarbonyl)methyl]-2-pyrrolidinon Durchführung in Methylenchlorid/N-Ethyl-diisopropylamin

Schmelzpunkt: 161-163°C

| Ber.: | C 64,85 | H 6,49 | N 8,73 |
|-------|---------|--------|--------|
| Gef.: | 64,85 | 6,46 | 8,65 |

(4) (3S,5S)-5-[[4'-(N-Isopropoxycarbonylamidino)-4-biphenylyl]oxymethyl]-3-[(methoxycarbonyl)methyl]-2-pyrrolidinon

Schmelzpunkt: 170-172°C

| Ber.: | C 64,23 | H 6,25 | N 8,99 |
|-------|---------|--------|--------|
| Gef.: | 64,26 | 6,35 | 8,95 |

(5) (3S,5S)-5-[[4'-(N-Ethoxycarbonylamidino)-4-biphenylyl]oxymethyl]-3-[(ethoxycarbonyl)methyl]-2-pyrrolidinon

Schmelzpunkt: 138-140°C
$R_f$-Wert: 0,39 (Kieselgel; Essigester)

(6) (3S,5S)-3-[(Ethoxycarbonyl)methyl]-5-[[4'-(N-methoxycarbonylamidino)-4-biphenylyl]oxymethyl]-2-pyrrolidinon

Schmelzpunkt: 190-192°C

| Ber.: | C 63,56 | H 6,00 | N 9,27 |
|-------|---------|--------|--------|
| Gef.: | 63,29 | 6,05 | 9,27 |

(7)     (3S,5S)-5-[[3'-Fluor-4'-(N-methoxycarbonylamidino)-4-biphenylyl]oxymethyl]-3-[(methoxycarbonyl)methyl]-2-pyrrolidinon

Schmelzpunkt: 156-158°C

| Ber.: | C 60,39 | H 5,29 | N 9,19 |
|-------|---------|--------|--------|
| Gef.: | 60,10 | 5,38 | 8,98 |

(8)     (3S,5S)-1-Acetyl-5-[[4'-(N-methoxycarbonylamidino)-4-biphenylyl]oxymethyl]-3-[(methoxycarbonyl)methyl]-2-pyrrolidinon x 0,25 Wasser

$R_f$-Wert: 0,23 (Kieselgel; Essigester)

| Ber.: | C 63,61 | H 6,25 | N 8,90 |
|-------|---------|--------|--------|
| Gef.: | 63,61 | 6,30 | 8,77 |

(9)     (3S,5S)-5-[[3'-Chlor-4'-(N-methoxycarbonylamidino)-4-biphenylyl]oxymethyl]-3-[(methoxycarbonyl)methyl]-2-pyrrolidinon

$R_f$-Wert: 0,70 (Kieselgel; Methylenchlorid/Methanol = 4:1)
Massenspektrum: $(M + H)^+ = 474$ und 476

(10) (3S,5S)-5-[[4'-(N-Benzoylamidino)-4-biphenylyl]oxymethyl]-3-[(methoxycarbonyl)methyl]-2-pyrrolidinon

Schmelzpunkt: 179-181°C

$R_f$-Wert: 0,38 (Kieselgel; Essigester)

Massenspektrum: $(M + H)^+ = 486$

(11) (3S,5S)-3-[(Isopropoxycarbonyl)methyl]-5-[[4'-(N-methoxycarbonylamidino)-4-biphenylyl]oxymethyl]-2-pyrrolidinon

Schmelzpunkt: 191°C (Zers.)

| Ber.: | C 64,23 | H 6,25 | N 8,99 |
|-------|---------|--------|--------|
| Gef.: | 64,24 | 6,25 | 8,92 |

(12) (3S,5S)-3-[(Ethoxycarbonyl)methyl]-5-[[4'-(N-phenoxycarbonylamidino)-4-biphenylyl]oxymethyl]-2-pyrrolidinon

Massenspektrum: $(M + H)^+ = 516$

(13) (3S,5S)-5-[(4'-Methoxycarbonylaminomethyl-4-biphenylyl)oxymethyl]-3-[(methoxycarbonyl)methyl]-2-pyrrolidinon

(14) (3S,5S)-3-[(Benzyloxycarbonyl)methyl]-5-[[4'-(N-methoxycarbonylamidino)-4-biphenylyl]oxymethyl]-2-pyrrolidinon

Schmelzpunkt: 203-205°C (Zers.)

(15) (3S,5S)-3-[(n-Butoxycarbonyl)methyl]-5-[[4'-(N-methoxycarbonylamidino)-4-biphenylyl]oxymethyl]-2-pyrrolidinon

Schmelzpunkt: 175-178°C (Zers.)

Beispiel 35

(3S,5S)-3-Carboxymethyl-5-[[4'-(N-hydroxyamidino)-4-biphenylyl)oxymethyl]-2-pyrrolidinon

450 mg (3S,5S)-5-[[4'-(N-Hydroxyamidino)-4-biphenylyl)oxymethyl]-3-[(methoxycarbonyl)methyl]-2-pyrrolidinon, 10 ml Methanol und 0,42 ml 4N Natronlauge werden 18 Stunden bei Raumtemperatur gerührt und 30 Minuten unter Rückfluß und Rühren erhitzt. Nach dem Abkühlen wird eingeengt, mit 20 ml halbkonzentrierter Salzsäure versetzt und 3 1/2 Stunden bei Raumtemperatur gerührt. Der erhaltene Niederschlag wird abgesaugt, gewaschen, getrocknet und dann in 100 ml Wasser, das mit Salzsäure auf ca. pH 1 gestellt worden ist, verrührt. Danach wird filtriert und das Filtrat auf pH 4 eingestellt. Der erhaltene Niederschlag wird abgesaugt, gewaschen und getrocknet.

Ausbeute: 200 mg (46 % der Theorie),

Schmelzpunkt: 218-220°C (Zers.)

$R_f$-Wert: 0,75 (reversed phase Kieselgel RP 8; Methanol/ 10%ige wäßrige Kochsalzlösung = 6:4)

| Ber.: | C 62,65 | H 5,52 | N 10,96 |
|-------|---------|--------|---------|
| Gef.: | 62,55 | 5,66 | 10,86 |

Beispiel 36

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke | ad 1,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 37

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 38

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.

Durchmesser der Tabletten: 9 mm.

Beispiel 39

Tablette mit 350 mg Wirkstoff

Zusammensetzung:

| (1) Wirkstoff | 350,0 mg |
|---|---|
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 40

Kapseln mit 50 mg Wirkstoff

Zusammensetzung:

| (1) Wirkstoff | 50,0 mg |
|---|---|
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

Beispiel 41

Kapseln mit 350 mg Wirkstoff

Zusammensetzung:

| (1) Wirkstoff | 300,0 mg |
|---|---|
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

1.  5-Gliedrige Alkyleniminoderivate der allgemeinen Formel

$$B - X - A - Y - E, \qquad (I)$$

in der

A einen gegebenenfalls durch die Reste $R_1$ und $R_2$ substituierten Pyrrolidin- oder 2-Pyrrolidinonring, in denen

$R_1$ eine Phenylgruppe, die durch eine Carboxy-, Methoxycarbonyl-, Aminocarbonyl-, Methylaminocarbonyl-, Ethylaminocarbonyl-, Dimethylaminocarbonyl-, Methansulfonylamino- oder Acetylaminogruppe substituiert sein kann,

eine Alkylgruppe mit 1 bis 4 Kohlenstoffatomen, die durch eine Hydroxy-, Methoxy-, Phenoxy-, Methylsulfenyl-, Methylsulfinyl-, Methylsulfonyl-, Phenylsulfenyl-, Phenylsulfinyl-, Phenylsulfonyl-, Amino-, Acetylamino-, Benzoylamino-, N-Methyl-acetylamino-, Methansulfonylamino- oder Benzolsulfonylaminogruppe substituiert sein kann, wobei diese Substituenten nicht in 1-Stellung stehen können, wenn $R_1$ an das Ringstickstoffatom des Restes A gebunden ist,

eine Alkylgruppe mit 1 bis 4 Kohlenstoffatomen, die durch zwei Phenylgruppen, durch eine Cyclohexylgruppe oder durch eine Phenylgruppe substituiert ist, wobei die letztere durch ein Fluor-, Chlor- oder Bromatom, durch ein Alkylgruppe mit 1 bis 4 Kohlenstoffatomen, durch eine Alkoxygruppe mit 1 bis 6 Kohlenstoffatomen, durch eine Phenyl-, Phenylmethyl-, Hydroxy-, Benzyloxy-, Methylsulfenyl-, Methylsulfonyl- oder Trifluormethylgruppe, durch zwei Methoxygruppen oder durch zwei Chloratome substituiert sein kann,

eine Methylgruppe, die durch eine Carboxy-, Methoxycarbonyl-, Aminocarbonyl-, Methylaminocarbonyl-, Ethylaminocarbonyl-, Dimethylaminocarbonyl-, Benzylaminocarbonyl-, Pyrrolidinocarbonyl-, Piperidinocarbonyl-, Hexamethyleniminocarbonyl-, Morpholinocarbonyl-, Thiomorpholinocarbonyl-, 1-Oxido-thiomorpholinocarbonyl-, 1,1-Dioxido-thiomorpholinocarbonyl-, Piperazinocarbonyl-, N-Methyl-piperazinocarbonyl-, N-Acetylpiperazinocarbonyl- oder N-Methansulfonyl-piperazinocarbonylgruppe substituiert ist,

oder auch, sofern $R_1$ nicht in 1-Stellung eines 2-Pyrrolidinonringes steht, eine Carbonylgruppe, die durch eine Methyl-, Phenyl-, Methoxymethyl-, Amino-, Methylamino-, Ethylamino-, Aminomethyl-, Dimethylamino-, Carboxy-, Methoxycarbonyl- oder Dimethylaminocarbonylgruppe substituiert ist,

oder auch, sofern R$_1$ auch nicht an einem zum Ringstickstoffatom benachbarten Kohlenstoffatom des 5-giedrigen Alkyleniminoringes A steht, eine durch eine Methyl-, Dimethylamino-, Phenyl- oder Methoxyphenylgruppe substituierte Sulfonylgruppe und

R$_2$ eine gegebenenfalls durch eine Phenylgruppe substituierte Alkylgruppe mit 1 bis 4 Kohlenstoffatomen darstellen,

B eine Aminoalkylgruppe mit 1 bis 5 Kohlenstoffatomen, eine Amino-, Amidino-, Guanidino- oder Guanidinoalkylgruppe mit 1 bis 5 Kohlenstoffatomen, wobei die vorstehend erwähnten Amino-, Aminoalkyl- oder Amidinogruppen an einem der Stickstoffatome durch eine Hydroxygruppe, durch eine Alkylgruppe mit 1 bis 4 Kohlenstoffatomen, durch eine Alkyloxycarbonylgruppe mit insgesamt 2 bis 6 Kohlenstoffatomen, durch eine Benzyloxycarbonyl-, Phenyloxycarbonyl- oder Benzoylgruppe substituiert sein können,

die Y-E-Gruppe eine geradkettige Alkylgruppe mit 1 bis 3 Kohlenstoffatomen, die durch eine Carboxy-, Phosphono-, O-Methylphosphono- oder Hydroxymethylgruppe, durch eine Alkoxycarbonylgruppe mit insgesamt 2 bis 7 Kohlenstoffatomen, durch eine Dialkylaminocarbonylmethoxycarbonylgruppe, in welcher jeder Alkylteil 1 bis 3 Kohlenstoffatome enthalten kann, durch eine Morpholinoethoxycarbonyl- oder (2-Oxo-1-pyrrolidinyl)ethoxycarbonylgruppe, durch eine Phenylalkoxycarbonylgruppe mit 1 bis 3 Kohlenstoffatomen im Alkoxyteil, wobei der Phenylkern durch eine oder zwei Methoxygruppen substituiert sein kann, oder durch eine Pyridylmethyloxycarbonylgruppe substituiert ist,
wobei der kürzeste Abstand zwischen dem Substituenten E und dem ersten Stickstoffatom der Gruppe B mindestens 10 Bindungen beträgt, und

X eine Gruppe der Formel

$$- X_1 - X_2 - ,$$

wobei X$_1$ an die Gruppe A und X$_2$ an die Gruppe B gebunden ist,

X$_1$ eine Bindung, eine Methylen- oder Ethylengruppe, wobei zwischen der Methylengruppe, sofern diese nicht an das Ringstickstoffatom der Gruppe A gebunden ist, und der benachbarten Gruppe X$_2$ zusätzlich noch ein Sauerstoff- oder Schwefelatom, eine Sulfonyl-, Imino-, -N(COCH$_3$)-, -N(SO$_2$CH$_3$)-, -CONH-, -NH-CO-, -NH-SO$_2$- oder -NH-CO-NH-Gruppe
oder zwischen der Ethylengruppe und der benachbarten Gruppe X$_2$ zusätzlich noch eine Imino-, -NHCO- oder -N(C$_2$H$_5$)CO-Gruppe stehen kann,

X$_2$ eine Phenylen- oder Biphenylylengruppe, die durch ein Fluor-, Chlor- oder Bromatom, durch eine Methyl-, Methoxy-, Ethoxy-, Trifluormethyl-, Methylsulfenyl-, Methylsulfinyl-, Methylsulfonyl-, Nitro-, Acetylamino- oder Methansulfonylaminogruppe oder durch eine weitere Methylgruppe substituiert sein können,

eine gegebenenfalls einfach oder mehrfach ungesättigte geradkettige Phenylenalkylengruppe mit 1 bis 3 Kohlenstoffatomen im Alkylteil, wobei jedoch eine Doppel- oder Dreifachbindung nicht benachbart zu einem Heteroatom stehen kann,

eine Phenylencycloalkylen- oder Cycloalkylenphenylengruppe mit jeweils 4 bis 6 Kohlenstoffatomen im Cycloalkylteil, eine Phenylennaphtylen-, Phenanthrenylen- oder Dihydrophenanthrenylengruppe oder eine Naphthylengruppe, die ganz oder teilweise hydriert sein kann, eine Fluorenylengruppe, in der die Methylengruppe durch eine Hydroxymethylen- oder Carbonylgruppe ersetzt sein kann, eine Indanylen-, Spiroundecylen- oder Phenylenbicycloheptylengruppe oder auch eine Phenylen-W-Phenylengruppe, in welcher W ein Sauerstoff- oder Schwefelatom, eine Imino-, Carbonyl-, Hydroxymethylen-, Sulfinyl- oder Sulfonylgruppe darstellt,
oder, falls auf X$_2$ nicht unmittelbar ein Heteroatom oder ein ungesättigtes Kohlenstoffatom der Gruppe B folgt, auch eine Oxyphenylen- oder Carbonylaminophenylengruppe bedeuten,

deren geometrische Isomere und deren Salze.

2.  5-Gliedrige Alkyleniminoderivate der allgemeinen Formel I gemäß Anspruch 1, in der

EP 0 483 667 B1

A einen gegebenenfalls durch die Reste $R_1$ und $R_2$ substituierten Pyrrolidin- oder 2-Pyrrolidinonring, in denen

$R_1$ eine Phenylgruppe, die durch eine Carboxy-, Methoxycarbonyl- oder Dimethylaminocarbonylgruppe substituiert sein kann,

eine geradkettige Alkylgruppe mit 1 bis 4 Kohlenstoffatomen, die endständig durch eine Phenylgruppe, welche durch eine Alkylgruppe mit 1 bis 4 Kohlenstoffatomen, durch eine Alkoxygruppe mit 1 bis 6 Kohlenstoffatomen, durch eine Phenyl-, Benzyl-, Methylsulfenyl-, Methylsulfonyl- oder Trifluormethylgruppe, durch zwei Methoxygruppen oder durch zwei Chloratome substituiert sein kann, oder durch eine Cyclohexylgruppe oder durch zwei Phenylgruppen substituiert sein kann,

eine Alkylgruppe mit 2 bis 4 Kohlenstoffatomen, die in 2-, 3- oder 4-Stellung durch eine Hydroxy-, Methoxy- oder Phenoxygruppe substituiert ist,

eine Methylgruppe, die durch eine Carboxy-, Methoxycarbonyl-, Aminocarbonyl-, Ethylaminocarbonyl-, Dimethylaminocarbonyl-, Benzylaminocarbonyl-, Pyrrolidinocarbonyl- oder Morpholinocarbonylgruppe substituiert ist,
oder auch, sofern $R_1$ nicht am Ringstickstoffatom des 2-Pyrrolidinonringes steht, eine Carbonylgruppe, die durch eine Methyl-, Phenyl-, Ethylamino-, Dimethylamino-, Methoxymethyl- oder Aminomethylgruppe substituiert ist,
oder auch, sofern $R_1$ auch nicht an einem zum Ringstickstoff benachbarten Kohlenstoffatom des 5-gliedrigen Alkyleniminoringes steht, eine durch eine Methyl-, Methoxyphenyl- oder Dimethylaminogruppe substituierte Sulfonylgruppe und

$R_2$ eine Alkylgruppe mit 1 bis 4 Kohlenstoffatomen darstellen,

B eine Guanidinomethylgruppe oder eine Amidinogruppe, die an einem der Stickstoffatome durch eine Alkylgruppe mit 1 bis 4 Kohlenstoffatomen oder durch eine Methoxycarbonyl-, Ethoxycarbonyl-, Isopropyloxycarbonyl-, Isobutyloxycarbonyl-, Phenyloxycarbonyl-, Benzyloxycarbonyl- oder Benzoylgruppe substituiert sein kann,

die Y-E-Gruppe eine Methylgruppe, die durch eine Carboxy-, Phosphono-, O-Methyl-phosphono- oder Dimethylaminocarbonylmethoxycarbonylgruppe oder durch eine Alkoxycarbonylgruppe mit insgesamt 2 bis 7 Kohlenstoffatomen, in der ein Methoxyteil durch eine Phenyl- oder Pyridylgruppe, der Ethoxyteil endständig durch eine Phenyl-, Dimethoxyphenyl-, Morpholino- oder 2-Oxo-1-pyrrolidinylgruppe und der n-Propoxyteil endständig durch einen Phenylrest substituiert sein kann, substituiert ist,
wobei der kürzeste Abstand zwischen dem Substituenten E und dem ersten Stickstoffatom der Gruppe B mindestens 10 Bindungen beträgt, und

X eine Gruppe der Formel

$$- X_1 - X_2 - ,$$

in welcher

$X_1$ eine Bindung, eine Methylengruppe, die, sofern diese nicht an das Ringstickstoffatom der Gruppe A gebunden ist, über ein Sauerstoffatom, eine Sulfonyl-, Imino-, -N(COCH$_3$)-, -NH-CO- oder -NH-SO$_2$-Gruppe an die benachbarte $X_2$-Gruppe gebunden ist,
oder eine Ethylengruppe, die über eine -NH-CO-Gruppe an die benachbarte $X_2$-Gruppe gebunden ist, und

$X_2$ eine Biphenylylengruppe, die durch ein Fluor-, Chlor- oder Bromatom, durch eine Methyl-, Methoxy-, Ethoxy-, Trifluormethyl-, Methylsulfenyl-, Methylsulfinyl-, Methylsulfonyl-, Nitro-, Acetylamino- oder Methansulfonylaminogruppe oder durch eine weitere Methylgruppe substituiert sein kann, eine Phenylencycloalkylengruppe mit insgesamt 10 bis 12 Kohlenstoffatomen, eine Phenylensulfenylphenylen-, Phenylensulfinylphenylen-, Dihydrophenanthrenylen-, Indanylen- oder Naphthylengruppe oder eine Fluorenylengruppe, in der die Methylengruppe durch eine Hydroxymethylen- oder Carbonylgruppe ersetzt sein kann, bedeuten,

170

deren geometrische Isomere und deren Salze.

3. 5-Gliedrige Alkyleniminoderivate der allgemeinen Formel I nach mindestens einem der Ansprüche 1 oder 2, in denen R$_1$ mit dem Ringstickstoffatom oder der Position 3 der Gruppe A verknüpft ist, deren geometrische Isomere und deren Salze.

4. Folgende 5-gliedrige Alkyleniminoderivate der allgemeinen Formel I gemäß Anspruch 1:

(3S,5S)-5-[(4'-Amidino-4-biphenylyl)oxymethyl]-3-[(methoxycarbonyl)methyl]-2-pyrrolidinon,

1-(4'-Amidino-4-biphenylyl)-4-phosphonomethyl-2-pyrrolidinon,

(3S,5S)-5-[(4'-Amidino-4-biphenylyl)oxymethyl)-3-carboxymethyl-1-(3-phenylpropyl)-2-pyrrolidinon,

(3S,5S)-5-[(4'-Amidino-4-biphenylyl)oxymethyl]-3-carboxymethyl-2-pyrrolidinon,

(3S,5S)-5-[(4'-Amidino-4-biphenylyl)oxymethyl]-3-carboxymethyl-1-phenyl-2-pyrrolidinon,

(3S,5S)-5-[(4'-Amidino-2,3-dimethyl-4-biphenylyl)oxymethyl]-3-carboxymethyl-1-(3-phenylpropyl)-2-pyrrolidinon,

(3S,5S)-5-[(7-Amidino-9-keto-2-fluorenyl)oxymethyl]-3-carboxymethyl-1-(3-phenylpropyl)-2-pyrrolidinon,

(3S,5S)-5-[[N-(4'-Amidino-4-biphenylyl)-N-acetyl]aminomethyl]-3-carboxymethyl-1-(3-phenylpropyl)-2-pyrrolidinon,

(3S,5S)-5-[[2-[(4-Amidinophenyl)amino]phenyl]carbonylaminomethyl]-3-carboxylmethyl-1-(3-phenylpropyl)-2-pyrrolidinon,

(3S,5S)-5-[[4-[(3-Amidinophenyl)carbonyl]phenyl]oxymethyl]-3-carboxymethyl-1-(3-phenylpropyl)-2-pyrrolidinon,

(3S,5S)-5-[(4'-Amidino-4-biphenylyl)oxymethyl]-3-carboxymethyl-1-[(dimethylaminocarbonyl)methyl]-2-pyrrolidinon,

(3S,5S)-1-Acetyl-5-[(4'-amidino-4-biphenylyl)oxymethyl]-3-carboxymethyl-pyrrolidin,

(3S,5S)-5-[(4'-Amidino-4-biphenylyl)oxymethyl]-3-carboxymethyl-1-(dimethylaminosulfonyl)-pyrrolidin,

(3S,5S)-5-[(4'-Amidino-4-biphenylyl)sulfonyl]aminomethyl]-3-carboxymethyl-1-(3-phenylpropyl)-2-pyrrolidinon,

(3R,S;4R,S)-4-[(4'-Amidino-4-biphenylyl)oxymethyl]-3-carboxymethyl-2-pyrrolidinon,

(3S,5S)-5-[(4'-Amidino-3'-fluor-4-biphenylyl)oxymethyl]-3-carboxymethyl-2-pyrrolidinon,

(3S,5S)-5-[[4-(4-Aminobutyl)phenyl]oxymethyl]-3-carboxymethyl-1-(3-phenylpropyl)-2-pyrrolidinon,

(3S,R;5S,R)-3-Carboxymethyl-5-[4-[(5-guanidinopentyl)oxy]phenyl]-1-(3-phenylpropyl)-2-pyrrolidinon,

(3S,5S)-5-[2-[(6-Amino-5,6,7,8-tetrahydro-2-naphtylcarbonyl)amino]ethyl]-3-carboxymethyl-1-(3-phenylpropyl)-2-pyrrolidinon,

(3S,5S)-5-[[4-[(3-Aminopropyl)carbonylamino]phenyl]oxymethyl]-3-carboxymethyl-1-(3-phenylpropyl)-2-pyrrolidinon,

(3S,5S)-3-Carboxymethyl-5-[[4'-(N-hydroxyamidino)-4-biphenylyl]oxymethyl]-2-pyrrolidinon,

(3S,5S)-5-[[4'-(N-Methoxycarbonylamidino)-4-biphenylyl]-oxymethyl]-3-[(methoxycarbonyl)methyl]-2-pyrrolidinon,

(3S,5S)-5-[[4'-(N-Ethoxycarbonylamidino)-4-biphenylyl]-oxymethyl]-3-[(methoxycarbonyl)methyl]-2-pyrrolidinon,

(3S,5S)-5-[[4'-(N-Ethoxycarbonylamidino)-4-biphenylyl]-oxymethyl]-3-[(ethoxycarbonyl)methyl]-2-pyrrolidinon und

(3S,5S)-3-[(Ethoxycarbonyl)methyl]-5-[[4'-(N-methoxycarbonylamidino)-4-biphenylyl]-oxymethyl]-2-pyrrolidinon
sowie deren Salze.

5. (3S,5S)-5-[(4'-Amidino-4-biphenylyl)oxymethyl]-3-carboxymethyl-2-pyrrolidinon und dessen Salze.

6. (3S,5S)-5-[[4'-(N-Methoxycarbonylamidino)-4-biphenylyl]-oxymethyl]-3-[(methoxycarbonyl)methyl]-2-pyrrolidinon und dessen Salze.

7. Physiologisch verträgliche Salze der Verbindungen nach mindestens einem der Ansprüche 1 bis 6 mit anorganischen oder organischen Säuren oder Basen.

8. Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmittel.

9. Verwendung einer Verbindung nach mindestens einem der Ansprüche 1 bis 6 oder ein physiologisch verträgliches Salz gemäß Anspruch 7 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

10. Verfahren zur Herstellung eines Arzneimittels gemäß Anspruch 8, dadurch gekennzeichnet, daß auf nichtchemischem Wege eine Verbindung nach mindestens einem der Ansprüche 1 bis 6 oder ein physiologisch verträgliches Salz gemäß Anspruch 7 in einen oder mehrere inerte Trägerstoffe und/oder Verdünnungsmittel eingearbeitet wird.

11. Verfahren zur Herstellung der 5-gliedrigen Alkyleniminoderivate gemäß den Ansprüchen 1 bis 8, dadurch gekennzeichnet, daß

a) zur Herstellung von Verbindungen der allgemeinen Formel I, in der B eine Amidinogruppe darstellt, welche an einem Stickstoffatom durch eine Hydroxygruppe oder durch eine Alkylgruppe mit 1 bis 4 Kohlenstoffatomen substituiert sein kann, eine gegebenenfalls im Reaktionsgemisch gebildete Verbindung der allgemeinen Formel

$$Z_1 - C(=NR_{10}) - X - A - Y - E \,,\qquad\qquad (II)$$

in der

A, E, X und Y wie in den Ansprüchen 1 bis 6 definiert sind, $R_{10}$ ein Wasserstoffatom oder eine Alkylgruppe mit 1 bis 4 Kohlenstoffatomen und
$Z_1$ eine Alkoxy-, Aralkoxy-, Alkylthio-, Aralkylthio- oder Aminogruppe darstellt, mit einem Amin der allgemeinen Formel

$$R_{11} - NH - R_{12} \,,\qquad\qquad (III)$$

in der
$R_{11}$ ein Wasserstoffatom, eine Hydroxygruppe oder eine Alkylgruppe mit 1 bis 4 Kohlenstoffatomen und
$R_{12}$ ein Wasserstoffatom bedeuten, oder mit deren Säureadditionssalzen umgesetzt wird oder

b) zur Herstellung von Verbindungen der allgemeinen Formel I, in der B eine Aminogruppe enthält, eine Verbindung der allgemeinen Formel

172

$$NC - X - A - Y - E \, , \qquad (IV)$$

in der

A, E, X und Y wie in den Ansprüchen 1 bis 6 definiert sind, reduziert wird oder

c) zur Herstellung von Verbindungen der allgemeinen Formel I, in der B einen Guanidinorest darstellt, eine Verbindung der allgemeinen Formel

$$H_2N - X - A - Y - E \, , \qquad (V)$$

in der

A, E, X und Y wie in den Ansprüchen 1 bis 6 definiert sind, oder dessen Säureadditionssalz mit Cyanamid umgesetzt wird oder

d) zur Herstellung von Verbindungen der allgemeinen Formel I, in der B einen Guanidinorest darstellt, welcher an einem der Stickstoffatome durch eine Hydroxygruppe oder durch eine Alkylgruppe mit 1 bis 4 Kohlenstoffatomen oder durch eine Methoxycarbonyl-, Ethoxycarbonyl-, Isopropyloxycarbonyl-, Isobutyloxycarbonyl-, Benzyloxycarbonyl- oder Phenyloxycarbonylgruppe substituiert sein kann, eine Verbindung der allgemeinen Formel

$$R_{13} - NH - X - A - Y - E \, , \qquad (VI)$$

in der

A, E, X und Y wie in den Ansprüchen 1 bis 6 definiert sind und
$R_{13}$ ein Wasserstoffatom oder eine Alkylgruppe mit 1 bis 4 Kohlenstoffatomen darstellt, mit einem Amidin der allgemeinen Formel

$$R_{14} - Z_2 \, , \qquad (VII)$$

in der
$R_{14}$ eine Amidinogruppe, die an einem der Stickstoffatome durch eine Hydroxygruppe oder durch eine Alkylgruppe mit 1 bis 4 Kohlenstoffatomen oder oder durch eine Methoxycarbonyl-, Ethoxycarbonyl-, Isopropyloxycarbonyl-, Isobutyloxycarbonyl-, Benzyloxycarbonyl- oder Phenyloxycarbonylgruppe substituiert sein kann, und
$Z_2$ einen abspaltbaren Rest darstellt, umgesetzt wird oder

e) zur Herstellung von Verbindungen der allgemeinen Formel I, in der B eine Aminogruppe darstellt, eine gegebenenfalls im Reaktionsgemisch gebildete Verbindung der allgemeinen Formel

$$Z_3 - X - A - Y - E \, , \qquad (VIII)$$

in der

A, E, X und Y wie in den Ansprüchen 1 bis 6 definiert sind und
$Z_3$ eine Nitro- oder Azidogruppe darstellt, reduziert wird oder

f) zur Herstellung von Verbindungen der allgemeinen Formel I, in der E eine Carboxygruppe darstellt, eine Verbindung der allgemeinen Formel

$$B - X - A - Y - E' \, , \qquad (IX)$$

in der

A, B, X und Y wie in den Ansprüchen 1 bis 6 definiert sind und
E', das an ein Kohlenstoffatom gebunden ist, eine mittels Hydrolyse, Behandlung mit einer Säure, Thermolyse oder Hydrogenolyse in eine Carboxygruppe überführbare Gruppe darstellt, mittels Hydrolyse, mittels Behandlung mit einer Säure, Thermolyse oder Hydrogenolyse in eine entsprechende Carboxylverbindung übergeführt wird oder

g) zur Herstellung von Verbindungen der allgemeinen Formel I, in der E einen der in den Ansprüchen 1 bis 6 erwähnten Esterreste darstellt, eine Verbindung der allgemeinen Formel

$$B - X - A - Y - COOH , \tag{X}$$

in der

A, B, X und Y wie in den Ansprüchen 1 bis 6 definiert sind, oder deren reaktionsfähige Derivate mit einer Verbindung der allgemeinen Formel

$$H - R_{15} , \tag{XI}$$

in der
$R_{15}$ eine Alkoxygruppe mit 1 bis 6 Kohlenstoffatomen, eine Dialkylaminocarbonylmethoxygruppe, in welcher jeder Alkylteil 1 bis 3 Kohlenstoffatome enthalten kann, eine Morpholinoethoxy- oder (2-Oxo-1-pyrrolidinyl)ethoxygruppe, eine Phenylalkoxygruppe mit 1 bis 3 Kohlenstoffatomen im Alkoxyteil, wobei der Phenylkern durch eine oder zwei Methoxygruppen substituiert sein kann, oder eine Pyridylmethyloxygruppe darstellt, umgesetzt wird oder

h) zur Herstellung von Verbindungen der allgemeinen Formel I, in der E eine Carboxygruppe darstellt, eine Verbindung der allgemeinen Formel

$$B - X - A - Y - CHO , \tag{XII}$$

in der

A, B, X und Y wie in den Ansprüchen 1 bis 6 definiert sind, oxidiert wird oder

i) zur Herstellung von Verbindungen der allgemeinen Formel I, in denen der B-X-A-Rest entweder eine B-$G_1$-T-$G_2$-A-Gruppe oder eine B-$G_1$-A-Gruppe darstellt, wobei $G_2$ einem Teil von X und $G_1$-T dem anderen Teil von X entspricht und zusätzlich $G_1$ und $G_2$ oder $G_1$-T auch eine Bindung darstellen können und $G_1$ auch für X stehen kann, und

T ein Sauerstoff- oder Schwefelatom, eine Imino-, -N(COCH$_3$)- oder -N(SO$_2$CH$_3$)-Gruppe bedeutet, eine Verbindung der allgemeinen Formel

$$B - G_1 - T - H , \tag{XIII}$$

mit einer Verbindung der allgemeinen Formel

$$Z_5 - G_2 - A - Y - E , \tag{XIV}$$

oder einer Verbindung der allgemeinen Formel

$$H - T - G_2 - A - Y - E , \tag{XV}$$

mit einer Verbindung der allgemeinen Formel

$$B - G_1 - Z_5 , \tag{XVI}$$

oder einer Verbindung der allgemeinen Formel

$$H - A - Y - E , \tag{XVII}$$

mit einer Verbindung der allgemeinen Formel

$$B - G_1 - Z_5 \,, \hspace{4cm} (XVI)$$

in denen

A, B, E und Y wie in den Ansprüchen 1 bis 6 definiert sind,

$G_2$ einem Teil von X und $G_1$-T dem anderen Teil von X entspricht,

wobei zusätzlich $G_1$ und $G_2$ oder $G_1$-T eine Bindung darstellen können und $G_1$ auch für X stehen kann sowie X jeweils wie in den Ansprüchen 1 bis 6 definiert ist,

T ein Sauerstoff- oder Schwefelatom, eine Imino-, -N(COCH$_3$)- oder -N(SO$_2$CH$_3$)-Gruppe und

$Z_5$ eine Austrittsgruppe bedeuten, oder mit den Alkali-, Erdalkalimetall- oder MgHal-Salzen einer Verbindung der allgemeinen Formeln XIII, XV oder XVII umgesetzt wird oder

j) zur Herstellung von Verbindungen der allgemeinen Formel I, in der $R_1$ einen der für $R_1$ in den Ansprüchen 1 bis 6 erwähnten Alkylreste darstellt, eine Verbindung der allgemeinen Formel

$$B - X - A' - Y - E \,, \hspace{4cm} (XVIII)$$

in der

B, E, X und Y wie in den Ansprüchen 1 bis 6 definiert sind und

A' einen der im Anpruch 1 erwähnten gegebenenfalls im Kohlenstoffgerüst durch $R_2$ substituierten 5-gliedrigen cyclische Alkyleniminogruppe darstellt, wobei $R_2$ wie in den Ansprüchen 1 bis 6 definiert ist, mit einer Verbindung der allgemeinen Formel

$$R_1' - Z_6 \,, \hspace{4cm} (XIX)$$

in der

$R_1'$ einen der für $R_1$ in den Ansprüchen 1 bis 6 erwähnten Alkylreste darstellt und

$Z_6$ eine Austrittsgruppe darstellt, alkyliert wird oder

k) zur Herstellung von Verbindungen der allgemeinen Formel I, in der $R_1$ einen der in den Ansprüchen 1 bis 6 erwähnten Acyl- oder Sulfonylreste und A einen Pyrrolidinring darstellen, eine Verbindung der allgemeinen Formel

$$B - X - A'' - Y - E \,, \hspace{4cm} (XX)$$

in der

B, E, X und Y wie in den Ansprüchen 1 bis 6 definiert sind und

A'' einen gegebenenfalls im Kohlenstoffgerüst durch $R_2$ substituierten Pyrrolidinring darstellt, wobei $R_2$ wie in den Ansprüchen 1 bis 6 definiert ist, mit einer Verbindung der allgemeinen Formel

$$R_1'' - Z_7 \,, \hspace{4cm} (XXI)$$

in der

$R_1''$ einen der für $R_1$ in den Ansprüchen 1 bis 6 erwähnten Acyl- oder Sulfonylreste darstellt und

$Z_7$ eine Hydroxygruppe oder eine Austrittsgruppe oder auch $Z_7$ zusammen mit dem Wasserstoffatom einer zur Carbonylgruppe benachbarten Iminogruppe eine weitere Kohlenstoff-Stickstoff-Bindung bedeuten, acyliert oder sulfoniert wird oder

l) zur Herstellung von Verbindungen der allgemeinen Formel I, in der E eine Hydroxycarbonylgruppe, eine Alkoxycarbonylgruppe mit insgesamt 2 bis 7 Kohlenstoffatomen, eine Pyridylmethyloxycarbonyl- oder Phenylalkoxycarbonylgruppe mit 1 bis 3 Kohlenstoffatomen im Alkoxyteil, in der der Phenylkern durch eine oder zwei Methoxygruppen substituiert sein kann, darstellt, eine Verbindung der allgemeinen Formel

$$B - X - A - Y - E'' \,, \hspace{4cm} (XXII)$$

in der

A, B, X und Y wie in den Ansprüchen 1 bis 6 definiert sind und
E" eine Vinyl- oder 1,2-Dihydroxyalkylgruppe darstellt, oxidiert und eine so erhaltene Verbindung erforderlichenfalls anschließend mit einem entsprechenden Alkohol verestert wird oder

m) zur Herstellung von Verbindungen der allgemeinen Formel I, in der E eine der in den Ansprüchen 1 bis 6 erwähnten durch eine Alkoxy-, Phenylalkoxy- oder Pyridylmethyloxygruppe substituierte Carbonylgruppe darstellt, eine Verbindung der allgemeinen Formel

$$B - X - A - Y - COOH , \qquad\qquad (XI)$$

in der

A, B, X und Y wie in den Ansprüchen 1 bis 6 definiert sind, mit einem Formamidacetal der allgemeinen Formel

$$(R_{16})_2N - CH(OR_{17})_2 , \qquad\qquad (XXIII)$$

in der
$R_{16}$ eine niedere Alkylgruppe und
$R_{17}$ eine Alkylgruppe mit 1 bis 6 Kohlenstoffatomen, eine im Phenylteil gegebenenfalls durch eine oder zwei Methoxygruppen substituierte Phenylalkylgruppe oder eine Pyridylmethylgruppe bedeuten, umgesetzt wird oder

n) zur Herstellung von Verbindungen der allgemeinen Formel I, in der A einen Pyrrolidinring und $R_1$ in 1-Stellung des Pyrrolidinringes eine Carbonylgruppe darstellt, die durch eine Amino-, Methylamino-, Ethylamino- oder Dimethylaminogruppe substituiert ist,
eine Verbindung der allgemeinen Formel

$$B - X - A''' - Y - E , \qquad\qquad (XXIV)$$

in der

B, E, X und Y wie in den Ansprüchen 1 bis 6 definiert sind und

A''' einen gegebenenfalls durch $R_2$ substituierten Pyrrolidinring darstellt, welcher in 1-Stellung durch eine Halogencarbonyl- oder N-Azolylcarbonylgruppe substituiert ist, mit einem Amin der allgemeinen Formel

$$H - N(R_5)_2 , \qquad\qquad (XXV)$$

in der

$R_5$ ein Wasserstoffatom, eine Methyl- oder Ethylgruppe darstellt, umgesetzt wird und
gewünschtenfalls anschließend eine so erhaltene Verbindung der allgemeinen Formel I, die eine Amino-, Methylamino- oder Iminogruppe enthält, mittels Acylierung, Sulfonylierung oder Alkylierung in eine entsprechende Verbindung der allgemeinen Formel I übergeführt wird und/oder

eine so erhaltene Verbindung der allgemeinen Formel I, die eine Hydroxygruppe enthält, mittels Alkylierung oder Acylierung in eine entsprechende Verbindung der allgemeinen Formel I übergeführt wird und/oder

eine so erhaltene Verbindung der allgemeinen Formel I, die eine Carbonylbrücke enthält, mittels Reduktion in eine entsprechende Hydroxymethylenverbindung der allgemeinen Formel I übergeführt wird und/oder

eine so erhaltene Verbindung der allgemeinen Formel I, die eine Carbonylbrücke enthält, mittels Reduktion in eine entsprechende Methylenverbindung der allgemeinen Formel I übergeführt wird und/oder

eine so erhaltene Verbindung der allgemeinen Formel I, die eine Sulfenyl- oder Sulfenylgruppe oder eine Thioetherbrücke enthält, mittels Oxidation in eine entsprechende S-Oxidverbindung der allgemeinen Formel I übergeführt wird und/oder

eine so erhaltene Verbindung der allgemeinen Formel I, die eine Sulfenyl- oder Sulfinylgruppe oder eine Thioetherbrücke enthält, oder eine S-Oxidverbindung der allgemeinen Formel I, mittels Oxidation in eine entsprechende S,S-Dioxidverbindung der allgemeinen Formel I übergeführt wird und/oder

eine so erhaltene Verbindung der allgemeinen Formel I, die einen durch eine Aminogruppe substituierten aromatischen Rest enthält, mittels Sandmeyer-Reaktion in eine entsprechende Cyanoverbindung der allgemeinen Formel I übergeführt wird und/oder

eine so erhaltene Verbindung der allgemeinen Formel I, die eine Estergruppe enthält, durch Umsetzung mit einem Amin in ein entsprechendes Amid übergeführt wird und/oder

eine so erhaltene Verbindung der allgemeinen Formel I, die eine Estergruppe enthält, mittels Reduktion eine eine entsprechende Hydroxymethylverbindung der allgemeinen Formel I übergeführt wird und/oder

eine so erhaltene Verbindung der allgemeinen Formel I, die eine Estergruppe enthält, mittels Umesterung in einen entsprechenden Ester übergeführt wird und/oder

eine so erhaltene Verbindung der allgemeinen Formel I, die eine Amidino- oder Guanodinogruppe enthält, mittels Acylierung in eine entsprechende Verbindung der allgemeinen Formel I übergeführt wird und

erforderlichenfalls ein während der Umsetzungen a) bis n) und während den vorstehend erwähnten nachträglichen Umsetzungen zum Schutze von reaktiven Gruppen verwendeter Schutzrest abgespalten wird und/oder

gewünschtenfalls eine so erhaltene Verbindung der allgemeinen Formel I in ihre cis-/trans-Isomere, in ihre Enantiomeren und/oder Diastereomeren aufgetrennt wird und/oder

eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

1. 5-membered alkyleneimino derivatives of general formula

$$B - X - A - Y - E , \qquad (I)$$

in which

A represents a pyrrolidine or 2-pyrrolidinone ring, optionally substituted by groups $R_1$ and $R_2$, wherein

$R_1$ represents a phenyl group which can be substituted by a carboxy, methoxycarbonyl, aminocarbonyl, methylaminocarbonyl, ethylaminocarbonyl, dimethylaminocarbonyl, methane sulphonylamino, or acetylamino group,

an alkyl group with 1 to 4 carbon atoms, which can be substituted by a hydroxy, methoxy, phenoxy, methylsulphenyl, methylsulphinyl, methylsulphonyl, amino, acetylamino, benzoylamino, N-methylacetylamino, methano-sulphonylamino, or benzene sulphonylamino group, whilst these groups cannot be in the 1-position if $R_1$ is linked to the cyclic nitrogen atom of group A,

an alkyl group with 1 to 4 carbon atoms, which is substituted by two phenyl groups, a cyclohexyl group or a phenyl group, whilst the latter can be substituted by a fluorine, chlorine or bromine atom, an alkyl group with 1 to 4 carbon atoms, an alkoxy group with 1 to 6 carbon atoms, a phenyl, phenylmethyl, hydroxy, benzyloxy, methylsulphenyl, methylsulphonyl or trifluoromethyl group, two methoxy groups or two chlorine atoms,

a methyl group, which is substituted by a carboxy, methoxycarbonyl, aminocarbonyl, methylaminocarbonyl, ethylaminocarbonyl, dimethylaminocarbonyl, benzylaminocarbonyl, pyrrolidinocarbonyl, piperidinocarbonyl, hexamethyleneiminocarbonyl, morpholinocarbonyl, thiomorpholinocarbonyl, 1-oxido-thiomorpholinocarbonyl, 1,1-dioxido-thiomorpholinocarbonyl, piperazinocarbonyl, N-methyl-piperazinocarbonyl, N-acetyl-piperazinocarbonyl, or N-methanesulphonylpiperazinocarbonyl group,

or, as long as $R_1$ is not in the 1-position of a 2-pyrrolidinone ring, a carbonyl group which is substituted by a methyl, phenyl, methoxymethyl, amino, methylamino, ethylamino, aminomethyl, dimethylamino, carboxy, methoxycarbonyl, or dimethylaminocarbonyl group,

or also, as long as $R_1$ is also not next to a carbon atom of the 5-membered alkyleneimino ring which neighbours the ring nitrogen atom, a sulphonyl group which is substituted by a methyl, dimethylamino, phenyl or methoxyphenyl group and

$R_2$ represents an alkyl group with 1 to 4 carbon atoms, optionally substituted by a phenyl group,

B represents an aminoalkyl group with 1 to 5 carbon atoms, an amino, amidino, guanidino or guanidinoalkyl group with 1 to 5 carbon atoms, whilst the aforementioned amino, aminoalkyl or amidino groups are substituted at one of the nitrogen atoms by a hydroxy group, an alkyl group with 1 to 4 carbon atoms, an alkyloxycarbonyl group with a total of 2 to 6 carbon atoms, a benzyloxycarbonyl, phenyloxycarbonyl or benzoyl group,

the Y-E group represents a straight-chained alkyl group, which can be substituted by a carboxy, phosphono, O-methyl-phosphono or hydroxymethyl group, an alkoxycarbonyl group with a total of 2 to 7 carbon atoms, a dialkylaminocarbonylmethoxycarbonyl group, in which each alkyl moiety can contain 1 to 3 carbon atoms, a morpholinoethoxycarbonyl or (2-oxo-1 pyrrolidinyl) ethoxycarbonyl group, a phenylalkoxycarbonyl group with 1 to 3 carbon atoms in the alkoxy moiety, whilst the phenyl nucleus can be substituted by one or two methoxy groups or is substituted by a pyridylmethoxycarbonyl group,

whilst the shortest distance between substituent E and the first nitrogen atom of group B is at least 10 bonds, and

X represents a group of the formula

$$- X_1 - X_2 - ,$$

wherein

$X_1$ is bound to group A and $X_2$ is bound to group B, $X_1$ represents a bond, a methylene or ethylene group, whilst between the methylene group, provided it is not bonded to the cyclic nitrogen atom of group A, and the adjacent group $X_2$, there may be an additional oxygen or sulphur atom or a sulphonyl, imino, -N(COCH$_3$), -N(SO$_2$CH$_3$), -CONH, -NH-CO, -NH-SO$_2$, or -NH-CO-NH group

or an additional imino, -NHCO or -N(C$_2$H$_5$)CO group may be between the ethylene group and the neighbouring group $X_2$,

$X_2$ represents a phenylene or biphenylene group, which may be substituted by a fluorine, chlorine, or bromine atom, or a methyl, methoxy, ethoxy, trifluoromethyl, methylsulphenyl, methylsulphinyl, methylsulphonyl, nitro, acetylamino or methane-sulphonylamino group, or by another methyl group,

or an optional mono- or polyunsaturated straight-chained phenylene-alkylene group with 1 to 3 carbon atoms in the alkyl part, although a double or triple bond cannot be adjacent to a hetero atom,

a phenylenecycloalkylene or cycloalkylenephenylene group, each with 4 to 6 carbon atoms in the cycloalkyl moiety, a phenylenenaphthylene, phenanthrenylene or dihydrophenanthrenylene group, or a naphthylene, which can be completely or partially hydrogenated, a fluorenylene group wherein the methylene group can be replaced by a hydroxymethylene or carbonyl group, an indanylene, spiroundecylene or phenylenebicycloheptylene group or a phenylene-W-phenylene group, wherein W represents an oxygen or sulphur atom, an imino, carbonyl, hydroxymethylene, sulphinyl or sulphonyl group,

or, if $X_2$ is not followed directly by a hetero atom or an unsaturated carbon atom of group B, can also mean an oxyphenylene or carbonylaminophenylene group,

the geometric isomers and the salts thereof.

2. 5-membered alkylene-imino derivative of general formula I according to claim 1, in which

A represents a pyrrolidine or 2-pyrrolidinone ring, optionally substituted by groups $R_1$ and $R_2$, wherein

$R_1$ represents a phenyl group which can be substituted by a carboxy, methoxycarbonyl or dimethylaminocarbonyl group,

a straight-chained alkyl group with 1 to 4 carbon atoms which may be terminally substituted by a phenyl group, which in turn may be substituted by an alkyl group with 1 to 4 carbon atoms, an alkoxy group with 1 to 6 carbon atoms, a phenyl, benzyl, methylsulphenyl, methylsulphonyl or trifluoromethyl group, by two methoxy groups or by two chlorine atoms, or by a cyclohexyl group or two phenyl groups,

an alkyl group with 2 to 4 carbon atoms, which is substituted in the 2-, 3- or 4-position by a hydroxy, methoxy or phenoxy group,

a methyl group which is substituted by a carboxy, methoxycarbonyl, aminocarbonyl, ethylaminocarbonyl, dimethylaminocarbonyl, benzylaminocarbonyl, pyrrolidinocarbonyl or morpholinocarbonyl group,
or also, if $R_1$ is not positioned at the cyclic nitrogen atom of the 2-pyrrolidinone ring, a carbonyl group, which is substituted by a methyl, phenyl, ethylamino, dimethylamino, methoxymethyl, or aminomethyl group,
or also, if $R_1$ is also not situated at a carbon atom of the 5-membered alkyleneimino ring which is adjacent to the cyclic nitrogen atom, a sulphonyl group substituted by a methyl, methoxyphenyl or dimethylamino group, and

$R_2$ represents an alkyl group with 1 to 4 carbon atoms,

B represents a guanidinomethyl group or an amidino group which can be substituted at one of the nitrogen atoms by an alkyl group with 1 to 4 carbon atoms or by a methoxycarbonyl, ethoxycarbonyl, isopropyloxycarbonyl, isobutyloxycarbonyl, phenyloxycarbonyl, benzyloxycarbonyl or benzoyl group,

the Y-E group represents a methyl group substituted by a carboxy, phosphono, O-methylphosphono, or dimethylaminocarbonylmethoxycarbonyl group or by an alkoxycarbonyl group with a total of 2 to 7 carbon atoms, wherein a methoxy moiety can be substituted by a phenyl or pyridyl group, the ethoxy moiety may be terminally substituted by a phenyl, dimethoxyphenyl, morpholino or 2-oxo-1-pyrrolidinyl group and the n-propoxy moiety may be terminally substituted by a phenyl group,
whilst the shortest distance between substituents E and the first nitrogen atom of group B is at least 10 bonds, and where

X represents a group of formula

$$- X_1 - X_2 -,$$

wherein

$X_1$ represents a bond, a methylene group, which, provided that it is not bound to the cyclic nitrogen atom of group A, is bound via an oxygen atom, a sulphonyl, imino, -N(COCH$_3$), -NH-CO- or -NH-SO$_2$- group to the adjacent $X_2$ group,
or an ethylene group bound via an -NH-CO- group to the adjacent $X_2$ group, and

$X_2$ represents a biphenylylene group which may be substituted by a fluorine, chlorine or bromine atom, by a methyl, methoxy, ethoxy, trifluoromethyl,
methylsulphenyl, methylsulphinyl, methylsulphonyl, nitro, acetylamino or methanesulphonylamino group or by another methyl group, a phenylenecycloalkylene group having a total of 10 to 12 carbon atoms, a phenylenesulphenylphenylene, phenylenesulphinylphenylene, dihydrophenanthrenylene, indanylene or naphthylene group or a fluorenylene group, in which the methylene group may be replaced by a hydroxymethylene or carbonyl group, the geometric isomers and the salts thereof.

3. 5-membered alkyleneimino derivatives of general formula I according to at least one of claims 1 or 2, wherein $R_1$ is linked to the cyclic nitrogen atom or position 3 of the group A, the geometric isomers and the salts thereof.

4. The following 5-membered alkyleneimino derivatives of general formula I according to claim 1:

(3S,5S)-5-[(4'-amidino-4-biphenylyl)oxymethyl]-3-[(methoxycarbonyl)methyl]-2-pyrrolidinone,

1-(4'-amidino-4-biphenylyl)-4-phosphonomethyl-2-pyrrolidinone,

(3S,5S)-5-[(4'-amidino-4-biphenylyl)oxymethyl)-3-carboxymethyl-1-(3-phenylpropyl)-2-pyrrolidinone,

(3S,5S)-5-[(4'-amidino-4-biphenylyl)oxymethyl]-3-carboxymethyl-2-pyrrolidinone,

(3S,5S)-5-[(4'-amidino-4-biphenylyl)oxymethyl)-3-carboxymethyl-1-phenyl-2-pyrrolidinone,

(3S,5S)-5-[(4'-amidino-2,3-dimethyl-4-biphenylyl)oxymethyl]-3-carboxymethyl-1-(3-phenylpropyl)-2-   pyrrolidinone,

(3S,5S)-5-[(7-amidino-9-keto-2-fluorenyl)oxymethyl]-3-carboxymethyl-1-(3-phenylpropyl)-2-pyrrolidinone,

(3S,5S)-5-[[N-(4'-amidino-4-biphenylyl)-N-acetyl]aminomethyl]-3-carboxymethyl-1-(3-phenylpropyl)-2-pyrrolidinone,

(3S,5S)-5-[[2-[(4-amidinophenyl)amino]phenyl]carbonylaminomethyl]-3-carboxylmethyl-1-(3-phenylpropyl)-2-pyrrolidinone,

(3S,5S)-5-[[4-[(3-amidinophenyl)carbonyl]phenyl]oxymethyl]-3-carboxymethyl-1-(3-phenylpropyl)-2-pyrrolidinone,

(3S,5S)-5-[(4'-amidino-4-biphenylyl)oxymethyl]-3-carboxymethyl-1-[(dimethylaminocarbonyl)methyl]-2-pyrrolidinone,

(3S,5S)-1-acetyl-5-[(4'-amidino-4-biphenylyl)oxymethyl]-3-carboxymethyl-pyrrolidine,

(3S,5S)-5-[(4'-amidino-4-biphenylyl)oxymethyl]-3-carboxymethyl-1-(dimethylaminosulphonyl)-pyrrolidine,

(3S,5S)-5-[(4'-amidino-4-biphenylyl)sulphonyl]aminomethyl]-3-carboxymethyl-1-(3-phenylpropyl)-2-pyrrolidinone,

(3R,S;4R,S)-4-[(4'-amidino-4-biphenylyl)oxymethyl]-3-carboxymethyl-2-pyrrolidinone,

(3S,5S)-5-[(4'-amidino-3'-fluoro-4-biphenylyl)oxymethyl]-3-carboxymethyl-2-pyrrolidinone,

(3S,5S)-5-[[4-(4-aminobutyl)phenyl]oxymethyl]-3-carboxymethyl-1-(3-phenylpropyl)-2-pyrrolidinone,

(3S,R;5S,R)-3-carboxymethyl-5-[4-[(5-guanidinopentyl)oxy]phenyl]-1-(3-phenylpropyl)-2-pyrrolidinone,

(3S,5S)-5-[2-[(6-amino-5,6,7,8-tetrahydro-2-naphthylcarbonyl)amino]ethyl]-3-carboxymethyl-1-(3-phenylpropyl)-2-pyrrolidinone,

(3S,5S)-5-[[4-[(3-aminopropyl)carbonylamino]phenyl]oxymethyl]-3-carboxymethyl-1-(3-phenylpropyl)-2-pyrrolidinone,

(3S,5S)-3-carboxymethyl-5-[[4'-(N-hydroxyamidino)-4-biphenylyl)oxymethyl]-2-pyrrolidinone and

(3S,5S)-5-[[4'-(N-methoxycarbonylamidino)-4-biphenylyl]oxymethyl]-3-[(methoxycarbonyl)methyl]-2-pyrrolidi-

(3S,5S)-5-[[4'-(N-ethoxycarbonylamidino)-4-biphenylyl]oxymethyl]-3-[(methoxycarbonyl)methyl]-2-pyrrolidinone,

(3S,5S)-5-[[4'-(N-ethoxycarbonylamidino)-4-biphenylyl]oxymethyl]-3-[(ethoxycarbonyl)methyl]-2-pyrrolidinone and

(3S,5S)-3-[(ethoxycarbonyl)methyl]-5-[[4'-(N-methoxycarbonylamidino)-4-biphenylyl]-oxymethyl]-2-pyrrolidinone,
and the salts thereof.

5. (3S,5S)-5-[(4'-amidino-4-biphenylyl)oxymethyl]-3-carboxymethyl-2-pyrrolidinone and the salts thereof.

6. (3S,5S)-5-[[4'-(N-methoxycarbonylamidino)-4-biphenylyl]-oxymethyl-2-pyrrolidinone and the salts thereof.

7. Physiologically-acceptable salts of the compounds according to at least one of claims 1 to 4 with inorganic or organic acids or bases.

8. Pharmaceutical compositions containing a compound according to at least one of claims 1 to 6 or a physiologically-acceptable salt according to claim 7 optionally together with one or more inert carriers and/or diluents.

9. Use of a compound according to at least one of claims 1 to 6 or a physiologically-acceptable salt according to claim 7 for preparing a pharmaceutical composition which is suitable for combatting or preventing diseases in which smaller or larger clumps of cells occur or in which cell-matrix interactions are involved.

10. Process for preparing a pharmaceutical composition according to claim 8, characterised in that a compound according to at least one of claims 1 to 6 or a physiologically-acceptable salt according to claim 7 is incorporated in a non-chemical manner in one or more inert carriers and/or diluents.

11. Process for preparing the 5-membered alkyleneimino derivatives according to claims 1 to 8, characterised in that

    a) in order to prepare compounds of general formula I wherein B represents an amidino group, which may be substituted at a nitrogen atom by a hydroxy group or an alkyl group with 1 to 4 carbon atoms,

    a compound of general formula

$$Z_1 - C(=NR_{10}) - X - A - Y - E \tag{II}$$

    optionally formed in the reaction mixture, wherein
    A, E, X and Y are defined as in claims 1 to 6,
    $R_{10}$ represents a hydrogen atom or a $C_{1-4}$-alkyl group and
    $Z_1$ represents an alkoxy, aralkoxy, alkylthio, aralkylthio or amino group,
    is reacted with an amine of general formula

$$R_{11} - NH - R_{12} \tag{III}$$

    wherein
    $R_{11}$ represents a hydrogen atom or a $C_{1-4}$-alkyl group and
    $R_{12}$ represents a hydrogen atom, or with the acid addition salts thereof, or

    b) in order to prepare compounds of general formula I wherein B contains an amino group,

    a compound of general formula

$$NC - X - A - Y - E \tag{IV}$$

    wherein

A, E, X and Y are defined as in claims 1 to 6, is reduced or

c) in order to prepare compounds of general formula I wherein B represents a guanidino group,

a compound of general formula

$$H_2N - X - A - Y - E \qquad (V)$$

wherein
A, E, X and Y are defined as in claims 1 to 6, or an acid addition salt thereof, is reacted with cyanamide, or

d) in order to prepare compounds of general formula I wherein B represents a guanidino group which may be substituted by a $C_{1-4}$-alkyl group or a methoxycarbonyl, ethoxycarbonyl, isopropyloxycarbonyl, isobutyloxycarbonyl, benzyloxycarbonyl or phenyloxycarbonyl group,

a compound of general formula

$$R_{13} - NH - X - A - Y - E \qquad (VI)$$

wherein
A, E, X and Y are defined as in claims 1 to 6 and
$R_{13}$ represents a hydrogen atom or a $C_{1-4}$-alkyl group, is reacted with an amidine of general formula

$$R_{14} - Z_2 \qquad (VII)$$

wherein
$R_{14}$ represents an amidino group which is substituted by a hydroxy group or by a $C_{1-5}$-alkyl group or a methoxycarbonyl, ethoxycarbonyl, isopropyloxycarbonyl, isobutyloxycarbonyl, benzyloxycarbonyl or phenyloxycarbonyl group and
$Z_2$ represents a cleavable group, or

e) in order to prepare compounds of general formula I wherein B represents an amino group,

a compound of general formula

$$Z_3 - X - A - Y - E \qquad (VIII)$$

optionally formed in the reaction mixture, wherein A, E, X and Y are defined as in claims 1 to 6 and $Z_3$ represents a nitro or azido group, is reduced or

f) in order to prepare compounds of general formula I wherein E represents a carboxy group,

a compound of general formula

$$B - X - A - Y - E' , \qquad (IX)$$

wherein
A, B, X and Y are defined as in claims 1 to 6 and
E', which is bound to a carbon atom, represents a group which can be converted into a carboxy group by hydrolysis, treatment with acids, thermolysis or hydrogenolysis, is converted into a corresponding carboxyl compound by hydrolysis, by treatment with acid, thermolysis or hydrogenolysis, or

g) in order to prepare compounds of general formula I wherein E represents an ester group as mentioned in claims 1 to 6, a compound of general formula

$$B - X - A - Y - COOH , \qquad (X)$$

wherein

A, B, X and Y are defined as in claims 1 to 6, or the reactive derivatives thereof, is reacted with a compound of general formula

$$H - R_{15} , \qquad (XI)$$

wherein
$R_{15}$ represents a $C_{1-6}$-alkoxy group, a dialkylaminocarbonylmethoxy group in which each alkyl moiety can contain 1 to 3 carbon atoms, a morpholinoethoxy or (2-oxo-1-pyrrolidinyl)ethoxy group, a phenylalkoxy group with 1 to 3 carbon atoms in the alkoxy moiety, wherein the phenyl nucleus can be substituted by one or two methoxy groups, or a pyridylmethyloxy group, is reacted or

h) in order to prepare compounds of general formula I wherein E represents a carboxy group,

a compound of general formula

$$B - X - A - Y - CHO , \qquad (XII)$$

wherein
A, B, X and Y are defined as in claims 1 to 6 is oxidised or

i) in order to prepare compounds of general formula I wherein the B-X-A group represents either a B-$G_1$-T-$G_2$-A group or a B-$G_1$-A group, whilst $G_2$ corresponds to part of X and $G_1$-T corresponds to the other part of X and additionally $G_1$ and $G_2$ or $G_1$-T may also represent a bond and $G_1$ may also represent X, and T represents an oxygen or sulphur atom or an imino, -N(COCH$_3$) or -N(SO$_2$CH$_3$) group,

a compound of general formula

$$B - G_1 - T - H \qquad (XIII)$$

is reacted with a compound of general formula

$$Z_5 - G_2 - A - Y - E \qquad (XIV)$$

or a compound of general formula

$$H - T - G_2 - A - Y - E \qquad (XV)$$

is reacted with a compound of general formula

$$B - G_1 - Z_5 \qquad (XVI)$$

or a compound of general formula

$$H - A - Y - E \qquad (XVII)$$

is reacted with a compound of general formula

$$B - G_1 - Z_5 \qquad (XVI)$$

wherein
A, B, E and Y are defined as in claims 1 to 6,
$G_2$ corresponds to a part of X and $G_1$-T corresponds to the other part of X, whilst additionally $G_1$ and $G_2$ or $G_1$-T may represent a bond and $G_1$ may also represent X and
X is defined as in claims 1 to 6,
T represents an oxygen or sulphur atom or an imino, -N(COCH$_3$) or -N(SO$_2$CH$_3$) group and
$Z_5$ represents a leaving group, or with the alkali metal, alkaline earth metal or MgHal salts of a compound of general formula XIII, XV or XVII is reacted, or

j) in order to prepare compounds of general formula I wherein $R_1$ represents one of the alkyl groups mentioned for $R_1$ in claims 1 to 6,

a compound of general formula

$$B - X - A' - Y - E ,\qquad\qquad (XVIII)$$

wherein
B, E, X and Y are defined as in claims 1 to 6 and
A' represents a 5-membered cyclic alkyleneimino group, mentioned in claim 1 which is optionally substituted in the carbon skeleton by $R_2$, $R_2$ being defined as in claims 1 to 6,
is alkylated with a compound of general formula

$$R_1' - Z_6 ,\qquad\qquad (XIX)$$

wherein
$R_1'$ represents one of the alkyl groups mentioned for $R_1$ in claims 1 to 6 and
$Z_6$ represents a leaving group, or

k) in order to prepare compounds of general formula I wherein $R_1$ represents one of the acyl or sulphonyl groups mentioned in claims 1 to 6 and A represents a pyrrolidine ring,

a compound of general formula

$$B - X - A'' - Y - E ,\qquad\qquad (XX)$$

wherein
B, E, X and Y are defined as in claims 1 to 6 and
A'' represents a pyrrolidine ring which is optionally substituted in the carbon skeleton by $R_2$, wherein $R_2$, as defined as in claims 1 to 6, is acylated or sulphonylated with a compound of general formula

$$R_1'' - Z_7\qquad\qquad (XXI)$$

wherein
$R_1''$ represents one of the acyl or sulphonyl groups mentioned for $R_1$ in claims 1 to 6 and
$Z_7$ represents a hydroxy group or a leaving group or $Z_7$ together with the hydrogen atom of an imino group adjacent to the carbonyl group represents another carbon-nitrogen bond, or

l) in order to prepare compounds of general formula I,
wherein E represents a hydroxycarbonyl, alkoxycarbonyl group with a total of 2 to 7 carbon atoms, a pyridylmethyloxycarbonyl or phenylalkoxycarbonyl group with 1 to 3 carbon atoms in the alkoxy moiety, in which the phenyl nucleus can be replaced by one or two methoxy groups, a compound of general formula

$$B - X - A - Y - E''\qquad\qquad (XXII)$$

wherein

A, B, X and Y are defined as in claims 1 to 6 and E'' represents a vinyl or 1,2-dihydroxyalkyl group, is oxidised and a compound thus obtained is, if necessary, subsequently esterified with a corresponding alcohol or

m) in order to prepare compounds of general formula I wherein E represents one of the carbonyl groups mentioned in claims 1 to 6 substituted by an alkoxy, phenylalkoxy or pyridylmethyloxy group,

a compound of general formula

$$B - X - A - Y - COOH ,\qquad\qquad (XI)$$

wherein

A, B, X and Y are defined as in claims 1 to 6, is reacted with a formamide acetal of general formula

$$(R_{16})_2N - CH(OR_{17})_2 ,\qquad\qquad\qquad (XXIII)$$

wherein

$R_{16}$ represents a lower alkyl group and
$R_{17}$ represents a $C_{1-6}$-alkyl group, a phenylalkyl group optionally substituted by one or more methoxy groups in the phenyl moiety or a pyridylmethyl group, or

n) in order to prepare compounds of general formula I wherein A represents a pyrrolidine ring and $R_1$ in 1-position of the pyrrolidinone ring represents a carbonyl group which is substituted by an amino, methylamino, ethylamino or dimethylamino group,

a compound of general formula

$$B - X - A''' - Y - E ,\qquad\qquad\qquad (XXIV)$$

wherein

B, E, X and Y are defined as in claims 1 to 6 and

$A'''$ represents a pyrrolidinone ring, optionally substituted by $R_2$, which is substituted in 1-position by a halocarbonyl or N-azolylcarbonyl group, is reacted with an amine of general formula

$$H - N(R_5)_2 ,\qquad\qquad\qquad (XXV)$$

wherein

$R_5$ represents a hydrogen atom, or a methyl or ethyl group, and
subsequently, if desired, a compound of general formula I thus obtained which contains an amino, methylamino or imino group, is converted by acylation, sulphonylation or alkylation into a corresponding compound of general formula I and/or

a compound of general formula I thus obtained which contains a hydroxy group is converted by alkylation or acylation into a corresponding compound of general formula I and/or

a compound of general formula I thus obtained which contains a carbonyl bridge is converted by reduction into a corresponding hydroxymethylene compound of general formula I and/or

a compound of general formula I thus obtained which contains a carbonyl bridge is converted by reduction into a corresponding methylene compound of general formula I and/or

a compound of general formula I thus obtained which contains a sulphenyl or sulphenyl group or a thioether bridge, is converted by oxidation into a corresponding S-oxide compound of general formula I, and/or

a compound of general formula I thus obtained which contains a sulphenyl or sulphinyl group or a thioether bridge, or an S-oxide compound of general formula I, is converted by oxidation into a corresponding S,S-dioxide compound of general formula I and/or

a compound of general formula I thus obtained which contains an aromatic group substituted by an amino group is converted by Sandmeyer reaction into a corresponding cyano compound of general formula I and/or

a compound of general formula I thus obtained which contains an ester group, is converted by reaction with an amine into a corresponding amide and/or

a compound of general formula I thus obtained which contains an ester group is converted by reduction into a corresponding hydroxymethyl compound of general formula I and/or

a compound of general formula I thus obtained which contains an ester group is converted by transesterification into a corresponding ester and/or

a compound of general formula I thus obtained which contains an amidino or guanidino group, is converted by acylation or cyanation into a corresponding compound of general formula I and

if necessary a protecting group used during reactions a) to n) and during the above-mentioned subsequent reactions in order to protect reactive groups is cleaved and/or

if desired a compound of general formula I thus obtained is separated into the cis/trans isomers, into the enantiomers and/or diastereomers thereof and/or

a compound of general formula I thus obtained is converted into the salts thereof, more particularly for pharmaceutical use into the physiologically-acceptable salts thereof with an inorganic or organic acid or base.

**Revendications**

1.  Dérivés alkylénimino à 5 chaînons de formule générale

$$B - X - A - Y - E \tag{I}$$

où

A représente un cycle pyrrolidine ou 2-pyrrolidinone éventuellement substitué par les restes $R_1$ et $R_2$ où
$R_1$ représente un groupe phényle qui peut être substitué par un groupe carboxyle, méthoxycarbonyle, aminocarbonyle, méthylaminocarbonyle, éthylaminocarbonyle, diméthylaminocarbonyle, méthanesulfonylamino ou acétylamino,

un groupe alkyle de 1 à 4 atomes de carbone, qui peut être substitué par un groupe hydroxyle, méthoxy, phénoxy, méthylsulfényle, méthylsulfinyle, méthylsulfonyle, phénylsulfényle, phénylsulfinyle, phénylsulfonyle, amino, acétylamino, benzoylamino, N-méthyl-acétylamino, méthanesulfonylamino ou benzènesulfonylamine, ces substituants ne pouvant pas être situés en position 1 lorsque $R_1$ est lié à l'atome d'azote cyclique du reste A,
un groupe alkyle de 1 à 4 atomes de carbone qui est substitué par deux groupes phényle, par un groupe cyclohexyle ou par un groupe phényle, ce dernier pouvant être substitué par un atome de fluor, de chlore ou de brome, par un groupe alkyle de 1 à 4 atomes de carbone, par un groupe alcoxy de 1 à 6 atomes de carbone, par un groupe phényle, phénylméthyle, hydroxyle, benzyloxy, méthylsulfényle, méthylsulfonyle ou trifluorométhyle, par deux groupes méthoxy ou par deux atomes de chlore,
un groupe méthyle qui peut être substitué par un groupe carboxyle, méthoxycarbonyle, aminocarbonyle, méthylaminocarbonyle, éthylaminocarbonyle, diméthylaminocarbonyle, benzylaminocarbonyle, pyrrolidinocarbonyle, pipéridinocarbonyle, hexaméthyléniminocarbonyle, morpholinocarbonyle, thiomorpholinocarbonyle, l-oxydo-thiomorpholinocarbonyle, 1,1-dioxydo-thiomorpholinocarbonyle, pipérazinocarbonyle, N-méthyl-pipérazinocarbonyle, N-acétylpipérazinocarbonyle ou N-méthanesulfonyl-pipérazinocarbonyle, ou encore, dans la mesure où $R_1$ n'est pas situé en position 1 d'un cycle 2-pyrrolidinone, un groupe carbonyle qui est substitué par un groupe méthyle, phényle, méthoxyméthyle, amino, méthylamino, éthylamino, aminométhyle, diméthylamino, carboxyle, méthoxycarbonyle ou diméthylaminocarbonyle, ou encore, dans la mesure où $R_1$ n'est pas situé non plus sur un atome de carbone du cycle alkylénimino à 5 chaînons A voisin de l'atome d'azote cyclique, un groupe sulfonyle substitué par un groupe méthyle, diméthylamino, phényle ou méthoxyphényle et

$R_2$ représente un groupe alkyle de 1 à 4 atomes de carbone éventuellement substitué par un groupe phényle,
B représente un groupe aminoalkyle de 1 à 5 atomes de carbone, un groupe amino, amidino, guanidino ou guanidinoalkyle de 1 à 5 atomes de carbone, les groupes amino, aminoalkyle ou amidino cités précédemment pouvant être substitués sur l'un des atomes d'azote par un groupe hydroxyle, par un groupe alkyle de 1 à 4

186

atomes de carbone, par un groupe alkyloxycarbonyle de 2 à 6 atomes de carbone au total, par un groupe benzyloxycarbonyle, phényloxycarbonyle ou benzoyle,

le groupe Y-E représente un groupe alkyle linéaire de 1 à 3 atomes de carbone, qui est substitué par un groupe carboxyle, phosphono, O-méthylphosphono ou hydroxyméthyle, par un groupe alcoxycarbonyle de 2 à 7 atomes de carbone au total, par un groupe dialkylaminocarbonylméthoxycarbonyle où chaque partie alkyle peut contenir 1 à 3 atomes de carbone, par un groupe morpholinoéthoxycarbonyle ou (2-oxo-1-pyrrolidinyl)éthoxycarbonyle, par un groupe phénylalcoxycarbonyle de 1 à 3 atomes de carbone dans la partie alcoxy, le noyau phényle pouvant être substitué par un ou deux groupes méthoxy, ou par un groupe pyridylméthyloxycarbonyle, la plus courte distance entre le substituant E et le premier atome d'azote du groupe B étant d'au moins 10 liaisons, et

X représente un groupe de formule

$$- X_1 - X_2 -$$

où

$X_1$ est lié au groupe A et $X_2$ est lié au groupe B, $X_1$ représente une liaison, un groupe méthylène ou éthylène, un atome d'oxygène ou de soufre, un groupe sulfonyle, imino, -N(COCH$_3$)-, -N(SO$_2$CH$_3$)-, -CONH-, -NH-CO-, -NH-SO$_2$- ou -NH-CO-NH- pouvant se trouver encore entre le groupe méthylène, dans la mesure où celui-ci n'est pas lié à l'atome d'azote cyclique du groupe A, et le groupe $X_2$ voisin, ou un groupe imino, -NHCO- ou -N(C$_2$H$_5$)CO- pouvant se trouver encore entre le groupe éthylène et le groupe $X_2$ voisin,

$X_2$ représente un groupe phénylène ou biphénylylène qui peut être substitué par un atome de fluor, de chlore ou de brome, par un groupe méthyle, méthoxy, éthoxy, trifluorométhyle, méthylsulfényle, méthylsulfinyle, méthylsulfonyle, nitro, acétylamino ou méthanesulfonylamino ou par un autre groupe méthyle,

un groupe phénylénalkylène linéaire de 1 à 3 atomes de carbone dans la partie alkyle, éventuellement substitué une ou plusieurs fois, une double ou triple liaison ne pouvant toutefois pas être voisine d'un hétéroatome, un groupe phénylènecycloalkylène ou cycloalkylènephénylène de 4 à 6 atomes de carbone dans la partie cycloalkyle dans chaque cas, un groupe phénylènenaphtylène, phénanthrénylène ou dihydrophénanthrénylène ou un groupe naphtylène qui peut être totalement ou partiellement hydrogéné, un groupe fluorénylène dans lequel le groupe méthylène peut être remplacé par un groupe hydroxyméthylène ou carbonyle, un groupe indanylène, spiroundécylène ou phénylènebicycloheptylène ou encore un groupe phénylène-W-phénylène où W représente un atome d'oxygène ou de soufre, un groupe imino, carbonyle, hydroxyméthylène, sulfinyle ou sulfonyle,

ou encore, au cas où un hétéroatome ou un atome de carbone insaturé du groupe B ne succède pas immédiatement à $X_2$, un groupe oxyphénylène ou carbonylaminophénylène, leurs isomères géométriques et leurs sels.

2. Dérivés alkylénimino à 5 chaînons de formule générale I selon la revendication 1 où

A représente un cycle pyrrolidine ou 2-pyrrolidinone éventuellement substitué par les restes $R_1$ et $R_2$ où

$R_1$ représente un groupe phényle qui peut être substitué par

un groupe carboxyle, méthoxycarbonyle ou diméthylaminocarbonyle,

un groupe alkyle linéaire de 1 à 4 atomes de carbone qui peut être substitué en position terminale par un groupe phényle, qui peut être substitué par un groupe alkyle de 1 à 4 atomes de carbone, par un groupe alcoxy de 1 à 6 atomes de carbone, par un groupe phényle, benzyle, méthylsulfényle, méthylsulfonyle ou trifluorométhyle, par deux groupes méthoxy ou par deux atomes de chlore, ou par un groupe cyclohexyle ou par deux groupes phényle,

un groupe alkyle de 2 à 4 atomes de carbone qui est substitué en position 2, 3 ou 4 par un groupe hydroxyle, méthoxy ou phénoxy,

un groupe méthyle qui est substitué par un groupe carboxyle, méthoxycarbonyle, aminocarbonyle, éthylaminocarbonyle, diméthylaminocarbonyle, benzylaminocarbonyle, pyrrolidinocarbonyle ou morpholinocarbonyle,

ou encore, dans la mesure où $R_1$ n'est pas situé sur l'atome d'azote cyclique du cycle 2-pyrrolidinone, un groupe carbonyle qui est substitué par un groupe méthyle, phényle, éthylamino, diméthylamino, méthoxyméthyle ou aminométhyle,

ou encore, dans la mesure où $R_1$ n'est pas situé non plus sur un atome de carbone du cycle alkylénimino à 5 chaînons voisin de l'atome d'azote cyclique, un groupe sulfonyle substitué par un groupe méthyle, méthoxyphényle ou diméthylamino, et

$R_2$ représente un groupe alkyle de 1 à 4 atomes de carbone,

B représente un groupe guanidinométhyle ou un groupe amidino qui peut être substitué sur l'un des atomes d'azote par un groupe alkyle de 1 à 4 atomes de carbone ou par un groupe méthoxycarbonyle, éthoxycarbonyle, isopropyloxycarbonyle, isobutyloxycarbonyle, phényloxycarbonyle, benzyloxycarbonyle ou benzoyle,

le groupe Y-E représente un groupe méthyle qui est substitué par un groupe carboxyle, phosphono, O-méthylphosphono ou diméthylaminocarbonylméthoxycarbonyle ou par un groupe alcoxycarbonyle de 2 à 7 atomes de carbone au total où une partie méthoxy peut être substituée par un groupe phényle ou pyridyle, la partie éthoxy peut être substituée en position terminale par un groupe phényle, diméthoxyphényle, morpholino ou 2-oxo-1-pyrrolidinyle et la partie n-propoxy peut être substituée en position terminale par un reste phényle,

la plus courte distance entre le substituant E et le premier atome d'azote du groupe B étant d'au moins 10 liaisons, et

X représente un groupe de formule

$$- X_1 - X_2 -$$

où

$X_1$ représente une liaison, un groupe méthylène qui, dans la mesure où celui-ci n'est pas lié à l'atome d'azote cyclique du groupe A, est lié au groupe $X_2$ voisin par un atome d'oxygène, un groupe sulfonyle, imino, -N(COCH$_3$)-, -NH-CO- ou -NH-SO$_2$-,

ou un groupe éthylène qui est lié au groupe $X_2$ voisin par un groupe -NH-CO- et

$X_2$ représente un groupe biphénylylène qui peut être substitué par un atome de fluor, de chlore ou de brome, par un groupe méthyle, méthoxy, éthoxy, trifluorométhyle, méthylsulfényle, méthylsulfinyle, méthylsulfonyle, nitro, acétylamino ou méthanesulfonylamino ou par un autre groupe méthyle, un groupe phénylènecycloalkylène de 10 à 12 atomes de carbone au total, un groupe phénylènesulfénylphénylène, phénylènesulfinylphénylène, dihydrophénanthrénylène, indanylène ou naphtylène ou un groupe fluorénylène où le groupe méthylène peut être remplacé par un groupe hydroxyméthylène ou carbonyle,

leurs isomères géométriques et leurs sels.

3. Dérivés alkylénimino à 5 chaînons de formule générale I selon au moins l'une des revendications 1 et 2, où $R_1$ est lié à l'atome d'azote cyclique ou à la position 3 du groupe A, leurs isomères géométriques et leurs sels.

4. Dérivés alkylénimino à 5 chaînons de formule générale I selon la revendication 1 suivants:

(3S,5S)-5-[(4'-amidino-4-biphénylyl)oxyméthyl]-3-[(méthoxycarbonyl)méthyl]-2-pyrrolidinone,

1-(4'-amidino-4-biphénylyl)-4-phosphonométhyl-2-pyrrolidinone,

(3S,5S)-5-[(4'-amidino-4-biphénylyl)oxyméthyl)-3-carboxyméthyl-1-(3-phénylpropyl)-2-pyrrolidinone,

(3S,5S)-5-[(4'-amidino-4-biphénylyl)oxyméthyl]-3-carboxyméthyl-2-pyrrolidinone,

(3S,5S)-5-[(4'-amidino-4-biphénylyl)oxyméthyl)-3-carboxyméthyl-1-phényl-2-pyrrolidinone,

(3S,5S)-5-[(4'-amidino-2,3-diméthyl-4-biphénylyl)oxyméthyl]-3-carboxyméthyl-1-(3-phénylpropyl)-2-pyrrolidinone,

(3S,5S)-5-[(7-amidino-9-céto-2-fluorényl)oxyméthyl]-3-carboxyméthyl-1-(3-phénylpropyl)-2-pyrrolidinone,

(3S,5S)-5-[[N-(4'-amidino-4-biphénylyl)-N-acétyl]aminométhyl]-3-carboxyméthyl-1-(3-phénylpropyl)-2-pyrrolidinone,

(3S,5S)-5-[[2-[(4-amidinophényl)amino]phényl]carbonylaminométhyl]-3-carboxylméthyl-1-(3-phénylpropyl)-2-pyrrolidinone,

(3S,5S)-5-[[4-[(3-amidinophényl)carbonyl]phényl]oxyméthyl]-3-carboxyméthyl-1-(3-phénylpropyl)-2-pyrrolidinone,

(3S,5S)-5-[(4'-amidino-4-biphénylyl)oxyméthyl]-3-carboxyméthyl-1-[(diméthylaminocarbonyl)méthyl]-2-pyrrolidinone,

(3S,5S)-1-acétyl-5-[(4'-amidino-4-biphénylyl)oxyméthyl]-3-carboxyméthyl-pyrrolidine,

(3S,5S)-5-[(4'-amidino-4-biphénylyl)oxyméthyl]-3-carboxyméthyl-1-(diméthylaminosulfonyl)-pyrrolidine,

(3S,5S)-5-[(4'-amidino-4-biphénylyl)sulfonyl]aminométhyl]-3-carboxyméthyl-1-(3-phénylpropyl)-2-pyrrolidinone,

(3R,S;4R,S)-4-[(4'-amidino-4-biphénylyl)oxyméthyl]-3-carboxyméthyl-2-pyrrolidinone,

(3S,5S)-5-[(4'-amidino-3'-fluoro-4-biphénylyl)oxyméthyl]-3-carboxyméthyl-2-pyrrolidinone,

(3S,5S)-5-[[4-(4-aminobutyl)phényl]oxyméthyl]-3-carboxyméthyl-1-(3-phénylpropyl)-2-pyrrolidinone,

(3S,R;5S,R)-3-carboxyméthyl-5-[4-[(5-guanidinopentyl)oxy]phényl]-1-(3-phénylpropyl)-2-pyrrolidinone,

(3S,5S)-5-[2-[(6-amino-5,6,7,8-tétrahydro-2-naphtylcarbonyl)-amino]éthyl]-3-carboxyméthyl-1-(3-phénylpropyl)-2-pyrrolidinone,

(3S,5S)-5-[[4-[(3-aminopropyl)carbonylamino]phényl]oxyméthyl]-3-carboxyméthyl-1-(3-phénylpropyl)-2-pyrrolidinone,

(3S,5S)-3-carboxyméthyl-5-[[4'-(N-hydroxyamidino)-4-biphénylyl)oxyméthyl]-2-pyrrolidinone,

(3S,5S)-5-[[4'-(N-méthoxycarbonylamidino)-4-biphénylyl]-oxyméthyl]-3-[(méthoxycarbonyl)méthyl]-2-pyrrolidinone,

(3S,5S)-5-[[4'-(N-éthoxycarbonylamidino)-4-biphénylyl]-oxyméthyl]-3-[(méthoxycarbonyl)méthyl]-2-pyrrolidinone,

(3S,5S)-5-[[4'-(N-éthoxycarbonylamidino)-4-biphénylyl]-oxyméthyl]-3-[(éthoxycarbonyl)méthyl]-2-pyrrolidinone et

(3S,5S)-3-[(éthoxycarbonyl)méthyl]-5-[[4'-(N-méthoxycarbonylamidino)-4-biphénylyl]-oxyméthyl]-2-pyrrolidinone ainsi que leurs sels.

5. (3S,5S)-5-[(4'-amidino-4-biphénylyl)oxyméthyl]-3-carboxyméthyl-2-pyrrolidinone et ses sels.

6. (3S,5S)-5-[[4'-(N-méthoxycarbonylamidino)-4-biphénylyl]-oxyméthyl]-3-[(méthoxycarbonyl)méthyl]-2-pyrrolidinone et ses sels.

7. Sels physiologiquement acceptables des composés selon au moins l'une des revendications 1 à 6 avec des acides ou bases inorganiques ou organiques.

8. Médicament contenant un composé selon au moins l'une des revendications 1 à 6 ou un sel physiologiquement acceptable selon la revendication 7 outre éventuellement un ou plusieurs supports et/ou diluants inertes.

9. Utilisation d'un composé selon au moins l'une des revendications 1 à 6 ou d'un sel physiologiquement acceptable selon la revendication 7 pour la préparation d'un médicament qui convient à la lutte contre ou à la prévention des maladies dans lesquelles des agrégats de cellules de taille relativement petite ou relativement grande apparaissent ou des interactions cellules-matrice jouent un rôle.

10. Procédé de préparation d'un médicament selon la revendication 8 caractérisé en ce que, par voie non chimique, un composé selon au moins l'une des revendications 1 à 6 ou un sel physiologiquement acceptable selon la revendication 7 est incorporé dans un ou plusieurs supports et/ou diluants inertes.

11. Procédé de préparation des dérivés alkylénimino à 5 chaînons selon les revendications 1 à 8 caractérisé en ce que

a) pour la préparation de composés de formule générale I où B représente un groupe amidino qui peut être substitué sur un atome d'azote par un groupe hydroxyle ou par un groupe alkyle de 1 à 4 atomes de carbone, un composé éventuellement formé dans le mélange réactionnel de formule générale

$$Z_1 - C(=NR_{10}) - X - A - Y - E \qquad (II)$$

où

A, E, X et Y sont définis comme dans les revendications 1 à 6,
$R_{10}$ représente un atome d'hydrogène ou un groupe alkyle de 1 à 4 atomes de carbone et
$Z_1$ représente un groupe alcoxy, aralcoxy, alkylthio, aralkylthio ou amino, est mis à réagir avec une amine de formule générale

$$R_{11} - NH - R_{12} \qquad (III)$$

où
$R_{11}$ représente un atome d'hydrogène, un groupe hydroxyle ou un groupe alkyle de 1 à 4 atomes de carbone et
$R_{12}$ représente un atome d'hydrogène, ou avec ses sels d'addition d'acide ou

b) pour la préparation de composés de formule générale I où B contient un groupe amino, un composé de formule générale

$$NC - X - A - Y - E \qquad (IV)$$

où

A, E, X et Y sont définis comme dans les revendications 1 à 6 est réduit ou

c) pour la préparation de composés de formule générale I où B représente un reste guanidino, un composé de formule générale

$$H_2N - X - A - Y - E \qquad (V)$$

où

A, E, X et Y sont définis comme dans les revendications 1 à 6 ou son sel d'addition d'acide est mis à réagir avec le cyanamide ou

d) pour la préparation de composés de formule générale I où B représente un reste guanidino qui peut être substitué sur l'un des atomes d'azote par un groupe hydroxyle ou par un groupe alkyle de 1 à 4 atomes de carbone ou par un groupe méthoxycarbonyle, éthoxycarbonyle, isopropyloxycarbonyle, isobutyloxycarbonyle, benzyloxycarbonyle ou phényloxycarbonyle, un composé de formule générale

$$R_{13} - NH - X - A - Y - E \qquad (VI)$$

où

A, E, X et Y sont définis comme dans les revendications 1 à 6 et $R_{13}$ représente un atome d'hydrogène ou un groupe alkyle de 1 à 4 atomes de carbone est mis à réagir avec une amidine de formule générale

$$R_{14} - Z_2 \qquad (VII)$$

où
$R_{14}$ représente un groupe amidino qui peut être substitué sur l'un des atomes d'azote par un groupe hydroxyle ou par un groupe alkyle de 1 à 4 atomes de carbone, ou par un groupe méthoxycarbonyle, éthoxycarbonyle, isopropyloxycarbonyle, isobutyloxycarbonyle, benzyloxycarbonyle ou phényloxycarbonyle, et
$Z_2$ représente un reste clivable, ou

e) pour la préparation de composés de formule générale I où B représente un groupe amino, un composé éventuellement formé dans le mélange réactionnel de formule générale

$$Z_3 - X - A - Y - E \qquad\qquad (VIII)$$

où

A, E, X et Y sont définis comme dans les revendications 1 à 6 et $Z_3$ représente un groupe nitro ou azido, est réduit ou

f) pour la préparation de composés de formule générale I où E représente un groupe carboxyle, un composé de formule générale

$$B - X - A - Y - E' \qquad\qquad (IX)$$

où

A, B, X et Y sont définis comme dans les revendications 1 à 6 et E', qui est lié à un atome de carbone, représente un groupe convertible en un groupe carboxyle par hydrolyse, traitement avec un acide, thermolyse ou hydrogénolyse, est converti en un composé carboxylé correspondant par hydrolyse, par traitement avec un acide, thermolyse ou hydrogénolyse, ou

g) pour la préparation de composés de formule générale I où E représente l'un des restes esters cités dans les revendications 1 à 6, un composé de formule générale

$$B - X - A - Y - COOH \qquad\qquad (X)$$

où

A, B, X et Y sont définis comme dans les revendications 1 à 6, ou ses dérivés réactifs, est mis à réagir avec un composé de formule générale

$$H - R_{15} \qquad\qquad (XI)$$

où
$R_{15}$ représente un groupe alcoxy de 1 à 6 atomes de carbone, un groupe dialkylaminocarbonylméthoxy où chaque partie alkyle peut contenir 1 à 3 atomes de carbone, un groupe morpholinoéthoxy ou (2-oxo-1-pyrrolidinyl)éthoxy, un groupe phénylalcoxy de 1 à 3 atomes de carbone dans la partie alcoxy, le noyau phényle pouvant être substitué par un ou deux groupes méthoxy, ou un groupe pyridylméthyloxy, ou

h) pour la préparation de composés de formule générale I où E représente un groupe carboxyle, un composé de formule générale

$$B - X - A - Y - CHO \qquad\qquad (XII)$$

où

A, B, X et Y sont définis comme dans les revendications 1 à 6, est oxydé ou

i) pour la préparation de composés de formule générale I où le reste B-X-A représente un groupe B-$G_1$-T-$G_2$-A ou un groupe B-$G_1$-A, où $G_2$ correspond à une partie de X et $G_1$-T correspond à l'autre partie de X et en outre $G_1$ et $G_2$ ou $G_1$-T peuvent aussi représenter une liaison et $G_1$ peut aussi représenter X, et

T représente un atome d'oxygène ou de soufre, un groupe imino, -N(COCH$_3$)- ou -N(SO$_2$CH$_3$)-, un composé de formule générale

$$B - G_1 - T - H \qquad\qquad (XIII)$$

est mis à réagir avec un composé de formule générale

$$Z_5 - G_2 - A - Y - E \qquad (XIV)$$

ou un composé de formule générale

$$H - T - G_2 - A - Y - E \qquad (XV)$$

est mis à réagir avec un composé de formule générale

$$B - G_1 - Z_5 \qquad (XVI)$$

ou un composé de formule générale

$$H - A - Y - E \qquad (XVII)$$

est mis à réagir avec un composé de formule générale

$$B - G_1 - Z_5 \qquad (XVI)$$

où
A, B, E et Y sont définis comme dans les revendications 1 à 6, $G_2$ correspond à une partie de X et $G_1$-T correspond à l'autre partie de X, où en outre $G_1$ et $G_2$ ou $G_1$-T peuvent représenter une liaison et $G_1$ peut aussi représenter X et X est défini dans chaque cas comme dans les revendications 1 à 6,
T représente un atome d'oxygène ou de soufre, un groupe imino, $-N(COCH_3)-$ ou $-N(SO_2CH_3)-$ et
$Z_5$ représente un groupe partant, ou avec les sels alcalins, alcalino-terreux ou de MgHal d'un composé de formule générale XIII, XV ou XVII ou

j) pour la préparation de composés de formule générale I où $R_1$ représente l'un des restes alkyle cités pour $R_1$ dans les revendications 1 à 6, un composé de formule générale

$$B - X - A' - Y - E \qquad (XVIII)$$

où

B, E, X et Y sont définis comme dans les revendications 1 à 6 et
A' représente l'un des groupes alkylénimino cycliques à 5 chaînons éventuellement substitués dans le squelette carboné par $R_2$ cités dans la revendication 1, où $R_2$ est défini comme dans les revendications 1 à 6, est alkylé avec un composé de formule générale

$$R_1' - Z_6 \qquad (XIX)$$

où
$R_1'$ représente l'un des restes alkyle cités pour $R_1$ dans les revendications 1 à 6 et
$Z_6$ représente un groupe partant, ou

k) pour la préparation de composés de formule générale I où $R_1$ représente l'un des restes acyle ou sulfonyle cités dans les revendications 1 à 6 et A représente un cycle pyrrolidine, un composé de formule générale

$$B - X - A'' - Y - E \qquad (XX)$$

où

B, E, X et Y sont définis comme dans les revendications 1 à 6 et A'' représente un cycle pyrrolidine éventuellement substitué par $R_2$ dans le squelette carboné, où $R_2$ est défini comme dans les revendications 1 à 6, est acylé ou sulfonylé avec un composé de formule générale

$$R_1'' - Z_7 \qquad (XXI)$$

où

$R_1''$ représente l'un des restes acyle ou sulfonyle cités pour $R_1$ dans les revendications 1 à 6 et
$Z_7$ représente un groupe hydroxyle ou un groupe partant ou encore $Z_7$ représente avec l'atome d'hydrogène d'un groupe imino voisin du groupe carbonyle une autre liaison carboneazote, ou

l) pour la préparation de composés de formule générale I où E représente un groupe hydroxycarbonyle, un groupe alcoxycarbonyle de 2 à 7 atomes de carbone au total, un groupe pyridylméthyloxycarbonyle ou phénylalcoxycarbonyle de 1 à 3 atomes de carbone dans la partie alcoxy, où le noyau phényle peut être substitué par un ou deux groupes méthoxy, un composé de formule générale

$$B - X - A - Y - E'' \qquad (XXII)$$

où

A, B, X et Y sont définis comme dans les revendications 1 à 6 et E'' représente un groupe vinyle ou 1,2-dihydroxyalkyle, est oxydé et, si nécessaire, un composé ainsi obtenu est ensuite estérifié avec un alcool correspondant ou

m) pour la préparation de composés de formule générale I où E représente l'un des groupes carbonyle substitués par un groupe alcoxy, phénylalcoxy ou pyridylméthyloxy cités dans les revendications 1 à 6, un composé de formule générale

$$B - X - A - Y - COOH \qquad (XI)$$

où

A, B, X et Y sont définis comme dans les revendications 1 à 6, est mis à réagir avec un formamide-acétal de formule générale

$$(R_{16})_2N - CH(OR_{17})_2 \qquad (XXIII)$$

où
$R_{16}$ représente un groupe alkyle inférieur et
$R_{17}$ représente un groupe alkyle de 1 à 6 atomes de carbone, un groupe phénylalkyle éventuellement substitué par un ou deux groupes méthoxy dans la partie phényle ou un groupe pyridylméthyle, ou

n) pour la préparation de composés de formule générale I où A représente un cycle pyrrolidine et $R_1$ représente en position 1 du cycle pyrrolidine un groupe carbonyle qui est substitué par un groupe amino, méthylamino, éthylamino ou diméthylamino,

un composé de formule générale

$$B - X - A''' - Y - E \qquad (XXIV)$$

où
B, E, X et Y sont définis comme dans les revendications 1 à 6 et A''' représente un cycle pyrrolidine éventuellement substitué par $R_2$, qui est substitué en position 1 par un groupe halogénocarbonyle ou N-azolylcarbonyle, est mis à réagir avec une amine de formule générale

$$H - N(R_5)_2 \qquad (XXV)$$

où
$R_5$ représente un atome d'hydrogène, un groupe méthyle ou éthyle, puis,
si on le souhaite, un composé de formule générale I ainsi obtenu, qui contient un groupe amino, méthylamino ou imino, est converti par acylation, sulfonylation ou alkylation en un composé de formule générale I correspondant et/ou un composé de formule générale I ainsi obtenu, qui contient un groupe hydroxyle, est converti par alkylation ou acylation en un composé de formule générale I correspondant et/ou
un composé de formule générale I ainsi obtenu, qui contient un pont carbonyle, est converti par réduction en un composé hydroxyméthylène de formule générale I correspondant et/ou un composé de formule générale I ainsi obtenu, qui contient un pont carbonyle, est converti par réduction en un composé méthylène de formule générale I correspondant et/ou

un composé de formule générale I ainsi obtenu, qui contient un groupe sulfényle ou sulfinyle ou un pont thioéther, est converti par oxydation en un composé S-oxyde de formule générale I correspondant et/ou

un composé de formule générale I ainsi obtenu, qui contient un groupe sulfényle ou sulfinyle ou un pont thioéther, ou un composé S-oxyde de formule générale I, est converti par oxydation en un composé S,S-dioxyde de formule générale I correspondant et/ou

un composé de formule générale I ainsi obtenu, qui contient un reste aromatique substitué par un groupe amino, est converti par la réaction de Sandmeyer en un composé cyano de formule générale I correspondant et/ou

un composé de formule générale I ainsi obtenu, qui contient un groupe ester, est converti par réaction avec une amine en un amide correspondant et/ou

un composé de formule générale I ainsi obtenu, qui contient un groupe ester, est converti par réduction en un composé hydroxyméthyle de formule générale I correspondant et/ou

un composé de formule générale I ainsi obtenu, qui contient un groupe ester, est converti par transestérification en un ester correspondant et/ou

un composé de formule générale I ainsi obtenu, qui contient un groupe amidino ou guanidino, est converti par acylation en un composé de formule générale I correspondant et

si nécessaire, un reste protecteur utilisé pendant les réactions a) à n) et pendant les réactions ultérieures citées précédemment pour la protection de groupes réactifs est clivé et/ou

si on le souhaite, un composé de formule générale I ainsi obtenu est résolu en ses isomères cis/trans, en ses énantiomères et/ou diastéréoisomères et/ou

un composé de formule générale I ainsi obtenu est converti en ses sels, en particulier pour l'utilisation pharmaceutique en ses sels physiologiquement acceptables avec un acide ou une base inorganique ou organique.